(19) Europäisches Patentamt
European Patent Office
Office européen des brevets

(11) **EP 4 684 781 A1**

(12) **EUROPEAN PATENT APPLICATION**
published in accordance with Art. 153(4) EPC

(43) Date of publication:
**28.01.2026 Bulletin 2026/05**

(21) Application number: **24774094.7**

(22) Date of filing: **19.03.2024**

(51) International Patent Classification (IPC):
***A61K 31/18*** (2006.01) ***A61K 31/4196*** (2006.01)
***A61P 13/12*** (2006.01) ***A61Q 1/00*** (2006.01)

(52) Cooperative Patent Classification (CPC):
**A61K 31/167; A61K 31/18; A61K 31/337; A61K 31/381; A61K 31/40; A61K 31/41; A61K 31/4192; A61K 31/4196; A61K 31/433; A61K 31/549; A61K 31/58; A61K 31/664; A61K 31/70; A61P 1/00; A61P 1/04;** (Cont.)

(86) International application number:
**PCT/CN2024/082357**

(87) International publication number:
**WO 2024/193529 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.03.2023 CN 202310299839**
**20.11.2023 CN 202311550676**

(71) Applicants:
- **Hangzhou Phecdamed Co., Ltd.**
  **Hangzhou, Zhejiang 311100 (CN)**
- **Nanjing Phecdamed Co., Ltd.**
  **Nanjing, Jiangsu 211122 (CN)**

(72) Inventors:
- **LIU, Dong**
  **Hangzhou, Zhejiang 311100 (CN)**
- **WU, Ronghai**
  **Hangzhou, Zhejiang 311100 (CN)**
- **TIAN, Zhen**
  **Hangzhou, Zhejiang 311100 (CN)**

(74) Representative: **Viering, Jentschura & Partner mbB**
**Patent- und Rechtsanwälte**
**Am Brauhaus 8**
**01099 Dresden (DE)**

(54) **USE OF SMALL-MOLECULE COMPOUND HAVING NAPHTHYLAMINE STRUCTURE**

(57) The present application discloses use of small-molecule compound having naphthalamine structure. In the present invention, it was found for first time that the general formula I compound can be used to treat diseases associated with kidney injury and diseases associated with mitophagy, and is expected to be an effective special drug for treating the diseases associated with mitophagy and kidney-associated diseases, especially chronic kidney disease.

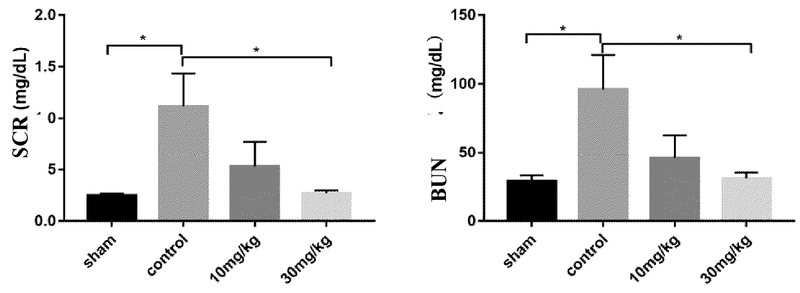


FIG.1

EP 4 684 781 A1

(52) Cooperative Patent Classification (CPC): (Cont.)
**A61P 1/16; A61P 9/00; A61P 9/04; A61P 9/10; A61P 11/00; A61P 13/08; A61P 13/12; A61P 15/08; A61P 17/00; A61P 25/00; A61P 25/08; A61P 25/14; A61P 25/16; A61P 25/24; A61P 25/28; A61Q 1/00; C07C 317/44; C07D 305/08**

## Description

### Cross References to Related Applications

This patent application claims priority to Chinese patent application No. 2023102998399 filed on March 20, 2023, entitled "Use of small-molecule compound having naphthylamine structure", which is incorporated herein by reference in its entirety.

This patent application claims priority to Chinese patent application No. 2023115506763, filed on November 20, 2023, entitled "Use of small-molecule compound having naphthylamine structure", which is incorporated herein by reference in its entirety.

### Technical Field

Embodiments of the present invention relate to the field of chemical drugs, and in particular to the Use of small-molecule compound having naphthylamine structure.

### Background Technology

The kidneys are necessary for physiological homeostasis as their roles include excretion, hormone production, blood pressure regulation, ionic homeostasis, osmotic homeostasis and pH balance. Nephropathy broadly refers to a variety of disorders of the kidneys, and due to the multiple functions of the kidneys, nephropathy often causes systemic consequences that make diagnosis and treatment extremely difficult and expensive.

The kidneys have the second highest mitochondrial content and oxygen consumption in the body, after the heart. Therefore, maintaining mitochondrial homeostasis is critical for normal renal function. Mitophagy is essential for maintaining renal cell homeostasis in vivo, and disruption of mitophagy has been associated with several renal diseases, including acute kidney injury (AKI) and chronic kidney disease (CKD).

Acute kidney injury (AKI) is a major renal disease characterized by a rapid decline in renal function. The major clinical causes of AKI include renal ischemia-reperfusion (IR), sepsis, and nephrotoxins. Recent studies have further shown that pre-existing renal diseases, such as chronic kidney disease (CKD) and diabetic kidney disease (DKD), increase susceptibility to AKI. Lethal and sublethal injury to the renal tubules are the main pathological features of AKI. After undergoing AKI, the remaining renal tubular cells undergo dedifferentiation, proliferation, migration, and redifferentiation into mature renal tubular cells to repair the injured tubules. Renal injury can be completely repaired after mild injury; however, severe or recurrent AKI usually results in aberrant repair leading to renal fibrosis and ultimately chronic kidney damage (CKD).

The main causes of CKD are primary glomerulonephritis, hypertensive renal microarteriosclerosis, diabetic nephropathy, secondary glomerulonephritis, tubulointerstitial lesions (chronic pyelonephritis, chronic uric acid nephropathy, obstructive nephropathy, drug-induced nephropathy, etc), ischemic nephropathy, and hereditary nephropathies (polycystic kidneys, hereditary nephritis). In developed countries, diabetic nephropathy and hypertensive renal small arteriosclerosis have become the main causes of chronic kidney disease; in China, there is also a significant trend of increase in these two diseases. The risk factors of CKD are age, family history of CKD, diabetes mellitus, hypertension, obesity-metabolic syndrome, high-protein diet, hyperlipidemia, autoimmune disorders, urinary tract infections or systemic infections, hepatitis viral infections, urinary stone, urethral obstruction, urinary tract or systemic tumors, history of application of nephrotoxic drugs, cardiovascular disease, anemia, smoking, and so on.

At present, for the different stages of CKD, three levels of protection are proposed. The first level of protection refers to the timely and effective treatment of existing renal patients or patients with disease that may cause renal injury (e.g., diabetes mellitus, hypertension, etc.), so as to prevent the occurrence of chronic renal failure. The second level of protection refers to the timely treatment of patients with mild or moderate chronic renal failure in order to slow down, stop or reverse the progression of chronic renal failure and prevent the occurrence of uremia. The third level of protection refers to early treatment for patients with uremia to prevent the occurrence of certain serious complications of uremia and improve the survival rate and quality of life of patients. The final result of the progression of chronic renal insufficiency is the end-stage renal failure, and patients will have to rely on renal replacement therapy to sustain their lives. Despite the great progress in dialysis treatment, the mortality rate of patients with end-stage renal failure is still high and the quality of survival is low. Therefore, the treatment of CKD patients includes the treatment to delay the progression of chronic renal insufficiency and the treatment for various complications.

At present, the main measures to delay the development and progression of chronic renal insufficiency are controlling blood pressure, low-protein diet, correcting the factors of acute exacerbation of chronic renal failure, and preventing and treating the complications, such as maintaining the water and electrolyte balance, correcting metabolic acidosis, preventing cardiovascular disease, correcting renal anemia, preventing and treating renal bone disease, etc.

There is no effective drug that can treat CKD patients. Currently, there is no effective drug that can treat chronic kidney injury and the various diseases caused by it.

Mitochondrial body dysfunction is considered to be a key factor in the pathogenesis of many diseases. The accumulation of dysfunctional mitochondria is recognized as a marker of pathological conditions and a key factor driving disease progression. A growing body of evidence suggests that mitochondria are damaged to varying degrees in a wide range of acute and chronic diseases, and that elimination of dysfunctional mitochondria is a powerful means of alleviating a wide range of diseases. Mitochondria-based therapeutic strategies and personalized drugs targeting mitochondria are the key of the future of drug therapy. However, existing reports lack effective drugs for the treatment of diseases associated with mitochondrial somatic dysfunction. Therefore, it is critical to find such therapeutic agents.

Summary of the invention

In order to address the above problems, it is an object of the present invention to provide uses for a small-molecule compound having naphthylamine structure.

It is another object of the present invention to provide a method of preventing and/or treating diseases associated with kidney injury.

The first aspect of the present invention provides uses of the compounds shown in the general formula (I) or its pharmaceutically acceptable salts, stereoisomers, solvates or prodrugs thereof, the uses are for:

(i) preparing a drug for preventing and/or treating diseases associated with kidney injury; and/or
(ii) preventing and/or treating diseases associated with kidney injury; and/or
(iii)preventing and/or treating diseases associated with mitochondrial dysfunction; and/or
(iv)preparing a drug for preventing and/or treating diseases associated with mitochondrial dysfunction; and/or
(v)preparing a cosmetic or medical instrument;

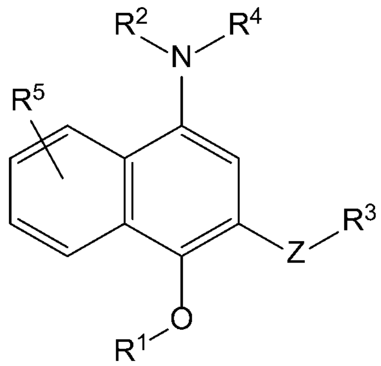

I

wherein Z is

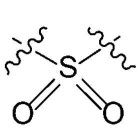 , 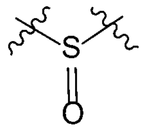 , 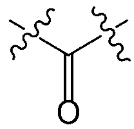 , 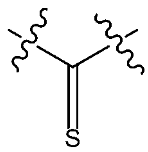

or a sulfur atom;

$R^1$ is hydrogen, $C_{1\sim6}$ alkyl,

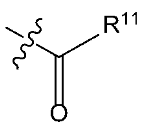 , 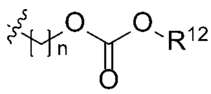 ,

wherein $R^{11}$ and $R^{12}$ are independently C1~6 alkyl or C1~6 cycloalkyl, respectively, and n is 1~4;

$R^2$ is hydrogen, $C_{1\sim6}$ alkyl, three- to six-membered cycloalkyl, three- to six-membered epoxyalkyl, phenyl, $C_{1\sim6}$

alkyl with at least one hydrogen substituted with $R^{2-1}$, phenyl with at least one hydrogen substituted with $R^{2-1}$, wherein $R^{2-1}$ is hydroxyl, halogen, amino, or $C_{1\sim6}$ alkoxy,
$R^3$ is

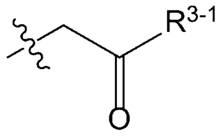 , 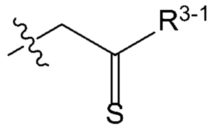 or 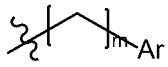 ;

wherein $R^{3-1}$ is hydrogen, hydroxyl, $C_{1\sim6}$ alkyl, $C_{1\sim6}$ alkoxy, $C_{3\sim6}$ cycloalkyl, three- to six-membered epoxyalkyl, amino, $C_{1\sim6}$ amine group, $-CH_2C(O)R^{3-2}$, $-CH_2C(O)OR^{3-2}$, $-CH_2C(O)N(R^{3-2}\ R^{3-2a})$, and $R^{3-2}$ and $R^{3-2a}$ are independently hydrogen, C1~6 alkyl, or three- to six-membered cycloalkyl respectively,
m is from 1 to 6, and
Ar is phenyl, and naphthyl, 5- or 6-membered monocyclic heteroaryl, 8- to 10-membered fused bicyclic heteroaryl, phenyl with at least one hydrogen atom substituted with $R^{3-3}$, and naphthyl with at least one hydrogen atom substituted with $R^{3-3}$, 5- or 6-membered monocyclic heteroaryl with at least one hydrogen atom substituted with $R^{3-3}$, 8- to 10-membered fused bicyclic heteroaryl with at least one hydrogen atom substituted with $R^{3-3}$, the $R^{3-3}$ is hydrogen, halogen, $C_{1\sim6}$ alkyl, three-to six-membered cycloalkyl, hydroxy, $C_{1\sim6}$ alkoxy, three- to six-membered epoxyalkyl, $C_{1\sim6}$ haloalkyl, $C_{2\sim6}$ alkenyl, $C_{2\sim6}$ alkynyl, $-N(R^{3-3a}R^{3-3b})$ or phenyl,
$R^{3-3a}$ and $R^{3-3b}$ are independently hydrogen, $C_{1\sim6}$ alkyl, or three- to six-membered cycloalkyl, respectively;
$R^4$ is

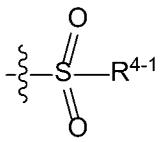 , 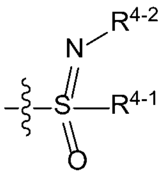 , 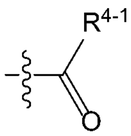 or 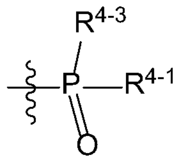 .

wherein $R^{4-1}$ is phenyl, and biphenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, $R^{4-11}$ is hydrogen, halogen, nitro, nitrile, hydroxyl, $C_{1\sim6}$ alkyl, $C_{13\sim6}$ cycloalkyl, $C_{1\sim6}$ alkoxy, $-N(R^{4-1a}R^{4-1b})$, phenyl, $C_{1\sim6}$ haloalkyl, $C_{1\sim6}$ haloalkoxy, $-C(O)OR^{4-12}$, $-C(O)R^{4-12}$, $-C(O)N(R^{4-1a}R^{4-1b})$, $-S(O)2R^{4-12}$, $-S(O)R^{4-12}$, $-\ OC(O)R^{4-12}$, $-OC(O)OR^{4-12}$ or

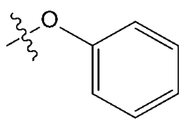 ,

$R^{4-12}$, $R4^{-1a}$ and $R^{4-1b}$ are independently hydrogen, respectively, $C_{1\sim6}$ alkyl, $C_{3\sim6}$ cycloalkyl, $C_{2\sim6}$ alkenyl, C2~6 alkynyl, C1~6 alkyl substituted with at least one hydrogen by a halogen, $C_{2\sim6}$ alkynyl with at least one hydrogen substituted with a halogen, $C_{3\sim6}$ cycloalkyl, C2~6 alkynyl with at least one hydrogen substituted with a halogen, and $R^{4-1a}$ and $R^{4-1b}$ are bondable to each other to form a ring,
$R^{4-2}$ is $C_{1\sim6}$ alkyl, $C_{3\sim6}$ cycloalkyl, $C_{1\sim6}$ alkyl with at least one hydrogen substituted with a hydroxyl, $C_{3\sim6}$ epoxyalkyl, or $R^{4-2}$ is bonded to R2 to form a 4- to 8-membered ring when $R^2$ is $C_{1\sim6}$ alkyl and $R^{4-2}$ is $C_{1\sim6}$ alkyl.
$R^{4-3}$ is C1~6 alkyl or C1~6 alkoxy;
the number of $R^5$ is 0~5, and at each occurrence, $R^5$ is independently hydrogen, halogen, nitro, nitrile group, $-N+(R^{5-1})_3$, $C_{1\sim6}$ haloalkyl, $-C(O)OR^{5-1}$, $-C(O)R^{5-1}$, $-C(O)N(R^{5-1}R^{5-1a})$, $-S(O)_2R^{5-1}$, $-S(O)R^{5-1}$, $-S(O)_2N(R^{5-1}R^{5-1a})$, $-S(O)N(R^{5-1}R^{5-1a})$, $-N=C(R^{5-1}\ R^{5-1a})$, hydroxyl, $C_{1\sim6}$ alkyl, phenyl, phenyl with at least one hydrogen substituted with $R^{5-1}$, $C_{1\sim6}$ alkoxy, $-N(R^{5-1}R^{5-1a})$, $-N(R^{5-1})C(O)R^{5-1a}$, $-N(R^{5-1})C(O)OR^{5-1a}$, $-OC(O)R^{5-1}$, $-OC(O)OR^{5-1}$, $-OC(O)\ N(R^{5-1}R^{5-1a})$ or $-SR^{5-1}$, $R^{5-1}$, $R^{5-1a}$ and $R^{5-1b}$ are independently hydrogen, $C_{1\sim6}$ alkyl, $C_{2\sim6}$ alkenyl, C2~6 alkynyl, $C_{1\sim6}$ alky with at least one hydrogen substituted with a halogen, $C_{2\sim6}$ alkenyl with at least one hydrogen substituted with a halogen or $C_{2\sim6}$ alkynyl with at least one hydrogen substituted with a halogen, respectively.

In some preferred embodiments, the Z is

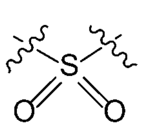, 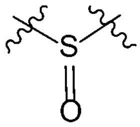, 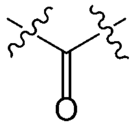,

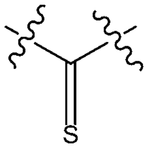

or a sulfur atom;

$R^1$ is hydrogen, $C_{1\sim4}$ alkyl,

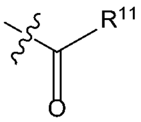 or 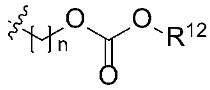,

wherein $R^{11}$ and $R^{12}$ are independently $C_{1\sim4}$ alkyl or $C_{1\sim4}$ cycloalkyl, respectively, and n is 1 or 2;

$R^2$ is hydrogen, $C_{1\sim4}$ alkyl, three- to six-membered cycloalkyl, three- to six-membered epoxyalkyl, phenyl, $C_{1\sim4}$ alkyl with at least one hydrogen substituted with $R^{2-1}$, and phenyl with at least one hydrogen substituted with $R^{2-1}$, wherein $R^{2-1}$ is hydroxy, halogen, amino, or $C_{1\sim4}$ alkoxy;

$R^3$ is

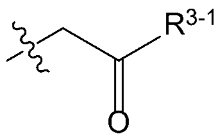, 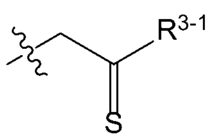 or 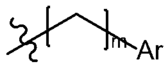;

wherein $R^{3-1}$ is hydrogen, hydroxyl, $C_{1\sim4}$ alkyl, $C_{1\sim4}$ alkoxy, $-N(R^{3-2}R^{3-2a})$,

$R^{3-2}$ and $R^{3-2a}$ are independently hydrogen or $C_{1\sim4}$ alkyl, respectively, $R^{3-3a}$ and $R^{3-3b}$ are independently hydrogen or $C_{1\sim4}$ alkyl, respectively,

m is 1~4.

Ar is phenyl, 5- or 6-membered monocyclic heteroaryl, 5- or 6-membered monocyclic heteroaryl with at least one hydrogen atom substituted with $R^{3-3}$, the $R^{3-3}$ is hydrogen, halogen, $C_{1\sim4}$ alkyl, hydroxyl, $C_{1\sim4}$ alkoxy, $C_{1\sim4}$ haloalkyl, or $-N(R^{3-3a}R^{3-3b})$;

$R^4$ is

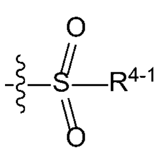, 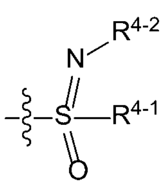, 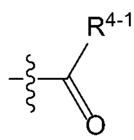 or 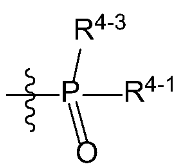,

wherein $R^{4-1}$ is phenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, and the $R^{4-11}$ is hydrogen, halogen, nitro, $C_{1\sim4}$ alkyl, $C_{3\sim6}$ cycloalkyl, $C_{1\sim4}$ alkoxy, $-N(R^{4-1a}R^{4-1b})$, phenyl, $C_{1\sim4}$ haloalkyl, $C_{1\sim4}$ haloalkoxy or

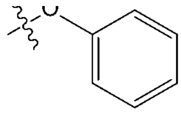,

$R^{4-1a}$ and $R^{4-1b}$ are independently hydrogen, $C_{1\sim4}$ alkyl, or $C_{3\sim6}$ cycloalkyl, respectively, and $R^{4-1a}$ and $R^{4-1b}$ are bondable to each other to form a ring,
$R^{4-2}$ is $C_{1\sim4}$ alkyl, $C_{3\sim5}$ cycloalkyl, $C_{1\sim4}$ alkyl with at least one hydrogen substituted with hydroxyl, $C_{3\sim5}$ epoxyalkyl, or $R^{4-2}$ is bonded to $R^2$ to form a 4- to 8-membered ring when $R^2$ is $C_{1\sim4}$ alkyl and $R^{4-2}$ is $C_{1\sim4}$ alkyl,
$R^{4-3}$ is $C_{1\sim4}$ alkyl or $C_{1\sim4}$ alkoxy;
at each occurrence, $R^5$ is independently hydrogen halogen, nitro, nitrile group, $-N+(R^{5-1})_3$, $C_{1\sim4}$ haloalkyl, $C(O)OR^{5-1}$, $-C(O)R^{5-1}$, $-C(O)N(R^{5-1}R^{5-1a})$, $-S(O)_2R^{5-1}$, $-S(O)R^{5-1}$, $-N{=}C(R^{5-1}\,R^{5-1a})$, hydroxyl, $C_{1\sim4}$ alkyl, phenyl, phenyl with at least one hydrogen substituted with $R^{5-1}$, $C_{1\sim4}$ alkoxy, $-N(R^{5-1}R^{5-1a})$, $-N(R^{5-1})C(O)R^{5-1a}$, or $-OC(O)R^{5-1}$, respectively, wherein $R^{5-1}$, $R^{5-1a}$, and $R^{5-1b}$ are independently hydrogen, $C_{1\sim4}$ alkyl, $C_{1\sim4}$ alkyl with at least one hydrogen substituted with a halogen, respectively.

In some preferred embodiments, the Z is

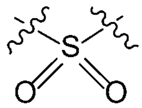, 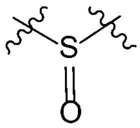, 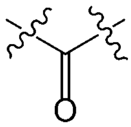,

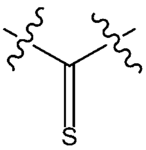

or a sulfur atom;

In some preferred embodiments, $R^1$ is hydrogen,

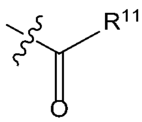

or

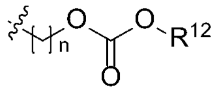,

wherein $R^{11}$ and $R^{12}$ are independently methyl, ethyl, n-propyl or isopropyl, respectively, and n is 1.

In some preferred embodiments, $R^2$ is hydrogen, methyl, ethyl, n-propyl, isopropyl, ethyl with one hydrogen substituted with hydroxyl, n-propyl with one hydrogen substituted with hydroxyl, phenyl,

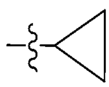, 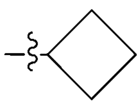, 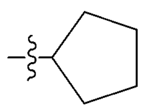,

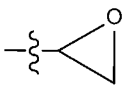, 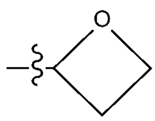, 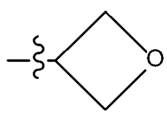, 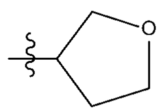 or 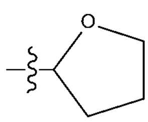.

In some preferred embodiments, $R^3$ is

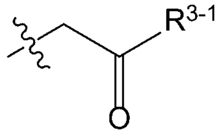,
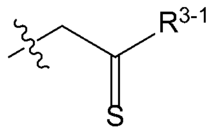

or

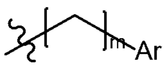;

wherein $R^{3-1}$ is hydrogen, hydroxyl, methyl, ethyl, n-propyl, isopropyl, methoxy, ethoxy, n-propoxy, isopropoxy, n-butoxy, tert-butoxy, sec-butoxy or isobutoxy,
m is 1,
Ar is phenyl, 5- or 6-membered nitrogen-containing monocyclic heteroaryl.

In some preferred embodiments, $R^4$ is

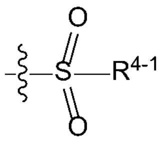,
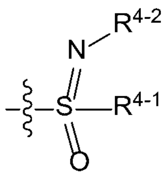,
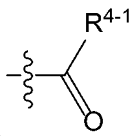

or

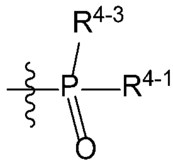,

wherein R4-1 is phenyl, phenyl with at least one hydrogen substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and the $R^{4-11}$ is halogen, nitro, methyl, ethyl, n-propyl, isopropyl,

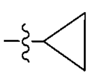, 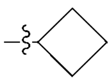, 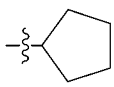, 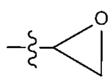, 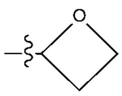, 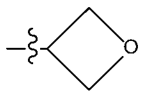,

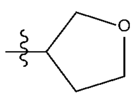, 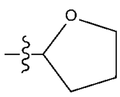, 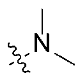, 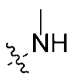, 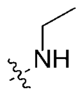, 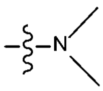, 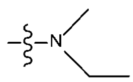, 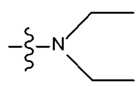,

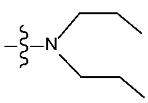, 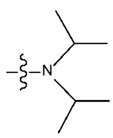, 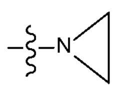, 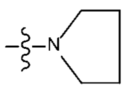, 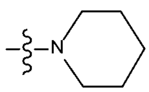,

fluoromethyl, fluoroethyl, fluoron-propyl, fluoroisopropyl, chloromethyl, chloroethyl, chloron-propyl, chloro isopropyl,

bromomethyl, bromoethyl, bromo -propyl, bromoisopropyl, iodomethyl, iodoethyl, iodon-propyl, iodoisopropyl, fluoromethoxy, fluoroethoxy, fluoron-propoxy, fluoroisopropoxy, chloromethoxy, chloroethoxy, chloron-propoxy, chloroisopropoxy, bromomethoxy, bromoethoxy, bromon-propoxy, bromoisopropoxy, Iodooxymethyl, iodoethoxy, iodon-propoxy, iodoisopropoxy, or

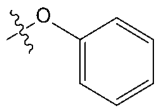,

$R^{4-2}$ is methyl, ethyl, n-propyl, isopropyl, n-butyl, isobutyl, tert-butyl, ethyl with one hydrogen substituted with hydroxyl, n-propyl with one hydrogen substituted with hydroxyl, n-butyl with one hydrogen substituted with hydroxyl,

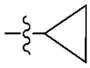, 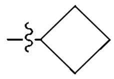,

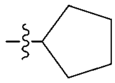, 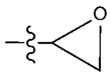, 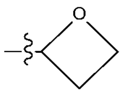, 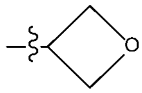, 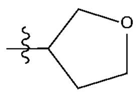, 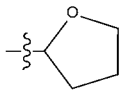

or phenyl, or, $R^{4-2}$ is bonded to $R^2$ to form a 4 to 8-membered ring when $R^2$ is methyl, ethyl or n-propyl and $R^{4-2}$ is methyl, ethyl,

$R^{4-3}$ is methyl, ethyl, n-propyl, isopropyl, methoxy, ethoxy, n-propoxy or isopropoxy.

In some preferred embodiments, at each occurrence, $R^5$ is independently hydrogen, halogen, nitro, nitrile group, $-N+(R^{5-1})^3$, fluoro-methyl, fluoroethyl, fluoron-propyl, fluoro-isopropyl, chloromethyl, chloroethyl, chloron-propyl, chloro-isopropyl, bromomethyl, bromoethyl, bromo-n-propyl, bromo-isopropyl, $-C(O)OR^{5-1}$, $-C(O)R^{5-1}$, $-C(O)N(R^{5-1}R^{5-1a})$, $-S(O)_2R^{5-1}$, $-S(O)R^{5-1}$, $-N=C(R^{5-1}\ R^{5-1a})$, hydroxyl, methyl, ethyl, n-propyl, isopropyl, phenyl, phenyl with at least one hydrogen substituted with $R^{5-1}$, methoxy, ethoxy, n-propoxy, isopropoxy, $-N(R^{5-1}R^{5-1a})$, $-N(R^{5-1})C(O)R^{5-1a}$, $-OC(O)R^{5-1}$, respectively, wherein $R^{5-1}$, $R^{5-1a}$, and $R^{5-1b}$ are independently hydrogen, methyl, ethyl, n-propyl, isopropyl, fluoro-methyl, fluoroethyl, fluoron-propyl, fluoro-isopropyl, chloro-methyl, chloroethyl, chloro n-propyl, chloro isopropyl, bromo-methyl, bromoethyl, bromo n-propyl, or bromo isopropyl, respectively.

In some preferred embodiments, $R^1$ is hydrogen,

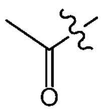, 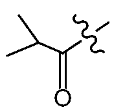

or

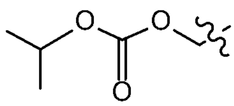.

In some preferred embodiments, $R^2$ is hydrogen, methyl,

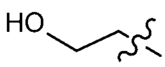, 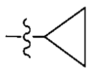,

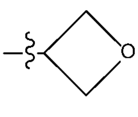

or phenyl.

In some preferred embodiments, $R^3$ is

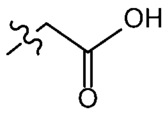 , 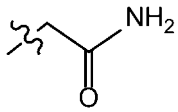 ,

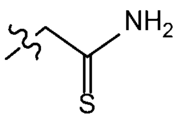 , 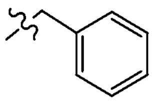 , 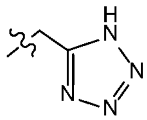 or 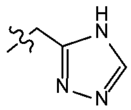 .

In some preferred embodiments, $R^4$ is

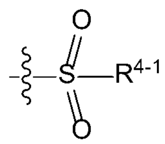 , 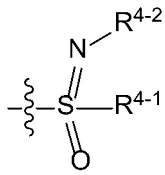 ,

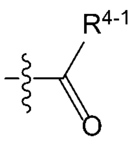 or 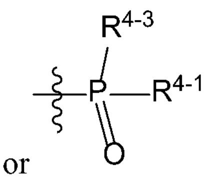 ,

wherein $R^{4-1}$ is phenyl, phenyl with at least one hydrogen substituted with $R^{4-11}$, five-membered monocyclic heteroaryl, five-membered monocyclic heteroaryl with at least one hydrogen substituted with $R^{4-11}$, nine-membered fused bicyclic heteroaryl, or naphthyl, wherein $R^{4-11}$ is bromine, fluorine, chlorine, methyl, nitro, phenyl, trifluoromethyl,

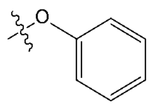 ,

methoxy, cyclopropyl, trifluoromethoxy, nitro or

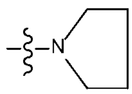 .

$R^{4-2}$ is methyl, ethyl,

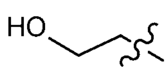 , 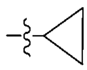 , 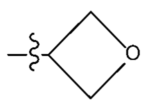

or phenyl, or, $R^{4-2}$ is bonded to $R^2$ to form a 4 to 6-membered ring when $R^2$ is methyl, ethyl, or n-propyl and $R^{4-2}$ is methyl, ethyl,

$R^{4-3}$ is methoxy, ethoxy, n-propoxy or isopropoxy.

In some preferred embodiments, at each occurrence, $R^5$ is independently halogen, nitro, nitrile, carboxyl, $-NHC(O)CH_3$, methoxy, or hydroxy, respectively.

In some preferred embodiments, the compound is selected from any one of the following compounds:

| | |
|---|---|
| I-1 | 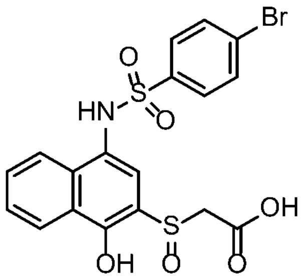 |
| I-2 | 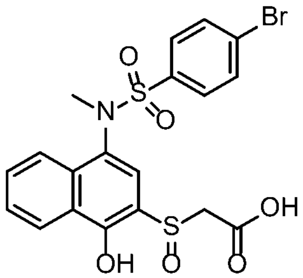 |
| I-3 | 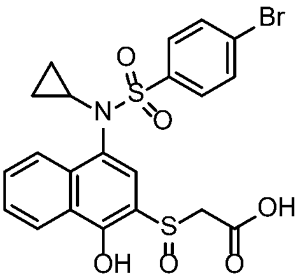 |
| I-4 | 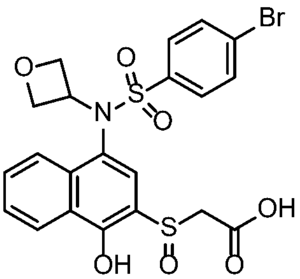 |
| I-5 | 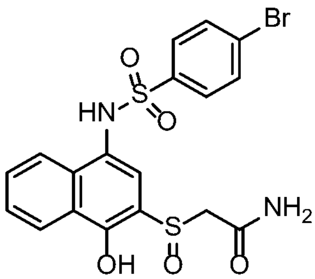 |

(continued)

| | |
|---|---|
| **I-6** | 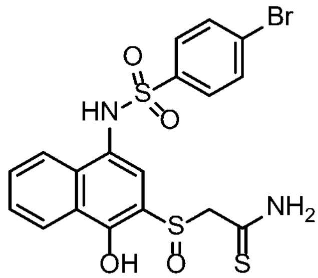 |
| **I-7-1** | 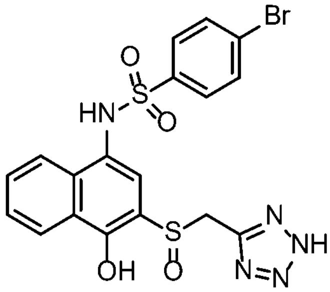 |
| **I-8** | 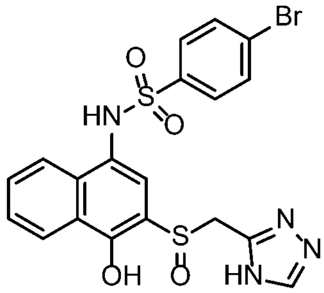 |
| **I-9-1** | 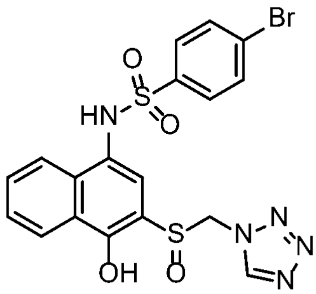 |
| **I-9-2** | 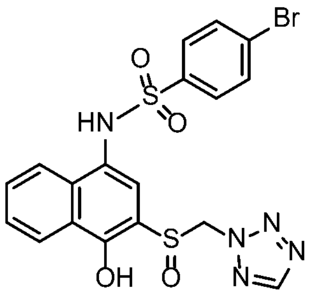 |

(continued)

| | |
|---|---|
| **I-10** | 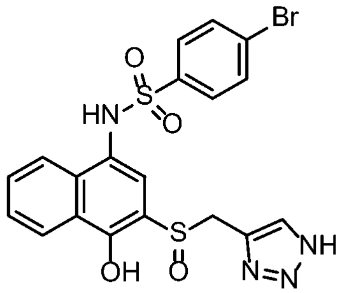 |
| **I-11-1** | 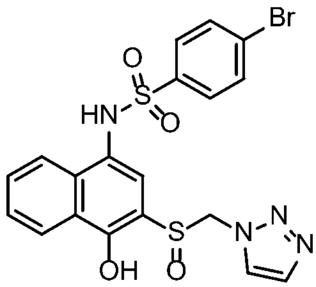 |
| **I-11-2** | 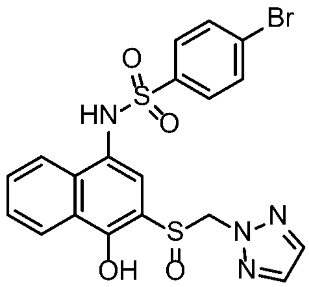 |
| **I-12** | 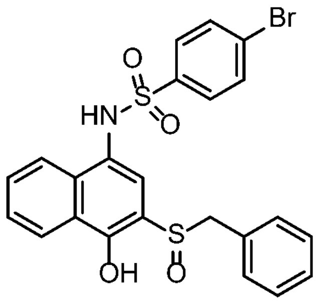 |
| **I-13-1** | 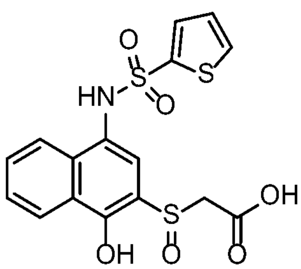 |

(continued)

| | |
|---|---|
| **I-14-1** | 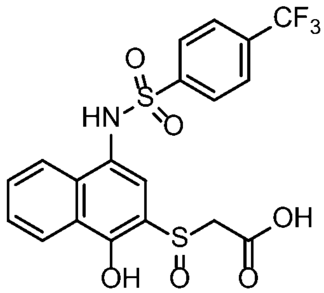 |
| **I-15-1** | 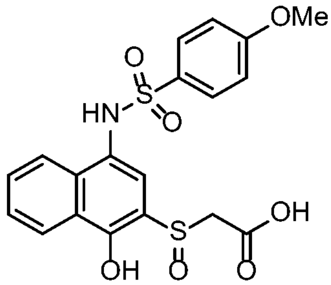 |
| **I-16-1** | 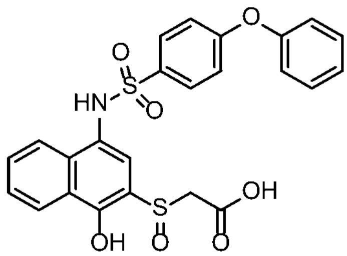 |
| **I-17-1** | 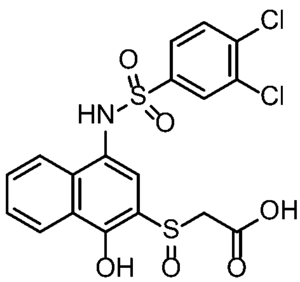 |
| **I-18-1** | 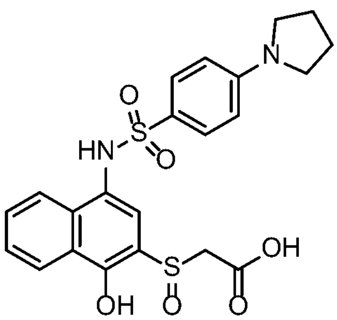 |

(continued)

| | |
|---|---|
| **I-19-1** | 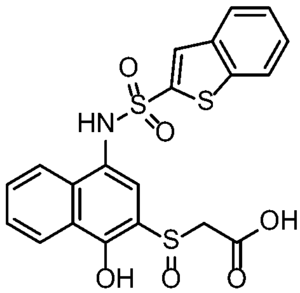 |
| **I-20-1** | 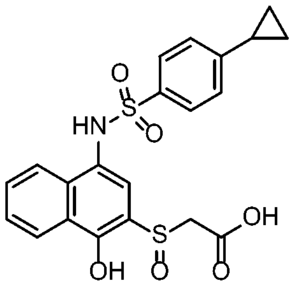 |
| **I-21-1** | 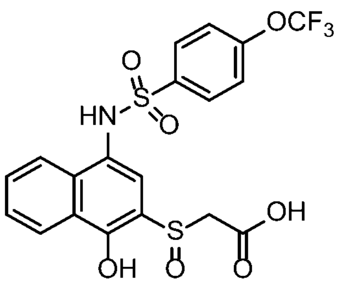 |
| **I-22-1** | 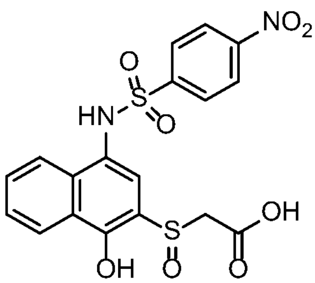 |
| **I-23-1** | 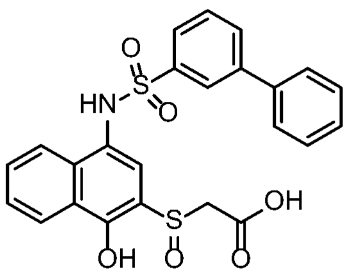 |

(continued)

| | |
|---|---|
| **I-24-1** | 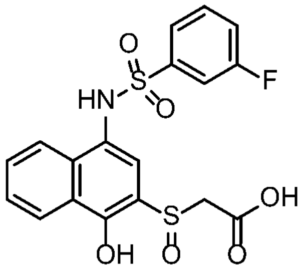 |
| **I-25-1** | 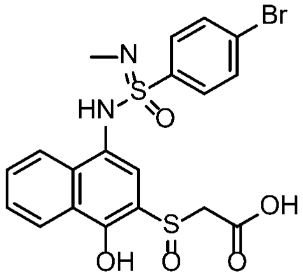 |
| **I-26** | 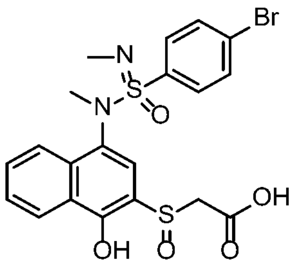 |
| **I-27** | 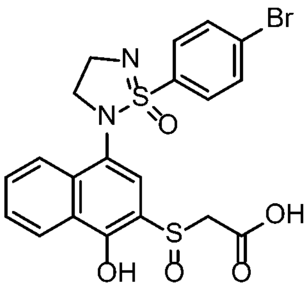 |
| **I-28** | 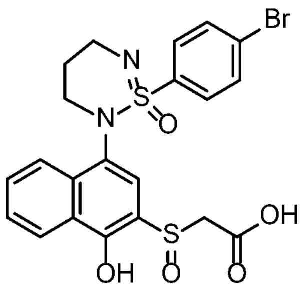 |

(continued)

| | |
|---|---|
| I-29 | 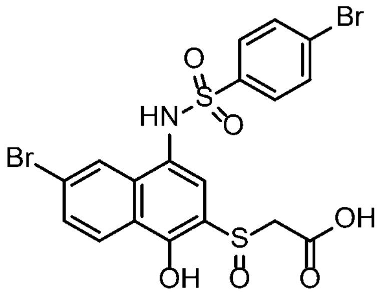 |
| I-30 | 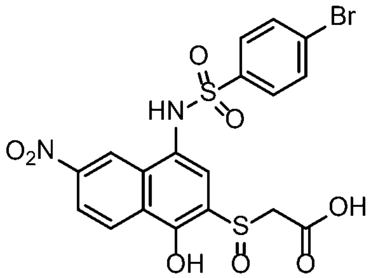 |
| I-31 | 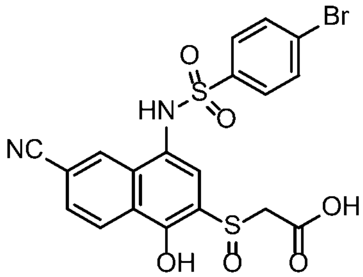 |
| I-32 | 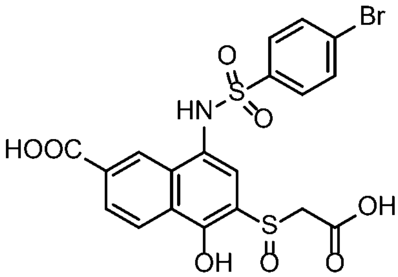 |
| I-33 | 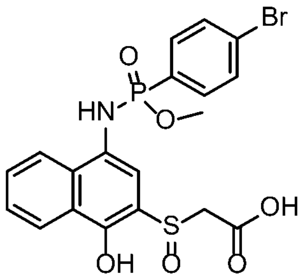 |

(continued)

| | |
|---|---|
| **I-34** | 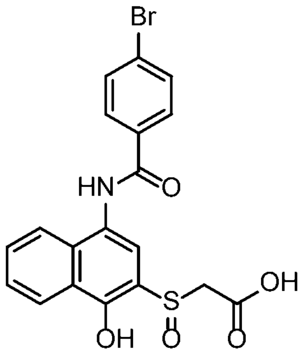 |
| **I-35-1** | 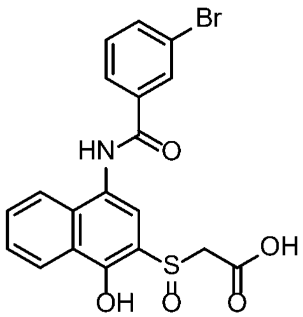 |
| **I-36** | 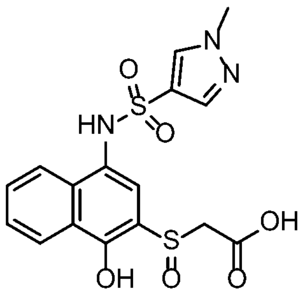 |
| **I-37** | 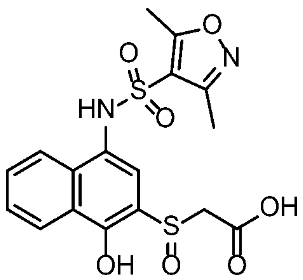 |
| **I-38** | 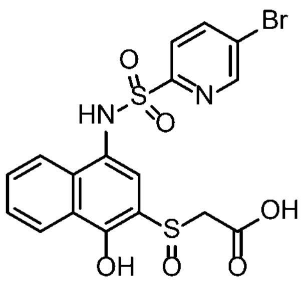 |

(continued)

| | |
|---|---|
| **I-39** | 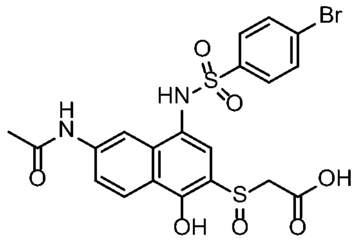 |
| **I-40-1** | 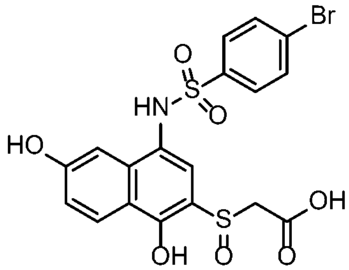 |
| **I-41-1** | 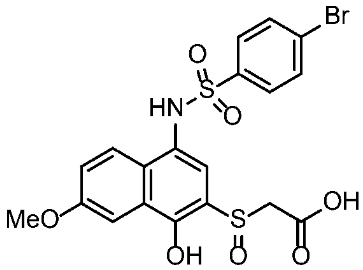 |
| **I-42** | 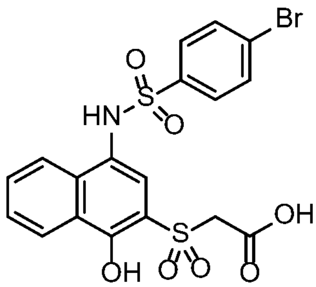 |
| **I-7-2** | 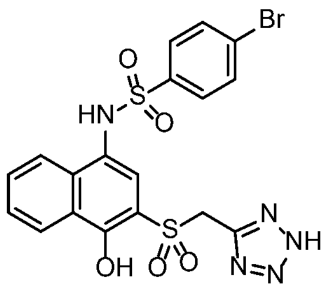 |
| **I-13-2** | 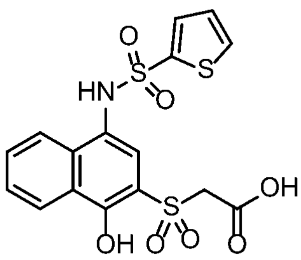 |

(continued)

| | |
|---|---|
| **I-14-2** | 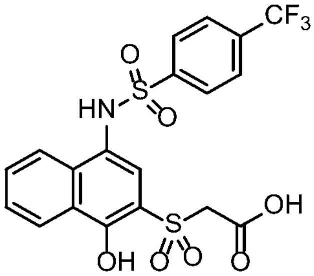 |
| **I-15-2** | 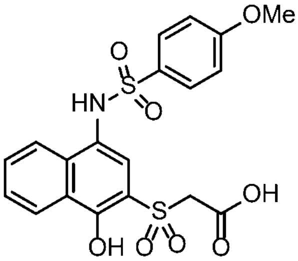 |
| **I-16-2** | 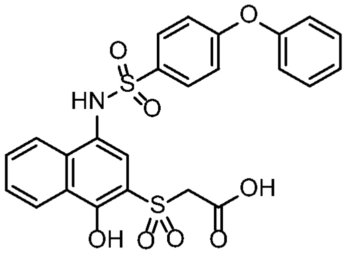 |
| **I-17-2** | 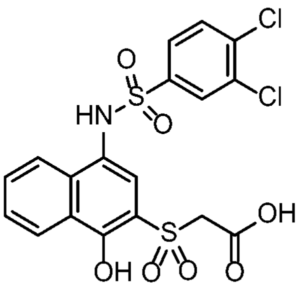 |
| **I-18-2** | 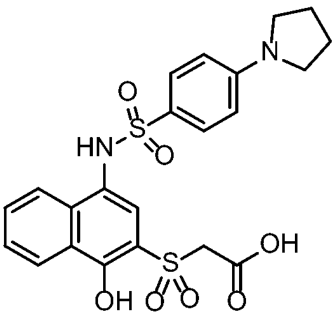 |

(continued)

| | |
|---|---|
| **I-19-2** | 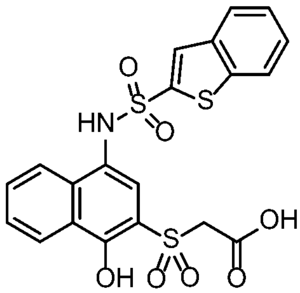 |
| **I-20-2** | 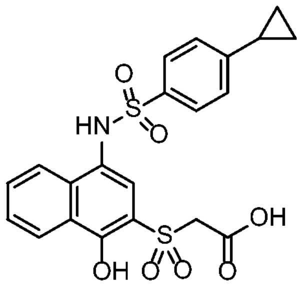 |
| **I-21-2** | 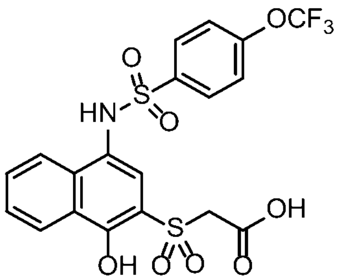 |
| **I-22-2** | 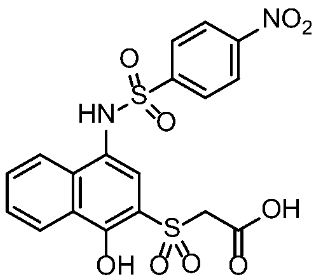 |
| **I-23-2** | 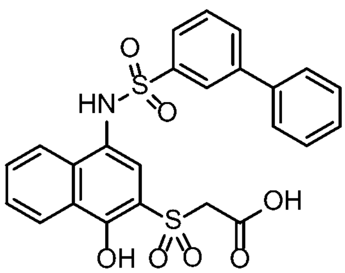 |

(continued)

| | |
|---|---|
| **I-24-2** | 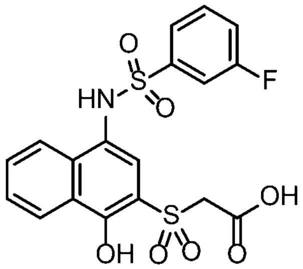 |
| **I-25-2** | 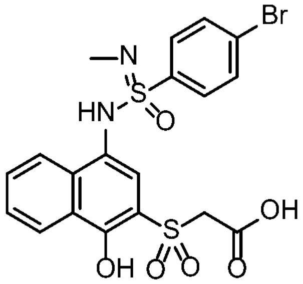 |
| **I-35-2** | 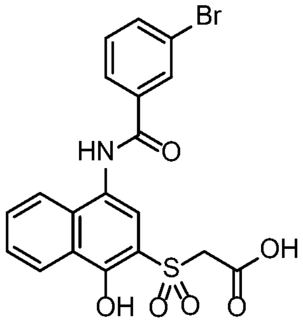 |
| **I-40-2** | 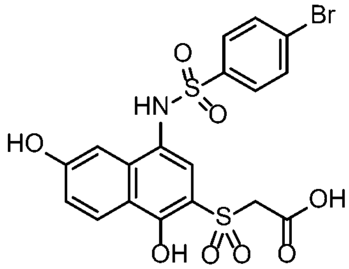 |
| **I-41-2** | 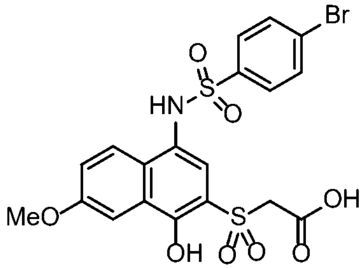 |

(continued)

| | |
|---|---|
| **I-43** | 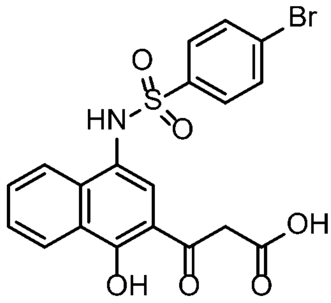 |
| **I-44** | 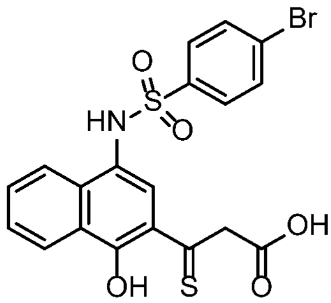 |
| **I-45** | 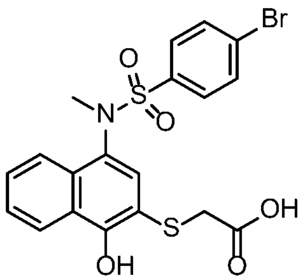 |
| **I-46** | 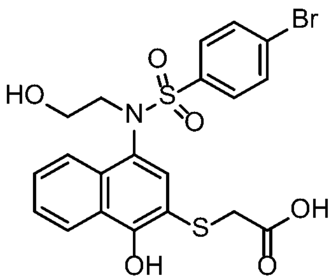 |
| **I-47** | 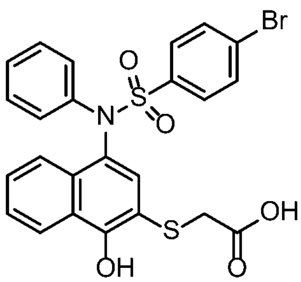 |

(continued)

| | |
|---|---|
| **I-48** | 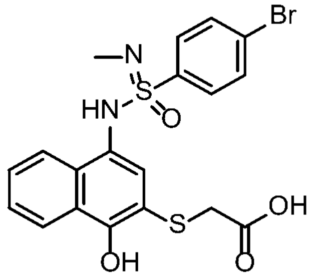 |
| **I-49** | 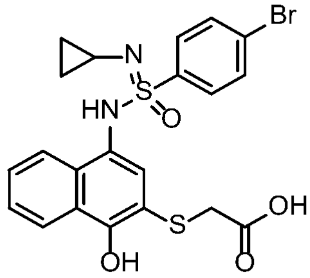 |
| **I-50** | 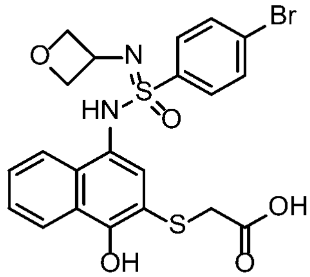 |
| **I-51** | 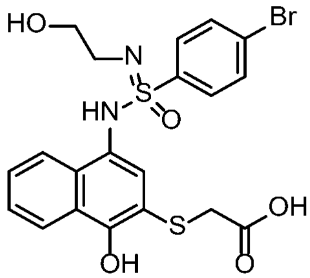 |
| **I-52** | 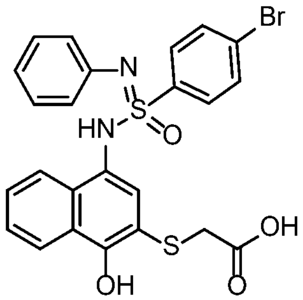 |

(continued)

| | |
|---|---|
| **I-53** | 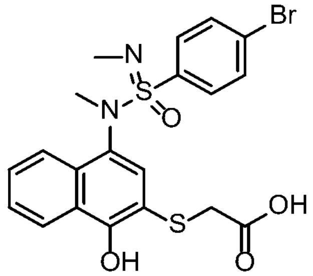 |
| **I-54** | 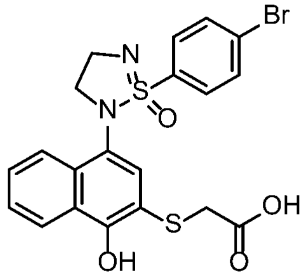 |
| **I-55** | 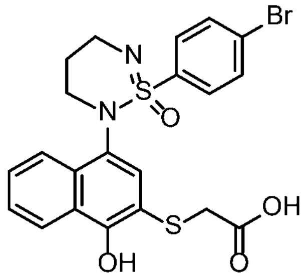 |
| **I-56** | 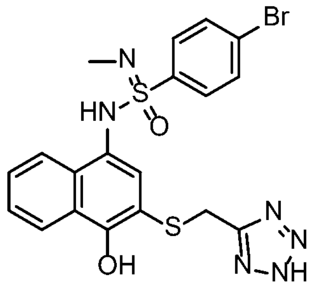 |
| **I-57** | 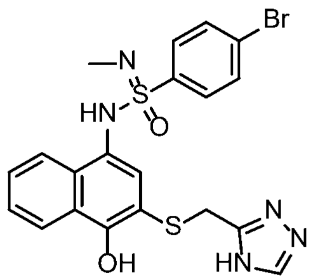 |

(continued)

| | |
|---|---|
| **I-58** | 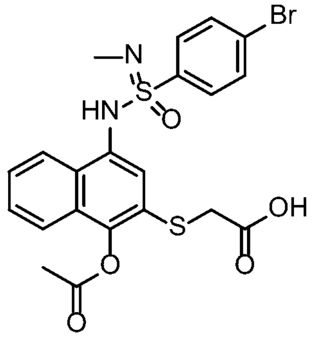 |
| **I-59** | 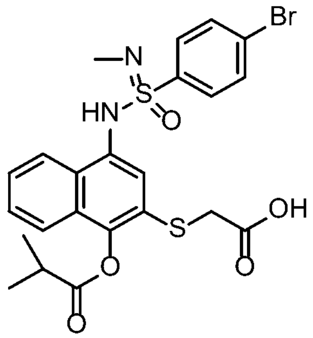 |
| **I-60** | 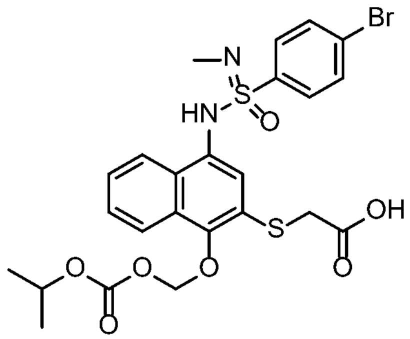 |
| **I-61** | 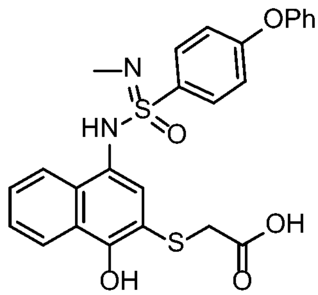 |
| **I-62** | 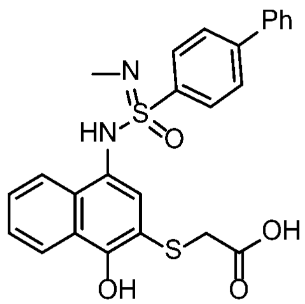 |

(continued)

| | |
|---|---|
| **I-63** | 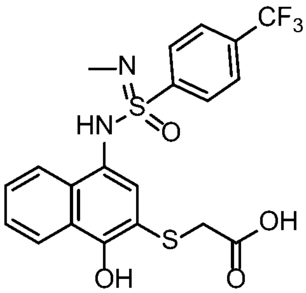 |
| **I-64** | 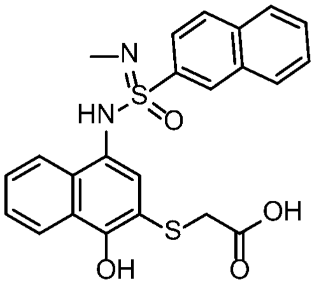 |
| **I-65** | 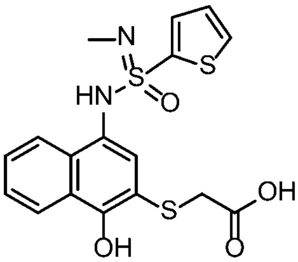 |
| **I-66** | 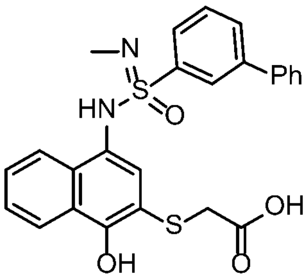 |
| **I-67** | 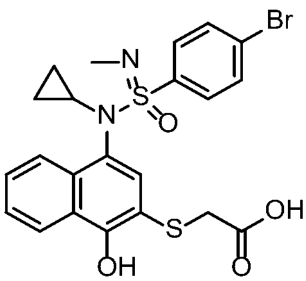 |

(continued)

| | |
|---|---|
| **I-68** | 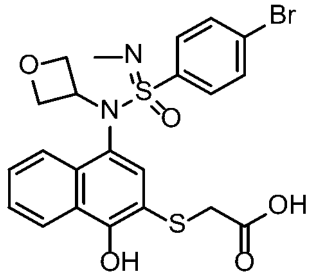 |
| **I-69** | 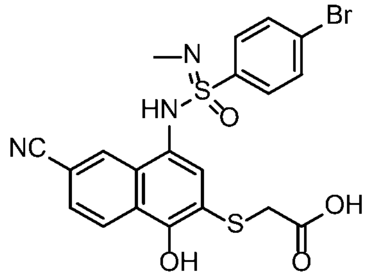 |
| **I-70** | 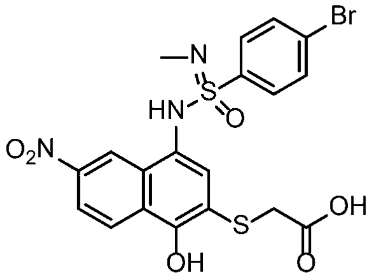 |

In some preferred embodiments, Z is not a sulfur atom.

In some preferred embodiments, Z is

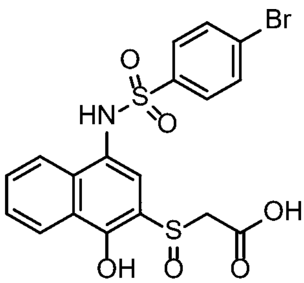

.

In some preferred embodiments, Z is not

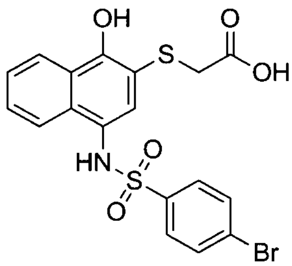

.

In some preferred embodiments, the diseases associated with kidney injury include acute kidney injury and chronic kidney injury, preferably chronic kidney injury.

In some preferred embodiments, the diseases associated with kidney injury are selected from acute renal ischemia-reperfusion injury, sepsis nephropathy, nephrotoxin injury, primary glomerulonephritis, hypertensive renal arteriosclerosis, diabetes nephropathy, secondary glomerulonephritis, renal tubulointerstitial lesions, ischemic nephropathy, lupus nephritis and hereditary nephropathy.

In some preferred embodiments, the renal tubulointerstitial lesions are selected from chronic pyelonephritis, chronic uric acid nephropathy, obstructive nephropathy, and drug-induced nephropathy.

In some preferred embodiments, the hereditary nephropathy is selected from polycystic kidney disease and hereditary nephritis.

In some preferred embodiments, the diseases associated with mitochondrial dysfunction are selected from at least one of the following: inflammatory bowel disease; lung injury; fibrotic diseases; sepsis; prostate disease; cardiovascular disease; neurological disorders; and aging-related diseases.

In some preferred embodiments, the inflammatory bowel disease is selected from at least one of ulcerative colitis and Crohn's disease.

In some preferred embodiments, the lung injury is selected from at least one of acute lung injury and chronic lung injury.

In some preferred embodiments, the chronic lung injury is chronic obstructive pulmonary disease.

In some preferred embodiments, the fibrotic disease is selected from at least one of renal tubulointerstitial fibrosis, interstitial lung disease (ILD), idiopathic pulmonary fibrosis (IPF), chronic obstructive pulmonary disease fibrosis, tissue fibrosis, joint fibrosis, liver fibrosis, skin fibrosis, fibromatosis, myelofibrosis, cardiac fibrosis, and cystic fibrosis.

In some preferred embodiments, the cardiovascular disease is selected from at least one of atherosclerosis, heart failure, myocardial ischemia/reperfusion injury and cardiovascular complications of hypertension, cardiomyopathy and diabetes.

In some preferred embodiments, the cardiomyopathy is myocarditis.

In some preferred embodiments, the neurological disorders are selected from at least one of sensory neuropathic hearing loss, developmental abnormalities in the brain, congenital hydrocephalus, congenital cranial nerve disease, congenital brain pathway abnormalities, metabolic dysfunction, congenital auditory aphasia, congenital visual aphasia, cerebral palsy, autism, depression, schizophrenia, bipolar disorder, paranoid mental disorder, manic disorder, obsessive-compulsive disorder, autism and other mental disorders, Parkinson's disease, Alzheimer's disease, brain injury, amyotrophic lateral sclerosis, epilepsy, Huntington's disease, spinocerebellar ataxia, cerebral ischemia (CI), stroke (preferably ischemic stroke), multiple sclerosis, and tinnitus.

In some preferred embodiments, the aging-related disease is premature aging.

In some preferred embodiments, the aging-related disease is early-onset ovarian insufficiency or naturally aging ovarian insufficiency.

In some preferred embodiments, the aging-related disease is skin aging, and more preferably, the aging-related disease is skin aging or damage caused by radiation.

A second aspect of the present invention provides a method for preventing and/or treating diseases associated with kidney injury, wherein administering a compound shown in formula (I) or a pharmaceutically acceptable salt, stereoisomer, solvate, or prodrug thereof to a subject.

A third aspect of the present invention provides a method for preventing and/or treating diseases associated with mitochondrial dysfunction, wherein the method comprises the steps of administering a therapeutically effective amount of a compound shown in formula (I) or a pharmaceutically acceptable salt, stereoisomer, solvate, or prodrug thereof to a subject.

In some preferred embodiments, the disease associated with mitochondrial dysfunction is cardiovascular disease, and the method comprises the steps of: administering a therapeutically effective amount of the compound shown in formula (I) or a pharmaceutically acceptable salt, stereoisomer, solvate or prodrug thereof, and Entresto to the subject in combination.

In some preferred embodiments, the disease associated with mitochondrial dysfunction is benign prostatic hyperplasia, and the method comprises the steps of: administering a therapeutically effective amount of the compound shown in formula (I) or a pharmaceutically acceptable salt, stereoisomer, solvate or prodrug thereof, and finasteride in combination to a subject.

In some combination drug schemes for treating benign prostatic hyperplasia, the compound shown in general formula (I) is compound I-1.

In some combination drug schemes for treating cardiovascular diseases, the compound shown in general formula (I) is compound I-1.

In some preferred embodiments, the single application dose of the compound shown in formula (I) is 0.1-100mg/kg, such as 0.5mg/kg, 1 mg/kg, 1.5 mg/kg, 2 mg/kg, 5 mg/kg, 10 mg/kg, 12 mg/kg, 15 mg/kg or 20 mg/kg, 50 mg/kg.

In some preferred embodiments, the administration frequency of the compound shown in formula (I) is once daily,

twice daily, three times daily, once every two days, once every three days, or once every week.

In some preferred embodiments, the administration period of the compound shown in formula (I) is I-365 days, such as 1 day, 7 days, 10 days, 15 days, 30 days, 60 days, 90 days, or 180 days.

In some preferred embodiments, the administration method of the compound shown in general formula (I) is selected from oral, intravenous, intratracheal, intramuscular, subcutaneous, intraperitoneal, intraspinal or local injection/-drip.

A fourth aspect of the present invention provides a cosmetic or medical instrument composition, wherein the cosmetic or medical instrument composition comprises a compound shown in general formula (I) and acceptable excipients in the cosmetic or medical instrument.

Compared to prior art, the present invention has at least the following advantages:

(1) The present invention first discovers that compounds of formula (I) can be used to treat diseases associated with kidney injury, and is expected to become effective specialized drugs for treating kidney related diseases, especially chronic kidney diseases;

(2) The present invention first discovered that compounds of formula (I) can be used to prepare drugs for the treatment of diseases associated with kidney injury;

(3) The present invention studies the use of compounds of general formula (I) as mitophagy inducers for treating various diseases associated with mitophagy or for preparing drugs for treating these diseases. Specifically, multiple disease models are studied at the animal level, confirming the excellent therapeutic effect of compounds of general formula (I) on these diseases;

(4) In the preferred embodiments of the present invention, the method of combining a compound of formula (I) (such as compound I-1) with other drugs is also explored.

It should be understood that within the scope of the present invention, the above-mentioned technical features of the present invention and the specific technical features described below (as embodiments) can be combined with each other to form new or preferred technical solutions. Due to space limitations, we will not elaborate on each one here.

Description of drawings

One or more embodiments are illustrated by the corresponding pictures in the accompanying drawings, which do not constitute limitations to the embodiments.

FIG. 1 is a statistical graph of blood creatinine and urea nitrogen in mice with renal ischemia-reperfusion after administration of I-1;

FIG. 2 is a HE staining graph of the renal tissue in mice with renal ischemia-reperfusion after administration of I-1;

FIG. 3 is a immunofluorescence graph of the renal in mice with renal ischemia-reperfusion after administration of I-1;

FIG. 4 is a statistical graph of weight of rats with 5/6 nephrectomy after 27 days of administration of I-1;

FIG. 5 is a statistical graph of blood creatinine in rats with 5/6 nephrectomy after 27 days of administration of I-1;

FIG. 6 is a statistical graph of urinary albumin in rats with 5/6 nephrectomy at the endpoint of I-1 administration for 27 days;

FIG. 7 is a statistical graph of creatinine clearance rate in rats with 5/6 nephrectomy after 27 days of administration of I-1;

FIG. 8 is a HE staining graph of renal tissue in rats with 5/6 nephrectomy after 27 days of administration of I-1;

FIG. 9 is a statistical graph of weight of rats of adenine induced kidney injury model after 21 days of administration of I-1;

FIG. 10 is a statistical graph of serum creatinine levels in rats of adenine induced kidney injury model after 21 days of administration of I-1;

FIG. 11 is a statistical graph of effect of serum uric acid levels in rats of adenine induced renal injury model after 21 days of administration of I-1;

FIG. 12 is a statistical graph of the effects of inflammatory factors on renal tissue in rats of adenine induced kidney injury model after 21 days of administration of I-1;

FIG. 13 is a HE staining graph of renal tissue in rats of adenine induced kidney injury model after 21 days of administration of I-1;

FIG. 14 is a statistical graph of weight increased after 27 days of administration of I-1 in rats of type I diabetes nephropathy model;

FIG. 15 is a statistical graph of serum creatinine in rats of type I diabetes nephropathy model after 27 days of administration of I-1;

FIG. 16 is a statistical graph of blood glucose level in rats of type I diabetes nephropathy model after 27 days of

administration of I-1;

FIG. 17 is a statistical graph of urinary protein level of type I diabetes nephropathy model rats after 27 days of administration of I-1;

FIG. 18 is a HE staining graph of kidney tissue in rats of type I diabetes nephropathy model after 27 days of administration of I-1;

FIG. 19 is the result graph of in vitro liver microsome stability experiments for compounds I-1 and UMI-77.

FIG. 20 is a schematic graph of weight changes in mice of the group of small molecule compound TJ01-013 injected intraperitoneally during DSS induction according to an embodiment of the present invention;

FIG. 21 is the disease acquisition index of the group of small molecule compound TJ01-013 injected intraperitoneally during DSS induction according to the embodiment of the present invention;

FIG. 22 is a graph of change in length of the colon in mice according to an embodiment of the present invention;

FIG. 23 is a schematic diagram of stained tissue sections and histopathological scores according to the embodiments of the present invention;

FIG. 24 is a graph of changes in inflammatory factors in mouse colorectal tissue according to the embodiments of the present invention;

FIG. 25 is a graph of changes in the total number of cells according to the embodiments of the present invention;

FIG. 26 is a graph of changes in inflammatory factors in alveolar lavage fluid according to an embodiment of the present invention;

FIG. 27 is a graph of changes in transcription levels of tissue inflammatory factors according to the embodiments of the present invention;

FIG. 28 is a survival curve of acute lung injury according to an embodiment of the present invention;

FIG. 29 is a Masson trichrome staining identification graph of the degree of fibrosis in renal tissue samples according to an embodiment of the present invention;

FIG. 30 is a graph of changes in mRNA levels of $\alpha$-smooth muscle actin, fibronectin, and type I collagen in renal tissue homogenate detected by fluorescence quantitative PCR according to the embodiment of the present invention;

FIG. 31 is a graph of quantitative analysis of the grayscale levels of $\alpha$-smooth muscle actin, fibronectin, and type I collagen according to an embodiment of the present invention;

FIG. 32 is a mitochondrial morphology graph of renal tubular epithelial cells according to an embodiment of the present invention;

FIG. 33 is a schematic diagram of the survival rate of sepsis according to an embodiment of the present invention;

FIG. 34 is a weight diagram of the rat prostate and urethra according to an embodiment of the present invention;

FIG. 35 is the coefficient diagram of rat prostate organs according to the embodiment of the present invention;

FIG. 36 shows the effects of TJ01-013, finasteride, and combined administration on the weight of the prostate and urethra in rats according to the embodiments of the present invention;

FIG. 37 shows the effect of TJ01-013, Novartis Entresto, and combined administration on the area of myocardial infarction in rats according to the embodiments of the present invention;

FIG. 38 shows the effect of TJ01-013, Novartis Entresto, and combined administration on the left ventricular end diastolic diameter of rats according to the embodiments of the present invention;

FIG. 39 shows the effect of TJ01-013, Novartis Entresto, and combined administration on the end systolic diameter of rats according to the embodiments of the present invention;

FIG. 40 shows the effect of TJ01-013, Novartis Entresto, and combined administration on the left ventricular ejection fraction (EF) in rats according to the embodiments of the present invention;

FIG. 41 shows the effect of TJ01-013, Novartis Entresto, and combined administration on the left ventricular short axis shortening rate in rats according to the embodiments of the present invention;

FIG. 42 shows the time consumption for finding the platform in the water maze experiment according to the embodiment of the present invention;

FIG. 43 is a trajectory diagram of the water maze experiment crossing the platform according to an embodiment of the present invention;

FIG. 44 shows the activation number of astrocytes in the hippocampal region of mice according to an embodiment of the present invention;

FIG. 45 is a diagram of A $\beta$ content in mouse brain tissue according to an embodiment of the present invention;

FIG. 46 shows the amplitude changes of the mouse hearing threshold at various frequencies according to the embodiments of the present invention;

FIG. 47 is a fluorescence graph of mouse hair cells labeled by Myo7a in the top, middle, and bottom gyrus according to the embodiment of the present invention;

FIG. 48 is a schematic diagram of the percentage of SA-$\beta$-gal positive cells in Con-O compared to Con-Y of mouse bone marrow mesenchymal stem cells according to an embodiment of the present invention;

FIG. 49 shows the level changes of DNA damage mark $\gamma$ - H2AX in old cells (Con-O) of mouse bone marrow

mesenchymal stem cells according to the embodiment of the present invention;

FIG. 50 is a schematic diagram of the expression level of heterochromatin modification H3K9me3 in Con-O cells in mouse bone marrow mesenchymal stem cells according to the embodiment of the present invention;

FIG. 51 is a schematic diagram of the percentage of SA-β-gal positive cells in CMC cells according to an embodiment of the present invention;

FIG. 52 is a schematic diagram of the level of gamma-H2AX in CMC cells according to an embodiment of the present invention;

FIG. 53 is a schematic diagram of the expression level of H3K9me3 in CMC cells according to an embodiment of the present invention;

FIG. 54 is a schematic diagram of the percentage of SA-β-gal positive cells in HGPS CMC cells according to an embodiment of the present invention;

FIG. 55 is a schematic diagram of the level of gamma H2AX in HGPS CMC cells according to an embodiment of the present invention;

FIG. 56 is a schematic diagram of the expression level of H3K9me3 in HGPS-CMC cells according to an embodiment of the present invention.

FIG. 57 shows the results of a mouse sucrose water preference test according to the embodiment of the present invention;

FIG. 58 shows the results of a mouse tail suspension test according to an embodiment of the present invention;

FIG. 59 is a distance graph of activity in a mouse open field test according to an embodiment of the present invention;

FIG. 60 is a time graph of locating in the edge area of the open field in a mouse open field test according to an embodiment of the present invention;

FIG. 61 shows the average movement speed in a mouse open field test according to an embodiment of the present invention.

FIG. 62 shows the quantitative analysis of serum TNF α and CXCL1 after administering compound TJ0113 to chronic obstructive pulmonary disease according to the embodiments of the present invention;

FIG. 63 shows a quantitative analysis of seizure levels after administering compound TJ01-013 to an epilepsy model according to an embodiment of the present invention;

FIG. 64 shows a quantitative analysis of the latency after stimulation in a Step-down test after the administration of compound TJ01-013 to the epilepsy model according to the embodiment of the present invention;

FIG. 65 shows the quantitative analysis of the latency after stimulation in a post-dark avoidance test after administering compound TJ01-013 to the epilepsy model according to the embodiment of the present invention;

FIG. 66 shows the quantitative analysis of serum inflammatory factors after administering compound TJ01-013 to the epilepsy model according to the embodiment of the present invention;

FIG. 67 shows the quantitative analysis of body weight, ovarian weight, and ovarian organ coefficient of mice after administrating compound TJ0113 in a chemotherapy induced ovarian premature failure model according to the embodiment of the present invention;

FIG. 68 shows the quantitative analysis of serum FSH in mice after administrating compound TJ0113 in a chemotherapy induced ovarian premature failure model according to the embodiment of the present invention;

FIG. 69 shows the quantitative analysis of serum E2 in mice after administrating compound TJ0113 in a chemotherapy induced ovarian premature failure model according to the embodiment of the present invention;

FIG. 70 shows the quantitative analysis of BDNF in ovarian tissue of mice after administrating compound TJ0113 in a chemotherapy induced ovarian premature failure model according to the embodiment of the present invention;

FIG. 71 shows the quantitative analysis of body weight, ovarian weight, and ovarian organ coefficient in mice after administrating compound TJ0113 in an ovarian insufficiency natural aging model according to the embodiment of the present invention;

FIG. 72 shows the quantitative analysis of FSH in mice after administrating compound TJ0113 in an ovarian insufficiency natural aging model according to the embodiment of the present invention;

FIG. 73 shows the quantitative analysis of E2 in mice after administrating compound TJ0113 in an ovarian insufficiency natural aging model according to the embodiment of the present invention;

FIG. 74 shows the quantitative analysis of random urine uPCR in mice after administering compound TJ0113 in a lupus nephritis model according to an embodiment of the present invention;

FIG. 75 shows the quantitative analysis of serum creatinine and urea nitrogen in mice after administering compound TJ0113 in a lupus nephritis model according to an embodiment of the present invention;

FIG. 76 shows the quantitative analysis of serum ANA in mice after administering compound TJ0113 in a lupus nephritis model according to an embodiment of the present invention;

FIG. 77 shows the quantitative analysis of serum dsDNA in mice after administering compound TJ0113 in a lupus nephritis model according to an embodiment of the present invention;

FIG. 78 shows the quantitative analysis of TNF α and IL-6 in renal tissue of mice after administering compound TJ0113

in a lupus nephritis model according to an embodiment of the present invention;
FIG. 79 shows the quantitative analysis of LVIDd and LVIDs in mice after administering compound TJ01-013 in a myocarditis model according to an embodiment of the present invention;
FIG. 80 shows the quantitative analysis of EF and FS in mice after administering compound TJ01-013 in a myocarditis model according to an embodiment of the present invention;
FIG. 81 shows the quantitative analysis of CK-MB levels in the serum of mice after administering compound TJ01-013 in a myocarditis model according to an embodiment of the present invention;
FIG. 82 shows a quantitative analysis of the escape latency after administrating compound TJ01-013 in a stroke model according to an embodiment of the present invention;
FIG. 83 shows a quantitative analysis of number of times crossing platform after administrating compound TJ01-013 in a stroke model according to an embodiment of the present invention;
FIG. 84 shows the quantitative analysis of neuronal apoptosis rate after administrating compound TJ01-013 in a stroke model according to the embodiment of the present invention;
FIG. 85 shows the quantitative analysis of tinnitus indexes after administering compound TJ0113 in tinnitus according to the embodiment of the present invention;
FIG. 86 shows a quantitative analysis of number of times a mouse entering an elevated plus maze after administrating compound TJ01-013 to mice in a multiple sclerosis model according to an embodiment of the present invention;
FIG. 87 shows a quantitative analysis of the distance of mice walking on an elevated plus maze after administering compound TJ01-013 to mice in a multiple sclerosis model according to an embodiment of the present invention;
FIG. 88 shows a quantitative analysis of the time for mice to enter an elevated plus maze after administrating compound TJ01-013 to mice in a multiple sclerosis model according to an embodiment of the present invention;
FIG. 89 shows a quantitative analysis of number of times crossing over platform after administrating compound TJ01-013 to mice in a multiple sclerosis model according to an embodiment of the present invention.

## Detailed description of the embodiments

Acute kidney injury is a major renal disease characterized by rapid decline in renal function. The kidney injury after mild acute kidney injury can be completely repaired. However, severe or recurrent acute kidney injury often leads to abnormal repair, leading to renal fibrosis and ultimately leading to chronic kidney disease. In the prior art, the commonly used treatment methods for chronic kidney disease are induction therapy and renal replacement therapy. Due to the complexity of the causes of this disease and the coexistence of multiple causes, the effect of induction therapy is not significant; the renal replacement therapy includes: 1) hemodialysis, 2) peritoneal dialysis, and 3) kidney transplantation, all of which require patients to undergo surgical trauma surgery, which is time-consuming and labor-intensive. Mitochondrial accumulation is considered a hallmark of pathological conditions and a key factor driving disease progression. Inducing mitophagy is expected to become an effective treatment for various acute and chronic diseases. The inventor has developed the compound shown in formula (I) through extensive and in-depth research, and conducted extensive experiments to confirm that the compound shown in formula (I) or a pharmaceutically acceptable salt, stereoisomer, solvate, or prodrug thereof can be used to prevent and/or treat diseases associated with kidney injury, and to prepare drugs for preventing and/or treating diseases associated with kidney injury, and/or to prevent and/or treat diseases associated with mitophagy, and/or to prepare drugs for preventing and/or treating diseases associated with mitophagy.

## Compound

In some embodiments of the present invention, the compound shown in general formula (I) is as follows:

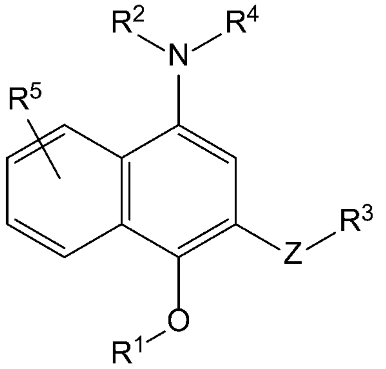

I

wherein Z is

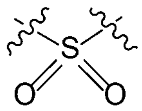, 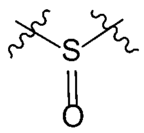, 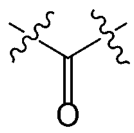, 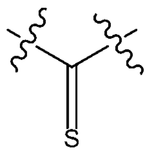

or a sulfur atom;

$R^1$ is hydrogen, $C_{1\sim6}$ alkyl,

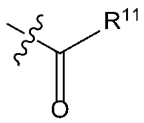, 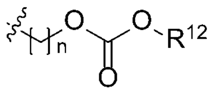,

wherein $R^{11}$ and $R^{12}$ are independently C1~6 alkyl or C1~6 cycloalkyl, respectively, and n is 1~4;

$R^2$ is hydrogen, $C_{1\sim6}$ alkyl, three- to six-membered cycloalkyl, three- to six-membered epoxyalkyl, phenyl, $C_{1\sim6}$ alkyl with at least one hydrogen substituted with $R^{2-1}$, phenyl with at least one hydrogen substituted with $R^{2-1}$, wherein $R^{2-1}$ is hydroxyl, halogen, amino group, or $C_{1\sim6}$ alkoxyl,

$R^3$ is

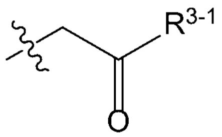, 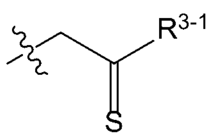 or 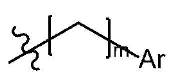;

wherein $R^{3-1}$ is hydrogen, hydroxyl, $C_{1\sim6}$ alkyl, $C_{1\sim6}$ alkoxy, $C_{3\sim6}$ cycloalkyl, three- to six-membered epoxyalkyl, amino group, $C_{1\sim6}$ amine group, $-CH_2C(O)R^{3-2}$, $-CH_2C(O)OR^{3-2}$, $-CH_2C(O)N$ ($R^{3-2}$ $R^{3-2a}$), and $R^{3-2}$ and $R^{3-2a}$ are independently hydrogen, C1~6 alkyl, or three- to six-membered cycloalkyl respectively,

m is from 1 to 6, and

Ar is phenyl, and biphenyl, 5- or 6-membered monocyclic heteroaryl, 8- to 10-membered fused bicyclic heteroaryl, phenyl with at least one hydrogen atom substituted with $R^{3-3}$, and biphenyl with at least one hydrogen atom substituted with $R^{3-3}$, 5- or 6-membered monocyclic heteroaryl with at least one hydrogen atom substituted with $R^{3-3}$, 8- to 10-membered fused bicyclic heteroaryl with at least one hydrogen atom substituted with $R^{3-3}$, the $R^{3-3}$ is hydrogen, halogen, $C_{1\sim6}$ alkyl, three-to six-membered cycloalkyl, hydroxy, $C_{1\sim6}$ alkoxy, three- to six-membered epoxyalkyl, $C_{1\sim6}$ haloalkyl, $C_{2\sim6}$ alkenyl, $C_{2\sim6}$ alkynyl, $-N(R^{3-3a}R^{3-3b})$ or phenyl,

$R^{3-3a}$ and $R^{3-3b}$ are independently hydrogen, $C_{1\sim6}$ alkyl, or three- to six-membered cycloalkyl, respectively;

$R^4$ is

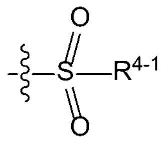, 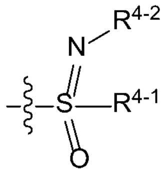, 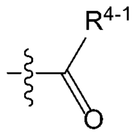 or 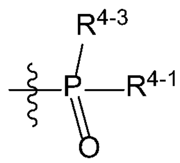.

wherein $R^{4-1}$ is phenyl, biphenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, $R^{4-11}$ is hydrogen, halogen, nitro, nitrile, hydroxyl, $C_{1\sim6}$ alkyl, $C_{13\sim6}$ cycloalkyl, $C_{1\sim6}$ alkoxy, -N($R^{4-1a}R^{4-1b}$), phenyl, $C_{1\sim6}$ haloalkyl, $C_{1\sim6}$ haloalkoxy, -C(O)O$R^{4-12}$, -C(O)$R^{4-12}$, -C(O)N($R^{4-1a}R^{4-1b}$), -S(O)2$R^{4-12}$, -S(O)$R^{4-12}$, - OC(O)$R^{4-12}$, -OC(O)O$R^{4-12}$ or

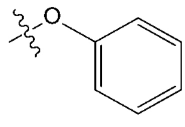,

$R^{4-12}$, R4$^{-1a}$ and $R^{4-1b}$ are independently hydrogen, respectively, $C_{1\sim6}$ alkyl, $C_{3\sim6}$ cycloalkyl, $C_{2\sim6}$ alkenyl, C2~6 alkynyl, C1~6 alkyl substituted with at least one hydrogen by a halogen, $C_{2\sim6}$ alkynyl with at least one hydrogen substituted with a halogen, $C_{3\sim6}$ cycloalkyl with at least one hydrogen substituted by halogen, C2~6 alkynyl with at least one hydrogen substituted with a halogen, and $R^{4-1a}$ and $R^{4-1b}$ are bondable to each other to form a ring,

$R^{4-2}$ is $C_{1\sim6}$ alkyl, $C_{3\sim6}$ cycloalkyl, $C_{1\sim6}$ alkyl with at least one hydrogen substituted with a hydroxyl group, $C_{3\sim6}$ epoxyalkyl, or $R^{4-2}$ is bonded to R2 to form a 4- to 8-membered ring when $R^2$ is $C_{1\sim6}$ alkyl and $R^{4-2}$ is $C_{1\sim6}$ alkyl.

$R^{4-3}$ is C1~6 alkyl or C1~6 alkoxy;

the number of $R^5$ is 0~5, and at each occurrence, $R^5$ is independently hydrogen, halogen, nitro, nitrile, -N+($R^{5-1}$)$_3$, $C_{1\sim6}$ haloalkyl, -C(O)O$R^{5-1}$, -C(O)$_R$$^{5-1}$, -C(O)N($R^{5-1}R^{5-1a}$), -S(O)$_2$$R^{5-1}$, -S(O)$R^{5-1}$, -S(O)$_2$N($R^{5-1}R^{5-1a}$), -S(O)N($R^{5-1}R^{5-1a}$), -N=C($R^{5-1}$ $R^{5-1a}$), hydroxyl, $C_{1\sim6}$ alkyl, phenyl, phenyl with at least one hydrogen substituted with $R^{5-1}$, $C_{1\sim6}$ alkoxy, -N($R^{5-1}R^{5-1a}$), -N($R^{5-1}$)C(O)$R^{5-1a}$, -N($R^{5-1}$)C(O)O$R^{5-1a}$, -OC(O)$R^{5-1}$, -OC(O)O$R^{5-1}$, -OC(O) N($R^{5-1}R^{5-1a}$) or -S$R^{5-1}$,

$R^{5-1}$, $R^{5-1a}$ and $R^{5-1b}$ are independently hydrogen, $C_{1\sim6}$ alkyl, $C_{2\sim6}$ alkenyl, C2~6 alkynyl, $C_{1\sim6}$ alky with at least one hydrogen substituted with a halogen, $C_{2\sim6}$ alkenyl with at least one hydrogen substituted with a halogen or $C_{2\sim6}$ alkynyl with at least one hydrogen substituted with a halogen, respectively.

In some preferred embodiments, the Z is

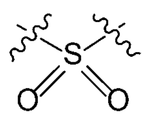, 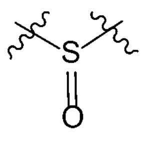, 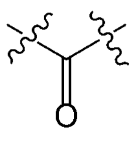,

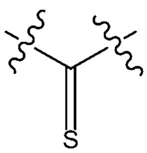

or a sulfur atom;

$R^1$ is hydrogen, $C_{1\sim4}$ alkyl,

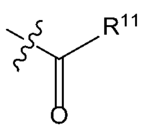 or 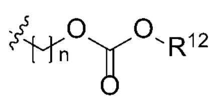,

wherein $R^{11}$ and $R^{12}$ are independently $C_{1\sim4}$ alkyl or $C_{1\sim4}$ cycloalkyl, respectively, and n is 1 or 2;

$R^2$ is hydrogen, $C_{1\sim4}$ alkyl, three- to six-membered cycloalkyl, three- to six-membered epoxyalkyl, phenyl, $C_{1\sim4}$ alkyl with at least one hydrogen substituted with $R^{2-1}$, and phenyl with at least one hydrogen substituted with $R^{2-1}$, wherein $R^{2-1}$ is hydroxy, halogen, amino group, or $C_{1\sim4}$ alkoxy;

$R^3$ is

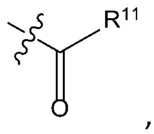 , 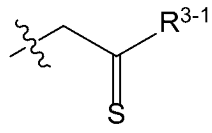 or 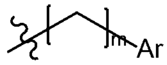 ;

wherein $R^{3-1}$ is hydrogen, hydroxyl, $C_{1\sim4}$ alkyl, $C_{1\sim4}$ alkoxy, -N($R^{3-2}R^{3-2a}$),

$R^{3-2}$ and $R^{3-2a}$ are independently hydrogen or $C_{1\sim4}$ alkyl, respectively, $R^{3-3a}$ and $R^{3-3b}$ are independently hydrogen or $C_{1\sim4}$ alkyl, respectively,

m is 1~4.

Ar is phenyl, 5- or 6-membered monocyclic heteroaryl, 5- or 6-membered monocyclic heteroaryl with at least one hydrogen atom substituted with $R^{3-3}$, the $R^{3-3}$ is hydrogen, halogen, $C_{1\sim4}$ alkyl, hydroxyl, $C_{1\sim4}$ alkoxy, $C_{1\sim4}$ haloalkyl, or -N($R^{3-3a}R^{3-3b}$);

$R^4$ is

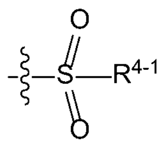 , 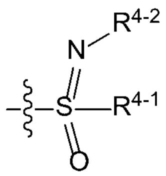 , 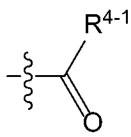 or 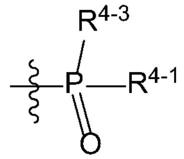 ,

wherein $R^{4-1}$ is phenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, and the $R^{4-11}$ is hydrogen, halogen, nitro, $C_{1\sim4}$ alkyl, $C_{3\sim6}$ cycloalkyl, $C_{1\sim4}$ alkoxy, -N($R^{4-1a}R^{4-1b}$), phenyl, $C_{1\sim4}$ haloalkyl, $C_{1\sim4}$ haloalkoxy or

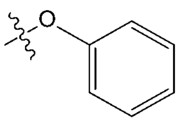 ,

$R^{4-1a}$ and $R^{4-1b}$ are independently hydrogen, $C_{1\sim4}$ alkyl, or $C_{3\sim6}$ cycloalkyl, respectively, and $R^{4-1a}$ and $R^{4-1b}$ are bondable to each other to form a ring,

$R^{4-2}$ is $C_{1\sim4}$ alkyl, $C_{3\sim5}$ cycloalkyl, $C_{1\sim4}$ alkyl with at least one hydrogen substituted with hydroxyl, $C_{3\sim5}$ epoxyalkyl, or $R^{4-2}$ is bonded to $R^2$ to form a 4- to 8-membered ring when $R^2$ is $C_{1\sim4}$ alkyl and $R^{4-2}$ is $C_{1\sim4}$ alkyl,

$R^{4-3}$ is $C_{1\sim4}$ alkyl or $C_{1\sim4}$ alkoxy;

at each occurrence, $R^5$ is independently hydrogen halogen, nitro, nitrile, -N+($R^{5-1}$)$_3$, $C_{1\sim4}$ haloalkyl, C(O)O$R^{5-1}$, -C(O)$R^{5-1}$, -C(O)N($R^{5-1}R^{5-1a}$), -S(O)$_2R^{5-1}$, -S(O)$R^{5-1}$, -N=C($R^{5-1}$ $R^{5-1a}$), hydroxyl, $C_{1\sim4}$ alkyl, phenyl, phenyl with at least one hydrogen substituted with $R^{5-1}$, $C_{1\sim4}$ alkoxy, -N($R^{5-1}R^{5-1a}$), -N( $R^{5-1}$)C(O)$R^{5-1a}$, or -OC(O)$R^{5-1}$, respectively, wherein $R^{5-1}$, $R^{5-1a}$, and $R^{5-1b}$ are independently hydrogen, $C_{1\sim4}$ alkyl, $C_{1\sim4}$ alkyl with at least one hydrogen substituted with a halogen, respectively.

In some preferred embodiments, the Z is

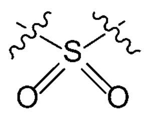 , 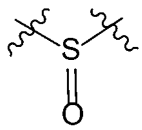 , 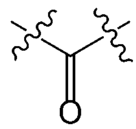 ,

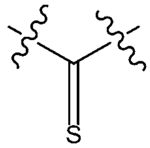

or a sulfur atom;

In some preferred embodiments, $R^1$ is hydrogen,

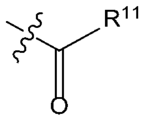

or

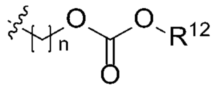

,

wherein $R^{11}$ and $R^{12}$ are independently methyl, ethyl, n-propyl or isopropyl, respectively, and n is 1.

In some preferred embodiments, $R^2$ is hydrogen, methyl, ethyl, n-propyl, isopropyl, ethyl with one hydrogen substituted with hydroxyl, n-propyl with one hydrogen substituted with hydroxyl, phenyl,

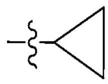, 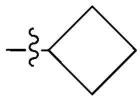, 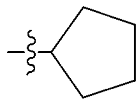,

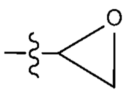, 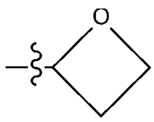, 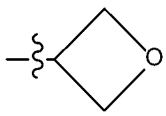, 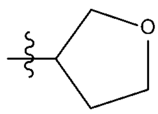 or 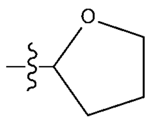.

In some preferred embodiments, $R^3$ is

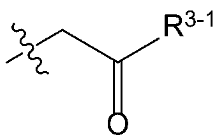, 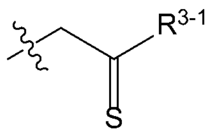

or

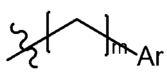;

wherein $R^{3-1}$ is hydrogen, hydroxyl, methyl, ethyl, n-propyl, isopropyl, methoxy, ethoxy, n-propoxy, isopropoxy, n-butoxy, tert-butoxy, sec-butoxy or isobutoxy,

m is 1,

Ar is phenyl, 5- or 6-membered nitrogen-containing monocyclic heteroaryl.

In some preferred embodiments, $R^4$ is

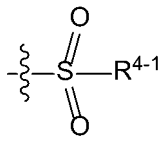, 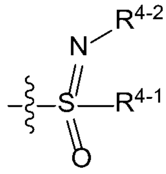, 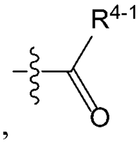

or

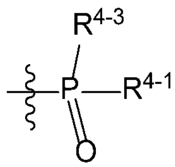,

wherein R4-1 is phenyl, phenyl with at least one hydrogen substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and the $R^{4-11}$ is halogen, nitro, methyl, ethyl, n-propyl, isopropyl,

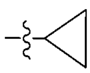, 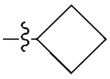, 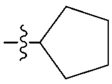, 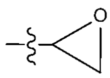, 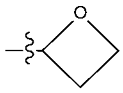, 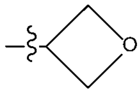,

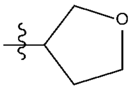, 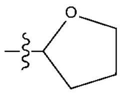, 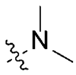, 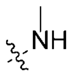, 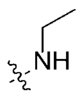, 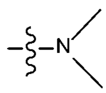, 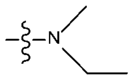, 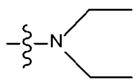,

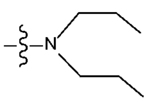, 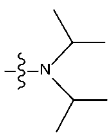, 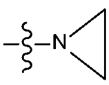, 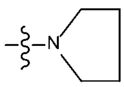, 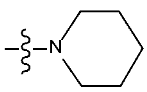,

fluoromethyl, fluorinated ethyl, fluorinated n-propyl, fluorinated isopropyl, chloromethyl, chlorinated ethyl, chlorinated n-propyl, chlorinated isopropyl, brominated methyl, bromoethyl, bromo n-propyl, bromo isopropyl, iodomethyl, iodoethyl, iodo n-propyl, iodo isopropyl, fluoromethoxy, fluoroethoxy, fluoroorthopropoxy, fluoroisopropoxy, chloromethoxy, chloroethoxy, chloro n-propoxy, chloroisopropoxy, bromomethoxy, bromoethoxy, bromo n-propoxy, bromoisopropoxy, iodomethoxy, iodoethoxy, iodo n-propoxy, iodoisopropoxy, or

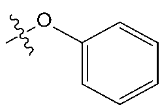,

$R^{4-2}$ is methyl, ethyl, n-propyl, isopropyl, n-butyl, isobutyl, tert-butyl, ethyl with one hydrogen substituted with hydroxyl, n-propyl with one hydrogen substituted with hydroxyl, n-butyl with one hydrogen substituted with hydroxyl,

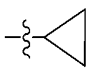, 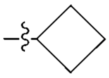,

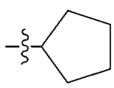, 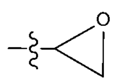, 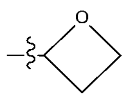, 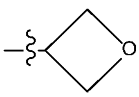, 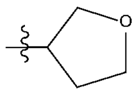, 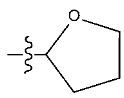

or phenyl, or, $R^{4-2}$ is bonded to $R^2$ to form a 4 to 8-membered ring when $R^2$ is methyl, ethyl or n-propyl and $R^{4-2}$ is methyl, ethyl,

$R^{4-3}$ is methyl, ethyl, n-propyl, isopropyl, methoxy, ethoxy, n-propoxy or isopropoxy.

In some preferred embodiments, at each occurrence, $R^5$ is independently hydrogen, halogen, nitro, nitrile, $-N+(R^{5-1})^3$, fluoro-methyl, fluoroethyl, fluoro-n-propyl, fluoro-isopropyl, chloromethyl, chloroethyl, chloro-n-propyl, chloro-isopropyl, bromomethyl, bromoethyl, bromo-n-propyl, bromo-isopropyl, $-C(O)OR^{5-1}$, $-C(O)R^{5-1}$, $-C(O)N(R^{5-1}R^{5-1a})$, $-S(O)_2R^{5-1}$, $-S(O)R^{5-1}$, $-N=C(R^{5-1}\ R^{5-1a})$, hydroxyl, methyl, ethyl, n-propyl, isopropyl, phenyl, phenyl with at least one hydrogen substituted with $R^{5-1}$, methoxy, ethoxy, n-propyloxy, isopropyloxy, $-N(R^{5-1}R^{5-1a})$, $-N(R^{5-1})C(O)R^{5-1a}$, $-OC(O)R^{5-1}$, respectively, wherein $R^{5-1}$, $R^{5-1a}$, and $R^{5-1b}$ are independently hydrogen, methyl, ethyl, n-propyl, isopropyl, fluoro-methyl, fluoroethyl, fluoro-n-propyl, fluoro-isopropyl, chloro-methyl, chloroethyl, chloro n-propyl, chloro isopropyl, bromo-methyl, bromoethyl, bromo n-propyl, or bromo isopropyl, respectively.

In some preferred embodiments, $R^1$ is hydrogen,

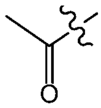 , 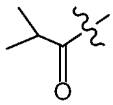

or

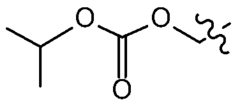 .

In some preferred embodiments, $R^2$ is hydrogen, methyl,

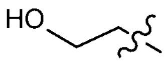 , 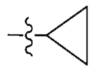 ,

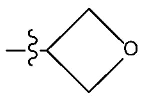

or phenyl.

In some preferred embodiments, $R^3$ is

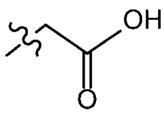 , 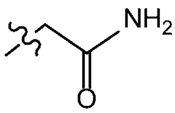 ,

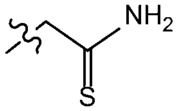 , 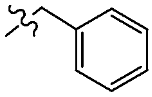 , 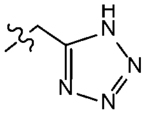 or 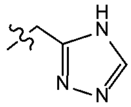 .

In some preferred embodiments, $R^4$ is

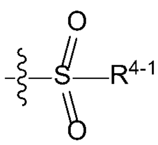 , 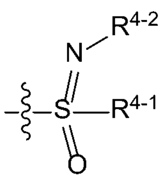 ,

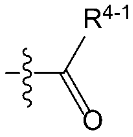 or 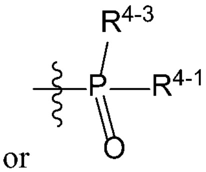 ,

wherein $R^{4-1}$ is phenyl, phenyl with at least one hydrogen substituted with $R^{4-11}$, five-membered monocyclic heteroaryl, five-membered monocyclic heteroaryl with at least one hydrogen substituted with $R^{4-11}$, nine-membered fused bicyclic heteroaryl, or naphthyl, wherein $R^{4-11}$ is bromine, fluorine, chlorine, methyl, nitro, phenyl, trifluoromethyl,

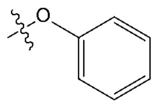 ,

methoxy, cyclopropyl, trifluoromethoxy, nitro or

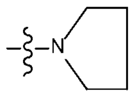 ,

$R^{4-2}$ is methyl, ethyl,

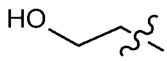 , 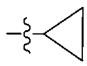 , 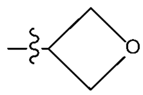

or phenyl, or, $R^{4-2}$ is bonded to $R^2$ to form a 4 to 6-membered ring when $R^2$ is methyl, ethyl, or n-propyl and $R^{4-2}$ is methyl, ethyl,
$R^{4-3}$ is methoxy, ethoxy, n-propoxy or isopropoxy.

In some preferred embodiments, at each occurrence, $R^5$ is independently halogen, nitro, nitrile, carboxyl, -NHC(O)CH, methoxy, or hydroxy, respectively.
In some preferred embodiments, the $C_{1\sim6}$ alkyl is $C_{1\sim4}$ alkyl, the $C_{1\sim4}$ alkyl is preferably methyl, ethyl, n-propyl, isopropyl, n-butyl, isobutyl or tert-butyl, e.g., methyl or ethyl.
In some preferred embodiments, the three to six-membered cycloalkyl is preferably $C_{3\sim5}$ cycloalkyl, said $C_{3\sim5}$ cycloalky is preferably

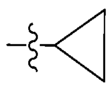 , 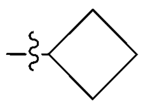

or

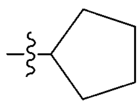 ,

for example

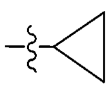 .

In some preferred embodiments, said three to six-membered cycloalkyl epoxide is preferably

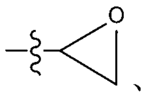, 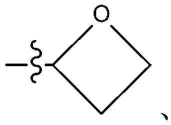, 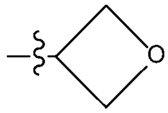,

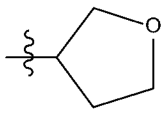 or 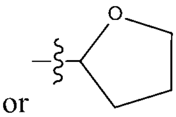,

for example:

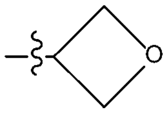.

In some preferred embodiments, the phenyl substituted with $C_{1\sim6}$ alkyl is preferably a phenyl substituted with $C_{1\sim4}$ alkyl, more preferably a phenyl substituted with any of methyl, ethyl, n-propyl, isopropyl, n-butyl, isobutyl, and tert-butyl.

In some preferred embodiments, the $C_{1\sim6}$ alkoxy is preferably $C_{1\sim4}$ alkoxy, more preferably methoxy, ethoxy, n-propoxy, isopropoxy, n-butoxy, tert-butoxy, sec-butoxy or isobutoxy.

In some preferred embodiments, the -N($R^{3-2}R^{3-2a}$), -N($R^{3-3a}R^{3-3b}$), -N($R^{4-1a}R^{4-1b}$), -N($R^{5-1}R^{5-1a}$) is preferably

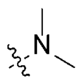, 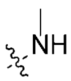, 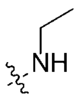, 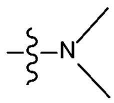, 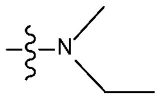,

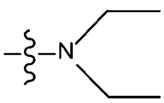, 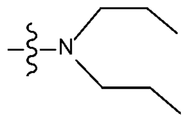, 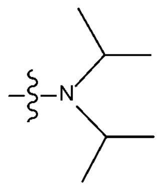, 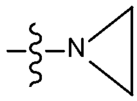, 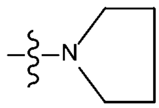

or

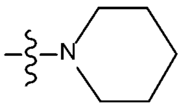.

In some preferred embodiments, the 5 or 6-membered monocyclic heteroaryl preferably is

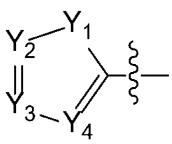, 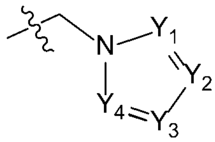, 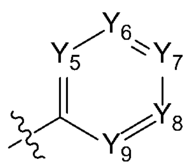,

wherein, $Y_1$, $Y_2$, $Y_3$, $Y_4$, $Y_5$, $Y_6$, $Y_7$, $Y_8$, $Y_9$ are independently selected from C, N, O, or S, respectively, and $Y_1$, $Y_2$, $Y_3$, $Y_4$ are not all C, $Y_5$, $Y_6$, $Y_7$, $Y_8$, $Y_9$ are not all C; the 5- or 6-membered monocyclic heteroaryl is more preferably pyrrolyl, furanyl, thienyl, pyrazolyl, imidazolyl, oxazolyl, isoxazolyl, thiazolyl, pyridyl, pyranyl, thiopyranyl, pyridazinyl, pyrimidinyl, pyrazinyl, piperazinyl, triazolyl, tetrazolyl; the 5- or 6-membered monocyclic heteroaryl is more preferably

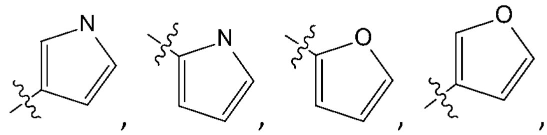

, , , ,

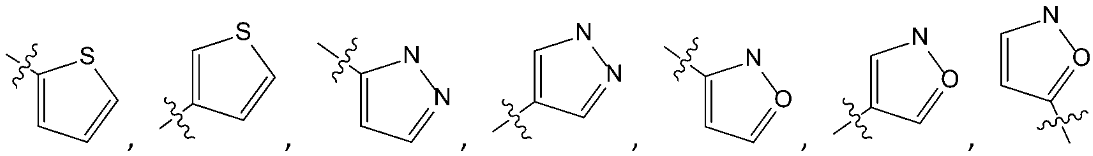

, , , , , , ,

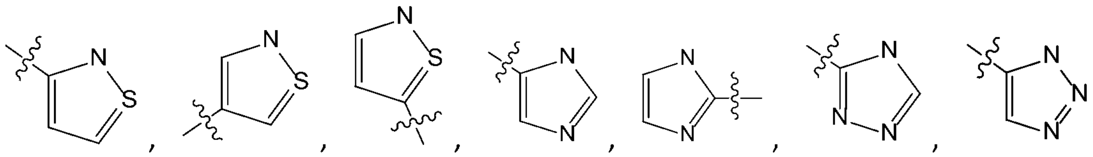

, , , , , , ,

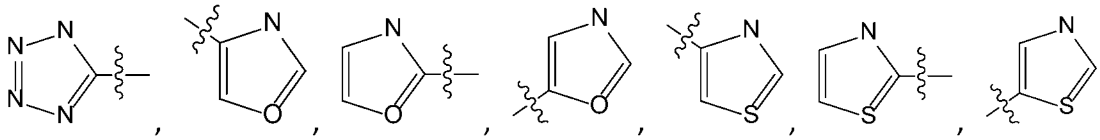

, , , , , , ,

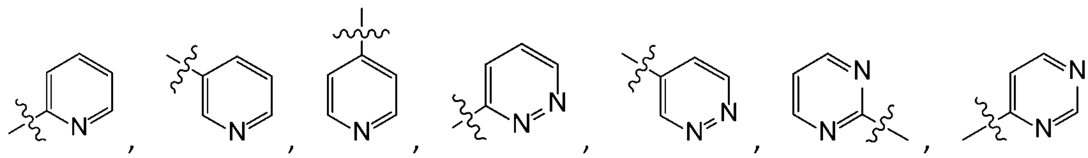

, , , , , , ,

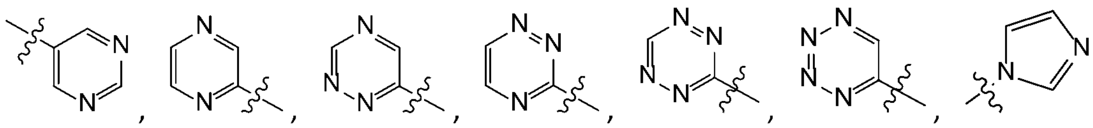

, , , , , , ,

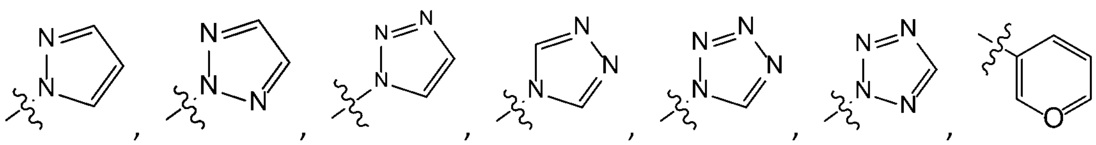

, , , , , , ,

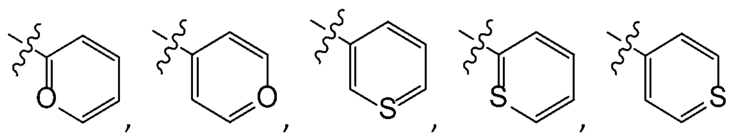

, , , , ;

more preferably, the 5- or 6-membered monocyclic heteroaryl is a 5 or 6-membered nitrogen-containing monocyclic heteroaryl, for example:

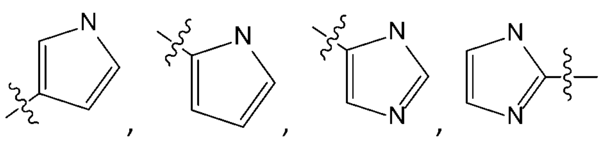

, , , ,

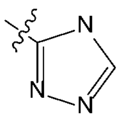, 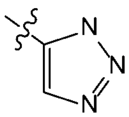, 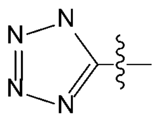, 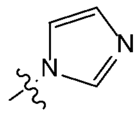, 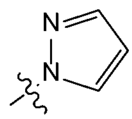, 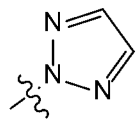, 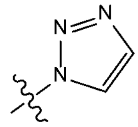,

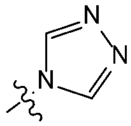, 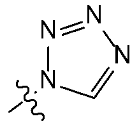, 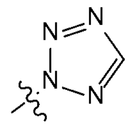 or 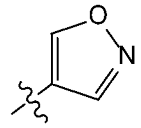;

most preferably, the 5 or 6-membered monocyclic heteroaryl is a 5-membered nitrogen-containing monocyclic heteroaryl, for example:

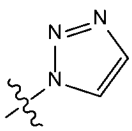, 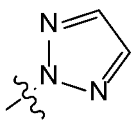 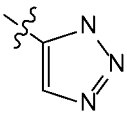, 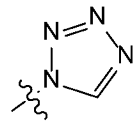, 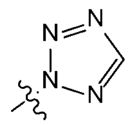

or

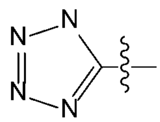.

In some preferred embodiments, the 8 to 10-membered fused bicyclic heteroaryl is

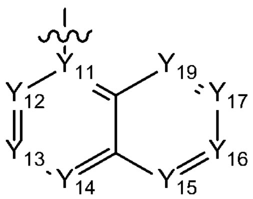, 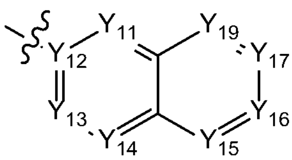, 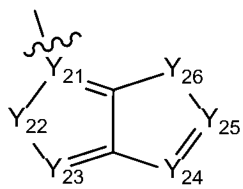,

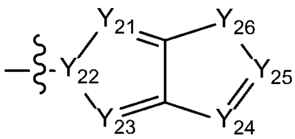, 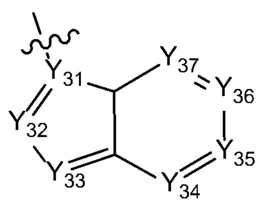, 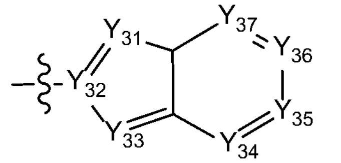,

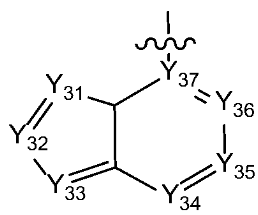 or 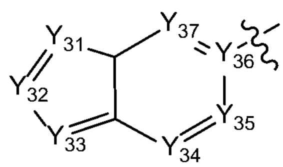;

wherein $Y_{11}$, $Y_{12}$, $Y_{13}$, $Y_{14}$, $Y_{15}$, $Y_{16}$, $Y_{17}$, $Y_{18}$, and $Y_{19}$ are independently selected from C, N, O, or S, respectively, and $Y_{11}$, $Y_{12}$, $Y_{13}$, $Y_{14}$, $Y_{15}$, $Y_{16}$, $Y_{17}$, $Y_{18}$, and $Y_{19}$ are not all C; $Y_{21}$, $Y_{22}$, $Y_{23}$, $Y_{24}$, $Y_{25}$ and $Y_{26}$ are independently selected from C, N, O or S, respectively, and $Y_{21}$, $Y_{22}$, $Y_{23}$, $Y_{24}$, $Y_{25}$ and $Y_{26}$ are not all C; $Y_{31}$, $Y_{32}$, $Y_{33}$, $Y_{34}$, $Y_{35}$, $Y_{36}$ and $Y_{37}$ are independently selected from C, N, O or S, respectively, and $Y_{31}$, $Y_{32}$, $Y_{33}$, $Y_{34}$, $Y_{35}$, $Y_{36}$ and $Y_{37}$ are not all C; the 8 to 10-membered thickened bicyclic heteroaryl is more preferably indolyl, benzindolyl, benzothienyl, carbazolyl, quinolinyl, pteridinyl,

purinyl; and said 8 to 10-membered thickened bicyclic heteroaryl is most preferably

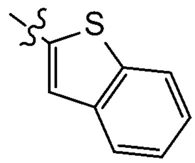

,

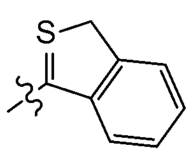

,

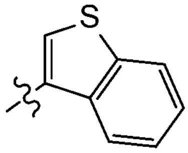

.

In some preferred embodiments, the halogen is preferably fluorine, chlorine, bromine or iodine.

In some preferred embodiments, the $C_{1\sim6}$ haloalkyl is preferably $C_{1\sim3}$ haloalkyl; more preferably fluoromethyl, fluoroethyl, fluoro n-propyl, fluoroisopropyl, chloromethyl, chloroethyl, chloro n-propyl, chloro isopropyl, bromomethyl, bromoethyl, bromo n-propyl, bromo isopropyl, iodomethyl, iodoethyl, iodo n-propyl, iodo isopropyl; most preferably trifluoromethyl.

In some preferred embodiments, the $C_{1\sim6}$ haloalkoxy is preferably $C_{1\sim3}$ haloalkoxy; more preferably fluoromethoxy, fluoroethoxy, fluoro n-propoxy, fluoroisopropoxy, chloromethoxy, chloroethoxy, chloroethoxy, chloro n-propoxy, chloroisopropoxy, bromomethoxy, bromoethoxy, bromo npropoxy, bromoisopropoxy, iodooxymethyl, iodoethoxy, iodo n-propoxy, iodo isopropoxy; most preferably trifluoromethoxy.

In some preferred embodiments, the $C_{2\sim6}$ alkenyl is preferably $C_{2\sim4}$ alkenyl, more preferably -CH=CH2, -CH=CH-CH3, -CH2-CH=CH2, -CH=CH-CH2-CH3, -CH=CH-CH=CH2.

In some preferred embodiments, the $C_{2\sim6}$ alkynyl is preferably a $C_{2\sim6}$ alkynyl, more preferably -C≡CH, -CH2-C=CH, -CH2-CH2-C=CH, -CH2-C=C-CH3.

Based on the beneficial effects of improving compound metabolic stability and reducing toxicity, in some preferred embodiments, Z is not a sulfur atom.

Furthermore, based on the beneficial effects of improving the efficacy of compound therapy for diseases associated with kidney, especially chronic kidney disease, in some preferred embodiments, Z is

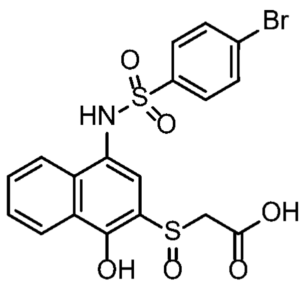

.

In some preferred embodiments, Z is not

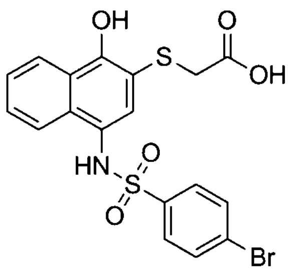

.

In the preferred embodiment of the present invention, the compound of formula I is compound I-1 (also known as TJ01-013 or TJ0113), with a structure as shown in the following formula;

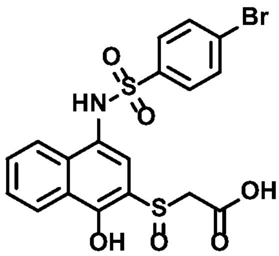

TJ01-013

.

In the present invention, compounds of the general formula I can be prepared by the method described in Chinese patent application 202111108417.6, which is incorporated herein by reference in its entirety.

**Pharmaceutical compositions**

The compound of general formula I of the present invention can be used to prepare a pharmaceutical composition for preventing and/or treating kidney injury, the pharmaceutical composition comprising: (i) an effective amount of the compound of general formula I, or a pharmaceutically acceptable salt thereof; and (ii) a pharmaceutically acceptable salt or excipient.

"Pharmaceutical composition" means a mixture of a compound described herein with an "excipient" such as a carrier, stabilizer, diluent, dispersant, suspending agent and/or thickener. The pharmaceutical composition facilitate the administration of a compound to an organism. Various techniques exist in the art for administering compounds, including, but not limited to: rectal, oral, intravenous, aerosol, parenteral, ocular, pulmonary, and topical administration.

The term "subject" refers to an animal, including, but not limited to, a primate (e.g., human), monkey, cow, pig, sheep, goat, horse, dog, cat, rabbit, rat, or mouse. The terms "subject" and "patient" are used interchangeably herein, e.g., with respect to a mammalian subject (e.g., human).

In some embodiments, the effective amount refers to a therapeutically effective amount.

"Effective amount" or "therapeutically effective amount" refers to a sufficient amount of a chemical entity (e.g., Compound I-1, or pharmaceutically acceptable salts and/or hydrates and/or co-crystals thereof;), when administered, the amount will provide some relief of one or more of the symptoms of the disease or condition being treated. Results include reduction and/or remission of signs, symptoms, or etiology of the disease or any other desired change in the biological system. The appropriate "effective" amount in any individual case is determined using any suitable technique such as dose-escalation studies.

In some embodiments, the present invention provides compositions comprising a compound of general formula I and at least one pharmaceutically acceptable carrier, wherein an amount of the compound of general formula I in the pharmaceutical composition is 0.005-5 grams.

In some embodiments, the present invention provides compositions comprising a compound of general formula I and at least one pharmaceutically acceptable carrier, wherein the amount of the compound of general formula I in the pharmaceutical composition is 0.005-3 grams.

In some embodiments, the present invention provides compositions comprising a compound of general formula I and at least one pharmaceutically acceptable carrier, wherein the amount of the compound of general formula I in the pharmaceutical composition is 0.005-2 grams.

In some embodiments, the present invention provides compositions comprising a compound of general formula I and at least one pharmaceutically acceptable carrier, wherein an amount of a compound of general formula I in the pharmaceutical composition is 0.005-1 gram.

In some embodiments, the present invention provides compositions comprising a compound of general formula I and at least one pharmaceutically acceptable carrier, wherein the amount of the compound of general formula I in the pharmaceutical composition is 0.005-0.5 grams.

In some embodiments, the present invention provides compositions comprising a compound of general formula I and at least one pharmaceutically acceptable carrier, wherein the amount of the compound of general formula I in the pharmaceutical composition is 0.005-0.2 grams.

In some embodiments, the present invention provides compositions comprising a compound of general formula I and at least one pharmaceutically acceptable carrier agent, wherein the amount of the compound of general formula I in the pharmaceutical composition is 0.005-0.15 grams.

In some embodiments, when an effective amount of a compound of formula I of the present invention or a pharmaceutically acceptable salt thereof is administered to a subject of application, the effective amount is 0.001-500 nmol/L, preferably 0.01-200 nmol/L.

Since the compounds of the general formula I of the present invention have excellent therapeutic effects in treating diseases associated with kidney injury, the pharmaceutical compositions having the compounds of the general formula I as the main active ingredient are usable for treating diseases associated with kidney injury.

The pharmaceutical compositions of the present invention comprise a compound of general formula I or a pharmaceutically acceptable salt thereof within a safe and effective amount and a pharmaceutically acceptable excipient or carrier. The term "safe and effective amount" means that the amount of the compound is sufficient to significantly improve the condition without causing serious side effects. Typically, the pharmaceutical composition contains 0.05-2 g of the compound/agent of general formula I of the present invention, preferably, 5-200 mg of the compound /agent of general formula I of the present invention.

"Pharmaceutically acceptable salt" may refer to a pharmaceutically acceptable addition salt prepared from a pharmaceutically acceptable non-toxic acid, including inorganic and organic acids. In some cases, the pharmaceutically acceptable salt is obtained by reacting the compounds described herein with an acid. The term "pharmaceutically acceptable salt" may also refer to a pharmaceutically acceptable addition salt prepared by reacting a compound having an acidic moiety with a base to form a salt or by other methods previously identified. A pharmaceutically acceptable salt is not particularly limited insofar as it can be used in a drug. Examples of salts formed by the compounds described herein with bases include the following: salts formed by reaction with inorganic bases such as sodium, potassium, magnesium, calcium, and aluminum; salts formed by reaction with organic bases such as methylamine, ethylamine, and ethanolamine; or salts formed by reaction with dicyclohexylamine, N-methyl-D-glucosamine, or tris(hydroxymethyl)methylamine; salts formed by reaction with basic amino acids such as lysine and ornithine; and ammonium salts. The salts may be acid addition salts, which are specifically exemplified as addition salts with the following: inorganic acids such as hydrochloric acid, hydrobromic acid, hydriodic acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; and acidic amino acids such as aspartic acid and glutamic acid.

"Excipient" or "pharmaceutically acceptable excipient" means a pharmaceutically acceptable material, composition or medium, such as a liquid or solid filler, diluent, carrier, solvent, or encapsulating material. In one embodiment, each component is compatible with the other components of the pharmaceutical preparation and suitable for contact with human and animal tissues or organs without excessive toxicity, irritation, allergic response, immunogenicity, or other problems or complications, and it is "pharmaceutically acceptable" in the sense of being commensurate with a reasonable benefit/risk ratio.

Unless otherwise indicated, the pharmaceutical compositions according to the present invention are prepared in a manner known per se, e.g. by means of conventional mixing, granulation, coating, dissolution or lyophilization processes. When preparing the compositions for oral dosage forms, any commonly used pharmaceutical medium may be employed, such as water, glycol, oil, ethanol; a carrier agent, such as starch, sugar, or microcrystalline cellulose; a diluent; a granulating agent; a lubricant; a binder; a disintegrating agent; and the like. Due to the ease of administration, tablets and capsules are the most favorable oral unit dosage forms, and solid pharmacy carriers are obviously employed.

In one embodiment, the present invention provides a pharmaceutical composition comprising a compound of general formula I and at least one pharmaceutically acceptable carrier, wherein the composition is a tablet or capsule.

In one embodiment, the present invention provides a pharmaceutical composition comprising a compound of general formula I and at least one pharmaceutically acceptable carrier, wherein the composition is a tablet.

In one embodiment, the present invention provides pharmaceutical compositions comprising a compound of general formula I and at least one pharmaceutically acceptable carrier, wherein the composition is a capsule.

In the context of treating a disease or disorder, the terms "treat, treating and treatment" are intended to include the alleviation or elimination of a disorder, disease or condition, wherein the term "disorder" as used herein shall always be understood to mean "disorder, disease or condition" or one or more of the symptoms associated with the disorder; or slowing the progression, spread or worsening of the disorder or condition or one or more of its symptoms.

**Cosmetic or medical instrument compositions**

In the present invention, a compound of general formula I (preferably compound TJ01-013) or a pharmaceutical composition may also be formed into a cosmetic composition with an excipient acceptable in cosmetics.

In the present invention, the term "cosmetic composition" means a chemical industrial product or fine chemical product which is dispersed on any part of the surface of the human body, such as the skin, hair, nails, lips and teeth, etc., by applying, spraying, or other similar methods for the purpose of cleansing, caring for, beautifying, modifying, and changing the appearance of the human body or correcting the odor of the human body to keep it in a good condition. The scope of the cosmetics includes toners, lotions, emulsions, makeup creams, essences, masks, cream gels, sprays, soaps, cleansers, body washes, shampoos, conditioners, foundations, creams, body lotions or massage creams, and the like.

In the present invention, the term medical instrument may be a Class III, Class II or Class I instrument, an

instrument, an apparatus, an appliance, an in vitro diagnostic reagent and calibrators, a material, and other similar or related items used directly or indirectly in the human body. The present invention includes, but is not limited to, masks, coatings, and the like.

The term "excipients acceptable in cosmetics or medical instruments" may be selected from solvents, solubilizers, preservatives, antioxidants, pH modifiers, permeation enhancers, liposomes, humectants, thickeners, chelating agents, skin-feel modifiers, surfactants, emulsifiers, propellants/propellants, flavors, pigments, and other potency additives.

**Indications and Uses**

In the present invention, a compound of general formula I (preferably compound TJ01-013) or a pharmaceutical composition is usable for (i) preparing a drug for the treatment of inflammatory bowel diseases; (ii) preventing and/or treating inflammatory bowel diseases. The term "Inflammatory bowel disease (IBD)" refers to an idiopathic inflammatory disease of the intestinal tract involving the ileum, rectum, and colon. Typical symptoms include diarrhea, weight loss and abdominal pain. Preferably, the inflammatory bowel diseases may include Crohn's Disease (CD) and Ulcerative Colitis (UC). Inflammatory bowel diseases have been found to be associated with mitochondrial dysfunction in the present invention, and the compound TJ01-013, which is a mitochondrial dysfunction therapeutic agent, has been found to be contributory in ameliorating the symptoms of inflammatory bowel diseases. In some embodiments, administration of compound TJ01-013 to the subjects can reduce the levels of expression of inflammatory factors in the diseased tissue, which include at least one of Cxcl1, G-CSF, IL6, IL-1$\beta$, S100A8, and TNF-$\alpha$. In some embodiments, administration of compound TJ01-013 to a subject can alleviate the aggregation of inflammatory cells (e.g., neutrophils, lymphocytes, monocytes, and eosinophils, among others) in the diseased tissue at the site where there is inflammation occurring. In some embodiments, administration of compound TJ01-013 to a subject prevents or slows weight loss in the subject.

In the present invention, a compound of general formula I (preferably compound TJ01-013) or a pharmaceutical composition is usable for (i) preparing a drug for the treatment of lung injury; and (ii) preventing and/or treating lung injury. The term "lung injury" refers to the destruction of the parenchymal structure of the lungs caused by the action of various factors inside and outside the lungs on the organism. Preferably, the lung injury here is an acute lung injury (ALI), an injury to alveolar epithelial cells and capillary endothelial cells caused by direct and indirect injury-causing factors leads to diffuse interstitial and alveolar oedema, resulting in an acute hypoxic respiratory insufficiency, and characterised by migratory, uncontrolled inflammatory response of trans-epithelial neutrophils, injury to the lung epithelium and endothelial cells and damage to the related cellular barrier. Preferably, the lung injury is a chronic lung injury, such as chronic obstructive pulmonary disease, which is a disease induced by common chronic lung injury that has a chronic bronchitis and/or emphysem characterized by airflow obstruction, and can further progress to a pulmonary heart disease and respiratory failure. In the present invention, it is found that mitochondrial dysfunction is associated with the progression of lung injury, and mitochondrial dysfunction may lead to lung cell death and even lung tissue damage, and compound TJ01-013, which is a mitochondrial dysfunction therapeutic agent, is useful in ameliorating symptoms associated with lung injury. In some embodiments, administration of compound TJ01-013 to a subject may result in a decrease in the level of inflammatory factors (including IL-6, CXCL1, CXCL2) and the level of inflammatory factor transcripts in diseased tissue (bronchoalveolar tissue). In some embodiments, administration of compound TJ01-013 to a subject reduces the number of neutrophils in the diseased tissue. In some embodiments, administration of compound TJ01-013 to a subject slows an increase in the level of inflammatory factors (including IL-6, CXCL1, CXCL2) and the level of inflammatory factor transcripts in the diseased tissue (bronchoalveolar tissue). In some embodiments, administration of compound TJ01-013 to a patient with acute or chronic lung injury increases the survival rate.

In the present invention, a compound of general formula I (preferably compound TJ01-013) or a pharmaceutical composition is usable for (i) preparing a drug for treating a fibrotic disease; (ii) preventing and/or treating a fibrotic disease. The term "fibrotic disease" refers to an increase in fibrous connective tissue and a decrease in parenchymal cells in the tissues of organs such as the heart, liver, kidneys, and lungs, which can progress to structural destruction and functional decline, or even failure of the organ, and accompanied by oxidative stress, hypoxia, and inflammation during the process of fibrosis of the injured tissue. Fibrotic diseases include renal tubulointerstitial fibrosis, interstitial lung disease (ILD), idiopathic pulmonary fibrosis (IPF), chronic obstructive pulmonary disease (COPD) fibrosis, tissue fibrosis, arthrofibrosis, hepatic fibrosis, dermal fibrosis, fibromatosis, myelofibrosis, cardiac fibrosis, and cystic fibrosis disease. In the present invention, it is found that mitochondrial dysfunction contributes to the development of fibrosis in diseased tissues, and compound TJ01-013 are able to significantly alleviate fibrosis symptoms. In some embodiments, administration of compound TJ01-013 to a subject can reduce the level of expression of fibrosis-related genes and/or proteins or slow the increase in the level of expression of fibrosis-related genes and/or proteins in the diseased tissue; preferably, the fibrosis-related genes comprise at least one of Acta2, Fn1, and Col1a1, and the fibrosis-related proteins comprise at least one of FN, collagen I, and $\alpha$-SMA.

In the present invention, a compound of general formula I (preferably compound TJ01-013) or a pharmaceutical

composition is usable for (i) preparing a drug for treating sepsis; (ii) preventing and/or treating sepsis. The term "sepsis" is a syndrome of systemic inflammatory response caused by the invasion of an organism by a pathogenic microorganism, such as a bacterium, characterized not only by an increase in inflammation, but also by immunosuppression, and this inappropriate response to an infection leads to cellular dysfunction and ultimately to organ failure. In the present invention, it is found that mitochondrial dysfunction have a critical impact in the pathogenesis of sepsis-induced organ damage, wherein during sepsis, various mitochondrial functions are altered, which include decreased oxidative phosphorylation, which results in the production of ATP, increased ROS production, increased apoptosis and altered mitochondrial biogenesis, and mitochondrial damage further enhances the immune response, causing in vivo ATP levels, baseline oxygen consumption rate, proton leakage oxygen consumption rate, maximum respiratory capacity oxygen consumption rate and ATP turnover oxygen consumption rate in patient to all reduce, and administration of compound TJ01-013 is able to significantly mitigate these adverse effects. In some embodiments, administration of Compound TJ01-013 to the patients with sepsis can increase their survival rate. In some embodiments, administration of compound TJ01-013 to the patients with sepsis can arrest or slow their rate of weight loss.

In the present invention, a compound of general formula I (preferably compound TJ01-013) or a pharmaceutical composition is usable for (i) preparing the drugs for treating prostate disorders; and (ii) preventing and/or treating prostate disorders. Prostate diseases in the present invention include prostatitis, prostate cancer and benign prostatic hyperplasia (BPH), etc.. In preferred embodiments of the present invention, the compound TJ01-013 is usable for (i) preparing the drugs for treating benign prostatic hyperplasia (BPH); (ii) preventing and/or treating benign prostatic hyperplasia (BPH). The term "benign prostatic hyperplasia (BPH)" manifests as cellular hyperplasia, and is the most common benign lesion responsible for dyspareunia in elder men. In the present invention, it is found that mitochondrial dysfunction is a possible contributing factor to BPH, and compound TJ01-013 is able to reduce BPH levels. In some embodiments, administration of compound TJ01-013 to a subject prevents the growth of prostate weight and/or volume or slows the rate of growth of prostate weight and/or volume.

In the present invention, the compound of general formula I (preferably compound TJ01-013) or the pharmaceutical composition is usable for (i) preparing the drugs for treating cardiovascular diseases; (ii) preventing and/or treating cardiovascular diseases. The term "cardiovascular diseases" refers to a variety of cardiovascular diseases in which mitophagy is involved, including atherosclerosis, heart failure, myocardial ischemia/reperfusion injury, hypertension, myocarditis, and cardiovascular complications of diabetes. In the present invention, it is found that Compound TJ01-013 is able to selectively remove dysfunctional mitochondria, maintain a balanced number of mitochondria in a cell, ensure mitochondrial structural and functional integrity, maintain homeostasis in vivo, and promote cell survival, these cells include cells of cardiovascular origin (e.g., cardiomyocytes, endothelial cells, and vascular smooth muscle cells). In some embodiments, administration of compound TJ01-013 to the subjects can reduce the myocardial infarction size or prevents the myocardial infarction size from expanding. In some embodiments, administration of compound TJ01-013 to the subjects can reduce left ventricular end-diastolic internal diameter. In some embodiments, administration of compound TJ01-013 to the subjects can reduce left ventricular end-systolic internal diameter. In some embodiments, administration of compound TJ01-013 to the subjects can increase left ventricular ejection fraction. In some embodiments, administration of compound TJ01-013 to the subjects can increase left ventricular fractional shortening.

In the present invention, a compound of general formula I (preferably compound TJ01-013) or a pharmaceutical composition is usable for (i) preparing the drugs for treating neurological diseases; (ii) preventing and/or treating neurological diseases. "Neuropsychiatric diseases" include neurodevelopmental defect diseases such as autism, sensorineural hearing loss, brain developmental anomalies, congenital hydrocephalus, congenital cranial nerve disorders, congenital cerebral penetrating pathway malformations, metabolic dysfunctions, congenital auditory agnosia, congenital visual agnosia, cerebral palsy, and etc.; and neurodegenerative diseases such as depression, schizophrenia, bipolar disorder, paranoid mental disorder, mania, obsessive-compulsive disorder, and other psychiatric disorders, and etc.; Parkinson’s disease (PD), Alzheimer’s disease (AD), brain injury (BI), Amyotrophic lateral sclerosis (ALS), epilepsy, Huntington’s disease, spinal cerebellar ataxia (SCA), cerebral ischemia (CI), stroke (preferably ischemic stroke), multiple sclerosis, tinnitus, etc.. In the present invention, it is found that mitochondrial dysfunction is an established hallmark of neurodegeneration in the present invention, and the compound TJ01-013 has therapeutic potential for neurological diseases. In some embodiments, administration of compound TJ01-013 to the subjects significantly improves his or her cognitive dysfunction. In some embodiments, administration of Compound TJ01-013 to the subjects ameliorates hearing impairment caused by cisplatin. In some embodiments, administration of compound TJ01-013 to the subjects reduces loss of cochlear basilar membrane cells in the patient.

In the present invention, a compound of general formula I (preferably compound TJ01-013) or a pharmaceutical composition is usable for (i) preparing the drugs for treating progeria; (ii) preventing and/or treating progeria (iii) preventing and treating skin aging or damage; and (iv) preparing the drugs for treating skin aging or damage. The full name of the term "progeria" is Hutchinson-Gilford Progeria syndrome, also known as Children Progeria, which manifests in the aging process of the body of a child being 5 to 10 times faster than normal, with the patient looking like an old man, and the organs also declining rapidly, resulting in a decline in physiological functions. The patient looks like an old man and his organs

decline rapidly, resulting in a decline in physiological functions. Symptoms include stature thin, hair loss and late teething. In the present invention, it is found that the compound TJ01-013 has a certain delaying effect on both natural physiological aging and pathological aging. In some embodiments, administration of compound TJ01-013 to the subjects is able to cause expression levels of heterochromatin-modified H3K9me3 in Con-O cells to increase. In some embodiments, administration of compound TJ01-013 to the subjects prevents or is able to cause a decrease in the expression level of heterochromatin-modified H3K9me3 in Con-O cells. In some embodiments, compound TJ01-013 non-therapeutically causes a decrease in the percentage of SA-β-gal+ cells in a population of mesenchymal stem cells in vitro.

In the present invention, a compound of general formula I (preferably compound TJ01-013) or a pharmaceutical composition is usable for (i) preparing the drugs for treating early onset ovarian insufficiency; (ii) preventing and/or treating early onset ovarian insufficiency (iii) preventing and treating natural ovarian insufficiency; and (iv) preparing the drugs for treating natural ovarian insufficiency. In some embodiments, administration of compound TJ01-013 to the subjects ameliorates is able to reduce ovarian dysfunction caused by chemotherapy or aging.

In the present invention, a compound or a pharmaceutical composition of general formula I is used for preventing and/or treating diseases associated with kidney injury, and for preparing the drugs for preventing and/or treating diseases associated with kidney injury. Preferably, the disease associated with kidney injury is chronic kidney disease or acute kidney injury, more preferably chronic kidney disease. Preferably, the diseases associated with kidney injury comprise acute kidney injury and chronic kidney disease, preferably chronic kidney injury. Preferably, the disease associated with kidney injury is selected from acute renal ischemia-reperfusion injury, septic nephropathy, nephrotoxic injury, primary glomerulonephritis, hypertensive renal small arteriosclerosis, diabetic nephropathy, secondary glomerulonephritis, tubulointerstitial lesions, ischemic nephropathy, lupus nephritis, and hereditary nephropathy. Preferably, the tubulointerstitial lesion is selected from chronic pyelonephritis, chronic uric acid nephropathy, obstructive nephropathy and pharmacologic nephropathy. Preferably, the hereditary nephropathy is selected from polycystic kidney and hereditary nephritis.

In the present invention, the compound of general formula I may also be added in cosmetics or medical devices for skin anti-aging and/or alleviating hair loss.

**Methods of treatment**

Some embodiments of the present invention also provide a method of treating disorders associated with kidney injury, the method comprising the step of: administering a compound of general formula I or a pharmaceutical composition comprising the same to a subject.

As used in the present invention, the term "administering" refers to physically introducing active ingredients of the drugs described herein into an individual using any of a variety of methods and delivery systems known to those skilled in the art. Routes of administration of the active ingredients of the drug of the present invention include oral, intravenous (e.g., infusion (also known as drip) or injection), airway, intramuscular, subcutaneous, intraperitoneal, spinal, topical, or other parenteral routes of administration. Accordingly, the active ingredients in the described drug of the present invention may be formulated as capsules, tablets, injections (including infusions or injectables), syrups, sprays, lozenges, liposomes, or suppositories, etc..

The mode of administration of the compound of general formula I or pharmaceutical composition in the present invention is not particularly limited and is preferably suitable for intestinal administration, e.g., oral or rectal administration; or parenteral administration, e.g., intramuscular, intravenous, transnasal, or transdermal administration, to warm-blooded animals (human and animal), with a representative mode of administration being intestinal administration.

In some embodiments, the disease associated with mitochondrial dysfunction is inflammatory bowel disease, and the mode of administration is intraperitoneal. In some embodiments, the disease associated with mitochondrial dysfunction is lung injury, and the mode of administration is airway drip. In some embodiments, the disease associated with mitochondrial dysfunction is fibrotic disease, and the mode of administration is oral gavage. In some embodiments, the disease associated with mitochondrial dysfunction is septicemia, and the mode of administration is intraperitoneal injection. In some embodiments, the disease associated with mitochondrial dysfunction is prostate disease, and the mode of administration is oral gavage. In some embodiments, the disease associated with mitochondrial dysfunction is cardiovascular disease, and the mode of administration is oral gavage. In some embodiments, the disease associated with mitochondrial dysfunction is neurological disease, and the mode of administration is oral gavage.

The compounds of general formula I in the present invention may be administered alone or in combination with other pharmaceutically acceptable compounds.

The use of the pharmaceutical compositions is the administration of a safe and effective amount of the compounds of the present invention to a mammal (e.g., a human being) in need of treatment, wherein the dose of administration is the pharmaceutically regarded effective dose to be administered, which is typically a daily dose of 1~2,000mg, or 5~1,000mg, or 10~500mg, preferably 20~500mg, more preferably 30~300g. Of course, the specific dosage should also take into account the route of administration, the patient's health condition and other factors, which are

within the skill of the skilled practitioner.

The compositions for enteral or parenteral administration are, for example, in unit dose form, such as tablets, capsules, suppositories or ampoules.

The unit content of the active ingredients in a single dose themselves need not be a therapeutically effective amount, since such an amount can be achieved by administering a plurality of dosage units. Compositions according to the present invention may contain about 10-100% of the therapeutically effective amount of the active ingredient, for example.

In the present invention, the frequency of administration of the compound of general formula I of the present invention or the pharmaceutical composition of the present invention to the subject may be once daily, twice daily, three times daily, once every two days, once every three days, or once weekly; and the period of administration of the compound TJ01-013 of the present invention or the pharmaceutical composition of the present invention to a subject may be 1~90 days, e.g., 90 days, 30 days, 28 days, 7 days, 8 days or 9 days.

In some preferred embodiments, the compound TJ01-013 of the present invention or the pharmaceutical compositions of the present invention may be administered in combination with other drugs. Exemplarily, in some embodiments, the present invention provides a method of treating cardiovascular disease, comprising the steps of: co-administering a subject a therapeutically effective amount of compound TJ01-013 and nosinostat (Entresto). The co-administration method of compound TJ01-013 and nosinostat (Entresto) may be administered simultaneously, or be administered one followed by the other. The co-administration of compound TJ01-013 and nociceptor (Entresto) may enhance the therapeutic effect on cardiovascular disease. In other embodiments, the present invention provides a method of treating benign prostatic hyperplasia, comprising the steps of: co-administering a therapeutically effective amount of compound TJ01-013 and finasteride to a subject. The co-administration of compound TJ01-013 and finasteride may further reduce the weight and volume of the prostate and ameliorates prostatic hyperplasia.

**Terminology**

As used herein, the term "disease associated with kidney injury" refers to acute kidney injury and chronic kidney disease. Acute kidney injury and chronic kidney disease mainly include acute renal ischemia/reperfusion injury, septic nephropathy, nephrotoxic injury, primary glomerulonephritis, hypertensive renal arteriolar sclerosis, diabetic nephropathy, secondary glomerulonephritis, tubulointerstitial lesions (chronic pyelonephritis, chronic uremic nephropathy, obstructive nephropathy, pharmacological nephropathy, etc.), ischemic nephropathy, hereditary nephropathies (polycystic kidneys, inherited nephritis) and others.), etc. As used herein, the term "acute kidney injury" refers to a structural or functional abnormality of the kidney that occurs within 48 hours, and lasts for no more than 3 months, including abnormality of renal injury markers in the terms of blood, urine, histology examination, or iconography. The etiology of acute renal failure is diverse and can be summarized into three broad categories: pre-renal, renal and post-renal. The diagnostic criteria for acute kidney injury are a sudden decrease in renal function within 48 hours, with an absolute increase in blood creatinine of more than 26.4 μmol/L (0.3 mg/dL); or an increase in blood creatinine of greater than 50% from a previous increase; or a decrease in urine output of less than 0.5 ml/kg/h for more than 6 hours (requiring the exclusion of urinary tract obstruction or other factors that can lead to a decrease in urine output). In embodiments of the invention, acute kidney injury includes diabetic nephropathy-induced kidney injury, acute kidney injury due to renal ischemia and reperfusion.

As used herein, the term "chronic kidney disease (CKD)" refers to 1) renal injury (structural or functional abnormality) resulting from various causes for ≥ 3 months, with or without a decrease in glomerular filtration rate (GFR), and 2) GFR < 60 ml/(min-1.73m$^2$) for ≥ 3 months, with or without evidence of renal injury. Chronic renal failure is a syndrome consisting of decreased glomerular filtration rate (GFR) caused by chronic kidney disease and associated metabolic disturbances and clinical symptoms. In the compensated stage and early stage of decompensation of chronic renal failure, patients may have no symptoms or only mild discomfort such as fatigue, lumbago, and increased nocturia; a few patients may have loss of appetite, metabolic acidosis, and mild anemia. After the renal failure stage, the above symptoms become more obvious. In the uremia stage, serious complications such as acute heart failure, severe hyperkalemia, gastrointestinal bleeding, central nervous system disorder, and even life-threatening complications may occur. In embodiments of the invention, chronic kidney injuries include adenine-induced kidney injury, nephrectomy-induced kidney injury.

As used herein, the terms "inflammatory bowel disease (IBD)" and "inflammatory bowel disease" are used interchangeably to refer to a group of chronic, recurrent non-specific inflammatory diseases of the intestinal tract of which have unknown etiology. Examples of inflammatory bowel disease include ulcerative colitis and Crohn's disease.

As used herein, the terms "pyemia" and "sepsis" are used interchangeably to refer to a syndrome of systemic inflammatory response caused by various infectious agents (bacteria, fungi, viruses, parasites, etc.).

As used herein, the term "lung injury" refers to the destruction of the parenchymal structure of the lungs caused by the action of various internal and external factors on the lungs. The meaning of lung injuries in the present invention includes both acute and chronic lung injury. The term "acute lung injury (ALI)" refers to the pathological changes of pulmonary edema and pulmonary atelectasis caused by damage to the alveolar capillary membrane following severe

infections, trauma and shock. The term "chronic lung injury" means a long-term lung disease characterized by fibrosis, including diffuse lung injury and chronic obstructive pulmonary disease. The term "chronic obstructive pulmonary disease (COPD)" refers to a common chronic disease that has chronic bronchitis and/or emphysema characterized by airflow obstruction, and may progress to pulmonary heart disease and respiratory failure.

As used herein, the term "cerebral ischemia (CI)" refers to a transient lack of blood supply to the brain with symptoms called a transient ischemic attack. Cerebral ischemia is a type of cerebrovascular disease. In severe cases, cerebral ischemia often results in limited ischemic necrosis or softening of brain tissue, leading to ischemic stroke.

As used herein, the term "stroke" refers to a disease caused by circulatory disorders of cerebral blood flow and functional or structural damage of brain tissue caused by cerebral vascular obstruction or rupture. The term "ischemic stroke" refers to a general term for necrosis of brain tissue due to insufficient blood supply to the brain as a result of narrowing or occlusion of the arteries supplying blood to the brain (carotid and vertebral arteries).

As used in the present invention, the term "cardiomyopathy" is a broad definition of a cardiomyopathy, which broadly includes a disease caused by a lesion of the myocardium, including a lesion caused by inflammation of the myocardium exemplarily such as myocarditis; a disease caused by ischemia or necrosis of the myocardium exemplarily such as myocardial infarction; and a disease characterized by abnormalities of the morphology, structure, and function of the myocardium exemplarily such as primary myocardial infarction and secondary myocardial infarction. Primary cardiomyopathies are exemplified by dilated cardiomyopathies, hypertrophic cardiomyopathies, restrictive cardiomyopathies, arrhythmogenic right ventricular cardiomyopathies, and undetermined cardiomyopathies, and secondary cardiomyopathies are exemplified by ischemic cardiomyopathies, alcoholic cardiomyopathies, perinatal cardiomyopathies, Kirschberg's disease, and neuromuscular cardiomyopathies.

As used in the present invention, the term "myocardial infarction (MI)" refers to ischemic necrosis of the myocardium resulting from severe and persistent ischemia of the corresponding myocardium due to a drastic reduction or interruption of coronary blood supply.

As used in the present invention, the term "myocarditis" refers to a localized or diffuse acute, subacute or chronic inflammatory lesion in the myocardium, which mostly affects children and young adults, and can lead to dilated cardiomyopathy and chronic heart failure if prolonged. Usually myocarditis can be divided into 3 categories, idiopathic, autoimmune and infectious myocarditis. Clinical treatment of myocarditis is mainly to intervene in the pre-acute inflammation, and once it progresses to the chronic phase, there is no specific treatment, and the main focus is on conservative treatment and reducing the cardiac load.

As used in the present invention, the term "aging-related disease" includes all physiological and pathological diseases caused by various factors such as changes in physiology, organs, etc. In the embodiment of the present invention, the term "aging-related disease" includes all physiological and pathological diseases. In the embodiment of the present invention, the aging-related disease includes skin aging, senilism, ovarian insufficiency (preferably early-onset ovarian insufficiency, natural aging ovarian insufficiency), and the like.

As used in the present invention, the term "senilism" refers to a phenomenon in which the number of cell division generations of an individual decreases, and the aging of certain organs and tissues multiplies and rapidly leads to disease and death. This is manifested by the appearance of characteristics of the elderly in minors, such as arteriosclerosis, wrinkled skin, gray hair, and an aged face and behavior.

As used in the present invention, the terms "premature ovarian insufficiency (POI)" and "premature ovarian failure" are used interchangeably, referring to the onset of ovarian insufficiency in a woman before the age of 40 years, mainly manifested by abnormal menstruation (amenorrhea, scanty or frequent menstruation), elevated gonadotropins [at least 2 serum basal follicle-stimulating hormone (FSH) levels >25 U/L], fluctuating decreases in estrogen levels, and the like.

As used in the present invention, the term "natural aging ovarian insufficiency" refers to the decline of ovarian function in a woman due to natural aging, which may cause endocrine disorders, premature ovarian failure, infertility, and other disorders.

As used in the present invention, the terms "lupus nephritis" and "lupus nephritis" are used interchangeably to refer to a type of immune complex nephritis caused by systemic lupus erythematosus involving the kidneys. In addition to the systemic manifestations of systemic lupus erythematosus, the main clinical manifestations are hematuria, proteinuria, and renal insufficiency.

As used in the present invention, the term "multiple sclerosis (MS)" is a complex pathogenesis, central nervous system autoimmune demyelination disease, which is characterized by multiple lesions, recurrent course, high disability rate, and seriously affects the normal life of patients. Its pathogenesis is unclear and may be caused by a variety of factors such as immunity, environment, and genetics.

As used in the present invention, the term "tinnitus" refers to the symptom of subjectively hearing a continuous sound without external sound stimulation. It is caused by pathological stimulation of auditory receptors and their conduction pathways or by lesions of the auditory center. In some embodiments, the tinnitus is preferably neurogenic tinnitus, the neurogenic tinnitus for example is sensorineural (originating from the cochlea), peripheral neurogenic

(originating from the auditory nerve), and central neurogenic tinnitus.

As used in the present invention, the terms "sensorineural hearing loss" and "sensorineural deafness" are used interchangeably, referring to hearing impairments caused by disorders of the inner ear, auditory nerve and auditory pathways.

As used in the present invention, the terms "abnormal brain development" and "brain hypogenesis" are used interchangeably, referring to a symptom characterized by mental retardation and growth retardation caused by a reduction in brain tissue and inadequate or damaged development of nerve cells in the brain due to a certain reason.

As used herein, the term "alkyl" refers to a straight or branched saturated aliphatic hydrocarbon group. The term "$C_{1\sim6}$ alkyl" refers to a straight or branched alkyl with 1 to 6 carbon atoms, non-limitingly, for example it is : methyl, ethyl, n-propyl, isopropyl, n-butyl, isobutyl, tert-butyl, sec-butyl, n-amyl, 1,1-dimethylpropyl, 1,2-dimethylpropyl, 2,2-dimethylpropyl, 1-ethylpropyl, 2-methylbutyl, 3-methylbutyl, n-hexyl, 1-ethy-2-dimethylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and their various branched isomers, etc. The term "$C_{1\sim4}$ alkyl" refers to a straight or branched alkyl with 1 to 4 carbon atoms, and if present at the end of the molecule, non-limitingly, for example, the $C_{1\sim4}$ alkyl is: methyl, ethyl, n-propyl, isopropyl, n-butyl, iso-butyl, sec-butyl, tert-butyl, or, when two portions of the molecule are connected by the alkyl, non-limitingly, for example, the $C_{1\sim4}$ alkyl is : $-CH_2-$, $-CH_2-CH_2-$, $-CH(CH_3)-$, $-CH_2-CH_2-CH_2-$, $-CH(C_2H_5)-$, $-C(CH_3)_2-$, and each hydrogen of the $C_{1\sim4}$ alkyl may be replaced by a substituent group as further enumerated herein.

As used herein, the term "alkenyl" refers to a straight or branched hydrocarbon chain containing at least one carbon-carbon double bond. Each hydrogen on the alkenyl carbon may be replaced by a substituent group as further enumerated herein. The term "$C_{2\sim6}$ alkenyl" refers to a straight or branched hydrocarbon chain containing at least one carbon-carbon double bond with 1 to 6 carbon atoms. If present at the end of the molecule, non-limitingly, for example it is: $-CH=CH_2$, $-CH=CH-CH_3$, $-CH_2-CH=CH_2$, $-CH=CH-CH_2-CH_3$, $-CH=CH-CH=CH_2$, or, when two portions of the molecule are connected by the alkenyl, non-limitingly, for example it is $-CH=CH-$ . Each hydrogen of the $C_{2\sim6}$ alkenyl may be replaced by a substituent group as further enumerated herein.

As used herein, the term "alkynyl" refers to a straight or branched hydrocarbon chain containing at least one carbon-carbon triple bond. Each hydrogen on the carbon of the alkynyl may be replaced by a substituent group as further enumerated herein. The term "$C_{2\sim6}$ alkynyl" refers to a straight or branched hydrocarbon chain containing at least one carbon-carbon triple bond with 1 to 6 carbon atoms. If present at the end of the molecule, non-limitingly, for example it is: $-C\equiv CH$, $-CH_2-C\equiv CH$, $-CH_2-CH_2-C\equiv CH$, $-CH_2-C\equiv C-CH_3$, or, when two portions of the molecule are connected by the alkynyl group, non-limitingly, for example it is $-C\equiv C-$. Each hydrogen of a C2~6 alkynyl may be replaced by a substituent group as further enumerated herein.

As used herein, the term "alkoxy" refers to a group having the structure "-O-alkyl", wherein the alkyl is defined as above. The term "$C_{1-6}$ alkoxy" refers to an alkoxy with 1 to 6 carbon atoms, non-limitingly, for example it is: methoxy, ethoxy, n-propoxy, isopropoxy, n-butoxy, tert-butoxy, isobutoxy, n-pentyloxy, and the like.

As used herein, the term "amine group" refers to a group formed by substitution of at least one hydrogen atom in an amino group with an alkyl, for example, in the amidogen

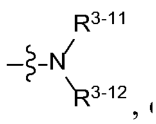

either one of $R^{3-11}$ and $R^{3-12}$ is alkyl, and the other is hydrogen; or both of $R^{3-11}$ and $R^{3-12}$ are alkyl; when both of $R^{3-11}$ and $R^{3-12}$ are alkyl, $R^{3-11}$ and $R^{3-12}$ are bondable to a ring.

As used herein, the term "haloalkyl" refers to an alkyl in which one or more (e.g., 1, 2, 3, 4, or 5) hydrogen atoms are substituted with halogens, wherein alkyl is defined as above.

As used herein, "(O)" refers to

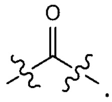

In one embodiment, $-CH_2C(O)R^{3-2}$ refers to

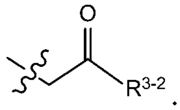

As used herein, the term "halooxyalkyl" refers to an alkoxy in which one or more hydrogen atoms are substituted with a halogen, wherein alkoxy is defined above.

As used herein, the terms "aryl", "aryl ring" and " aromatic ring" are used interchangeably, referring to an all-carbon monocyclic ring, an all-carbon non-fused polycyclic ring (ring to ring connected by covalent bonds, non-fused) or an all-carbon polycyclic ring. non-condensed) or all-carbon fused polycyclic (i.e., rings sharing adjacent carbon atom pairs), in which at least one ring is aromatic, i.e., has a ring-forming conjugated $\pi$-electron system.

As used herein, the term "heteroaryl" refers to an aryl in which at least one of the ring carbon atoms constituting the aryl is replaced by a heteroatom, the heteroatom is a non-carbon atom, such as S, N or O.

As used herein, the term "monocyclic heteroaryl" refers to a heteroaryl having only one aromatic ring, wherein heteroaryl is defined as above. The term "5- or 6-membered monocyclic heteroaryl" refers to a monocyclic heteroaryl having 5 or 6 ring atoms, wherein 1, 2, or 3 ring atoms are heteroatoms selected from nitrogen, oxygen, or S(=O)m' (wherein m' is an integer 0 to 2), and non-limitingly, for example, it is: thiophene, furan, thiazole, isothiazole, imidazole, oxazole, pyrrole, pyrazole, 20 triazole, 1,2,3-triazole, 1,2,4-triazole, 1,2,5-triazole, 1,3,4-triazole, tetrazole, isoxazole, oxadiazole, 1,2,3-oxadiazole, 1,2,4-oxadiazole, 1,2,5-oxadiazole, 1,3,4-oxadiazole, thiadiazole, pyridine, pyridazine, pyrimidine, and the like.

As used herein, the term " fused ring heteroaryl" refers to having at least two aromatic rings in which two ring atoms are shared and adjacent to each other, wherein heteroaryl is defined as above. The term "fused bicyclic heteroaryl" refers to a fused ring heteroary having two aromatic rings, wherein the fused ring heteroary is defined as above. The term "8 to 10-membered fused bicyclic heteroaryl" refers to a fused bicyclic heteroaryl having 8 to 10 ring atoms, wherein 1, 2, 3, 4, or 5 ring atoms are heteroatoms selected from nitrogen, oxygen, or S(=O)m' (wherein m' is an integer 0 to 2), non-limiting examples include: benzo[d]isoxazole, 1H-indole, isoindole, 1H-benzo[d]imidazole, benzo[d]isothiazole, 1H-benzo[d][1,2,3]triazole, benzo[d]oxazole, benzo[d]thiazole, indazole, benzofuran, benzo[b]thiophene, quinoline, isoquinoxaline, quinazoline, quinoxaline, cinnoline, pyrido[3,2-d] pyrimidine, pyrido[2,3-d]pyrimidine, pyrido[3,4-d]pyrimidine, pyrido[4,3-d]pyrimidine, 1,8-naphthyridine, 1,7-naphthyridine, 1,6-naphthyridine, 1,5-naphthyridine, pyrazolo[1,5-a]pyrimidine, imidazo[1,2-b]pyridazine and the like.

In order to make the objects, technical solutions and advantages of the embodiments of the present invention clearer, the present invention is further elaborated below in connection with specific embodiments. It should be understood that these embodiments are used only to illustrate the present invention and are not intended to limit the scope of the present invention. Experimental methods for which specific conditions are not indicated in the following embodiments are generally in accordance with conventional conditions, or in accordance with conditions recommended by the manufacturer. Unless otherwise indicated, percentages and parts are weight percentages and weight parts. Experimental materials and reagents used in the following embodiments are available from commercially available sources if not otherwise indicated.

Unless otherwise indicated, technical and scientific terms used herein have the same meanings as are commonly understood by one of ordinary skill in the art to which this application belongs, and it is to be noted that the terminology used herein is intended only to describe specific embodiments and is not intended to limit the exemplary embodiments of the present application.

**Experimental example 1 alleviating acute kidney injury caused by renal ischemia/reperfusion and inhibiting apoptosis of renal tissue cells caused by renal ischemia/reperfusion**

1) Unilateral renal ischemia-reperfusion

8-week-old C57BL/6 mice (weighing about 20-25g) were injected intraperitoneally with 50mg/kg sodium pentobarbital. After the mice were anesthetized, carried out preserved skin and placed on a thermostatic table, the skin and muscle of the right side were incised from the back and the right kidney was exposed, and the tissue around the pedicle of the kidney was stripped off with forceps to expose the blood vessels and the ureter. The arterial and venous blood vessels and the ureter were ligated with sutures. After removing the kidney, the muscle and skin were sutured with sutures and placed at 37°C to wake it up, and after waking up, the mice were given intraperitoneal injection of I-1. The experiments were divided into a sham group (exposing the right side of the kidney without removing it), a control group (giving solvent treatment by oral gavage), a 10mg/kg group (10mg/kg I-1 by oral gavage) and a 30mg/kg group (30mg/kg I-1 by oral gavage), with being administered once a day, and left kidney ischemia-reperfusion was performed on the sixth day, the specific plan was: mice were injected intraperitoneally with 50mg/kg sodium pentobarbital, after anesthetizing, carried out preserved skin and placed on a thermostatic table, the skin and muscle of the left side were cut from the back and the left kidney was exposed, and the tissue around the pedicle of the kidney was stripped off with forceps to expose the blood vessels, and the arterial clip were used to tighten the blood vessel, after ischemia for 45 min, the arterial clip were released and the kidney tissue was returned to its place with a cotton swab, and finally the muscle and skin were sutured with a suture lines and the mice were placed at 37°C to wake it up, and given another drug treatment after waking up. Blood was

taken from the eyeball after 24h of reperfusion, and the blood was centrifuged at 12000g for 10min at 4°C after standing at room temperature for 45min, and the supernatant (serum) was taken to detect the level of creatinine and urea nitrogen in the serum with a biochemical analyzer.

As shown in Fig. 1, serum creatinine and serum urea nitrogen levels were significantly reduced in mice with renal ischemia-reperfusion for 24h after oral gavage of 10mg/kg and 30mg/kg of I-1, indicating that I-1 can significantly reduce blood creatinine levels after renal ischemia-reperfusion, and has a certain protective effect on acute kidney injury.

2) HE staining analysis of kidney tissues

After blood extraction from the mouse eyeballs, cardiac perfusion was performed with PBS, after mice were anesthetized and fixed on a foam plate with a needle, the skin and ribs of the chest and abdomen were cut open to expose the heart, the injection needle was inserted into the left ventricle of mice and the pericardium was cut open at the same time, and the perfusion was performed with PBS at a speed of 7rpm until the liver turned white and then the perfusion was stopped, the kidneys were taken out, and the renal peritoneum was removed, and the kidneys were put into 4% paraformaldehyde and fixed and immersed for 72 h. Subsequently, tissue embedding was carried out. Then tissue embedding, sectioning and HE staining were performed as follows:

**Tissue embedding**

The steps of tissue embedding were as follows: ethanol dehydration: the tissues were dehydrated by different concentrations of ethanol solution step by step to 75%, 85%, 95%, 100%, 100%, each time dehydrated for 40 min; transparency: the tissues were sequentially immersed in three xylene jars, each jar for 30 min; wax infiltration: the tissues were sequentially immersed in three wax jars, the first one for 1h, the second one for 1.5h, and the third one for 2h; Embedding: poured the liquid paraffin into the mold box, and then placed the wax-impregnated tissue blocks flat on the bottom, paying attention to the cut surface facing downward, remove the embedding frame after the paraffin solidifies, and then trim the wax block after it cools down and hardens completely, with the paraffin on the periphery of the tissues retained moderately to facilitate sectioning.

**Sectioning**

The pre-cooled wax block was fixed on the wax slicer, with the cut surface of the block being parallel to knife edge, and the knife was usually inclined at 15 degrees, and the thickness of the section was adjusted to 3 μm by rotating the rotary advancer to make sections of uniform thickness. The left hand held the brush, the right hand rotated the handle of the slicer, the section was gently held up with the brush after being brought out, and then the wax section was gently picked up with tweezers, and put into the spreading box with the front side, and the water temperature was about 40°C. The section was fished out after flattened. Attaching the sections, one end of the slide was held with the left hand, and vertically inserted into the water to attach the section, tweezers were held by the right hand to assist in promoting section to attach to two-thirds of the slide. After attaching, the section was put in the air to dry slightly, then put on the 60°C baking machine for 1h, and then put in the oven for baking for 2h.

**Dewaxing wax sections to water**

The wax sections were put sequentially into xylene I for 15min-xylene II for 15min-anhydrous ethanol I for 10min-anhydrous ethanol II for 10min-95% alcohol for 10min-85% alcohol for 10min to dewax in gradient.

**HE staining**

The wax section was stained with hematoxylin for 5-10 min, rinsed with tap water, differentiated with 1% hydrochloric acid in alcohol for a few seconds, rinsed with tap water, and then returned to blue in saturated aqueous lithium carbonate for 1 min, washed with running water for a few seconds, and stained with eosin stain solution for a few seconds, rinsed with running water.

**Dehydration, sealing and image acquisition**

The wax section was put into 75% ethanol for 2min -85% ethanol for 2min - anhydrous ethanol for 2min - anhydrous ethanol for 2min - xylene for 2min for transparency, and then the section was taken out from xylene and sealed with neutral gum. Microscopic examination was performed and images were captured and analyzed.

As shown in FIG. 2, renal ischemia and reperfusion caused damage to normal renal tissues, and I-1 treatment

alleviated the damage to renal tissues caused by renal ischemia and reperfusion.

3) Immunostaining of renal tissue

Tissue was dewaxed after embedding and sectioning, and performed antigen repair, 5% goat serum sealing solution was added dropwise at 37°C for 40min, 50μl of KIM-1 and caspase3 primary antibody (1:500) was added dropwise, overnight at 4°C; after rewarming at 37°C for 30min, the tissue was washed by PBS four times, each time for 5min. And then fluorescence secondary antibody was added dropwise for 1h, and the tissue was washed by PBS four times, each time for 5min, finally, the tissue was stained by DAPI for 10min, sealed with nail polish, and images were collected by cytation 3.

As shown in FIG.3, both KIM-1 and cleaved-caspase3 were significantly expressed after renal ischemia-reperfusion, and I-1 treatment inhibited the levels of KIM-1 and cleaved-caspase3 after renal ischemia-reperfusion.

In addition, the results showed that the treatment of renal ischemia-reperfusion injury in the UMI-77 group was less effective than the improvement of renal ischemia-reperfusion injury by I-1.

The mice used in the experiments were purchased from GEMPHARMATECH CO., LTD.

**Experimental example 2: Treatment of 5/6 nephrectomy-induced renal injury**

5/6 nephrectomy directly leads to a large reduction of renal units, high workload of residual renal units, and combined hypertension (or non-hypertension), and these features are similar to the symptoms of patients with clinical nephrectomy, CKD hypertensive syndrome, and solitary kidney, and like, and the pathological changes are dominated by glomerulosclerosis and tubulo-fibrosis, which is a good model for the study of CKD. The model was prepared as follows, 230-250g male SD rats were anesthetized with isoflurane gas, and an incision of about 3 cm at 1 cm left and below of the cribriform ridge corner was made, fully exposing the left kidney, and after exposing the kidney in the sham group, the muscle and skin were sutured with sutures, during which there was no ligation and no resection. In the model group, about 1/3 of the cortex of the upper and lower left kidney was cut with ophthalmic surgical scissors, and was pressed with the absorbable gelatin sponge to stop bleeding for 3 min, then the hemostatic clip was released, and whether there is obvious bleeding was observed and the residual kidney was reset, and the muscle and skin were sutured with sutures. After 1 week, the second surgery was performed, and after fully exposing the right kidney, the renal pedicle and the ureter were ligated, and the right kidney was resected. Similarly, in the sham group, after exposing the kidney, the muscle and skin were sutured with sutures, during which there was no ligation and no resection. Penicillin (80 kU/rat) was routinely injected for three days after surgery. Serum was taken weekly starting 1 week after the end of the right nephrectomy surgery to determine the serum creatinine level of rats in each model group, and the modeling was considered successful if the blood creatinine level was significantly higher than that of the sham group. In the experiment, 10d after the end of the right nephrectomy surgery, the serum creatinine level of rats increased, which was statistically significant compared to the sham group, and began to be administered in groups.

The experiments were conducted in sham group (exposing both kidneys without resection), Control group (model group, given vehicle treatment by oral gavage), and 15 mg/kg I-1 group (15 mg/kg by oral gavage), respectively, during the experimental period, a drug was administered once a day for a total of 27 days, and the 24-hour urine of the rats in each group was collected by metabolism cages on the 28th day to measure the volume of urine, urine creatinine, and urine protein. At the same time, blood was collected from the heart, and the blood was centrifuged at 12000 g for 10 min at 4°C after standing at room temperature for 45 min, the supernatant (serum) was taken to measure the creatinine level in the serum, and finally the creatinine clearance was calculated. After euthanasia of rats at endpoint of the test, the kidneys were dissected and removed, in which four kidneys of rats were fixed with 4% paraformaldehyde for 48h, paraffin-embedded, sectioned, and HE stained as described above.

As shown in FIG. 4, compared with the sham group, the body weight of rats in the model control group decreased significantly ($P < 0.001$), and the administration of I-1 15 mg/kg for 27 days resulted in a significant increase in the body weight of the model group ($P < 0.05$), indicating that I-1 had a certain protective effect on the recovery of body weight in rats.

As shown in FIG. 5, compared with the sham group, the blood creatinine level of rats in the model control group was significantly increased ($P < 0.001$), and the I-1 treatment significantly reduced the blood creatinine level of rats in the model group ($P < 0.001$), indicating that I-1 had a certain ameliorative effect on creatinine level of renal injury caused by 5/6 nephrectomy.

After 27 days of oral gavage administration, 24h urine was collected and tested as shown in Fig. 6. Compared with the sham group, the 24h urine protein content of rats in the model control group was significantly elevated, and the I-1 treatment significantly reduced the urine protein content of the model group, suggesting that I-1 has a certain protective effect on the leakage of protein caused by chronic kidney injury. As shown in FIG. 7, there is also an improvement trend in creatinine clearance rate after 27 days of I-1 treatment compared with the model control group.

HE staining results were shown in FIG. 8, statistical analysis was performed on four cases of renal histopathological sections, the results were show in Table 1, sham group: in four cases of kidneys, glomeruli and tubules were structurally intact, cell morphology was clear, the interstitium did not see obvious abnormalities (4/4, no abnormality); model control group: in four cases of kidneys, under low magnification, renal cortex significant thickening was visible and expressed as compensatory hypertrophy; and under high-magnification, renal tubular cytoplasm was sparse and granular, vacuoles were partly visible, powder stained material was visible in a small number of tubular lumens, and inflammatory cell infiltration and thickening of the renal peritoneum (1/4, mild, 3/4, moderate) were visible in individual kidney interstitium; in administration group, varying degrees of renal cortical thickening were visible and expressed as compensatory hypertrophy; and under high-magnification, renal tubular cytoplasm was sparse and granular, vacuoles were partly visible, powder stained material was visible in a small number of tubular lumens, and inflammatory cell infiltration and thickening of the renal peritoneum (1/4, mild, 3/4, moderate) were visible in individual kidney interstitium. Compared with the sham group, renal tissues of rats in the model control group were significantly damaged, and the I-1 treatment improved the degree of lesions in the renal tissues of rats in the model group. In addition, UMI-77 was at least less protective than the I-1 group against 5/6 nephrectomy-induced renal injury, at least in the above indexes. The rats used in the experiments were purchased from SPF (Beijing) Biotechnology Co.,Ltd.

Table 1 Summary of histologic examination of 5/6 nephrectomized rats (n=4)

| Group | Degree of lesion | Renal lesions |
|---|---|---|
| sham group | - | 4 |
| | ± | 0 |
| | + | 0 |
| | ++ | 0 |
| | +++ | 0 |
| model control group | - | 0 |
| | ± | 0 |
| | + | 1 |
| | ++ | 3 |
| | +++ | 0 |
| 15 mg/kg I-1 group | - | 0 |
| | ± | 0 |
| | + | 3 |
| | ++ | 1 |
| | +++ | 0 |
| Note: - no lesions seen; ± slight lesions; + mild lesions; ++ moderate lesions; +++ severe lesions; n=4 | | |

**Experimental Example 3, Treatment of Adenine-Induced Kidney Injury**

Adenine-induced kidney injury is an animal model of chronic kidney injury. The model was prepared as follows: 1500 mg/kg of potassium oxonate and 50 mg/kg of adenine were ground and suspended in 0.5% carboxymethylcellulose sodium (CMC-Na) solution and gavaged once a day in a volume of 10 mL/kg for 180-200 g male SD rats, and an equal dose of 0.5% CMC-Na solution was gavaged for the sham group. After 1 week of modeling, serum creatinine (Cr) was measured weekly to determine whether the model was successful or not; in this experiment, there was a statistically significant difference in serum creatinine elevation on the 21st day of modeling compared with that of the sham group, at this time, administration in group was initiated, and the shock modeling with 1500 mg/kg of potassium oxonate and 50 mg/kg of adenine was continued daily along with the administration. The test was divided into three groups according to the serum creatinine level, sham operation group (sham group, without adenine modeling and vehicle treatment by oral gavage), control group (model control group, vehicle treatment by oral gavage), and 15mg/kg group (15mg/kg compound I-1 by oral gavage), and the dosage was once a day in the volume of 10 ml/kg for 21 consecutive days. The dose was administered once a day in a volume of 10 ml/kg for 21 days. At the end of the administration, blood was collected from the heart, and the blood was centrifuged at 12000g for 10 minutes at 4°C after standing at room temperature for 45 minutes, and the supernatant (serum) was extracted to measure the levels of creatinine and uric acid in the serum. After euthanasia of rats at endpoint of test, rats were dissected and kidneys were taken, of which 4 kidneys were fixed with 4% paraformaldehyde for 48h, embedded in paraffin, sectioned, and HE stained, the specific staining operation was as described above. The left kidney tissue was taken and ground, and the inflammatory factors IL-1β and TNFα were detected by Elisa method.

As shown in FIG. 9, compared with the sham group, there was a tendency of decreasing body weight in the model control group of rats, and there was no significant change in body weight in the compound I-1 treatment.

As shown in FIG. 10, compared with the sham group, serum creatinine level was significantly increased ($P < 0.001$) in the model control rats. Serum creatinine decreased by 20.7% ($P < 0.05$) after 21 days of I-1 treatment, suggesting that I-1 significantly alleviated the increase in creatinine level caused by adenine-induced renal injury.

As shown in FIG. 11, compared with the sham group, the serum urea nitrogen level in the model control rats was significantly elevated ($P < 0.001$) 21 days after administration, and the serum urea nitrogen level was significantly decreased ($P < 0.05$) 21 days after I-1 15 mg/kg treatment.

As shown in FIG. 12, compared with the sham group, the levels of inflammatory factors TNF-$\alpha$ and IL-1$\beta$ in the kidney tissues of rats in the model group were significantly increased ($P < 0.05$-$0.01$), and the levels of inflammatory factors TNF-$\alpha$ in the kidney tissues were significantly decreased ($P < 0.05$-$0.01$) and IL-1$\beta$ had a tendency to be decreased after 21 days of I-1 15mg/kg treatment, indicating that compound I-1 was able to inhibit the increase of inflammation level caused by adenine-induced renal injury.

The HE staining results are shown in FIG. 13, and the histopathologic statistical results of the kidneys of 4 rats are shown in Table 2. In 4 cases of the kidneys in the sham group, the glomeruli and tubules were structurally intact, with a clear cellular morphology, and any obvious abnormality was invisible in the mesenchyme (4/4, no abnormality was visible). In the model control group, dilatation, edema, basophilic changes, basement membrane thickening were visible in 4 cases of renal tubules, and grass-green crystals were visible in a small number of small blood vessels and the lumen of the renal tubules, some of the glomerular capsule was thicken, a small number of glomerular atrophy, and inflammatory cell infiltration and fibrous tissue hyperplasia (3/4, moderate, 1/4, severe) were visible in the interstitium. In the administered group (15mg/kg Compound I-1), different degrees of renal tubular dilatation, edema, basophilic changes, basement membrane thickening also were visible, grass-green crystals were visible in a small number of small blood vessels and the lumen of the renal tubules, some of the glomerular capsule was thicken, a small number of glomerular atrophy, and inflammatory cell infiltration and fibrous tissue hyperplasia (1/4, mild, 3/4, moderate) were visible in the interstitium. The results showed that I-1 improved the histopathology of adenine-induced renal injury to some extent. Specific results are shown in Table 2. In addition, UMI-77 was less effective in treating adenine-induced renal injury than I-1 in ameliorating adenine-induced renal injury, at least in the above indexes. The rats used in the experiments were purchased from SPF (Beijing) Biotechnology Co. Ltd, and adenine and potassium oxonate were purchased from Shanghai yuanye Bio-Technology Co., Ltd.

Table 2 Summary of renal histologic examination in rats with adenine-induced renal injury (n=4)

| Group | Degree of lesion | Renal lesion |
|---|---|---|
| sham group | - | 4 |
| | ± | 0 |
| | + | 0 |
| | ++ | 0 |
| | +++ | 0 |
| model control group | - | 0 |
| | ± | 0 |
| | + | 0 |
| | ++ | 3 |
| | +++ | 1 |
| 15mg/kg I-1 Treatment Group | - | 0 |
| | ± | 0 |
| | + | 1 |
| | ++ | 3 |
| | +++ | 0 |
| Note: - no lesions; ± slight lesions; + mild lesions; ++ moderate lesions; +++ severe lesions; n=4 | | |

**Experimental example 4, treatment of kidney injury induced by type 1 diabetic nephropathy**

150-170g SD male rats were fasted but not deprived of water for 12h before surgery, and a longitudinal incision was made at the intersection of the lower edge of the right rib arch, which was about 1 cm long. Then the three layers of skin, mucous membrane, and muscle were separated layer by layer to expose the perinephric fat, and the perinephric fat was

clamped by hemostatic forceps and lifted upward to expose the right kidney, and the right kidney pedicle was ligated and the right kidney was resected, and after no hemorrhage was checked, the tissues were sutured layer by layer. One week after surgery, 1% streptozotocin (STZ) 50 mg/kg was injected intraperitoneally in a volume of 2 mL/kg. Rats in the sham group only had the abdominal cavity opened, the right renal adipose capsule was peeled off, and the right kidney was not resected; the abdominal cavity was closed, the incision was sutured and an equal volume of citrate solution was injected into the tail vein one week later.

Random blood glucose was measured 72 h after STZ injection, and blood glucose ≥16.65 mmol /L was considered as successful diabetes modeling. The successful modeling rats were selected to continue to be fed with normal chow and free water. 14 days after STZ modeling, random blood glucose ≥16.65 mmol/L and serum creatinine elevation, which was significantly different from that of the sham group, were regarded as the success of the model, and the drugs were administered in randomized groups according to the value of creatinine. The experiment was divided into sham group (sham group, without STZ modeling and given vehicle by oral gavage), Control group (model control group, STZ modeling and given vehicle treatment by oral gavage), and 15 mg/kg I-1 group (STZ modeling and given 15 mg/kg I-1 by oral gavage), and the dosage of the drug was administered once a day in the volume of 10 ml/kg for 27 days. At the end point of the experiment, metabolic cages were used to collect 24h urine from each group of rats to check urine volume, urinary protein and creatinine, and blood was collected from the heart, and the blood was centrifuged at 12,000g for 10min at 4°C after standing at room temperature for 45min, the supernatant (serum) was extracted to measure the levels of creatinine and urea nitrogen in the serum, and finally the creatinine clearance was calculated. At the end point of the experiment, the rats were euthanized, the kidneys were removed after dissection, and four kidneys were taken to be fixed with 4% paraformaldehyde for 48h, paraffin-embedded, sectioned and HE stained, and the specific staining procedure was as described above.

As shown in FIG. 14, compared with the sham group, the body weight of rats in the model control group decreased significantly ($P < 0.001$), and the I-1 treatment caused a trend of increasing body weight of rats.

As shown in FIG. 15, compared with the sham group, the serum creatinine level of rats in the model control group was significantly elevated ($P < 0.01$~$0.001$), and the serum creatinine level of the model group was significantly decreased after 27 days of I-1 treatment ($P < 0.5$), indicating that the I-1 treatment was able to alleviate the increase in creatinine caused by type I diabetic nephropathy.

As shown in FIG. 16, compared with the sham group, the blood glucose level of rats in the model control group increased significantly, and I-1 treatment had no significant effect on the blood glucose level of rats in the model group.

As shown in FIG. 17, compared with the sham group, the 24-h urine protein of rats in the model-control group was significantly elevated ($P < 0.001$), and the I-1 treatment caused a decrease in the 24-h urine protein level of rats, but there was no significant difference.

The results of HE staining were shown in FIG. 18, and the statistical analysis of pathology and histology of the four cases was shown in Table 3. In the sham group, one case of kidney had localized glomerular capillary dilatation, tubular edema, and dilatation (1/4, mild); and in 3 cases of kidneys, glomeruli and tubules of the kidneys were structurally intact, with a clear cellular morphology, and the interstitium did not have any obvious abnormality (3/4, no abnormalities were visible). In the model control group, in 4 cases of kidneys, some glomerular and tubular necrosis were visible, some glomerular capillaries were dilatated, mesangial matrix was increased, powder-stained exudate was visible in the renal capsule, a small amount of glomerular has structural destruction, and edema, vacuolar degeneration, basophilic degeneration, and dilatation (1/4, mild, 1/4, moderate, and 2/4, severe) were visible in the renal tubules. In the I-1-treated group, some glomerular and tubular necrosis was visible, some glomerular capillaries were dilatated, mesangial matrix was increased, powder-stained exudate was visible in the renal capsule, a small amount of glomerular has structural destruction, and edema, vacuolar degeneration, basophilic degeneration, and dilatation (3/4, mild, 1/4, severe) were visible in the renal tubules, suggesting that I-1 resulted in a significant reduction in the degree of renal pathology due to diabetic nephropathy as compared to the model group. In addition, the results showed that UMI-77 was less protective against kidney injury in type 1 diabetes than the I-1 group, at least in the above indexes.

The rats used in the experiments were purchased from SPF (Beijing) Biotechnology Co., Ltd. and the STZ were purchased from Shanghai Taoshu Biotechnology Co., LTD.

Table 3 Summary of histologic examination of rats with type I diabetic nephropathy (n=4)

| Group | Degree of lesion | Renal lesion |
|---|---|---|
| sham group | - | 3 |
| | ± | 0 |
| | + | 1 |
| | ++ | 0 |
| | +++ | 0 |

(continued)

| Group | Degree of lesion | Renal lesion |
|---|---|---|
| model control group | - | 0 |
| | ± | 0 |
| | + | 1 |
| | ++ | 1 |
| | +++ | 2 |
| 15mg/kg I-1 treatment group | - | 0 |
| | ± | 0 |
| | + | 3 |
| | ++ | 0 |
| | +++ | 1 |
| Note: - no lesions; ± slight lesions; + mild lesions; ++ moderate lesions; +++ severe lesions; n=4 | | |

**Experimental Example 5, In vitro liver microsomal stability test**

Ketanserin was selected as the reference compound. The specific method was as follows:
0.1 M K3PO4 (pH 7.4) buffer and 3×NADPH stock solution (6 mM, 5 mg/mL) were configured and preheated in a 37°C water bath; configuration of the test compounds and the control compounds (spiking solution): adding 5 μL of the compound stock solution (10 nM) into 95 μL of acetonitrile; configuration of 1.5 μM spiking solution in microsomes (0.75 mg/mL) : adding 1.5 μL of spiking solution and 18.75 μL of liver microsomal solution (20 mg/mL) into 479.75 μL of K3PO4 buffer; adding 30 μL of spiking solution in microsomes into the multiwell plate, and incubating at 37°C for 5 min; adding 15 μL of NADPH storage solution into each well to start to react, and timing; adding 150 μL of acetonitrile solution containing IS at 0 min, 5 min, 15 min, 30 min, and 45 min, respectively, stopping the reaction; oscillating for 10 min, and then centrifuging at 6,000 rpm for 15 min; taking 80 μL of supernatant from each well for LC/MS detection, and calculating T1/2. The test results are shown in FIG. 19.

Fig. 19 shows the elimination of the compounds in the in vitro mouse or human liver microsomal environment, which was measured by the elimination half-life T1/2. In vitro liver microsomal stability experiments of UMI-77 showed that UMI-77 was unstable in the liver microsomal environment and would be eliminated rapidly, while compounds I-1 and I-2 demonstrated superior liver microsomal stability.
Note: Mice and human liver microsomes used in the experiments were purchased from Xenotech.

**Example 6 Alleviation of inflammatory bowel disease**

In IBD models, DSS-induced enteritis is the most commonly used mouse disease model. DSS is a polyanionic derivative of dextran, formed by the esterification reaction of dextran and chlorosulfonic acid, with the molecular formula $(C_6H_7Na_3O_{14}S_3)n$, molecular weights ranging from 36,000 to 50,000, and a sulfur content of typically 17%-20%. Current studies have suggested that DSS increases intestinal permeability, destroys the intestinal mucosal barrier, upregulates cytokines (tumor necrosis factor, interleukin, interferon, IL-10 and IL-12), activates pathways (NF-κB pathway and TRPV1 pathway), and dysregulates intestinal flora. It has also been reported that DSS induction can lead to upregulation of intracellular reactive oxygen species. Acute colitis modeling is one of the commonly used models of colitis, which is ideal model for studying the pathogenesis of UC and evaluating the efficacy of drugs because of its simplicity in preparation, high success rate, and similarity to human UC lesions.

(1) Model preparation

Eight-week-old C57BL/6J male mice were randomly divided into six groups, of which, G1 blank control group (8 mice), G2 positive control group (intraperitoneal injection of infliximab 10 mg /kg, 8 mice), G3 group (intraperitoneal injection of small-molecule drug TJ01-013, 5 mg/kg, 12 mice), G4 group (intraperitoneal injection of small-molecule drug TJ01-013, 10 mg/kg, 12 mice) and G5 group (intraperitoneal injection of small-molecule drug TJ01-013, 10 mg/kg, 12 mice). The configuration method of intraperitoneal injection drug was 5% DMSO + 30% PEG400 + 65% ddH2O, the small-molecule compound was first dissolved in 5% DMSO, and then 30% PEG400 and 65% H2O were added sequentially, after 2 weeks of acclimatization, 2% DSS solution was given as drinking water for 6 days, and then was replaced with normal drinking water. During the experiment, the mice were injected intraperitoneally with the corresponding concentration of small-molecule compound every day; and the mice were recorded daily for weight change, fecal shape and blood in stool.

During the experiment, the fecal shape and blood in stool were scored, and the disease activity index was calculated on day 3th, day 6th, and day 8th.

As shown in Figs. 20, 21, during the DSS induction period, the enteritis symptoms of mice in the intraperitoneally injected small molecule compound TJ01-013 group improved to a certain extent, which was mainly reflected in the slowing down of the weight loss of the mice and the reduction of the disease activity index.

(2) Observation of colorectal morphology and measurement of colorectal length

At the end of the experiment, after the mice were put to death with $CO_2$, all the intestinal segments from the cecum to the anus were cut, photographed and measured for length. As shown in FIG. 22, the colorectal length of mice in the small molecule compound TJ01-013 group was significantly greater than that of the model control group.

(3) HE staining analysis of colorectal tissue

The middle about 1 cm intestinal segment was taken into the embedding box and immersed in 4% paraformaldehyde, fixed and immersed for 72h to prepare pathological specimens for HE staining, and the histopathological results were scored; tissue embedding, sectioning and HE staining were performed as follows:

(a) Tissue embedding

Ethanol dehydration: the tissues were dehydrated step by step with different concentrations of ethanol solution 75%, 85%, 95%, 100%, 100%, 40min each time; transparency: the tissues were sequentially immersed in three xylene jars, 30min each; wax dipping: the tissues were sequentially immersed in three wax jars, the first one for 1h, the second one for 1.5h, and the third one for 2h; embedding: liquid wax was poured into a mold box, then put the wax-impregnated tissue blocks flat on the bottom, noting that the direction of the cut surface is facing down, the embedding frame was removed after the wax solidified, and the wax block was trimmed after completely cooling and hardening, the wax at the periphery of the tissue was retained moderately for sectioning.

(b) Preparation of sections

Fixing the pre-cooled wax block on the wax slicer so that the cut surface of the wax block is parallel to the knife edge, and the knife is usually tilted at 15 degrees, turning the wheel advancer to adjust the slice thickness to 3μm, and cutting the section with uniform thickness. The left hand held the brush, the right hand rotated the slicer handle, the section was gently held up with the brush after bringing out, and then the wax section was gently picked up with tweezers, and put into the spreading box with the front side, and the water temperature was about 40°C. The section was retrieved after spreading. Attaching the sections, one end of the slide was held with the left hand, and vertically inserted into the water to attach the section, tweezers were held by the right hand to assist in pushing section to attach to two-thirds of the slide. After attaching, the section was put in the air to dry slightly, then put on the 60°C baking machine for 1h, and then put in the oven for 2h.

(c) Wax section dewaxing

The wax section was successively put into xylene I for 15 minutes-xylene II for 15 minutes-anhydrous ethanol I for 10 minutes-anhydrous ethanol II for 10 minutes-95% alcohol for 10 minutes -85% alcohol for 10 minutes to dewax in gradient.

(d) HE staining

The wax section was stained with hematoxylin for 5-10 minutes, rinsed with tap water, differentiated with 1% hydrochloric acid in alcohol for a few seconds, rinsed with tap water, and then returned to blue in saturated aqueous lithium carbonate for 1 minutes, washed with running water for a few seconds, and stained with eosin stain for a few seconds, rinsed with running water.

(e) Dewatering and sealing and image acquisition

The wax section was successively put into 75% ethanol for 2min -85% ethanol for 2min - anhydrous ethanol for 2min - anhydrous ethanol for 2min - xylene for 2min for transparency, and then the section was taken out from xylene and sealed with neutral gum. Microscopic examination was performed and images were captured and analyzed.

The results are shown in FIG. 23, tissue section staining showed that the colorectal tissue morphology of mice in the small molecule compound TJ01-013 treatment group was more intact and the inflammatory infiltration was reduced compared with the model control group. Histopathological scores also showed that small molecule compound TJ01-013 treatment significantly reduced the pathological changes of inflammatory bowel inflammation and the tissue morphology was more intact.

(4) Colorectal tissue q-PCR to detect the transcript levels of inflammatory factors

The remaining intestinal segments were washed with pre-cooled saline using a 50mL syringe, and after washing out the remaining liquid in the intestinal lumen, the tissues were cut into pieces with small scissors and mixed evenly, wrapped in tin foil and then rapidly frozen in liquid nitrogen, and all the operations were controlled to be completed within 10 minutes after the death of the mice. The tissues were lysed by Trizol, and RNA was extracted according to the instructions of Trizol; the obtained RNA was subjected to reverse transcription reaction with the Reverse Transcription Kit from ABI company; SYBR Green method was used to detect the inflammatory factors such as Cxcl1, G-CSF, IL6, IL-1β, S100A8, and TNF-α in the reversed transcription products, and the housekeeping gene Actin was used as an internal reference, and calculated and analyzed by 2-ΔΔCt method.

As shown in FIG. 24, the results of real-time quantitative PCR showed that the expression levels of inflammatory factors Cxcl1, G-CSF, IL6, IL-1β, S100A8 and TNF-α were significantly reduced in the colorectal tissues of the mice in the small molecule compound TJ01-013 treatment group compared with those in the model control group.

**Example 7 Mitigation of acute lung injury**

Lipopolysaccharide (LPS) is the main bioactive component of the cell wall of Gram-negative bacteria, and has been widely used to induce an acute lung injury model, which is similar to the pathological characteristics of human acute lung injury. The model was prepared according to the following methods:

(1) Acute lung injury

Male mice C57BL/6J aged 6-8 weeks and weighing 18-22 g were selected for the study. After fixing the mice, 50 μl of PBS was injected into the airways of the mice under direct laryngoscopic vision to form a control group, and 5 mg/kg of LPS (E.coil) was dissolved in 50 μl of PBS and dripped to form a model group. The experiments were divided into five groups, each having 6 mice, namely, NS group (control group, airway drip of 50 μl PBS), LPS group (model control group, airway drip of 50 μl LPS), LPS+10 mg/kg group (airway drip of 50 μl LPS and given 10 mg/kg TJ01-013 treatment), LPS+20 mg/kg group (airway drip of 50 μ l LPS and given 20 mg/kg TJ01-013 treatment), LPS+30 mg/kg group (airway drip of 50 μl LPS and given 30 mg/kg TJ01-013 treatment), LPS+Sivelestat group (Positive control group, airway drip of 50 μl LPS and given 5 mg/kg Sivelestat treatment). All groups were administered once by intraperitoneal injection in a volume of 200 μl 1h and 7h after airway drip, and the mice were euthanized 24h later, bronchoalveolar lavage fluid (BALF) was taken, and the levels of inflammatory factors in the bronchoalveolar lavage fluid were detected and the changes in the transcriptional levels of tissue-associated inflammatory factors were observed.

(2) taking and observing of Bronchoalveolar lavage fluid (BALF)

Use chloral hydrate intraperitoneal injection to make mice die of rapid overdose anesthesia, place mice in supine position on the dissection table, fix the limbs, disinfect the skin of the neck of the mice with 75% ethanol, incise the skin along the midline of the neck, bluntly separate the subcutaneous connective tissues and muscles with hemostatic forceps, expose the trachea, separate connective tissues on both sides of the trachea and between the trachea and the esophagus, and free the trachea. Two surgical sutures about 20 cm long were threaded between the trachea and esophagus. A 24G intravenous cannula needle was inserted at a 30-degree angle from the connective tissue membrane between the thyroid cartilage and the cricoid cartilage, and the intravenous cannula needle was delivered in the direction of the tracheal eminence. A surgical suture placed between the tracheo-esophageal was ligated at the entry of the cannula into the trachea and at the distal end of the cannula, respectively, and tied as tightly as possible to secure the cannula and trachea. 0.8 ml of PBS was aspirated with a 1 ml syringe, and the syringe was connected to the inlet end of the intravenous cannula needle, and PBS was slowly injected into the trachea, stayed for 30 seconds and then was withdrawn back, and a milky white frothy liquid withdrawn back was visible, and the lavage was repeated three times, and the liquid was centrifuged at 1500 rpm/min for 10 min, and the supernatant was used for cytokine assay, and the cell precipitates were coated after resuspending with PBS for Giemsa staining and sorting and counting.

As shown in FIG. 25, the total number of cells increased sharply after LPS treatment and decreased after TJ01-013 treatment, and the total number of cells decreased to a better extent in TJ01-013 20 mg/kg treatment than in the

positive control Sivelestat sodium. Sorting and counting the alveolar lavage fluid showed that TJ01-013 treatment had no significant effect on the number of macrophages, however, it was able to significantly reduce the number of neutrophils, indicating that TJ01-013 was able to alleviate the increase in the level of inflammation caused by acute lung injury.

(3) Measurement of inflammatory factors

The levels of inflammatory factors (inflammatory factors including IL-6, CXCL1, CXCL2, etc.) in BALF supernatant were detected according to the instructions of the ELISA test kit.

As shown in FIG. 26, the levels of inflammatory factors IL-6, CXCL1, and CXCL2 in the alveolar lavage fluid after LPS treatment were significantly increased compared with the control group, and the levels of inflammatory factors associated with the TJ01-013 treatment were significantly decreased in a concentration-dependent manner. The positive control Sivelestat only significantly decreased CXCL1 levels, and had no significant effect on IL-6 and CXCL2 levels. This further indicates that TJ01-013 is able to alleviate the increase in inflammation levels caused by acute lung injury.

(4) A small piece of lung tissue was took and ground with liquid nitrogen, and added into the lysis solution containing β-mercaptoethanol to lyse the cells, the RNA of the relevant lung tissues was extracted according to the instructions of the RNA extraction kit, and the concentration and quality of RNA was detected, 1 μg of RNA was took to formulate into the corresponding reaction system and then incubated in the 37 °C for 15min, 85 °C for 5s, followed by placed on ice, and reversely transcribed into cDNA. The obtained cDNA was amplified on a 96-well PCR plate using a LightCycler 480 system, and the gene level was determined by using glyceraldehyde 3-phosphate dehydrogenase (GAPDH) as an internal standard.

As shown in FIG. 27, the transcript levels of tissue inflammatory factors IL-6, CXCL1, and CXCL2 were all significantly increased after LPS treatment compared with the control group, and the transcript levels of related inflammatory factors were all significantly decreased after TJ01-013 treatment in a concentration-dependent manner. Positive control Sivelestat only significantly decreased CXCL1 levels, and had no significant effect on IL-6 and CXCL2 levels. This indicates that TJ01-013 is able to alleviate the increase in inflammatory transcript levels caused by acute lung injury.

**Example 8 Increasing survival in acute lung injury**

The model was constructed as follows, male mice C57 aged 6-8 weeks and weighing 18-22 g were anesthetized and fixed, and then 20 mg/kg LPS (E.coil) was dissolved in 50 μl of PBS and dripped under direct laryngoscopic vision to form a model group. The experiment was divided into three groups, each having six mice, LPS group (model control group, airway drip of 50 μl LPS), LPS+20 mg/kg group (airway drip of 50 μl LPS, and given 20 mg/kg of TJ01-013 treatment), LPS+Sivelestat group (positive control group, airway drip of 50 μl LPS and given 5 mg/kg of Sivelestat treatment). The mode of administration was intraperitoneal injection, the volume of administration was 5ml/kg, the frequency of administration was once a day, and the biological deaths and changes in body weights of the mice were observed and recorded every day, and the mice were regarded as physiologically dead if their body weights decreased by 20% or more compared with that before the administration of LPS. Survival curves were plotted based on deaths observed up to 9 days after administration.

As shown in FIG. 28, the survival rate in the LPS group decreased by about 70%, and the 20 mg/kg TJ01-13 treatment significantly elevated the survival rate, indicating that TJ01-13 can significantly increase the survival rate of acute lung injury.

**Example 9 Alleviation of UUO-induced fibrosis**

The UUO model is currently the most widely used experimental animal model for studying renal tubulointerstitial fibrosis. By ligating a unilateral ureter to cause obstruction of the renal drainage system, leading to acute alterations in renal function and chronic damage to renal structure, it simulates renal interstitial injury caused by ureteral obstruction, which is common in clinical practice. The modeling was performed as follows:

(1) On the first day of the test, mice were given 50 mg/kg sodium pentobarbital anesthesia, after confirming that the mice were under anesthesia and preparing and disinfecting the skin, an incision of about 1 cm at 1 cm left and below of the costovertebral angle was made, the left side of the kidney was fully exposed and the ureter was sought downward along the renal hilum, and the ureter was ligated with a 6-0 nylon thread, and after ligation, the muscle and the skin layers were sutured layer by layer. In the sham group, only the kidney was exposed, but it was not ligated, and sutured directly. The experiments were divided into a sham group (Sham, oral gavage of vehicle), a model control group (vehicle, oral gavage of vehicle), a TJ01-013 10 mg/kg treatment group (oral gavage of 10 mg/kg TJ01-013) and a TJ01-013 20 mg/kg treatment group (oral gavage of 20 mg/kg TJ01-013), and the vehicle used is 0.5% CMC-Na,

administered at a dose of 10 ml/kg, and the frequency of administration was once a day. The drug was administered continuously for 7 days after UUO surgery, at the endpoint of the test, immediately after the mice were put to death, saline was perfused through the heart until the blood in the kidneys was completely drained and the kidneys were transformed from a dark red color to khaki. The operated kidney on the side was collected, the kidney was cut through the short axis of the kidney, and the kidney tissue samples were stored separately according to the requirements of the test.

(2) Renal histopathology

Kidney tissues were fixed with 4% paraformaldehyde for 48 hours, paraffin-embedded, sectioned at a thickness of 4 μm, and Masson stained. Specific experimental procedures were performed as described previously, and after sectioning and scanning the images, the results were analyzed semi-quantitatively using Image J software.

As shown in FIG. 29, the degree of fibrosis in the kidney tissue samples was identified by Masson trichrome staining, with collagen fibers appearing blue. Stained images of the renal cortex and renal medulla were randomly collected from each mouse sample separately, and the proportion of area occupied by the blue area, i.e., Collagen volume fraction%, was analyzed and quantified by ImageJ software. As shown, UUO modeling resulted in significant tubular dilatation, tubular cell compression, and concomitant significant renal interstitial fibrosis compared to the sham group. UUO modeling significantly increased the degree of interstitial fibrosis in the renal cortical tissues (Sham group: 0.28% vs. model-control group: 4.67%, $p<0.05$); whereas, compared to the model control group (UUO+Vehicle), the 10 mg/kg TJ01-013 intervention had no significant effect (5.58% vs. model control: 4.67%), 30 mg/kg TJ01-013 significantly reduced the degree of interstitial fibrosis in the renal cortex (0.61% vs. model control: 4.67%, $p<0.05$). Similar to the trend of the renal cortex, UUO modeling also caused a certain degree of fibrosis in the renal medulla, and TJ01-013 also had a reducing effect on fibrosis. In conclusion TJ01-013 treatment significantly alleviated renal fibrosis caused by UUO.

(3) Fibrosis-related gene and protein expression

Total RNA was extracted from kidney tissue samples by Tissue RNA Rapid Extraction Kit, and the concentration and purity of RNA were determined by micro-ultraviolet spectrophotometer, and then operated according to the instruction steps of HiScript® II 1st Strand cDNA Synthesis Kit, the cDNA was reversely transcribed with 1 μg of total amount of cDNA, followed by a 15ul amplification system, with 5 μL of cDNA, 7.5 μL of 2×SYBR Premix Ex TaqTM, 0.3 μL of each of forward and reverse primers, and the rest was supplemented with double-distilled water. The conditions of the Bio-Rad reaction were 95 °C for 5 min, denaturation at 95 °C for 10 seconds, annealing/extension at 60 °C for 30 seconds for 40 cycles, and 65 °C for 15 min. The expression of the target gene was calculated by $2\text{-}\Delta\Delta Ct$ relative quantification. The Ct value of the target gene was first corrected by the internal reference gene (β-actin), and then compared with the control group to derive the relative expression level.

As shown in FIG. 30, the mRNA levels of three key indexes of renal fibrosis, i.e., α-smooth muscle actin (α-SMA, corresponding gene: Acta2), fibronectin (Fn1) and type I collagen (Col1a1) in renal tissue homogenates, were detected by fluorescence quantitative PCR. The results showed that compared with the sham group, UUO modeling induced the expression of fibrosis-related genes, and the mRNA levels of the fibrosis key genes, Acta2, Fn1 and Collal, were significantly increased; compared to UUO+Vehicle, the TJ01-013 intervention significantly reduced the expression of the Fn1 gene, and there was a certain tendency to inhibit the Col1a1 gene as well.

(4) Fibrosis-related protein expression

Kidney tissues were added with RIPA lysis buffer (with protease inhibitor) and then ground into homogenate, incubated on ice for 30 min, centrifuged at 12000 g for 10 min, and the supernatant was taken and added with an equal volume of 2×Loading buffer, heated at 100°C for 10 min, and then centrifuged with 10%-12% SDS-PAGE gels, and then transferred to PVPV membrane using the BioRad Semi-Dry Transfer System 0.2A, and the membrane was closed in PBST buffer containing 5% (w/v) skimmed milk for 1 h, and was incubated in PBST containing 5% (w/v) BSA at 4°C overnight with the corresponding antibodies, including fibronectin (FN), collagen I, α-smooth muscle acin (α-SMA) in the PBST containing 5% (w/v) BSA, and fibrosis-related protein expression was detected by HRP-labeled secondary antibody and chemiluminescent reagent after PBST washing. Quantitative analysis of gray scale was performed using ImageJ 1.53a.

As shown in FIG. 31, UUO modeling caused a significant increase in the levels of three key fibrosis proteins (FN, collagen I, and α-SMA) compared to the sham group, and the TJ01-013 treatment significantly reduced the protein levels of fibronectin FN and collagen type I (collagen I) compared to the UUO+Vehicle group.

(5) Mitochondrial morphology analysis of renal tissues

Renal tubules are the main site of injury in the UUO model and an important pathogenetic basis leading to renal interstitial fibrosis. Transmission electron microscopy was utilized to observe the subcellular structure of renal tubular epithelial cells and assess the mitochondrial morphology to further determine the therapeutic effect of TJ01-013. Three mouse kidney samples from each of the four groups were collected for electron microscopic observation, the renal cortex was excised at a size of about 1 cubic millimeter and fixed in 2.5% glutaraldehyde at 4°C overnight; the next day, rinsed with PBS for 10 minutes three times; and fixed 1% in osmium acid for 1 hour, and rinsed with double-distilled water for 10 minutes three times; stained with 2% uranyl acetate for 30 min; subsequently dehydrated with 50%, 70%, 90%, and 100% ethanol, 100% acetone, completely embedded overnight, and ultrathin sections (Leica UC7) were stained with uranyl acetate and lead nitrate for 30 min, respectively. It was observed and shot with a transmission electron microscopy, and the mitochondrial morphology of tubular epithelial cells was observed with 4,000x and 10,000x.

As shown in FIG. 32, normal tubular epithelial cells have a structurally clear brush border and a large number of dense, elongated mitochondria in cell; UUO modeling resulted in tubular dilatation, tubular epithelial cells being shortened by extrusion, and the brush border being disappeared; the mitochondria morphology were swollen, irregular or nearly rounded, and reduced in density; the morphology of TJ01-013 intervened renal tubular epithelial cells and mitochondria were in between the former two, while more autophagic vesicles with double-layer or multi-layer membrane structure could be seen in the cell.

The above experimental results showed that TJ01-013 was able to improve mitochondrial swelling and homeostatic imbalance of tubular epithelial cells in the UUO model, and improve renal interstitial fibrosis.

### Example 10 Increasing Survival in Septicemia

A constructed preclinical sepsis model is a LPS-induced sepsis in mice. Injection of purified LPS into mice by intraperitoneal route or intravenous route can lead to activation of the innate immune system and simulate fulminant Gram-negative bacterial infections, the specific modeling method is as follows: 20-25g C57 mice were selected, and 20mg/kg LPS was injected intraperitoneally according to the body weight (the LPS was dissolved with PBS), and the experiment was divided into three groups, a model control group (LPS), a positive control treatment group (prednisone, 10mg/kg), TJ01-013 treatment group (TJ01-013, 10mg/kg), the mode of administration was intraperitoneal injection, the volume of administration was 5ml/kg, the frequency of administration was once a day, and the biological deaths and changes in body weights of the mice were observed and recorded every day, and the mice were regarded as physiologically dead if their body weights decreased by 20% or more compared with that before the administration of LPS. Survival curves were plotted based on deaths observed up to 8 days after administration.

As shown in FIG. 33, the survival rate of sepsis due to LPS treatment decreased to about 20% on 8th day, the survival rate of hormone prednisone treatment could be maintained at 100%, and the survival rate of TJ01-013 treatment was maintained at 80%, indicating that TJ01-013 treatment significantly increased the survival rate of sepsis.

### Example 11 Reduction of prostate hyperplasia levels

Androgen-induced benign prostatic hyperplasia in rats has been used in many research fields. The specific model was constructed as follows: 180g-200g male SD rats were given 30 mg/kg androgen intramuscularly every other day, and 15 consecutive androgen injections were given for modeling, and the experiments were divided into four groups, a negative control group (Sham), a model control group (Control), TJ01-013 treatment group (TJ01-013, 30 mg/kg), Finasteride treatment group (Finasteride, 10 mg/kg), and TJ01-013+Finasteride treatment group (13+F). The drug was administered at the same time as the androgen modeling, the mode of administration was by oral gavage, the volume of the administration was 10 ml/kg, the frequency of administration was daily, and the administration was ended on the 30th day, the rats were weighed and euthanized, and the prostate and urethra of the rats were taken and weighed, and the organ coefficients (weight of the prostate gland/weight of the rats) were finally calculated.

As shown in FIG. 34, the prostate weight was significantly increased in the model group (Control) compared to the negative control group (Sham), the positive drug Finasteride treatment reduced the prostate weight, and the TJ01-013+Finasteride co-administration treatment further reduced the prostate weight. In terms of the organ coefficient of prostate, as shown in FIG. 35, the organ coefficient of prostate was significantly increased in the model group (Control) compared to the negative control group (Sham), the Finasteride treatment reduced the organ coefficient of prostate, but there was no significant difference, and TJ01-013+Finasteride co-administration treatment significantly reduced the organ coefficient of prostate. Meanwhile, we weighed the weight of urethra and bladder separately, and the results were shown in Fig. 36, the weight of urethra and bladder was significantly increased in the model group (Control) compared with the negative control group (Sham), and there was a tendency of decreasing its weight in Finasteride treatment, and the TJ01-013+Finasteride co-administration treatment significantly reduced the weight of urethra and bladder. In summary, TJ01-013 can be

combined with prostate enlargement drugs to further reduce the weight and volume of the prostate.

**Example 12 Relief of myocardial infarction**

The heart, as the largest energy-consuming organ in the human body, has mitochondria as its main source of energy. Cardiomyocytes are among the cells with the largest number of mitochondria, accounting for 40-60% of the total volume. Under human physiological conditions, cardiomyocyte mitochondria provide energy for normal contraction and metabolism of cardiomyocytes, thus maintaining cardiomyocyte homeostasis.

(1) Model preparation and a mode of administration

180g-200g SD rats were anesthetized by chloral hydrate after fasting for 12h, the hair from the mandible to the upper part of the abdominal cavity and the ventral surfaces of the limbs was removed cleanly, and they were fixed on the surgical table, firstly, a small incision of about 1.5cm was made in the neck with a scalpel, and then the subcutaneous muscle layer was bluntly separated with surgical scissors to expose the trachea, and then the tracheal tube was intubated and connected to the animal respiratory machine. After sterilizing with iodophor, the skin was cut on the left side of the chest between the 3rd and 4th ribs, and the muscles were bluntly separated to expose the ribs. The 3rd rib was cut with surgical scissors, hemostatic forceps were used to clamp and break the cut rib, and an eyelid opener was placed to peel the pericardium. After the pericardium was cut, a cotton swab moistened with saline was used to gently pivot the heart to locate the left auricle and coronary arteries. A threaded suture needle was clamped with a microscopic needle holder, and passed through the myocardium from right to left at 2-3 mm below the left auricle to ligate the coronary artery. At the end of the ligation, the organs in the thoracic cavity were recovered with saline-soaked cotton swabs, and figure-of-eight sutures were applied to the third and fourth ribs. After the rib layer suture was completed, subcutaneous muscle layering suture was performed, and the skin was finally sutured. After dialing out the tracheal tube, the rats were closely observed whether they had spontaneous respiration or not, and if there was no respiration they should be immediately pressed on the chest cavity for cardiopulmonary resuscitation. The electrocardiograph was connected to measure the postoperative electrocardiogram. ST-segment elevation was seen in successful infarction modeling, and grouped according to the ECG data, which was mainly divided into five groups, namely, the sham group (Sham), the model control group (MI), the TJ01-013 administration group (30 mg/kg), the Entresto administration group (Entresto, 10 mg/kg), and the TJ01-013+Entrosto co-administration group. The drug was administered orally by gavage in a volume of 10 ml/kg, and the frequency of administration was once a day for 4 weeks, and then the rats were anesthetized for cardiac ultrasound.

(2) Echocardiographic examination

The rats were anesthetized by intraperitoneal injection of sodium pentobarbital 40mg/kg, and the limbs were fixed on a wooden board in supine position, and the board was tilted to the left at 30 degrees. The chest was shaved and a II lead ECG was connected. A ULTRAMARK 9 ultrasonic diagnostic instrument from ATL, USA was used with a wide bandwidth line array probe with a frequency of 10 MHz-5 MHz. The Doppler detection frequency was 6.0 MHz, the length of the probe was 38 mm, the gain of the instrument was fixedly set as 50 dB in the experiment, and the depth of the image was adjusted to 2.5 cm. The probe was placed on the left side of the sternum at 10-30 degrees from the midline of the sternum to display the parasternal left ventricular long-axis view, and the probe was rotated clockwise by 90 degrees to display the left ventricular view. Doppler flow measurements of the mitral and aortic valves were performed in the parasternal left ventricular long-axis view and the pulmonary artery long-axis view. Ultrasound measurement indexes included left ventricular end-diastolic internal diameter (LVDd), end-systolic internal diameters (LVDs), left ventricular ejection fraction EF, left ventricular short-axis shortening rate FS, and infarct size.

As shown in FIG. 37, the infarct size increased sharply in the model control group compared with the sham group, and the TJ01-013 treatment and the positive drug Entresto treatment reduced the infarct size, and the combined treatment of TJ01-013 and Entresto further reduced the infarct size. LVDd refers to the left ventricular end-diastolic diameter, which is an important index of cardiac color ultrasound, and it can reflect whether there is any abnormality in the size of the left ventricle. As shown in FIG. 38, the left ventricular end-diastolic diameter (LVDd) increased significantly in the model control group compared with the sham group, and the TJ01-013 treatment and the positive drug Entresto treatment had no significant effect on the left ventricular end-diastolic diameter (LVDd), but the combined treatment of TJ01-013 and Entresto could significantly reduce the left ventricular end-diastolic diameter. LVDs represent the left ventricular end-systolic diameter, which is mainly used to reflect the contractile function of the left ventricle. As shown in FIG. 39, the end-systolic diameter (LVDs) of the model control group increased significantly compared with those of the sham group, and theTJ01-013 treatment and the positive drug Entresto treatment did not have a significant effect on the end-systolic diameters (LVDs), but the combined treatment of TJ01-013 and Entresto was able to significantly reduce the end-systolic internal diameters. EF refers to the ejection fraction of the heart, which represents the quality of the cardiac function, and is

an important index to determine the reduction of cardiac function. As shown in FIG. 40, EF of left ventricular ejection fraction was significantly reduced in the model control group compared with the sham group, and TJ01-013 treatment significantly increased the left ventricular ejection fraction in the model group, and the positive drug Entresto treatment did not have a significant effect on the left ventricular ejection fraction, but the combined treatment of TJ01-013 and Entresto was able to further increase the left ventricular ejection fraction. Left ventricular short-axis fractional shortening FS is one of the parameters of left ventricular systolic function index. As shown in FIG. 41, FS was significantly reduced in the model control group compared to the sham group, and TJ01-013 treatment significantly increased FS in the model group, and positive drug Entresto treatment had no significant effect on FS, but the combined treatment of TJ01-013 and Entresto was able to further increase the left ventricular short-axis fractional shortening.

**Example 13 Alleviation of cognitive dysfunction in APP/PS1 mice**

Clinical symptoms of Alzheimer's disease (AD) are mainly full-blown dementia manifestations such as memory impairment, aphasia, apraxia, agnosia, visuospatial skill impairment, executive dysfunction, and other comprehensive dementia manifestations. Pathologic manifestations are mainly brain tissue atrophy, neurofibrillary tangles, senile plaques, and massive amyloid deposits. In addition to the Aβ toxicity hypothesis, abnormal Tau protein metabolism, etc., mitochondrial dysfunction is also one of the possible pathogenic mechanisms of AD. APP/PS1 double transgenic mice can express mutant human presenilin 1 (PS1-dE9) and amyloid precursor protein (APPswe), the expression of both genes are initiated by mouse prion protein promoter, and these mutations can lead to early-onset Alzheimer's disease, a classic model for studying Alzheimer's disease. Its specific modeling and administration methods are described below:

(1) Model construction

Male transgenic APP/PS1 mice were purchased from the Model Animal Research Center of Nanjing University, and after being raised to 12 weeks old, the APP/PS1 mice were divided into three groups: normal C57 group (WT), model control group (APP/PS1+Vehicle), and TJ01-013 treatment group (APP/PS1+TJ01-013). The mode of administration was by oral gavage, normal group and model control group were administered vehicle by oral gavage, the concentration of TJ01-013 was 30mg/kg, the gavage volume was 5ml/kg, the frequency of administration was once a day, and the administration period was 3 months.

(2) Water maze experiment

The water maze device is a circular pool (140cm in diameter) filled with dissolved titanium dioxide water, keeping the water temperature at 22°C. A platform with a diameter of 12 cm was placed at a fixed position 1 cm below the water surface, and the mice were trained in four quadrants every day for five consecutive days. Each quadrant lasted 60 seconds or until the mice found the platform. If the mice did not find the platform within the specified time, the experimenter was required to guide the mice to stand on the platform for 20 s. All parameters were recorded by a video tracking system.

As shown in FIG. 42, compared with wild-type control mice WT, APP/PS1 mice took significantly longer time to find the platform and showed obvious cognitive dysfunction. 30 mg/kg TJ01-013 treatment significantly reduced the time to find the platform and improved the learning and spatial memory ability of APP/PS1 mice. As shown in FIG. 43, the number of crossing over the platform in the APP/PS1 mice vehicle group was significantly less than that of the wild-type mice WT group, and the number of crossing over the platform in mice in the TJ01-013 treatment group was significantly larger than that of the APP/PS1 mice Vehicle group, which further verified that TJ01-013 improved the cognitive dysfunction of APP/PS1 mice.

(3) Immunohistochemistry

Mouse heart perfusion for brain extraction: APP/PS1 mice and control C57BL/6J mice were anesthetized, and after they became unconscious, the limbs were fixed on a dissection table in a fuming hood, and the abdominal skin was cut with scissors after alcohol disinfection to expose the liver. The diaphragm was quickly cut, and the heart was exposed by turning upward after clamping the xiphoid with hemostatic forceps. Prepared PBS was injected into the left ventricle, noting that the direction was toward the aorta. The peristaltic pump was turned on after opening the right auricle and the blood was drained at a low rate at first. When the heart stopped beating or the fluid pumped at the right auricle was clarified and colorless, the mouse liver can be seen to turn grayish white. The fur was cut open along the midline of the mouse hindbrain with scissors, and the skull was exposed, then forceps were inserted from the criss-cross of the mouse's brain shell, and scissors were used to assist in peeling off the skull, and then the intact whole brain was removed. The fresh mouse whole brain was halved and placed in a 5 mL centrifuge tube, and 4 mL of 4% paraformaldehyde fixative was added to fix for 24 h, sections were made for antigenic repair, incubated for 30 min with dropwise adding of BSA at a concentration of 3-5%, and

stained overnight with polyclonal rabbit anti-GFAP antibody (1:100) and mouse anti-β-amyloid protein 1-16 antibody (1:100), and after the sections were washed in PBS three times, the corresponding fluorescent secondary antibody was added dropwise to cover the tissue, and the sections were incubated in dark for 1 h, the slides were washed and stained with DAPI staining solution added dropwisely for 10 min, and the sections were sealed with resin sealer, and finally imaged with Cytation3.

GFAP is a marker of astrocyte activation, and the proliferation of astrocytes is a pathological marker of structural lesions in the central nervous system. As shown in FIG. 44, model control mice showed astrocyte activation in the hippocampal region along with a large number of Aβ plaques. The astrocyte activation in the hippocampal region of mice in the TJ01-013-treated group was significantly reduced, along with a significant decrease in the number and size of Aβ plaques, indicating that the treatment of TJ01-013 was able to reduce the astrocyte activation in APP/PS1 mice and the number and size of Aβ plaques.

(4) Elisa assay for the amount of Aβ in brain tissue homogenates

Mouse brain tissue homogenates were prepared according to the brain tissue homogenization method described in the Life Technologies ELISA Technical Guide. About 100 mg of brain tissue was weighed and brought into a centrifuge tube, then added about 8 times the volume of grinding buffer (5 M guanidine hydrochloride, 50 mM Tris) and transfered to a homogenizer to grind evenly, and put on an oscillator at room temperature to mix by shaking for 3-4 hours, then diluted 10-fold with PBS containing protease inhibitors, and centrifuged for 20 min at 16,000 g at 4°C, the supernatant was extracted and put on ice for reserve. Elisa assay was performed according to the Aβ assay kit instructions. As shown in FIG. 45, the amount of Aβ in the brain tissues of APP/PS1 mice in the model control group was significantly increased, and the amount of Aβ in the brain tissues of the mice after TJ01-013 treatment was significantly decreased, indicating that TJ01-013 treatment was able to alleviate the cognitive dysfunction in the APP/PS1 mice by reducing the Aβ content.

## Example 14 Protective effect against hearing damage caused by cisplatin

Cisplatin-associated ototoxicity is a key side effect of chemotherapy and can lead to irreversible hearing loss. The cisplatin-induced hearing impairment model in mice is an important model for simulating hearing loss caused by ototoxic drugs. Its model construction method is as follows:

(1) Model construction

Select 8-week-old C57 male mice weighing 22-25 g, according to the previous studies and the previous laboratory exploration, mice were induced to have hearing damage by 4 mg/kg of cisplatin, and a certain amount of cisplatin was dissolved in 0.9% saline of the model control group, and then intraperitoneally injected into the mice for four consecutive days, with the volume of the intraperitoneal injection of 5 ml/kg, and then intraperitoneally injected into the mice for four consecutive days with the volume of 0.9% saline for recovery and alleviation, the injection was prescribed at the same time point every day, and no treatment was done on the 9th day to conduct hearing test or dissect the mice for subsequent experiments. TJ01-013 was administered by gavage every day, and the vehicle used for the gavage was 0.5% CMC-Na, and the dosage of the drug administered was 30 mg/kg in a volume of 5 ml/kg, for a total of 8 days.

(2) Auditory function test in mice: Auditory Brainstem Response (ABR) test

Pentobarbital sodium was prepared in 0.9% saline and injected intraperitoneally into the mice for audiometry at a dose of 50 mg/kg, so that the mice were deeply anesthetized and placed on a 37°C heating pad to maintain their body temperature, and then transferred to a soundproof room. Three electrodes for audiometry were inserted into the corresponding positions of the mice: the active electrode was inserted into the cranial apex of both ears, the grounding electrode was inserted into the dorsal subcutaneous area, and the reference electrode was inserted into the root of the ear. The electrode index light as well as the resistive frequency were checked, and one ear was ensured to be about 10 cm away from the loudspeaker, and was stimulated with click and different frequencies of sound (4 kHz, 8 kHz, 12 kHz, 16 kHz, 24 kHz, and 32 kHz), which were detected starting from 90 dB, and then gradually reduced by 10 dB or 5 dB each time, and the TDT system was used to collect the signal and record the response, and the final hearing thresholds were recorded using the SigGen32 software, Multiple mice were audiometrically tested in sequence, and since the ABR is a test of the auditory nerve, each mouse could be audiometrically tested multiple times, but it was ensured that the mice remained under anesthesia during the audiometric test, and the data were finally statistically and analytically analyzed.

As shown in FIG. 46, ABR audiometry revealed that the model control group Cis had significantly higher hearing thresholds than the negative control group, i.e., wild-type rats, at all the measured frequencies (4 kHz, 8 kHz, 16 kHz, 24 kHz, 32 kHz), and the TJ01-013 administration treatment significantly decreased the hearing thresholds at the measured

frequencies (4 kHz, 8 kHz, 16 kHz, 24 kHz, 32 kHz) hearing thresholds. This indicates that TJ01-013 has a protective effect against hearing damage in mice.

(3) Immunofluorescence staining of cochlear basement membrane

The mice were sacrificed by neck dissection, and the cochlea was removed from mice by neck dissection, and processed under the anatomical microscope to expose the round window and the oval window, the cochlear apex was perforate, and the round window, the oval window and the cochlear apex were perfused with 4% PFA solution, and then the whole cochlea was put into 4% PFA solution for fixation, and was shaken overnight at 4 °C. The cochlea was washed in PBS, and then decalcified in 10% EDTA for 2h-4h, with a reasonable stop time according to the hardness of the tissues. After the tissue was softened, the basement membrane was removed under the dissecting microscope and segmented, and the apical gyrus, middle gyrus, and basal gyrus were cut with a microtome and put into a U-shaped 96-well plate and labeled. The plate was permeabilized with 0.2% Triton-X100 solution for 15 min and closed with 10% goat serum for 1 h. They were incubated with primary antibody overnight, washed with PBS and incubated with fluorescent secondary antibody protected from light at room temperature for 1 h. The basement membrane was removed slightly with forceps and spread on a slide with a drop of sealer (containing DAPI staining solution), with the front side of the hair cells facing upwards, then was pressed by the coverslip, and the periphery of the coverslip was sealed with nail polish. Finally, photographs were taken using a fluorescence microscope.

As shown in FIG. 47, using Myo7a as a marker for hair cells, cisplatin-treated mice in the model group showed loss of hair cells s in fluorescence spreads at the apical gyrus, middle gyrus, and base gyrus position, and TJ01-013 treatment significantly reduced the loss of hair cells. These results indicate that TJ01-013 treatment has a protective effect on hearing damage caused by cisplatin.

**Example 15 Alleviation of Aging-Related Diseases and Cell Damage**

In addition to natural physiological aging, the living organism also has pathological aging, which mainly refers to aging accelerated by physiological changes due to various factors that lead to the emergence of a disease or a chronic disease, and whose representative pathological aging diseases include progeria, which is a rare, autosomal dominantly inherited childhood disease. Bone mesenchymal stromal cells (BMSCs) are a kind of pluripotent stem cells with self-renewal and multidirectional differentiation ability, which can be senesced by successive generations in vitro. The specific experimental methods are as follows:

(1) Experiment of mouse bone marrow mesenchymal stem cells

Extraction of bone marrow mesenchymal stem cells: C57 mice weighing about 25g were selected, and after anesthesia with 30mg/kg chloral hydrate, the femur and tibia were removed and put into a Petri dish, one end of the femur and tibia were cut off to fully expose the bone marrow cavity, and a 1ml syringe was used to extract the DMEM/F12 medium, and the tip of the needle was pricked into the bone marrow cavity to rinse the bone marrow, and a 15ml centrifuge tube was connected underneath to make the rinsed liquid flow into the centrifuge tube, and the rinsing was repeated several times until the bone marrow cavity was rinsed clean. The rinsed suspension was blown repeatedly with a pipette to make the cells in the rinsed liquid as single-cell as possible, and the supernatant was transferred to another tube after standing still for a few minutes, and the cell suspension was continued to blow to make the cells well dispersed. The obtained cell suspension was centrifuged at 1000 rpm for 5 min, and after removing the residual supernatant, 1 ml of DMEM/F12 medium was added, and after repeated blowing, the CD45-Sca-1+PDGFR$\alpha$+ high-purity primary mouse bMSC cell line was sorted out by flow cytometric sorting, and the sorted cells were dispensed into culture flasks. The cells were observed under the microscope and placed in the incubator at 37°C, 5% CO2 to incubate, and the solution was replaced for 72 h, the drug administration treatment was performed at the first passaging, and then 10 $\mu$M TJ01-013 was given to each passaging thereafter, labeled as Con-Y at the 2nd generation, and labeled as Con-O at the 9th generation, and the drug administration treatment groups were given until the 9th generation, and all the cells were placed under the microscope for observing the morphology of the cells, and the proportion of the SA-$\beta$-gal+ cells was counted. The levels of the DNA damage marker $\gamma$-H2AX and the heterochromatin modification H3K9me3 after TJ01-013 treatment were detected by immunofluorescence technique (procedure described previously).

Senescence-associated $\beta$-galactosidase (SA-$\beta$-gal) is one of the most widely used senescence markers. The proportion it occupies in cells can reflect the degree of cell aging. As shown in FIG. 48, the percentage of SA-$\beta$-gal+ cells in mouse bone marrow mesenchymal stem cells Con-O was significantly increased compared with that in Con-Y, and the percentage of SA-$\beta$-gal+ cells in the 9th generation after treatment with TJ01-013 was significantly decreased compared with that in Con-O, indicating that TJ01-013 inhibited the senescence process of mouse mesenchymal stem cells. As shown in FIG. 49, the level of the DNA damage marker $\gamma$-H2AX was increased in aged cells (Con-O), and its level was

significantly reduced after TJ01-013 treatment. Also as shown in FIG. 50, heterochromatin-modified H3K9me3 was expressed at a lower level in Con-O cells, and TJ01-013 treatment restored its expression level to some extent.

(2) Experiment of normal human derived mesenchymal stem cells

Mesenchymal stem cells(MSC) differentiated from normal human pluripotent stem cells (iPSC) were taken for culture, and the experiment was divided into three groups: young cells cultured for 6 generations, labeled as Con-Y; senescent cells cultured for 16 generations, labeled as Con-O; and the administration group, to which 10 μM TJ01-013 was added during the process of culture, similarly cultured up to the 16th generation, and labeled as Con-O + TJ01-013, and after the end of the treatment, all cells were placed under the microscope to observe the morphology of the cells, and the percentage of SA-β-gal+ cells was count . The levels of the DNA damage marker γ-H2AX and the heterochromatin modification H3K9me3 after TJ01-013 treatment were detected by immunofluorescence technique (procedure described previously).

As shown in FIG. 51, the percentage of SA-β-gal+ cells in CMC cells was significantly increased in Con-O compared to Con-Y, and the percentage of SA-β-gal-positive cells was significantly decreased in TJ01-013 treated cells compared to Con-O, suggesting that TJ01-013 inhibited the senescence process of human-derived CMC cells. As shown in FIG. 52, the level of DNA damage marker γ-H2AX was increased in aged cells (Con-O), and its level was significantly reduced in TJ01-013 treatment. Also as shown in FIG. 53, heterochromatin-modified H3K9me3 was expressed at a lower level in Con-O cells, and TJ01-013 treatment restored its expression level to some extent.

(3) Experiment of patient with Hutchinson-Gilford Progeria Syndrome (HGPS) derived mesenchymal stem cells

Mesenchymal stem cells HGPS-MSC differentiated from pluripotent stem cells (iPSC) of patients with Hutchinson-Gilford Progeria Syndrome (HGPS) were taken for culture, and the experiment was divided into three groups, namely, young cells cultured for 7 generations, labeled as Con-Y; senescent cells cultured for 12 generations, labeled as Con-O; and the administration group to which 10 μM of TJ01-013 was added during the process of culture, similarly cultured up to the 12th generation, labeled as Con-O+TJ01-013; the drug administration group to which 10 μM positive drug Ionafarnib was added during the process of culture, similarly cultured up to the 12th generation, and labeled as Con-O+ Ionafarnib; after the end of the treatment, all the cells were placed under the microscope to observe the morphology of the cells, and the proportion of SA-β-gal+ cells was counted. The levels of DNA damage marker γ-H2AX and heterochromatin modification H3K9me3 after TJ01-013 treatment and Ionafarnib treatment were detected by immunofluorescence technique (the specific procedure was described previously).

As shown in FIG. 54, the percentage of SA-β-gal+ cells in HGPS-CMC cells was significantly increased in Con-O compared to Con-Y, and the percentage of SA-β-gal+ cells was significantly decreased in TJ01-013 treatment and Ionafarnib treatment compared to Con-O, indicating that both TJ01-013 and Ionafarnib inhibited senescence process of HGPS- CMC cells. As shown in FIG. 55, the level of DNA damage marker γ-H2AX was increased in aged cells (Con-O), and the level of DNA damage was significantly reduced in TJ01-013 treatment and Ionafarnib treatment. Also as shown in FIG. 56, heterochromatin-modified H3K9me3 was expressed at a lower level in Con-O cells, and both TJ01-013 treatment and Ionafarnib treatment restored its expression level to some extent. In summary, TJ01-013 was able to delay the aging process of normal human and HGPS patients to a certain extent.

(4) Experiment of human epidermal melanocytes HEMn-LP and NHEM-Neo

The skin is a high-turnover organ whose constant renewal depends on the rapid proliferation of its progenitor cells. The energy requirements of these cells are met by mitochondrial respiration, which is an ATP-producing process driven by a series of protein complexes. However reactive oxygen is inevitably produced during respiration and can damage macromolecules and cellular structures if not quenched by antioxidant systems, and oxidative damage induced by mitochondrial ROS production has been identified as the molecular basis for a variety of pathophysiological states. Mitochondria are the primary organelle affected during age and UV-induced skin aging, which manifests as a direct consequence of mitochondrial dysfunction. Recent studies have shown that mitochondria have innate roles in the maintenance of skin homeostasis and pigmentation, and that these roles are compromised when essential mitochondrial functions are impaired. A number of common and rare skin disorders have mitochondrial involvement, including the cutaneous manifestations of primary mitochondrial disorders and congenital skin disorders caused by damaged mitochondria. There is growing evidence of a strong link between mitochondria and skin health. Skin aging is caused by a combination of intrinsic and extrinsic factors that ultimately compromise the structural integrity and physiological function of the skin. Recent studies have shown that melanocytes expressing markers of aging accumulate in human skin, which is significantly associated with increased facial wrinkles, increased perceived age, and age-related elastin formation in the dermis. The specific experimental solution is as follows:

Human epidermal melanocytes HEMn-LP and NHEM-Neo were taken for culture, and the experiment was divided into four groups: normal cultured human epidermal melanocytes, labeled as Control; the administration group to which 10 μM TJ0113 was added during the process of normal culture, labeled as Control+TJ0113; normal cultured human epidermal melanocytes were irradiated with 10 Gy X-rays to induce melanocyte senescence, labeled as senescence; the administration group to which 10 μM TJ0113 was added during the process of normal cultured human epidermal melanocytes being irradiated with 10Gy X-rays to induce melanocyte senescence, labeled as senescence+TJ0113; the period of all experimental treatments and the administrations was 12 days, and at the end of the treatments, all the cells were placed under the microscope to observe the morphology of the cells and count the percentage of SA-β-gal positive cells. The levels of DNA damage marker γ-H2AX and heterochromatin modification H3K9me3 were detected by immunofluorescence technique (specific procedure as described previously) after TJ0113 treatment.

**Example 16 Treatment of depression-like disorders**

Depression, also known as depressive disorder, is characterized by significant and long-lasting depressed state of mind as the main clinical feature. The CUMS animal model of depression is a chronic unpredictable animal stress model, which simulates part of the stressor, the external material environmental stimulus, and will cause dysfunctions in the animal organism through different stress stimuli, leading to occurrence of the psychiatric disease depression. After the chronic unpredictable stress, the animals showed the phenomenon of reduced locomotor ability, lack of pleasure and elevated plasma corticosterone, which is similar to human depression, and can more realistically simulate the depression patient's certain etiology and symptoms, and is a more ideal and reliable animal model of depression, and its model construction method is as follows: 8-week-old C57BL/6J male mice were selected, and the model was constructed in accordance with the following methods:

(1) Fasting food and water: depriving the mice of food and drinking water and maintaining for 24 hours.
(2) Plantar electrical stimulation: placing mice in a fear-memory device and stimulating intermittently with a current of 0.3 mA for 2 s at 30-s intervals for 500 s.
(3) wetting the bedding: pouring water into the dry bedding until the bedding was completely wet, and placing the mice in the wet bedding for 24 hours.
(4) Cage tilting: Placing the mouse cage against the wall with a tilting angle of 45°, and feeding the mice for 24 hours.
(5) Behavioral restraint: Placing the mice in a behavioral restraint device to ensure that they are unable to carry out normal activities in a confined space and maintaining for 1 hour. During this period, carefully observing the status of each mouse.
(6) Basket shaking: Placing the mice in an empty basket and shaking the basket drastically, with the criterion that the mice in the basket are unable to stand firm and sway with the shaking of the basket, for 1 hour.
(7) Olfactory stimulation: Placing anise and other spices in the mice's baskets as a means of olfactory stimulation.

The above seven experiments were conducted randomly during a week as a way to achieve unpredictability, with 4 weeks of continuous stimulation.

| Day1 | Day2 | Day3 | Day4 | Day5 | Day6 | Day7 |
|---|---|---|---|---|---|---|
| Fasting food and water | Cage tilting | Basket shaking | Olfactory stimulatio n | Behavioral restraint | Plantar electrical stimulation | wetting the bedding |
| Day8 | Day9 | Day10 | Day11 | Day12 | Day13 | Day14 |
| Cage tilting | Basket shaking | wetting the bedding | Fasting food and water | Plantar electrical stimulation | Behavioral restraint | Olfactory stimulation |
| Day15 | Day16 | Day17 | Day18 | Day19 | Day20 | Day21 |
| wetting the bedding | Behavioral restraint | Fasting food and water | Basket shaking | Cage tilting | Plantar electrical stimulation | Behavioral restraint |

(continued)

| Day22 | Day23 | Day24 | Day25 | Day26 | Day27 | Day28 |
|---|---|---|---|---|---|---|
| Behavioral restraint | Fasting food and water | Olfactory stimulation | wetting the bedding | Basket shaking | Cage tilting | Plantar electrical stimulation |

At the end of the modeling, an open field test experiment was conducted to screen the modeled animals and group the successful modeled animals into five groups: normal group (Control), model control group (Model), positive drug treatment group (Fluoxetine), TJ0113 10 mg/kg treatment group (10 mg/kg), TJ0113 30 mg/kg treatment group (10 mg/kg), TJ0113 30 mg/kg treatment group (30 mg/kg). There were 10 mice in each group, and the administration treatment was administered by gavage, the normal and model control groups were given vehicle treatment, the vehicle was 0.5% CMC-Na, the positive drug fluoxetine hydrochloride was solubilized in saline and administered at a dose of 30 mg/kg, and the TJ0113 vehicle was 0.5% CMC-Na, and administered at a dose of 10 mg/kg and 30 mg/kg, respectively. 4 weeks after the end of the administration, behavioral tests were performed.

Sugar water preference test

(1) Adaptive feeding: Adaptive feeding with 1% sucrose water and plain water for 2d before the start of the test, at 1d, interchanging the sugar water and plain water to avoid positional differences for the animals, and fasting food and water for 1d after adaption;
(2) Formal test: after fasting food and water, 1% sucrose water and plain water were given to the animals (the initial mass of sugar water and plain water is known), and weighing the remaining mass of sugar water/plain water of each animal after 24h, and calculating the consumption rates of sugar water A and plain water B;
(3) Data analysis: sugar water preference rate = (A/A+B) $\times$ 100%.

Hanging Tail Test

(1) adapting to the environment: placing the animals in the test environment for 60 min to adapt to the environment before the test;
(2) Formal test: fixing the rear 1/3 of the mouse tail with tape and suspending on the stand of the experimental apparatus, with the head 15 cm from the bottom;
(3) starting timing camera, and stopping after 6 min, and recording the immobility time of the mice within 4 min;
(4) At the end of the test, using 75 % alcohol to wipe the bottom and inner wall of the chamber.

Open field test

(1) adapting to the environment: placing the animals in the test environment for 60 min to adapt to the environment before the test;
(2) Formal test: placing the animals to be tested in the middle of the open field, and using the ANY-Maze software to collect the data, the test time is 10 min, and the feces and urine must be removed at the end of each round and disinfected and wiped dry with 75% alcohol;
(3) Analysis of results: total moved distance and average speed during the activity, time spent in the center area and time spent in the corner area.

After the data were summarized and counted, they were analyzed using SPSS data statistics software, and the images were plotted according to the results of SPSS analysis using Graph Pad software.

As shown in FIG. 57, in the sugar water preference experiment, both TJ0113 30 mg/kg treatment and positive drug Fluoxetine treatment significantly increased the sugar water preference of mice. As shown in FIG. 58, in the tail-hanging experiment, the model control mice remained immobile in despair for a significantly longer time, and both TJ0113 30 mg/kg treatment and the positive drug Fluoxetine treatment significantly decreased the time that the mice were in

despair. As shown in FIG. 59, in the open field experiment, the distance moved by the mice in the model control group was significantly reduced compared with that of the mice in the normal group, and both TJ0113 30mg/kg treatment and the positive drug Fluoxetine treatment significantly increased the distance moved by the model mice. As shown in FIG. 60, in the open field experiment, the time that the model control mice were in the edge area of the open field was significantly increased compared with the normal mice, and both TJ0113 30mg/kg treatment and the positive drug Fluoxetine treatment significantly decreased the time that the model mice were in the edge area of the open field. As shown in FIG. 61, in the open field experiment, the average movement speed of the model control mice was significantly reduced compared with the normal mice, and the TJ0113 10mg/kg and 30mg/kg treatments and the positive drug Fluoxetine treatment significantly increased the average movement speed of the model mice. The results of the above behavioral experiments indicated that TJ0113 was able to significantly alleviate the depression-like phenotype of the mice.

**Example 17 Treatment of chronic obstructive pulmonary disease**

After 5-week-old male SD rats were adaptively fed for 1 week, they were randomly grouped according to their body weights into five groups, i.e., a normal control group (normal, n=10), a model control group (Control, n=10), a TJ01-013 7.5 mg/kg treatment group (7.5 mg/kg, n=10), a TJ01-013 15 mg/kg treatment group ( 15mg/kg, n=10), TJ01-013 30mg/kg treatment group (30mg/kg, n=10). The model was replicated using the intra-airway injection of lipopolysaccharide method combined with passive smoking method, which is a recognized model for chronic obstructive pulmonary disease. Except for the normal control group, all groups were anesthetized with sodium pentobarbital via intraperitoneal injection on the first day of modeling, the rats were fixed on the rat platform, the neck fur was removed and disinfected, an incision of about 0.5 cm was made in the middle of the neck, the subcutaneous tissues and fascia were separated, and the trachea of the rats was exposed to the field of view, the head of the rats was elevated at 45 degrees, and the 1 ml syringe was selected to suck up the concentration of 1 mg/mL of 0.2 ml of LPS, and slowly inject into the rat airway, after the injection was completed, the rat was lifted by the tail, slowly rotated and shaken, so that the drug was uniformly distributed in the rat airway and both lungs, and finally the surgical wound was sutured. On the second day after surgery, the rats were subjected to passive smoking, in which cigarettes were inserted into the smoking box and smoked once a day for 1 hour, consuming about 10 cigarettes, and the smoking box was knocked once at intervals of about 5 min during smoking, so as to avoid the phenomenon of non-uniform concentration of smoke dispersal in the airway and the lung tissues. The normal control group was subjected to sham operation and was not smoked. From the second day of modeling, the normal control group and the model control group were treated with vehicle (0.5% CMC-Na) by gavage, and the TJ01-013 treatment group was treated with the corresponding concentration of TJ01-013 in a volume of 10 ml/kg for a total of 3 weeks, and blood was collected from the posterior bulbous vein of the rat after administration, and the blood was centrifuged at 6,000 rpm for 10 min after standing still at room temperature for 30 min, then the upper layer of serum was taken, and the levels of TNF$\alpha$ and CXCL1 in serum were detected by enzyme-linked immunosorbent assay (ELISA).

As shown in FIG. 62, the serum levels of TNF$\alpha$ and CXCL1 in the model group were significantly higher than those in the normal control group, and both 15 mg/kg and 30 mg/kg TJ01-013 treatments were able to reduce the serum CXCL1 level; the 30 mg/kg TJ01-013 treatment also reduced the serum TNF$\alpha$ level, and the difference was significant. The results showed that TJ01-013 treatment could reduce the rise of inflammatory indexes caused by COPD and alleviate the symptoms.

**Example 18 Treatment of Epilepsy, Alleviation of Epilepsy-Induced Behavioral and Cognitive Disorders and Reduction of Inflammatory Factor Levels**

Clean grade 8-10 week old male ICR mice were selected for the preparation of a chronic epilepsy mouse model. They were randomly divided into four groups: normal control group (Control, 0.5% CMC-Na treatment by oral gavage, n=8), model group (Model, 0.5% CMC-Na treatment by oral gavage, n=8), TJ01-013 treatment group (TJ01-013, 30mg/kg TJ01-013 by oral gavage, n=8), I-14-1 treatment group (I-14-1, 30 mg/kg I-14-1 by oral gavage, n=8). Starting from the first day of the experiment, at 10-11 am every day, mice in all groups except the normal control group were injected intraperitoneally with PTZ 40 mg/kg and the normal control group was injected with the corresponding volume of saline for a total of 21 days. The drug administration treatment was started right after the start of modeling, and the drug was administered once a day for a total of 21 days, and the corresponding tests were performed at the end of the drug administration.

(1) Animal behavioral observation

Epileptic seizure grading standard is based on Racine grading: Grade 0, no convulsive response, manifested as normal behavior state; Grade I, facial muscle spasm, manifested as rhythmic chewing, blinking, moving whiskers, etc.; Grade II, neck muscle spasm, manifested as head nodding with or without tail erecting; Grade III, forelimb lifting, clonic or

rhythmic twitching; Grade IV, hindlimb tonic or standing tonic; Grade V, generalized clonic, standing tonic and falling down; loss of balance. The animal behavior was observed after the experiment, and the results are shown in FIG. 63, the normal control group did not have a seizure status, seizures in the model group were in Grade V status, TJ0113 and I-14-1 administration treatment are able to reduce the seizure grade, and TJ0113 has a significant difference in reducing seizures, indicating that the TJ01-013 treatment effect is significantly better than I-14-1.

(2) Platform jumping experiment

The platform jumping experiment is an experimental method designed to utilize the habit of experimental mice to jump down from a high place immediately to explore the surrounding environment. The experimental mice are placed on a high platform, and at the moment of jumping down, they are given electrical stimulation. Then in order to avoid the electric stimulus, the animals will not jump off the platform, while the mice that are not good at learning and memorizing will maintain their habit and jump off the platform. At the end of each round of the experiment, 75% alcohol were sprayed for wiping to avoid odor interference between the mice. The experiment was conducted after the administration, and the experimental device was a cubic box with a (10 cm$\times$10 cm) electric fence at the bottom and a height of 30 cm, with a platform of 3.2 cm in height and 4.2 cm in diameter inside. The experimental steps and procedures of this experiment were divided into: (1) The mice were put into the bottom of the experimental device, which was not energized at this time, so that they were free to explore and adapt to the environment for 10 s. (2) The mice were placed on the high platform inside the experimental device, and the time at which the mice jumped off the high platform was recorded as Initial latency, and were given electrical stimulation (50 Hz, 20 V, 5 s). (3) After 24 h, the mice were placed on the platform again, and the time at which they jumped off the platform was recorded as Step-down latency. The residence time of the mice on the high platform should not exceed 60 s, and the time exceeding 60 s was still recorded as 60 s. The results are shown in Fig. 64, the model group showed a significant decrease in the Step-down latency compared with the normal control group, indicating that cognitive dysfunction appeared in the model mice, and the TJ0113 and I-14-1 treatments were able to increase the Initial latency to a certain degree after the administration, and the TJ0113 had significant differences, indicating that the treatment effect of TJ01-013 was significantly better than that of I-14-1.

(3) Step through Dark Avoidance experiment

The step through dark avoidance experiment is an experimental method designed to utilize the tendency of mice to move around in dark environments more than in bright environments. The experimental mice are placed in a bright environment of a bright and dark environment connecting device, and then electrically stimulated when they enter the dark environment. Then in order to avoid the electric stimulus, they will move into the light environment, while mice that are not good at learning and memorizing will maintain their habit and enter the dark environment. At the end of each round of the experiment, 75% alcohol were sprayed for wiping to avoid odor interference between mice. This experiment was conducted at the end of the administration. The experimental steps and procedures of this experiment were divided into: (1) The mice were placed into the light chamber with their backs facing the dark chamber, the time at which the mice entered the dark chamber was recorded as Initial latency, and the door was closed, then they were given electrical stimulation (50 Hz, 20 V, 5 s). (2) After 24 h, the mice were put into the bright chamber again, and the time at which the mice entered the dark chamber was recorded as the Step-through latency. The results are shown in FIG. 65, the model group significantly reduced the Step-through latency compared to the normal control group, indicating that the model group of mice appeared cognitive dysfunction, TJ0113 and I-14-1 treatment were able to increase the latency to a certain extent after the administration, and TJ0113 had a significant difference, indicating that the therapeutic effect of TJ01-013 was significantly better than that of I-14-1.

(4) Detecting the levels of TNF-$\alpha$ and IL-1$\beta$ in serum by blood sampling after completion of the experiments in mice

24 h after the completion of the water maze experiment, cutting off the whiskers of the mice, removing the eyeballs for blood collection, collecting about 0.5 mL of blood into a centrifuge tube, standing still for 2 h, centrifuging (3000 rpm/min, 10 min), extracting the supernatant, and frozen storing for use. The levels of TNF-$\alpha$ and IL-1$\beta$ in serum were detected by Elisa method.

Principle of enzyme-linked immunosorbent assay (ELISA): Specimen, standard and HRP-labeled detection antibody were added sequentially to the coated micro-wells pre-coated with mouse TNF-$\alpha$ and IL-1$\beta$ capture antibody, and then incubating and washing thoroughly. The color was developed with the substrate TMB, which was converted to blue color by catalysis of peroxidase and finally to yellow color by acid. The absorbance (OD value) was measured at a wavelength of 450 nm using an enzyme meter and the sample concentration was calculated. The results are shown in FIG. 66, the levels of TNF-$\alpha$ and IL-1$\beta$ in the serum of the model group were significantly increased compared to the normal control group, indicating that the model caused the level of inflammation in the serum to increase. Both TJ0113 and I-14-1 were able to reduce the levels of TNF-$\alpha$ and IL-1$\beta$ in the serum of the model group to a certain degree after administration,

and the therapeutic effect of TJ01-013 was significantly better than that of I-14-1.

**Example 19 Relief of early-onset ovarian insufficiency and delaying the progression of ovarian insufficiency**

1) Mouse model of chemotherapy-induced POF

Infertility after premature ovarian failure is one of the most serious side effects of chemotherapy in young female cancer patients, but there is no effective treatment for gonadotoxic damage in the ovaries. Cyclophosphamide (Cy) is a commonly used chemotherapeutic agent, and induces severe ovarian damage and is a recognized risk for POF. Cyclophosphamide can induce primordial follicular failure.

Eight-week-old C57BL/6J mice (weighing about 20-25 g) were selected and adaptively fed for 7 d firstly, after which they were randomly divided into three groups according to their body weights, namely, the control group (Control, n=8), the model group (Cy, treated with the solvent 0.5% CMC-Na, n=8), and the TJ01-013 treatment group (Cy+TJ01-013, 30 mg/kg TJ01-013 by oral gavage, n=8). kg TJ01-013, n=8), I-14-1 treatment group (Cy+ I-14-1, 30 mg/kg I-14-1 by oral gavage, n=8). The model group and the TJ01-013 treatment group received a single intraperitoneal injection of cyclophosphamide (75 mg/kg, 200-300 μl), and the control group received a single intraperitoneal injection of an equal volume of saline. Samples were collected after 12 days of administration, and body weights, ovary weights, and levels of the serum hormones E2 and FSH of the mice were measured.

2) Determination of ovarian organ coefficient

The body weight and bilateral ovarian wet weight of each group of mice were weighed with an electronic balance and an analytical balance respectively, and the ovarian organ coefficient of each group was calculated according to the body weight of mice (g) and bilateral ovarian wet weight (mg). The formula is:

Testicular organ coefficient = bilateral ovarian wet weight (mg)/mouse body weight (g).

As shown in FIG. 67, there was no significant change in body weight of mice after chemotherapy and treatment for 14 days, but chemotherapy resulted in a significant decrease in ovarian weight, which was significantly elevated after TJ01-013 and I-14-1 treatments. Similarly, chemotherapy resulted in a significant decrease in ovarian coefficient (the ratio of ovarian weight to body weight), which was also significantly elevated after TJ01-013 and I-14-1 treatments, with TJ01-013 being more effective than I-14-1.

3) Serum hormone level Elisa assay

After the experiment reached the end point, blood was collected from the orbit, and the blood was centrifuged at 12,000g for 10 min after standing still at room temperature for 45 min, and the supernatant (serum) was taken, and the levels of E2, FSH and BDNF in serum were detected by ELISA.

As shown in FIG. 68, the serum FSH concentration significantly increased after chemotherapy modeling compared with the normal modeling group, and the serum FSH level significantly decreased after TJ01-013 and I-14-1 treatments. As shown in FIG. 69, serum E2 level decreased significantly after chemotherapy modeling and increased significantly after I-14-1 and TJ01-013 treatments, indicating that both TJ01-013 and I-14-1 treatments significantly improved the function of the ovaries in mice.

4) Ovarian tissue BDNF ELISA assay

Brain-derived neurotrophic factor (BDNF) is a widely studied neurotrophic factor, and now also known as ovarian endocrine factor, and a large number of evidence suggests that BDNF plays a role in ovarian follicle development. The mice were euthanized after reaching the end point of the experiment, and the levels of BDNF in the ovarian tissues were assayed by ELISA after the ovaries were extracted and ground.

As shown in FIG. 70, BDNF level in ovarian tissues was decreased significantly after chemotherapy modeling, and was increased significantly after TJ01-013 and I-14-1 treatments, indicating that TJ01-013 and I-14-1 treatments improved the function of the ovaries in mice.

The improvement of FSH concentration, serum E2 level and BDNF level by TJ0113 was better than by I-14-1, especially the advantage of effect on serum FSH concentration was more significant.

**Example 20: Treatment of naturally aging ovarian insufficiency**

1) Natural aging POF (NA-POF) modeling

The Natural Aging POF model (NA-POF) simulates conditions of progressive ovarian degeneration. Female C57BL/6J mice of 9-10 months of age were purchased and raised to 12 months old, and then the mice were randomly divided into three groups, namely, the control group (Control, 0.5% CMC-Na by oral gavage, n=8), the TJ01-013-treated group (TJ01-013, 30 mg/kg TJ01-013 by oral gavage, n=8), the I-14-1 treatment group (I-14-1,30 mg/kg I-14-1 by oral gavage, n=8), and were administered daily for 30 days. At the endpoint of the experiment, body weight, ovarian weight, ovarian organ coefficient, serum hormone E2 and FSH levels in mice were measured.

2) Determination of ovarian organ coefficient

The body weight and bilateral ovarian wet weight of each group of mice were weighed with an electronic balance and an analytical balance respectively, and the ovarian organ coefficient of each group of mice was calculated according to the body weight of the mice (g) and the wet weight(mg) of the bilateral ovaries. The formula is:

Ovarian organ coefficient = bilateral ovarian wet weight (mg)/mouse body weight (g).

The results are shown in Fig. 71, there was no significant change in the body weight of mice natural aging and treated with TJ01-013 and I-14-1, but the aging led to a significant decrease in the ovarian weight, which was significantly elevated after the TJ01-013 and I-14-1 treatments, and similarly, the aging led to a significant decrease in the ovarian coefficient, which was significantly elevated after the TJ01-013 treatment, and the I-14-1 treatment had an rising trend in the ovarian coefficient, although the ovarian coefficient did not rise significantly.

3) Serum hormone level Elisa assay.

After the experiment reached the end point, orbital blood collection was carried out, and the blood was centrifuged at 12000 g for 10 min after standing still at room temperature for 45 min, and the supernatant (serum) was taken, and the levels of E2 and FSH in serum were detected by ELISA.

As shown in FIG. 72, the serum FSH level was decreased significantly after treatment with TJ01-013 after aging. As shown in FIG. 73, serum E2 levels were increased significantly after treatment with TJ01-013 and I-14-1 after aging. The results indicate that both TJ01-013 and I-14-1 treatments were able to improve the decrease in ovarian function of mice caused by aging to a certain extent, with TJ0113 being more effective.

**Example 21 Treatment of lupus nephritis**

1) Model preparation and administration treatment

MRL/lpr mice are the most classical animal model for lupus and immune-related diseases. The MRL/lpr animal model eventually develops into a systemic lupus erythematosus-like disease characterized by generalized lymph node enlargement, skin lesions, alopecia areata, invasive arthritis, and immune-complex-depositing glomerulonephritis. Its onset is usually at 14-16 weeks of age. Considering that the pathogenicity of SLE is related to gender, and its incidence rate is about 1:9 in men and women, the animals used in this study are female MRL/lpr mice.

The 10-week-old female MRL/lpr mice were fed in an SPF environment with a light-dark cycle of 12 h and the temperature maintained at 24-26°C. The mice were randomly divided into three groups according to their body weights, namely, the model group (Control, n=8, 0.5% CMC-Na by oral gavage), TJ01-013 10mg/kg treatment group (10mg/kg, n=8, 10mg/kg TJ01-013 by oral gavage), TJ01-013 30mg/kg treatment group (30mg/kg, n=8, 30mg/kg TJ01-013 by oral gavage), the animals were adaptively fed for one week, and were administered every day starting from 11th week, and the animals were monitored for the concentration of randomized urinary proteins and urinary creatinine of the mice in each group every 2 weeks starting from week 11, the mouse urine protein to creatinine concentration ratio (uPCR) was calculated. The animals were administered for a total of 10 weeks, and blood was collected from the orbits at the end of administration and serum was collected. Serum creatinine, urea nitrogen, serum ANA and anti-dsDNA concentrations were detected.

2) Detection of urine protein and urine creatinine concentration

Urine protein and urinary creatinine levels were detected by utilizing Caumas Brilliant Blue staining. The results were shown in FIG. 74, the trend of uPCR in the continuous monitoring in the normal group and the TJ01-013-treated group

after 10 weeks of administration was basically the same, and both were significantly lower than that of the MRL/lpr model group, indicating that the TJ01-013 treatment was able to alleviate the occurrence of proteinuria.

3) Detection of Serum creatinine and urea nitrogen

Serum creatinine and urea nitrogen were detected by biochemical analyzer. The results are shown in FIG. 75. Serum creatinine and urea nitrogen levels of MRL/lpr model mice were significantly increased compared to normal mice, and creatinine and urea nitrogen levels were significantly decreased after TJ01-013 treatment compared to the model group, indicating that the TJ01-013 treatment was able to alleviate renal function significantly.

4) Detection of Serum ANA, anti-dsDNA concentration

Serum ANA and anti-dsDNA levels were detected by ELISA. The results are shown in FIG.s 76-77. Serum ANA and dsDNA levels of MRL/lpr model mice were significantly increased compared with those of normal mice, and the levels of ANA and dsDNA were significantly decreased compared with those of the model group after the treatment of TJ01-013, indicating that the TJ01-013 treatment was able to significantly reduce the level of systemic lupus erythematosus -specific antibodies.

5) Detection of inflammatory factor levels in kidney tissues

Kidney tissue was placed in a homogenizer and minced into pieces thoroughly with clean scissors; 400μl of lysate was added to the homogenizer, homogenized and then placed on ice, and the grinding was repeated several times on ice until the kidney tissue was ground as thoroughly as possible; after 30min of lysis, the lysate was transferred to a 1.5ml centrifuge tube with a pipette, and then centrifuged for 10min at 12,000rpm under the condition of 4°C, and the supernatant was taken and stored at -20°C. Inflammatory factors TNFα and IL-6 in kidney tissue proteins were detected using ELISA.

The results are shown in FIG. 78, in which the levels of TNFα and IL-6 in the kidney tissues of MRL/lpr model mice were significantly increased compared to normal mice, and the levels of TNFα and IL-6 were significantly reduced after TJ01-013 treatment compared to the model group, indicating that the TJ01-013 treatment was able to significantly reduce the level of inflammation in the kidney tissues.

**Example 22 Treatment of myocarditis**

(1) Model construction

8-week-old male SPF-grade Lewis rats were adaptively fed for one week to construct an autoimmune myocarditis model. The rats were divided into two groups, normal control group (normal rats, routine feeding) and model group, in which porcine cardiac myosin used to induce autoimmunity and Freund's adjuvant were fully mixed in 1:1 in the model group(purified porcine cardiac myosin was dissolved in PBS solution, with its concentration being controlled to 10g/L, under sterile conditions the solution was mixed with the complete Freund's adjuvant CFA in 1:1 equal volume and emulsified completely, so that it became a homogeneous emulsion), a total of 0.2 ml of the immunization mixture was injected subcutaneously into multiple points such as the bilateral groin and axilla of the rats. Normal control rats were immunized with myosin-free PBS and CFA emulsion. From the 14th day after immunization, the model groups were administrated in groups, and were divided into three groups: model group (model, treated with 0.5% CMC-Na by oral gavage, n=8) TJ01-013 treatment group (TJ01-013, 30mg/kg TJ01-013 by oral gavage, n=8), I-14-1 treatment group (I-14-1, 30mg/kg I -14-1 by oral gavage, n=8). A normal control group (Control, treated with 0.5% CMC-Na by oral gavage, n=8) was further provided, and the administration of the drug was started immediately after grouping, and the drug was administered for a total of 4 weeks. At the end of the administration, rats were weighed, anesthetized and blood was taken, and serum was extracted and the corresponding physiological indexes were detected. After euthanasia, the rats' hearts were stripped and weighed, and the organ coefficients of the hearts were calculated.

(2) Echocardiographic evaluation of cardiac function

After the end of administration, rats were weighed, anesthetized with 10% chloral hydrate via intraperitoneal injection, and then subjected to echocardiography in the supine and slightly left lateral position after chest hair removal, and the average value was taken from 3 repetitions. The detection indexes include left ventricular end-diastolic transverse diameter LVIDd, left ventricular end-systolic transverse diameter LVIDs, short-axis fractional shortening FS, and ejection fraction EF. The results are shown in FIG. 79. The LVIDd and LVIDs were significantly increased in the model group after

immunization modeling compared with the normal control group. Both TJ01-013 and IL-14-1 treatments were able to reduce the levels of LVIDd and LVIDs, and the treatment of TJ01-013 was significantly better than that of I-14-1. As shown in FIG. 80, EF and FS were significantly decreased in the model group after immunization modeling compared with the normal control group, and both TJ01-013 and IL-14-1 treatments were able to increase the levels of EF and FS, and the treatment of TJ01-013 was significantly better than that of I-14-1. As shown in FIG. 81, CK-MB levels were significantly increased in the model group after immunization modeling compared with the normal control group, both TJ01-013 and I-14-1 treatments were able to decrease CK-MB level, and the treatment of TJ01-013 was significantly better than that of I-14-1.

**Example 23 Treatment of stroke**

Stroke is a disease that seriously jeopardizes human health, and the global incidence of stroke is on the rise year by year, and it is also the disease with the highest disability rate in China, which brings a serious burden to society and economy. The post-stroke rehabilitation is a key and difficult problem that needs to be solved in clinical practice.

Stroke is a serious cerebrovascular disease and one of the main causes of long-term disability and premature death. The majority of stroke cases (about 80%) are ischemic strokes caused by vascular occlusion, i.e., cerebral ischemia, with arterial thrombosis as the main cause. At present, stroke is a disease that seriously jeopardizes human health, and the global incidence of stroke is on the rise year by year, and it is also the disease with the highest disability rate in China, which brings a serious burden to the society and the economy. The post-stroke rehabilitation is a key and difficult problem that needs to be solved urgently in clinical practice. The most effective treatment for ischemic stroke is to rapidly restore blood supply, but ischemia-reperfusion injury caused by revascularization can further aggravate brain injury and neuromotor dysfunction, and restoration of neurological function has become a key problem to be solved urgently.

(1) Model construction

The middle cerebral artery occlusion (MCAO) model is a focal cerebral ischemia model and the most widely used cerebral ischemia model. The pathogenesis of this model is similar to that of ischemic stroke, and there are the characteristics of easy operation and accurate control of reperfusion. The middle cerebral artery (MCA) involves the largest number of blood-supplying regions, and the obstruction mainly leads to cortical and striatal lesions, and the degree of infarction depends on the location and duration of the occlusion as well as the amount of collateral circulating blood in the MCA.

7-8 weeks-old SD male rats were selected and adaptively fed for one week to establish a middle artery occlusion model in rats. After inducing anesthesia in rats using a small animal anesthesia machine, the anesthesia was immediately switched to maintenance, the rats were fixed in the supine position on the operating table, the neck in the middle and the right side of the operative part were shaved, and an incision was made in the middle of the neck after disinfection with alcohol and iodine vapors, and the carotid artery was separated along the tissues under the microscope, to avoid damaging blood vessels and nerves and to reduce the amount of hemorrhage; the electrocoagulation pen was used to cut off small arteries in the paracarotid branch and ligate the external carotid artery, and an another loose knot was played for backup; arterial clips were used to clip the proximal end of internal carotid artery and common carotid artery; the external carotid artery was cut with surgical scissors in the 45° direction, the filament was inserted carefully, and the marking point reached the bifurcation of internal and external carotid arteries, which meant that the head of the filament has already reached the middle cerebral artery; the skin was sutured, the symptoms were observed when the animal waked up, and the neurological function was evaluated; the suture line was cut open two hours after the surgery, the filament was drawn out, and ligature the remnant of external carotid artery was ligated tightly. The skin was re-sutured and attention was paid to the postoperative care of the rats. After modeling, drug administration was started in groups, which were divided into three groups: model group (model, treated with 0.5% CMC-Na by oral gavage, n=8) TJ01-013 treatment group (TJ01-013, 30mg/kg TJ01-013 by oral gavage, n=8), and I-14-1 treatment group (I-14-1, 30mg/kg I-14-1 by oral gavage, n=8). 8). Another normal control group (Control, 0.5% CMC-Na treatment by oral gavage, n=8) was set up, with performing the corresponding surgical operation, but no carotid artery was ligated and no filament insertion was performed, and the normal control group was administered for a total of 8 weeks, and at the end of the administration, the cognitive function of the rats was examined by using the water maze.

Ischemic stroke leads to insufficient perfusion of brain tissue, which is prone to ischemia and hypoxia during surgery, and a large accumulation of calcium ions in the brain tissue can damage the brain tissue, leading to postoperative cognitive function decline.

(2) Water maze experiment

The night before the experiment begins, clean the laboratory and the water maze, the old water in the water maze

is drained, the animal excreta should be cleaned up, and the laboratory is ventilated and free of special odor. The water maze was filled with fresh water, and the height of the water surface should be about 2 cm higher than the platform, and the four groups of rats underwent the water maze acclimatization training for 5 days. The above training was divided into a 4-day localization navigation experiment and a 1-day spatial exploration experiment. The water maze device was a circular pool, which was divided into quadrants I, II, III and IV with four equidistant points on the wall, and a circular hidden platform was placed in the center of the fourth quadrant. References were around the pool and kept unchanged, the pool and the platform were black, and black ink was poured into the water and mixed thoroughly so that the rats could not see the circular platform. The temperature of the water was kept at 22~24°C and the light in the room was stabilized. For each group of rats, a water maze test was performed at the end of drug administration to examine the cognitive status of the experimental animals. Each time, rats were placed into the pool facing the wall from the three quadrants except for the platform quadrant, and if the rats did not find the hidden platform under the water surface of the target quadrant within 2min, the rats were guided to stay on the platform in the target quadrant for 15s. The four groups of rats underwent this training for 4 days, four times a day, two times in the morning and two times in the afternoon. At the end of each day, the experimenter thoroughly cleaned the water maze apparatus to prevent the odor of the rats in the water maze from affecting the results of the second day of the experiment. The time from when the rats were put into the water facing the pool wall to when they boarded the hidden platform in the target quadrant was recorded, which was defined as the evasion latency, and the average of the four results was taken. 60s was the time limit for each localization and navigation experiment, and if the rats didn't find the hidden platform in the target quadrant within the limited time, the experimenters guided the rats to the hidden platform, with the purpose of making the rats stay in the hidden platform for 15s to memorize it, and the escape latency of the rats in this experiment was recorded as 60s. A towel, hairdryer and other items were prepared for the experiment, and the rats were wiped with a towel and then dried with a hairdryer after each swimming session to prevent them from catching a cold. The experimental process was as quiet as possible to avoid interference. As shown in FIG. 82, the escape latency of the model group was significantly increased compared with the normal control group, and both TJ0113 and I-14-1 treatments were able to shorten the escape latency, indicating that TJ0113 and I-14-1 have therapeutic functions, and the therapeutic effect of TJ01-013 was significantly better than that of I-14-1.

After the above experiments, the platform was removed for space exploration experiments. The rats were place into the pool facing the pool wall from the middle position of the quadrant facing the circular hidden platform, and the rats swam in the pool for 60s. The number that the rats crossed the location of the circular platform in the center of the target quadrant within 60s was recorded as the number of crossing over the platform. As shown in FIG. 83, compared with the normal control group, the number of crossing over the platform was significantly reduced in rats in the model group, and the number of rats crossing the platform could be increased after TJ0113 and I-14-1 treatments, and TJ01-013 was able to significantly shorten the evasion latency, indicating that TJ0113 and I-14-1 had therapeutic functions, and the therapeutic effect of TJ01-013 was significantly better than that of I-14-1.

Determination of apoptosis rate of rat hippocampal neurons by flow cytometry method

After the end of drug administration and behavioral experiments, all groups of rats were put into death by intraperitoneal injection with chloral hydrate, the rats were decapitated rapidly and brains were removed, and the hippocampal tissues were rapidly seperated on ice. The hippocampal tissue was weighed in the required amount of about 1 g, and placed on a 100 mesh copper mesh, the surface connective tissue membrane was cut off, after adding buffer, the hippocampal tissue was cut into pieces and gently rubbed with tweezers to filter and prepare single-cell suspension, and then the cell suspension was secondarily filtered with 300 mesh nylon to filter out the large agglomerates, the collected cell suspensions was centrifuged in the centrifuge for 5 min, at 3000 r/min, with the centrifugal radius of 10cm and centrifugal force of 13000g, after discarding the supernatant, 500μl of 1X Buffer was added to prepare single-cell suspensions at a concentration of $1\times10^5$~$5\times10^5$/L, 5μl of phospholipid-binding protein V (Annexin V) was added firstly, and then 10μl of propidium iodide (PI) was added, and then the sample was incubated with protected from light for 5min after sufficient mixing, the procedure was carried out according to the instruction manual and the instructions, and with the practical experience. The fluorescence intensity of apoptotic hippocampal neurons was detected by flow cytometry, and the apoptosis rate of hippocampal neurons was calculated. As shown in FIG. 84, the apoptosis rate of rat hippocampal neurons in the model group was significantly increased compared to the normal control group, and both TJ0113 and I-14-1 treatments were able to significantly reduce the apoptosis rate of hippocampal neurons, indicating that TJ0113 and I-14-1 have therapeutic functions, and the therapeutic effect of TJ01-013 was significantly better than that of I-14-1.

**Example 24 Treatment of tinnitus**

7-week-old C57BL/6 male mice without tinnitus were screened for tinnitus-free mice using the acoustic startle (AS) response and gap pre-pulse inhibition of the acoustic startle (GPIAS). The mice were randomly divided into five groups, normal control group (normal, n=10), model control group (Control, n=10), TJ01-013 7.5 mg/kg treatment group (7.5 mg/kg, n=10), TJ01-013 15 mg/kg treatment group (15 mg/kg, n=10), and TJ01-013 30 mg/kg treatment group (30mg/kg, n=10). Except for the normal control group, all the other groups were injected intraperitoneally with 450 mg/kg

sodium salicylate to construct the model, in which the normal control group was injected intraperitoneally with the corresponding volume of saline, and the administration treatment was started on the day of modeling, in which the normal control group and the model control group were administrated vehicle (0.5% CMC-Na) by gavage, and the TJ01-013 treatment group was given the treatment of TJ01-013 with the corresponding concentration of TJ01-013 in a volume of 10 ml/kg, and the acoustic startle was measured again 3 hours after the last dose after 4 consecutive days of administration. Behavioral test, AS response paired with GPLAS response was the behavioral experiment to detect the presence of tinnitus. All mice were placed in a transparent box to adapt for 15 min in consecutive 3 days prior to the start of the experiment. The background noise given in the experiment was 8 and 16 kHz narrowband noise with a bandwidth of 1 kHz, respectively, to get an idea of the frequency range in which tinnitus occurs. To perform the GPIAS experiment, each mouse was placed in a transparent box located in a dark room, the transparent box was placed on a sensitive piezoelectric transducer that produces a voltage proportional to the magnitude of the response caused by the sound generated by the digital signal processor. The output of the platform is amplified, sampled, stored in a computer, and analyzed offline. The GPIAS consists of 100 tests with anterior gap (gap tests) and 100 tests without anterior gap (no gap tests), the anterior gap test and the no gap test uses a randomized paired method. The degree of tinnitus was calculated as the percentage of the ratio of the amplitude of the response caused by the stimulus with anterior gap to the amplitude of the response caused by the stimulus without anterior gap (Ratio=gap/no gap×100%).

As shown in FIG. 85, the AS response amplitude ratio of the tinnitus model group injected with sodium salicylate was significantly higher than that of the vehicle group at 8 kHz and 16 kHz noise exposure, indicating that the modeling was successful; the AS response amplitude ratios of the protected group with TJ0113 decreased significantly compared with that of the model group and showed a dose-dependence, indicating that TJ0113 could significantly improve tinnitus disease in mice.

**Lab Example 25 Treatment of Multiple Sclerosis**

The currently recognized pathogenesis of Multiple Sclerosis (MS) is that abnormal immune attack leads to damage of the blood-brain barrier, and a variety of inflammatory factors and immune cells enter the central nervous system and act on microglia and immune cells in the central nervous system, causing inflammatory reactions, resulting in myelin desheathing, neuronal death, and axonal damage. Current treatments for multiple sclerosis are mainly anti-inflammatory, but are only effective in the relapsing and remitting phases of the disease. Therefore, the development of therapeutic approaches for MS has become an urgent problem.

The CUP model can be used to simulate multiple sclerosis by causing mice to develop demyelination symptoms through the dietary addition of CUP. CUP is a copper chelator that is added to the animal's diet through dietary addition and ultimately causes oligodendrocytes to apoptosis, leading to demyelination.

1. Model construction

After one week of adaptive feeding in 7-8 weeks C57BL/6 female rats, they were randomly divided into four groups according to their body weights: the normal control group (Control, 0.5% CMC-Na treatment by oral gavage, n=8), the model group (model, 0.5% CMC-Na treatment by oral gavage, n=8), TJ01-013 treatment group (TJ01-013, 30 mg/kg TJ01-013 by oral gavage, n=8), I-14-1 treatment group (I-14-1, oral gavage 30 mg/kg I-14-1, n=8). The normal control group was fed with the basal diet throughout for 12 weeks, and the model group, TJ01-013 treatment group, and I-14-1 treatment group were fed with 0.2% CUP diet throughout. All groups were administered 5 ml/kg in a volume of 5 ml/kg for a total of 12 weeks starting 6 weeks after modeling.

2. Behavioral tests

(1) Elevated cross maze test

The elevated cross maze consisted of two open arms (35cm×6cm) and two closed arms (35cm×6cm×14cm), with the middle area (6cm×6cm) and a height of 70cm above the ground, and the mice were placed in the middle area facing the open arms, and were active for 5min in a 50cm×50cm open field prior to the start of the test. During the test, the activity time, the activity distance and the number of open arm entries in the mice were counted. Each mouse was wiped with 75% alcohol on the maze after the test. The experimental results are shown in FIG. 86. Compared with the control group, the number of the open arm entries in mice in the model group was significantly increased.

the number of open arm entries in mice was significantly reduced after TJ01-013 and I-14-1 treatment, and both were statistically different, and treatment of TJ01-013 was significantly better than that of I-14-1. As shown in Fig. 87, compared with the control group, the walking distance was significantly increased in mice in the model group, and the walking distance was reduced in mice after TJ01-013 and I-14-1 treatment, and treatment of TJ01-013 was significantly

better than that of I-14-1 and statistically different. As shown in FIG. 88, compared with the control group, the open arm time in mice in the model group was significantly increased, and the open arm time in mice was significantly decreased after TJ01-013 and I-14-1 treatments, and both of them were statistically different, and treatment of TJ01-013 was significantly better than that of I-14-1.

(2) Water maze test

The mice were placed in a basin with a diameter of 70cm and a height of 30cm, and the water temperature was about 22-24°C. The basin was divided into four quadrants, and a platform with a diameter of 5 cm was placed in the target quadrant, which was placed 1 cm below the surface of the water. The mice were acclimatized for 1 day before the official test and learned for 4 days, during learning, the mice entered the water in the direction of facing the wall of the basin, and were given 60s to search for the platform, or else were guided to learn for 30s on the platform, and the time that the mice found the platform during learning was recorded. The platform was withdrawn on the day of the test, and the number of crossing over the platform after entering from the opposite quadrant of the target quadrant in mice was recorded to determine the spatial learning and memory ability of the mice. As shown in FIG. 89, the number of crossing over the platform was significantly reduced in the model group compared to the normal group, and the number of crossing over the platform in mice was increased after TJ01-013 and I-14-1 treatments, with the treatment of TJ01-013 being significantly better than that of I-14-1 and statistically different.

It can be understood by those of ordinary skill in the art that each of the above embodiments is a specific embodiment for realizing the present invention, and that in practical application, various changes can be made thereto in form and detail without departing from the spirit and scope of the present invention.

## Claims

1. Uses of the compounds shown in the general formula (I) or of pharmaceutically acceptable salts, stereoisomers, solvates or prodrugs thereof, whererin the uses are for:

    (i) preparing a drug for preventing and/or treating diseases associated with kidney injury; and/or
    (ii) preventing and/or treating diseases associated with kidney injury; and/or
    (iii)preventing and/or treating diseases associated with mitochondrial dysfunction; and/or
    (iv)preparing a drug for preventing and/or treating diseases associated with mitochondrial dysfunction; and/or
    (v)preparing a cosmetic or medical instrument;

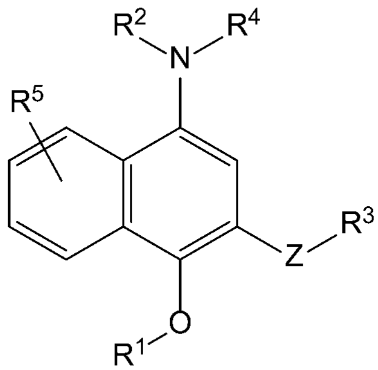

I

wherein Z is

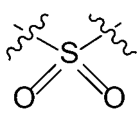 , 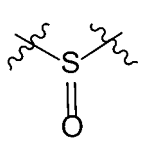 , 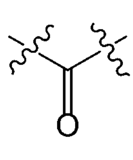 , 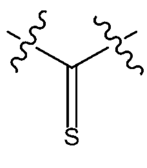

or a sulfur atom;

$R^1$ is hydrogen, $C_{1\sim6}$ alkyl,

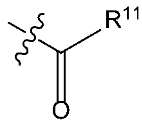 , 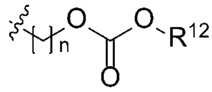 ,

wherein $R^{11}$ and $R^{12}$ are independently C1~6 alkyl or C1~6 cycloalkyl, respectively, and n is 1~4;
$R^2$ is hydrogen, $C_{1\sim6}$ alkyl, three- to six-membered cycloalkyl, three- to six-membered cyclooxyalkyl, phenyl, $C_{1\sim6}$ alkyl with at least one hydrogen substituted with $R^{2-1}$, phenyl with at least one hydrogen substituted with $R^{2-1}$, wherein $R^{2-1}$ is hydroxyl, halogen, amino, or $C_{1\sim6}$ alkoxy,
$R^3$ is

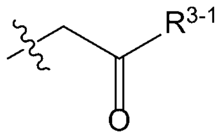 , 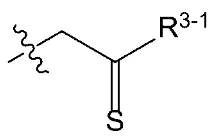 or 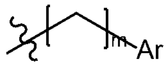 ;

wherein $R^{3-1}$ is hydrogen, hydroxyl, $C_{1\sim6}$ alkyl, $C_{1\sim6}$ alkoxy, $C_{3\sim6}$ cycloalkyl, three- to six-membered cyclooxyalkyl, amino, $C_{1\sim6}$ amine group, $-CH_2C(O)R^{3-2}$, $-CH_2C(O)OR^{3-2}$, $-CH_2C(O)N$ $(R^{3-2}$ $R^{3-2a})$, and $R^{3-2}$ and $R^{3-2a}$ are independently hydrogen, C1~6 alkyl, or three- to six-membered cycloalkyl respectively, m is from 1 to 6, and
Ar is phenyl, and acene, 5- or 6-membered monocyclic heteroaryl, 8- to 10-membered fused bicyclic heteroaryl, phenyl with at least one hydrogen atom substituted with $R^{3-3}$, and acene with at least one hydrogen atom substituted with $R^{3-3}$, 5- or 6-membered monocyclic heteroaryl with at least one hydrogen atom substituted with $R^{3-3}$, 8- to 10-membered fused bicyclic heteroaryl with at least one hydrogen atom substituted with $R^{3-3}$, and the $R^{3-3}$ is hydrogen, halogen, $C_{1\sim6}$ alkyl, three- to six-membered cycloalkyl, hydroxy, $C_{1\sim6}$ alkoxy, three- to six-membered epoxyalkyl, $C_{1\sim6}$ haloalkyl, $C_{2\sim6}$ alkenyl, $C_{2\sim6}$ alkynyl, $-N(R^{3-3a}R^{3-3b})$ or phenyl,
$R^{3-3a}$ and $R^{3-3b}$ are independently hydrogen, $C_{1\sim6}$ alkyl, or three- to six-membered cycloalkyl, respectively;
$R^4$ is

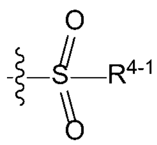 , 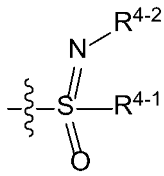 , 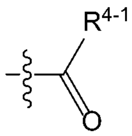 or 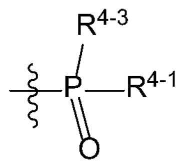 .

wherein $R^{4-1}$ is phenyl, and acene, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and $R^{4-11}$ is hydrogen, halogen, nitro, nitrile, hydroxyl, $C_{1\sim6}$ alkyl, $C_{13\sim6}$ cycloalkyl, $C_{1\sim6}$ alkoxy, $-N(R^{4-1a}R^{4-1b})$, phenyl, $C_{1\sim6}$ haloalkyl, $C_{1\sim6}$ haloalkoxy, $-C(O)OR^{4-12}$, $-C(O)R^{4-12}$, $-C(O)N(R^{4-1a}R^{4-1b})$, $-S(O)2R^{4-12}$, $-S(O)R^{4-12}$, - $OC(O)R^{4-12}$, $-OC(O)OR^{4-12}$ or

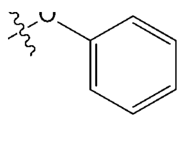 ,

$R^{4-12}$, R4$^{-1a}$ and $R^{4-1b}$ are independently hydrogen, respectively, $C_{1\sim6}$ alkyl, $C_{3\sim6}$ cycloalkyl, $C_{2\sim6}$ alkenyl, C2~6 alkynyl, C1~6 alkyl substituted with at least one hydrogen by a halogen, $C_{2\sim6}$ alkynyl with at least one hydrogen substituted with a halogen, $C_{3\sim6}$ cycloalkyl, C2~6 alkynyl with at least one hydrogen substituted with a halogen, and R4$^{-1a}$ and $R^{4-1b}$ are bondable to each other to form a ring,
$R^{4-2}$ is C1~6 alkyl, $C_{3\sim6}$ cycloalkyl, C1~6 alkyl with at least one hydrogen substituted with a hydroxyl group,

$C_{3\sim6}$ epoxyalkyl, or $R^{4-2}$ is bonded to R2 to form a 4- to 8-membered ring when $R^2$ is C1~6 alkyl and $R^{4-2}$ is C1~6 alkyl.

$R^{4-3}$ is C1~6 alkyl or C1~6 alkoxy;

the number of $R^5$ is 0~5, and at each occurrence, $R^5$ is independently hydrogen, halogen, nitro, nitrile, -N+($R^{5-1}$)$_3$, C1~6 haloalkyl, -C(O)O$R^{5-1}$, -C(O)$R^{5-1}$, -C(O)N($R^{5-1}R^{5-1a}$), -S(O)$_2R^{5-1}$, -S(O)$R^{5-1}$, -S(O)$_2$N($R^{5-1}R^{5-1a}$), -S(O)N($R^{5-1}R^{5-1a}$), -N=C($R^{5-1}$ $R^{5-1a}$), hydroxyl, $C_{1\sim6}$ alkyl, phenyl, phenyl with at least one hydrogen substituted with $R^{5-1}$, C1~6 alkoxy, -N($R^{5-1}R^{5-1a}$), -N($R^{5-1}$)C(O)$R^{5-1a}$, -N($R^{5-1}$)C(O)O$R^{5-1a}$, -OC(O)$R^{5-1}$, -OC(O)O$R^{5-1}$, -OC(O) N($R^{5-1}R^{5-1a}$) or -S$R^{5-1}$, $R^{5-1}$, $R^{5-1a}$ and $R^{5-1b}$ are independently C1~6 alkyl, $C_{2\sim6}$ alkenyl, C2~6 alkynyl, $C_{1\sim6}$ alky with at least one hydrogen substituted with a halogen, $C_{2\sim6}$ alkenyl with at least one hydrogen substituted with a halogen or $C_{2\sim6}$ alkynyl with at least one hydrogen substituted with a halogen, respectively.

**2.** The Uses according to claim 1, wherein the Z is

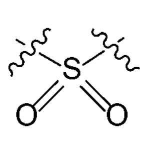,
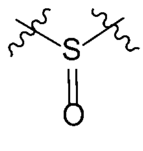,

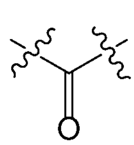,
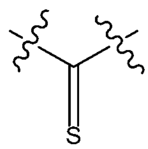

or a sulfur atom;

$R^1$ is hydrogen, $C_{1\sim4}$ alkyl,

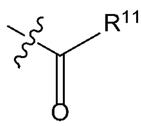 or 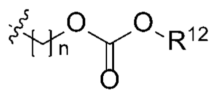,

wherein $R^{11}$ and $R^{12}$ are independently $C_{1\sim4}$ alkyl or $C_{1\sim4}$ cycloalkyl, respectively, and n is 1 or 2;

$R^2$ is hydrogen, $C_{1\sim4}$ alkyl, three- to six-membered cycloalkyl, three- to six-membered cyclooxyalkyl, phenyl, $C_{1\sim4}$ alkyl with at least one hydrogen substituted with $R^{2-1}$, and phenyl with at least one hydrogen substituted with $R^{2-1}$, wherein $R^{2-1}$ is hydroxy, halogen, amino, or $C_{1\sim4}$ alkoxy;

$R^3$ is

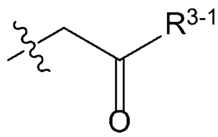, 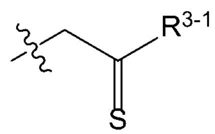 or 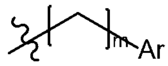;

wherein $R^{3-1}$ is hydrogen, hydroxyl, $C_{1\sim4}$ alkyl, $C_{1\sim4}$ alkoxy, -N($R^{3-2}R^{3-2a}$),

$R^{3-2}$ and $R^{3-2a}$ are independently hydrogen or $C_{1\sim4}$ alkyl, respectively, $R^{3-3a}$ and $R^{3-3b}$ are independently hydrogen or $C_{1\sim4}$ alkyl, respectively,

m is 1~4.

Ar is phenyl, 5- or 6-membered monocyclic heteroaryl, 5- or 6-membered monocyclic heteroaryl with at least one hydrogen atom substituted with $R^{3-3}$, the $R^{3-3}$ is hydrogen, halogen, $C_{1\sim4}$ alkyl, hydroxyl, $C_{1\sim4}$ alkoxy, $C_{1\sim4}$ haloalkyl, or -N($R^{3-3a}R^{3-3b}$);

$R^4$ is

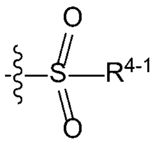, 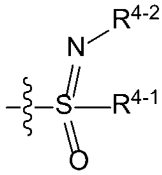, 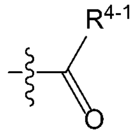 or 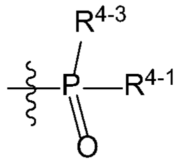,

wherein $R^{4-1}$ is phenyl, phenyl with at least one hydrogen atom substituted with $R^{4-11}$, 5- or 6-membered monocyclic heteroaryl, 5- or 6-membered monocyclic heteroaryl with at least one hydrogen atom substituted with $R^{4-11}$, and the $R^{4-11}$ is hydrogen, halogen, nitro, $C_{1\sim4}$ alkyl, $C_{3\sim6}$ cycloalkyl, $C_{1\sim4}$ alkoxy, -N($R^{4-1a}R^{4-1b}$), phenyl, $C_{1\sim4}$ haloalkyl, $C_{1\sim4}$ haloalkoxy or

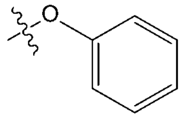,

R4$^{-1a}$ and $R^{4-1b}$ are independently hydrogen, $C_{1\sim4}$ alkyl, or $C_{3\sim6}$ cycloalkyl, respectively, and $R^{4-1a}$ and $R^{4-1b}$ are bondable to each other to form a ring,
$R^{4-2}$ is $C_{1\sim4}$ alkyl, $C_{3\sim5}$ cycloalkyl, $C_{1\sim4}$ alkyl with at least one hydrogen substituted with hydroxyl, $C_{3\sim5}$ cyclooxyalkyl, or $R^{4-2}$ is bonded to $R^2$ to form a 4- to 8-membered ring when $R^2$ is $C_{1\sim4}$ alkyl and $R^{4-2}$ is $C_{1\sim4}$ alkyl,
$R^{4-3}$ is $C_{1\sim4}$ alkyl or $C_{1\sim4}$ alkoxy;
at each occurrence, $R^5$ is independently hydrogen halogen, nitro, nitrile, -N+($R^{5-1}$)$_3$, $C_{1\sim4}$ haloalkyl, C(O)O$R^{5-1}$, -C(O)$R^{5-1}$, -C(O)N($R^{5-1}R^{5-1a}$), -S(O)$_2R^{5-1}$, -S(O)$R^{5-1}$, -N=C($R^{5-1}$ $R^{5-1a}$), hydroxyl, $C_{1\sim4}$ alkyl, phenyl, phenyl with at least one hydrogen substituted with $R^{5-1}$, $C_{1\sim4}$ alkoxy, -N($R^{5-1}R^{5-1a}$), -N( $R^{5-1}$)C(O)$R^{5-1a}$, or -OC(O)$R^{5-1}$, respectively, wherein $R^{5-1}$, $R^{5-1a}$, and $R^{5-1b}$ are independently hydrogen, $C_{1\sim4}$ alkyl, $C_{1\sim4}$ alkyl with at least one hydrogen substituted with a halogen, respectively.

**3.** The Uses according to claim 1, wherein the Z is

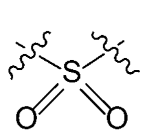, 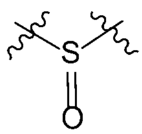,

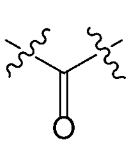, 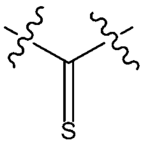

or a sulfur atom;

and/or, $R^1$ is hydrogen,

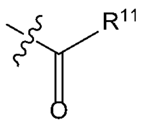 or 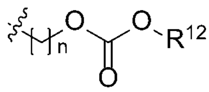,

wherein $R^{11}$ and $R^{12}$ are independently methyl, ethyl, n-propyl or isopropyl, respectively, and n is 1;
and/or, $R^2$ is hydrogen, methyl, ethyl, n-propyl, isopropyl, ethyl with one hydrogen substituted with hydroxyl, n-propyl with one hydrogen substituted with hydroxyl, phenyl,

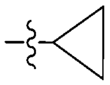, 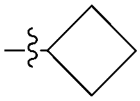, 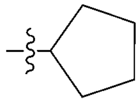, 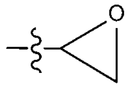, 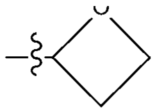,

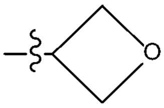, 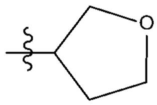 or 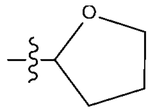;

and/or, $R^3$ is

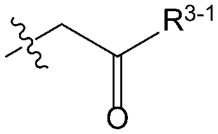, 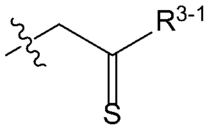 or 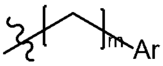;

wherein $R^{3-1}$ is hydrogen, hydroxyl, methyl, ethyl, n-propyl, isopropyl, methoxy, ethoxy, n-propoxy, isopropoxy, n-butoxy, tert-butoxy, sec-butoxy or isobutoxy,
m is 1,
Ar is phenyl, 5- or 6-membered nitrogen-containing monocyclic heteroaryl;
and/or, $R^4$ is

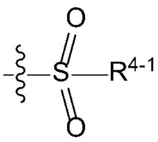, 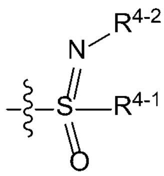, 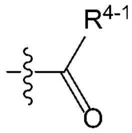 or 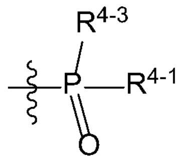,

wherein R4-1 is phenyl, phenyl with at least one hydrogen substituted with $R^{4-11}$, 5- or 6-membered monocyclic heteroaryl, 5- or 6-membered monocyclic heteroaryl with at least one hydrogen atom substituted with $R^{4-11}$, 8- to 10-membered fused bicyclic heteroaryl, 8- to 10-membered fused bicyclic heteroaryl with at least one hydrogen atom substituted with $R^{4-11}$, and the $R^{4-11}$ is halogen, nitro, methyl, ethyl, n-propyl, isopropyl,

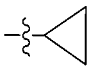, 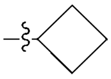, 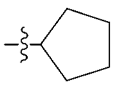, 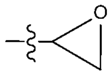, 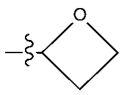, 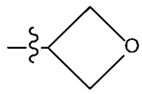,

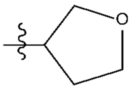, 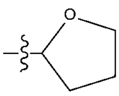, 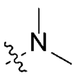, 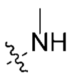, 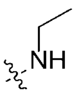, 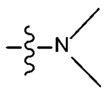, 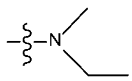, 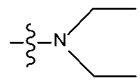,

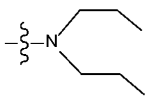, 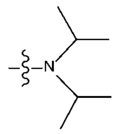, 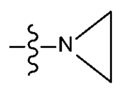, 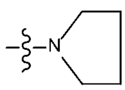, 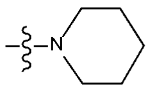,

fluoromethyl, fluorinated ethyl, fluorinated n-propyl, fluorinated isopropyl, chloromethyl, chlorinated ethyl, chlorinated n-propyl, chlorinated isopropyl, bromomethyl, bromoethyl, bromo n-propyl, bromo isopropyl, iodomethyl, iodoethyl, iodo n-propyl, iodo isopropyl, fluoroethoxy, fluoroethoxy, fluoroorthopropoxy, fluoroisopropoxy, chloromethoxy, chloroethoxy, chloro n-propoxy, chloroisopropoxy, bromomethoxy, bromoethoxy, bromo n-propoxy, bromoisopropoxy, iodomethoxy, iodoethoxy, iodo n-propoxy, iodoisopropoxy, or

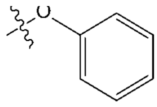,

$R^{4-2}$ is methyl, ethyl, n-propyl, isopropyl, n-butyl, isobutyl, tert-butyl, ethyl with one hydrogen substituted with hydroxyl, n-propyl with one hydrogen substituted with hydroxyl, n-butyl with one hydrogen substituted with hydroxyl,

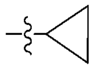, 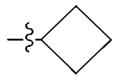,

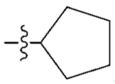, 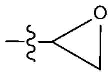, 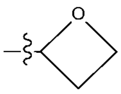, 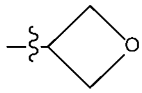, 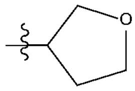, 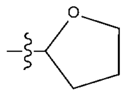

or phenyl, or, $R^{4-2}$ is bonded to $R^2$ to form a 4 to 8-membered ring when $R^2$ is methyl, ethyl or n-propyl and $R^{4-2}$ is methyl, ethyl,

$R^{4-3}$ is methyl, ethyl, n-propyl, isopropyl, methoxy, ethoxy, n-propoxy or isopropoxy;

and/or, at each occurrence, $R^5$ is independently hydrogen, halogen, nitro, nitrile, -N+$(R^{5-1})^3$, fluoro-methyl, fluoroethyl, fluoro-n-propyl, fluoro-isopropyl, chloromethyl, chloroethyl, chloro-n-propyl, chloro-isopropyl, bromomethyl, bromoethyl, bromo-n-propyl, bromo-isopropyl, -C(O)OR$^{5-1}$, -C(O)R$^{5-1}$, -C(O)N(R$^{5-1}$R$^{5-1a}$), -S(O)$_2$R$^{5-1}$, -S(O)R$^{5-1}$, -N=C(R$^{5-1}$ R$^{5-1a}$), hydroxyl, methyl, ethyl, n-propyl, isopropyl, phenyl, phenyl with at least one hydrogen substituted with R$^{5-1}$, methoxy, ethoxy, n-propyloxy, isopropyloxy, -N(R$^{5-1}$R$^{5-1a}$), -N(R$^{5-1}$) C(O)R$^{5-1a}$, -OC(O)R$^{5-1}$, respectively, wherein R$^{5-1}$, R$^{5-1a}$, and R$^{5-1b}$ are independently hydrogen, methyl, ethyl, n-propyl, isopropyl, fluoro-methyl, fluoroethyl, fluoro-n-propyl, fluoro-isopropyl, chloro-methyl, chloroethyl, chloro n-propyl, chloro isopropyl, bromo-methyl, bromoethyl, bromo n-propyl, or bromo isopropyl, respectively.

**4.** The Uses according to claim 1, wherein the Z is

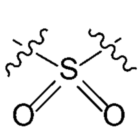, 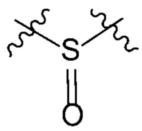,

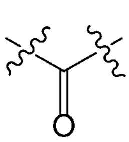, 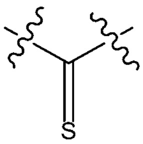

or a sulfur atom;

and/or, $R^1$ is hydrogen,

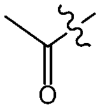, 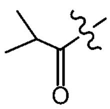 or 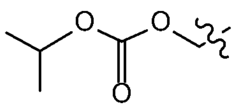;

and/or, $R^2$ is hydrogen, methyl,

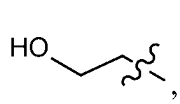, 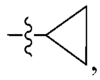, 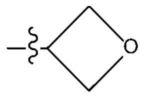

or phenyl;
and/or, $R^3$ is

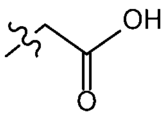, 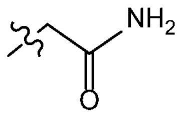, 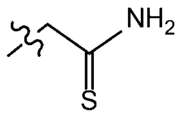, 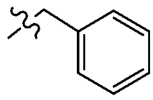,

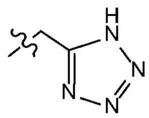 or 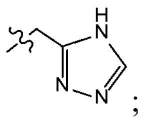;

and/or, $R^4$ is

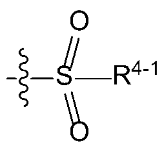, 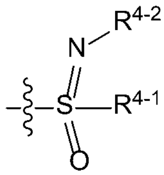, 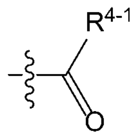 or 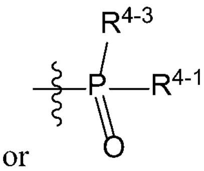,

wherein $R^{4-1}$ is phenyl, phenyl with at least one hydrogen substituted with $R^{4-11}$, five-membered monocyclic heteroaryl, five-membered monocyclic heteroaryl with at least one hydrogen substituted with $R^{4-11}$, nine-membered fused bicyclic heteroaryl, or naphthyl, wherein $R^{4-11}$ is bromine, fluorine, chlorine, methyl, nitro, phenyl, trifluoromethyl,

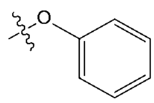,

methoxy, cyclopropyl, trifluoromethoxy, nitro or

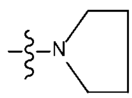;

$R^{4-2}$ is methyl, ethyl,

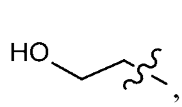, 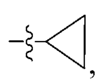, 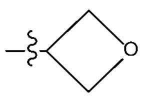

or phenyl, or, $R^{4-2}$ is bonded to $R^2$ to form a 4 to 6-membered ring when $R^2$ is methyl, ethyl, or n-propyl and $R^{4-2}$ is methyl, ethyl,
$R^{4-3}$ is methoxy, ethoxy, n-propoxy or isopropoxy;
and/or, at each occurrence, $R^5$ is independently halogen, nitro, nitrile, carboxyl, -NHC(O)$CH_3$, methoxy, or hydroxy, respectively;

**5.** The Uses according to claim 1, wherein the compound is selected from any one of the following compounds:

| | |
|---|---|
| **I-1** | 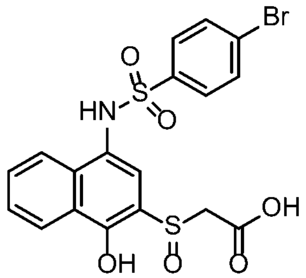 |
| **I-2** | 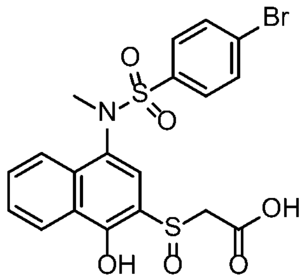 |
| **I-3** | 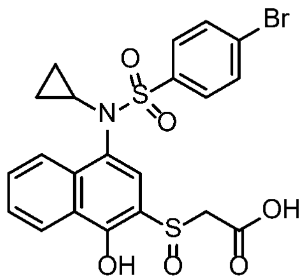 |
| **I-4** | 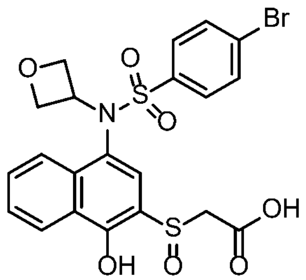 |
| **I-5** | 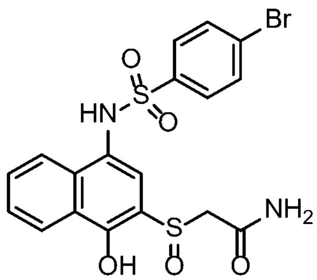 |
| **I-6** | 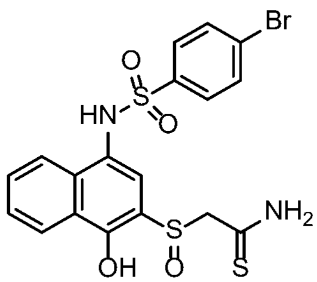 |

(continued)

| | |
|---|---|
| **I-7-1** | 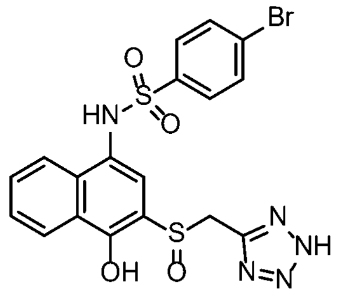 |
| **I-8** | 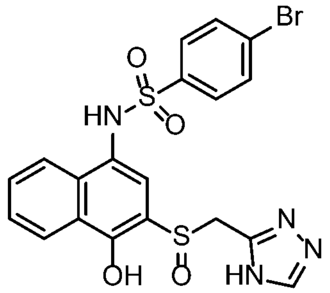 |
| **I-9-1** | 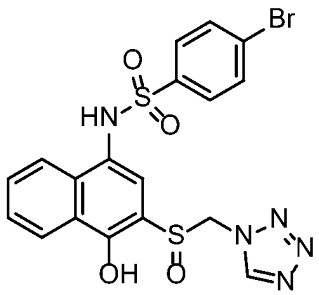 |
| **I-9-2** | 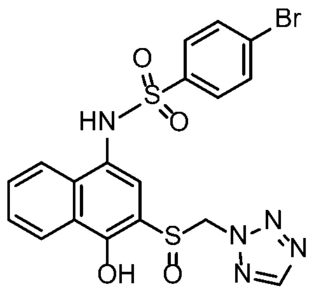 |
| **I-10** | 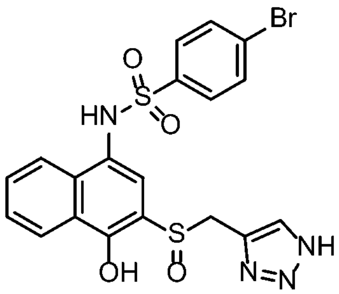 |

(continued)

| | |
|---|---|
| **I-11-1** | 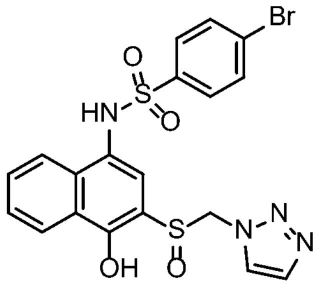 |
| **I-11-2** | 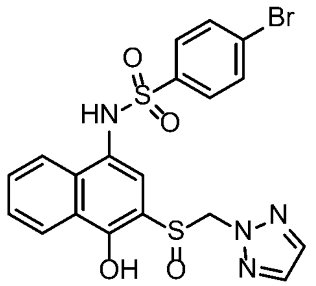 |
| **I-12** | 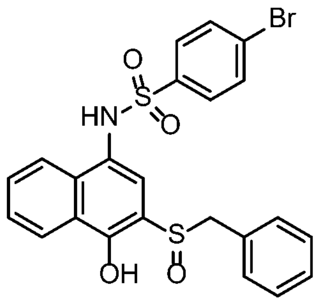 |
| **I-13-1** | 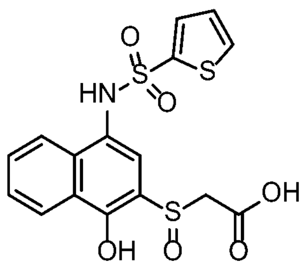 |
| **I-14-1** | 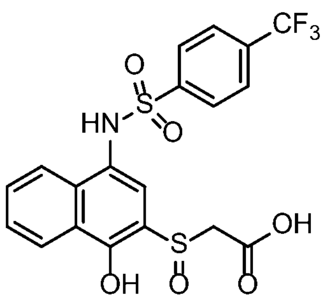 |

(continued)

| | |
|---|---|
| **I-15-1** | 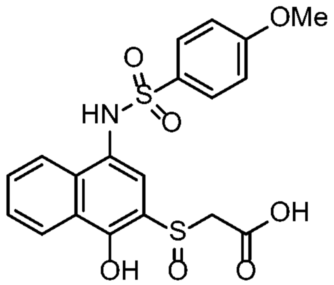 |
| **I-16-1** | 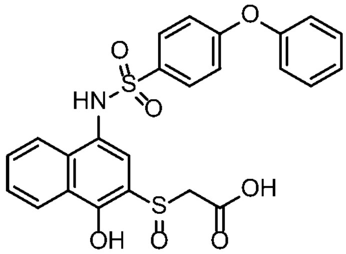 |
| **I-17-1** | 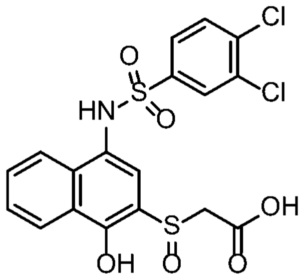 |
| **I-18-1** | 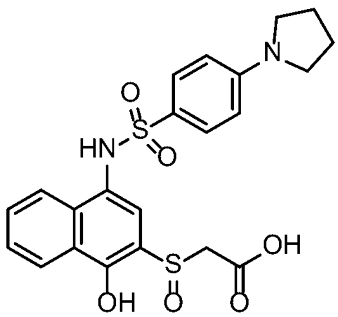 |
| **I-19-1** | 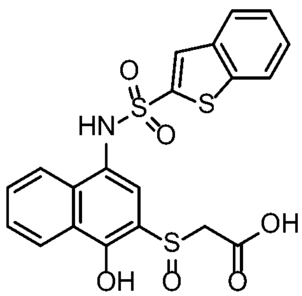 |

(continued)

| | |
|---|---|
| **I-20-1** | 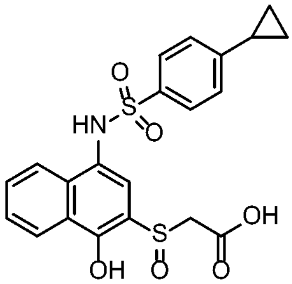 |
| **I-21-1** | 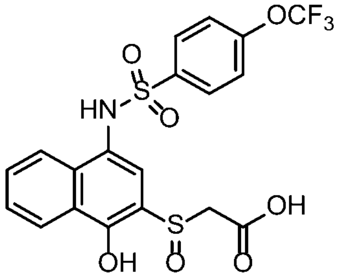 |
| **I-22-1** | 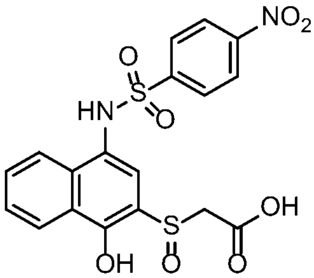 |
| **I-23-1** | 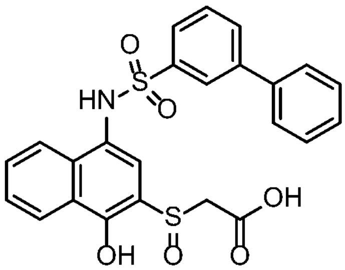 |
| **I-24-1** | 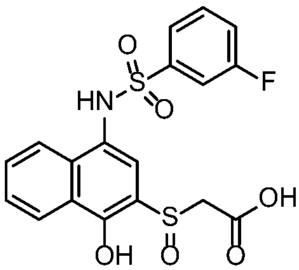 |
| **I-25-1** | 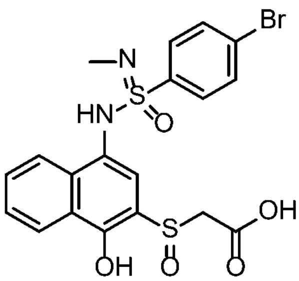 |

(continued)

| | |
|---|---|
| **I-26** | 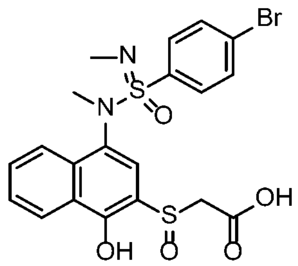 |
| **I-27** | 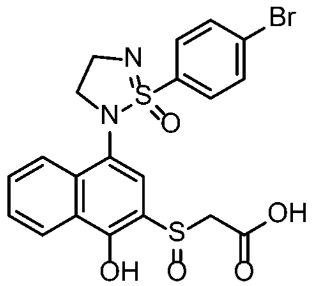 |
| **I-28** | 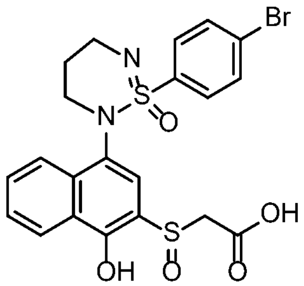 |
| **I-29** | 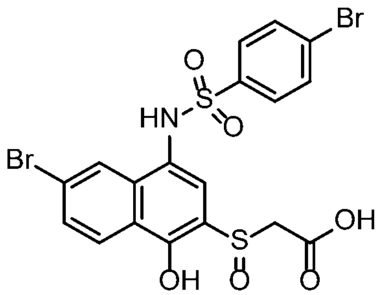 |
| **I-30** | 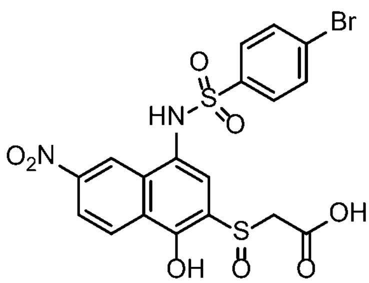 |

(continued)

| | |
|---|---|
| **I-31** | 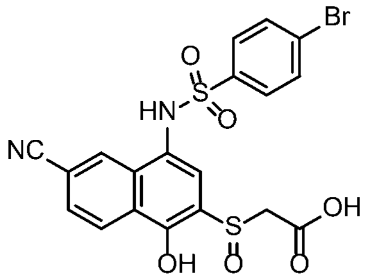 |
| **I-32** | 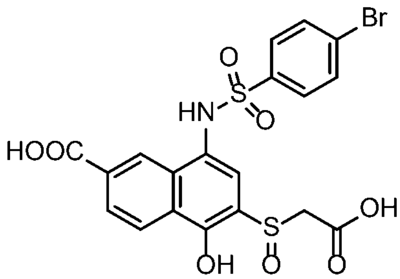 |
| **I-33** | 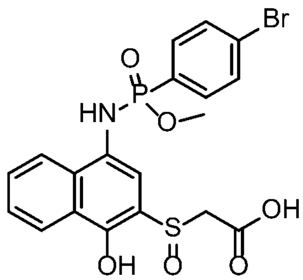 |
| **I-34** | 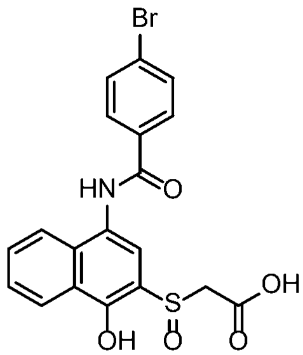 |
| **I-35-1** | 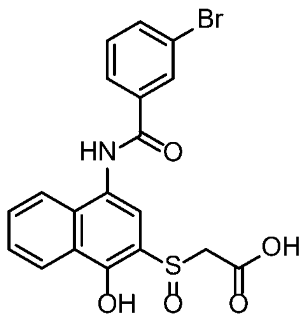 |

(continued)

| | |
|---|---|
| **I-36** | 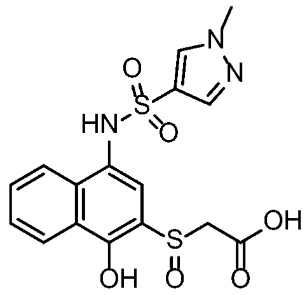 |
| **I-37** | 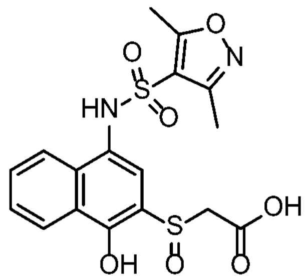 |
| **I-38** | 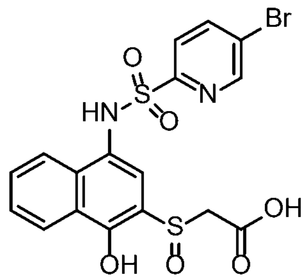 |
| **I-39** | 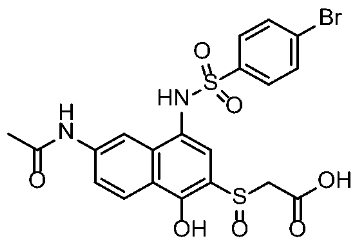 |
| **I-40-1** | 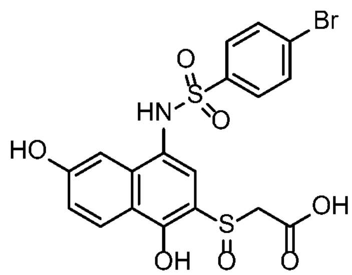 |
| **I-41-1** | 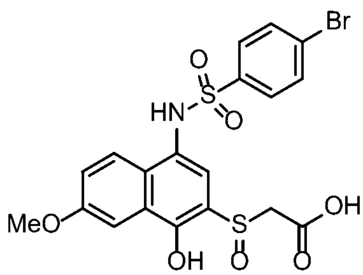 |

(continued)

| | |
|---|---|
| **I-42** | 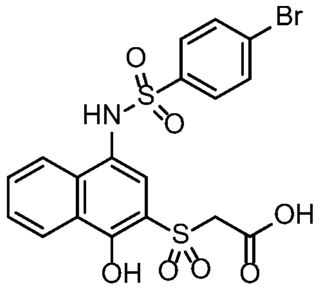 |
| **I-7-2** | 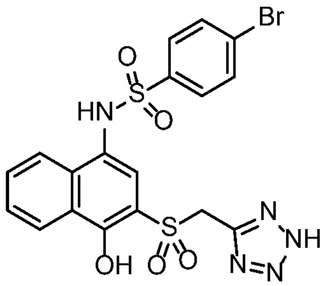 |
| **I-13-2** | 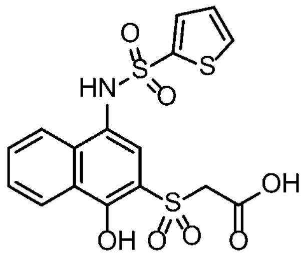 |
| **I-14-2** | 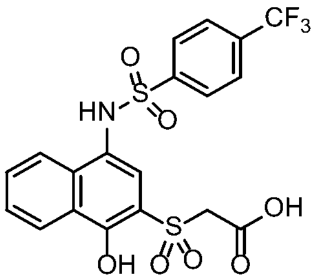 |
| **I-15-2** | 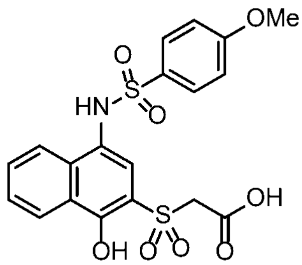 |
| **I-16-2** | 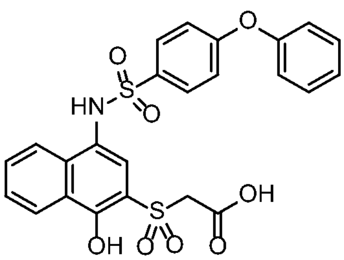 |

(continued)

| | |
|---|---|
| **I-17-2** | 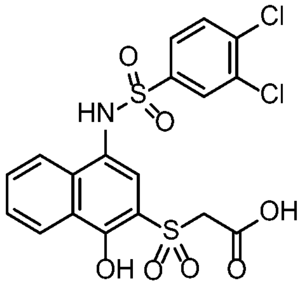 |
| **I-18-2** | 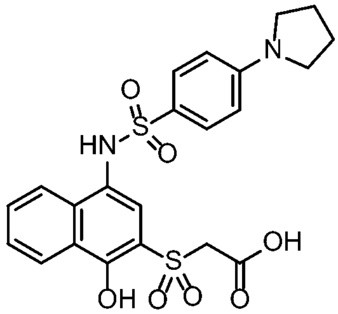 |
| **I-19-2** | 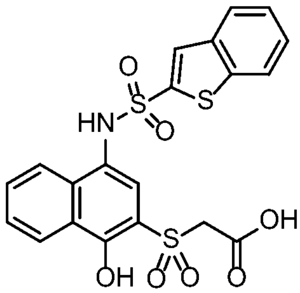 |
| **I-20-2** | 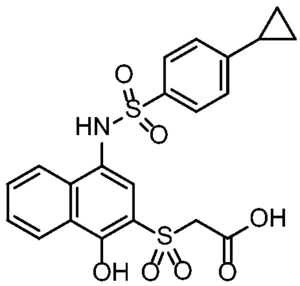 |
| **I-21-2** | 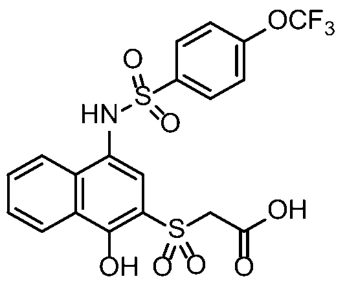 |

(continued)

| | |
|---|---|
| **I-22-2** | 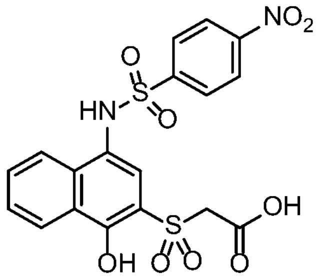 |
| **I-23-2** | 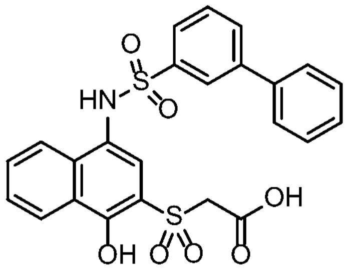 |
| **I-24-2** | 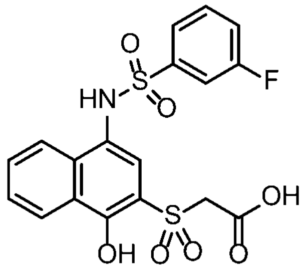 |
| **I-25-2** | 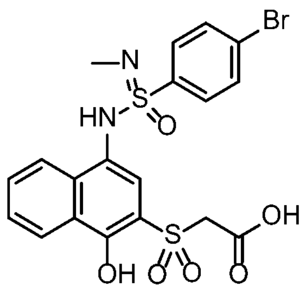 |
| **I-35-2** | 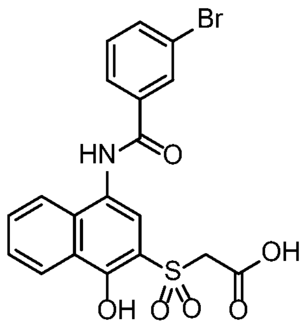 |

(continued)

| | |
|---|---|
| **I-40-2** | 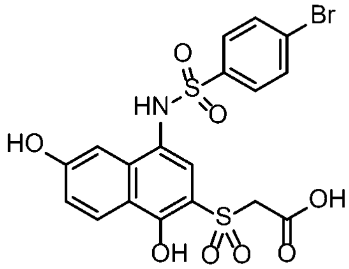 |
| **I-41-2** | 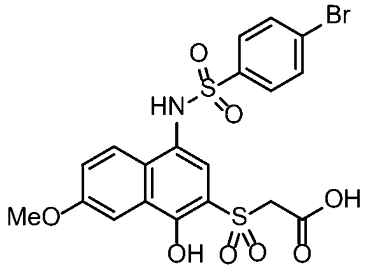 |
| **I-43** | 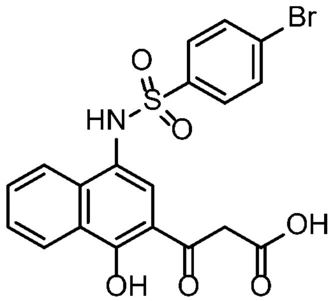 |
| **I-44** | 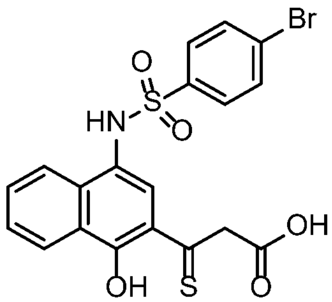 |
| **I-45** | 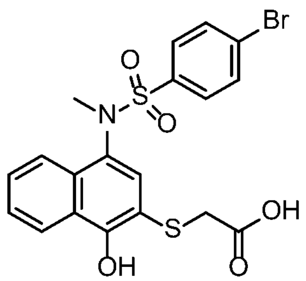 |

(continued)

| | |
|---|---|
| **I-46** | 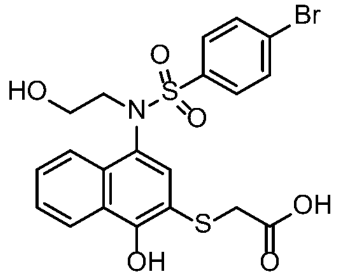 |
| **I-47** | 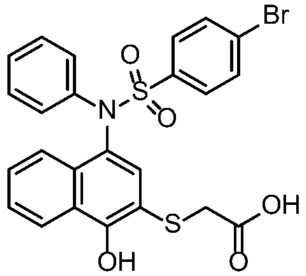 |
| **I-48** | 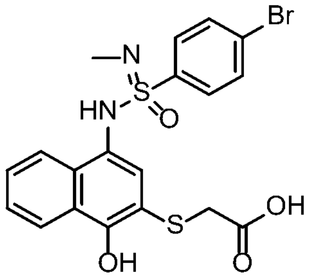 |
| **I-49** | 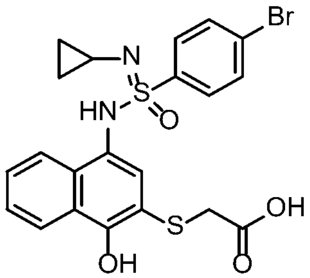 |
| **I-50** | 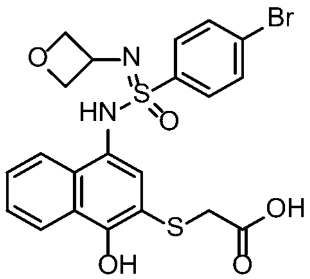 |
| **I-51** | 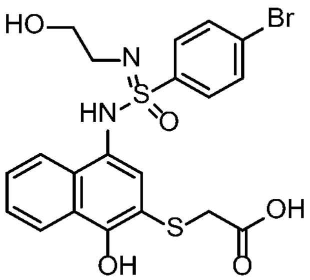 |

(continued)

| | |
|---|---|
| **I-52** | 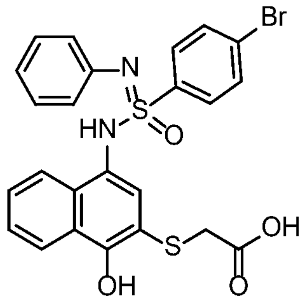 |
| **I-53** | 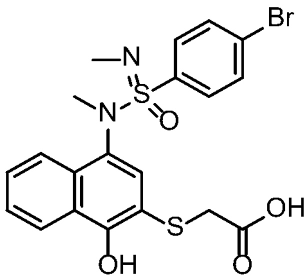 |
| **I-54** | 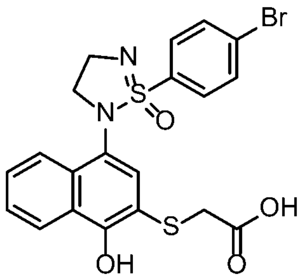 |
| **I-55** | 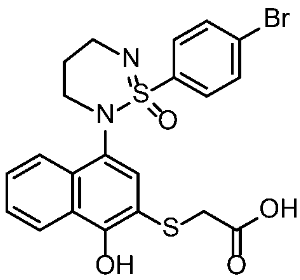 |
| **I-56** | 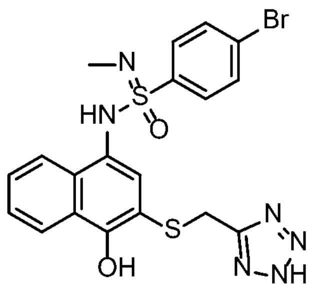 |

(continued)

| | |
|---|---|
| **I-57** | 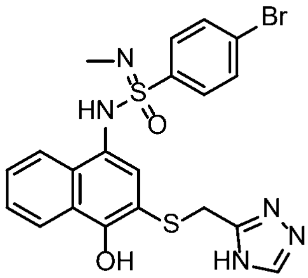 |
| **I-58** | 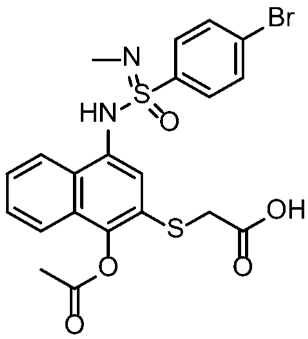 |
| **I-59** | 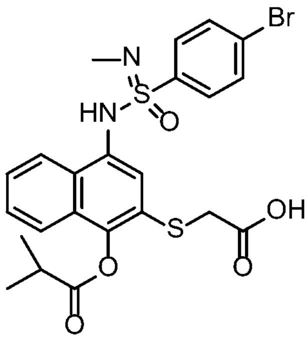 |
| **I-60** | 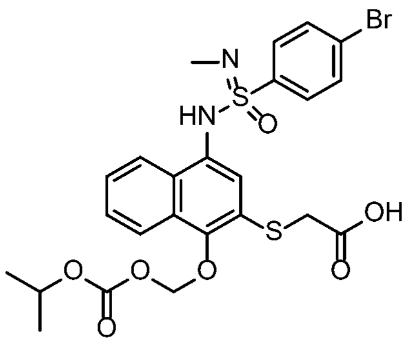 |
| **I-61** | 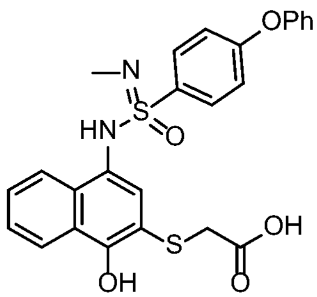 |

(continued)

| | |
|---|---|
| **I-62** | 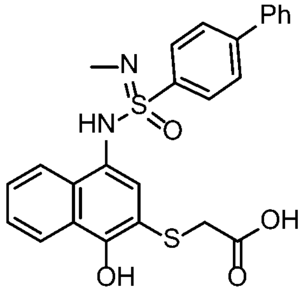 |
| **I-63** | 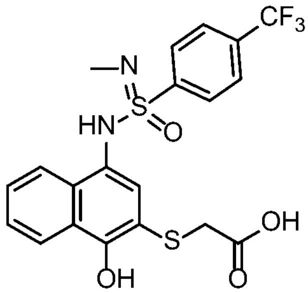 |
| **I-64** | 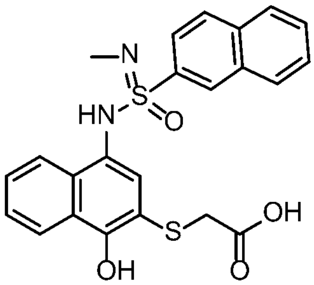 |
| **I-65** | 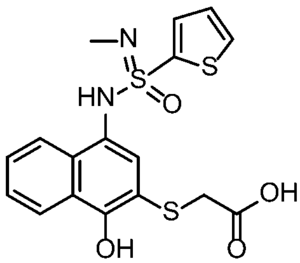 |
| **I-66** | 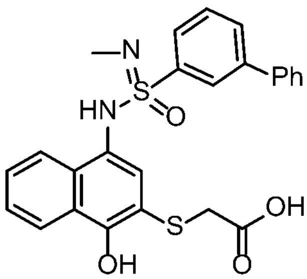 |

(continued)

| | |
|---|---|
| **I-67** | 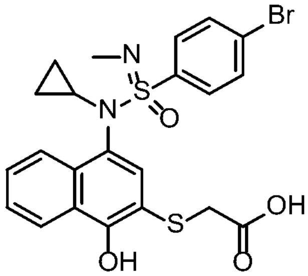 |
| **I-68** | 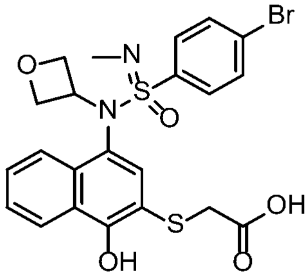 |
| **I-69** | 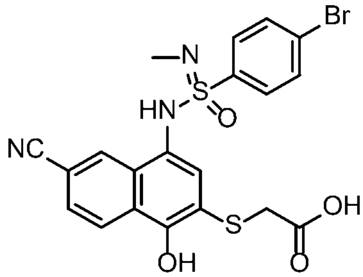 |
| **I-70** | 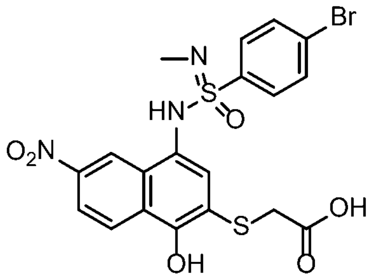 |

**6.** The Uses according to any one of claim 1~5, wherein the diseases associated with kidney injury include acute kidney injury and chronic kidney injury, preferably chronic kidney injury;
and/or, the diseases associated with mitochondrial dysfunction are selected from at least one of the following: inflammatory bowel disease; lung injury; fibrotic diseases; sepsis; prostate disease; cardiovascular disease; neurological disorders; and aging-related diseases.

**7.** The Uses according to any one of claim 6, wherein the diseases associated with kidney injury are selected from acute renal ischemia-reperfusion injury, sepsis nephropathy, nephrotoxin injury, primary glomerulonephritis, hypertensive renal arteriosclerosis, diabetes nephropathy, secondary glomerulonephritis, renal tubulointerstitial lesions, ischemic nephropathy, lupus nephritis and hereditary nephropathy;

and/or, the inflammatory bowel disease is selected from at least one of ulcerative colitis and Crohn's disease;
and/or, the lung injury is selected from at least one of acute lung injury and chronic lung injury;
and/or, the fibrotic disease is selected from at least one of renal tubulointerstitial fibrosis, interstitial lung disease (ILD), idiopathic pulmonary fibrosis (IPF), chronic obstructive pulmonary disease fibrosis, tissue fibrosis, joint fibrosis, liver fibrosis, skin fibrosis, fibromatosis, bone marrow fibrosis, cardiac fibrosis, and cystic fibrosis;
and/or, the cardiovascular disease is selected from at least one of atherosclerosis, heart failure, myocardial

ischemia/reperfusion injury and cardiovascular complications of hypertension, cardiomyopathy and diabetes;
and/or, the neurological disorders are selected from at least one of sensory neuropathic hearing loss, developmental abnormalities in the brain, congenital hydrocephalus, congenital cranial nerve disease, congenital brain pathway abnormalities, metabolic dysfunction, congenital auditory aphasia, congenital visual aphasia, cerebral palsy, autism, depression, schizophrenia, bipolar disorder, paranoid mental disorder, manic disorder, obsessive-compulsive disorder, autism and other mental disorders, Parkinson's disease, Alzheimer's disease, brain injury, amyotrophic lateral sclerosis, epilepsy, Huntington's disease, spinocerebellar ataxia, cerebral ischemia (CI), stroke (preferably ischemic stroke), multiple sclerosis, and tinnitus;
and/or, the aging-related disease is premature aging, early-onset ovarian insufficiency, naturally aging ovarian insufficiency or skin aging.

**8.** The Uses according to any one of claim 7, wherein the renal tubulointerstitial lesions are selected from chronic pyelonephritis, chronic uric acid nephropathy, obstructive nephropathy, and drug-induced nephropathy;
and/or, the chronic lung injury is chronic obstructive pulmonary disease.

**9.** The Uses according to any one of claim 7, wherein the hereditary nephropathy is selected from polycystic kidney disease and hereditary nephritis.

**10.** A method for preventing and/or treating diseases associated with kidney injury, wherein administering a compound shown in formula (I) or a pharmaceutically acceptable salt, a stereoisomer, a solvate, or a prodrug thereof to a subject.

**11.** A method for preventing and/or treating a disease associated with mitochondrial dysfunction, wherein the method comprises the steps of administering a therapeutically effective amount of a compound shown in formula (I) or a pharmaceutically acceptable salt, stereoisomer, solvate, or prodrug thereof to a subject.

**12.** The method according to claim 11, wherein the disease associated with mitochondrial dysfunction is cardiovascular disease, and the method comprises the steps of: co-administering a therapeutically effective amount of the compound shown in formula (I) or a pharmaceutically acceptable salt, a stereoisomer, a solvate or a prodrug thereof and Entresto to the subject.

**13.** The method according to claim 11, wherein the disease associated with mitochondrial dysfunction is benign prostatic hyperplasia, and the method comprises the steps of: administering a therapeutically effective amount of the compound shown in formula (I) or the pharmaceutically acceptable salt, the stereoisomer, the solvate or the prodrug thereof and finasteride to the subject.

**14.** A composition, wherein the composition is a cosmetic composition or a medical instrument composition comprising a compound shown in general formula (I) and acceptable excipients in the cosmetic or medical instrument.

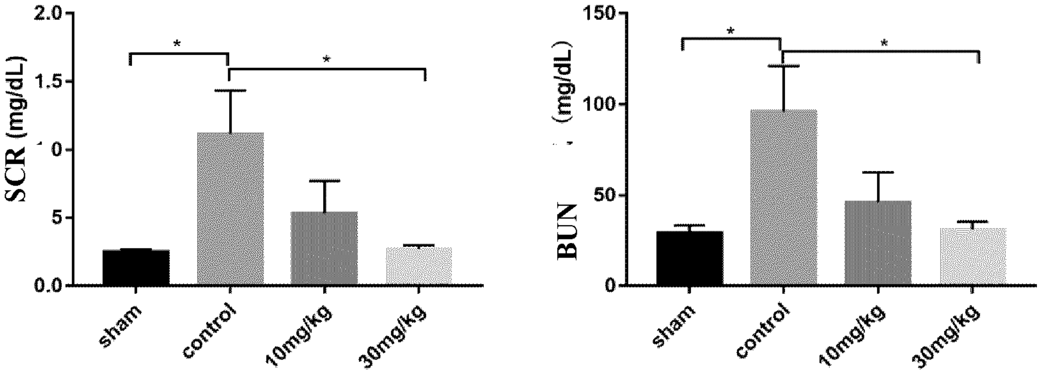
SCR (mg/dL)
2.0
1.5
1.0
0.5
0.0
*
*
sham
control
10mg/kg
30mg/kg
BUN (mg/dL)
150
100
50
0

FIG.1

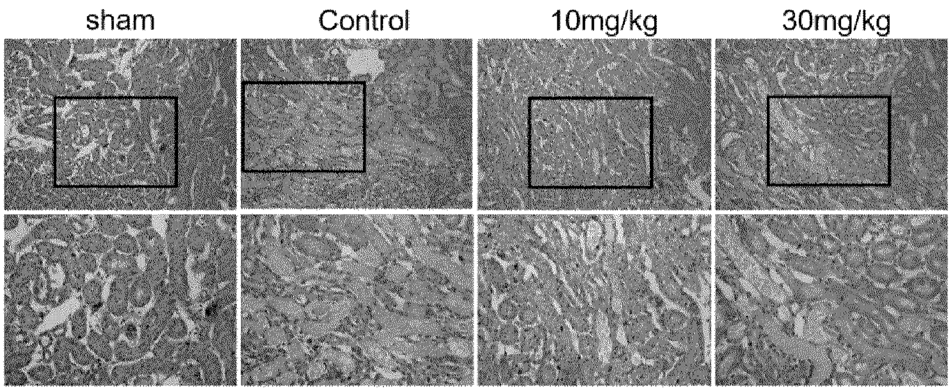
sham
Control
10mg/kg
30mg/kg

FIG.2

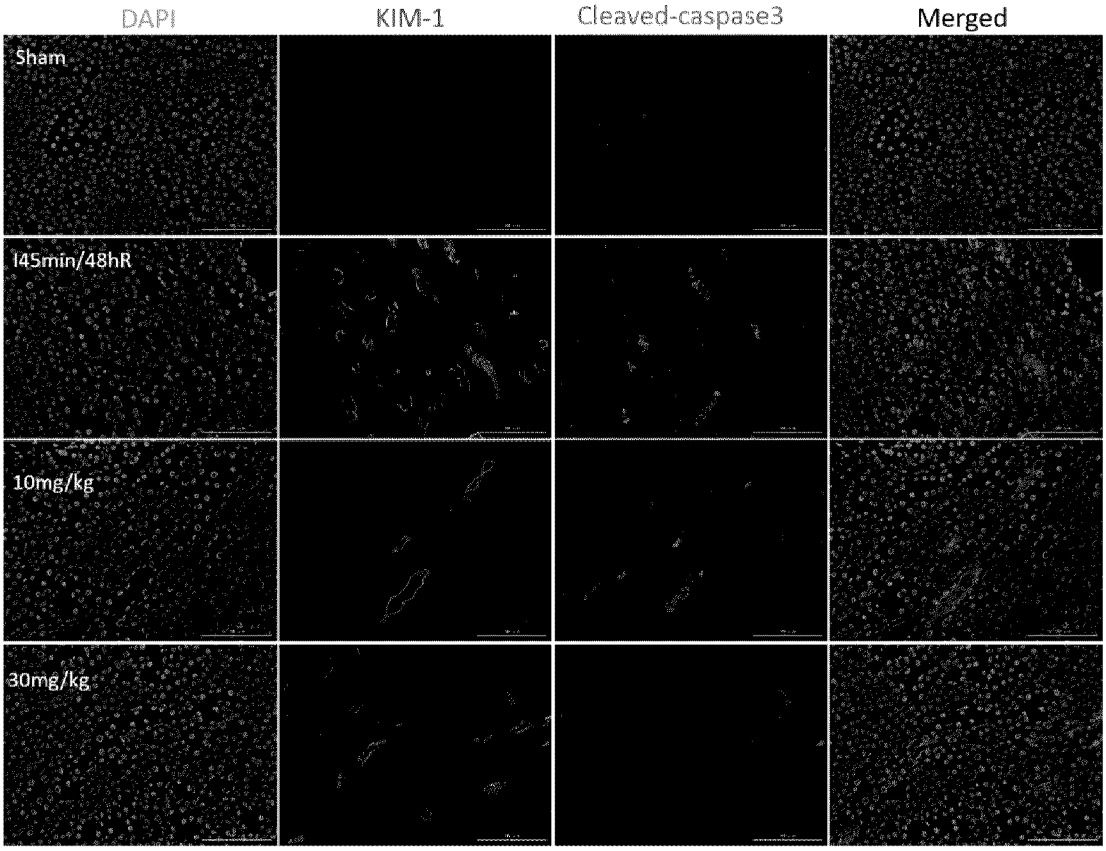
DAPI
KIM-1
Cleaved-caspase3
Merged
Sham
I45min/48hR
10mg/kg
30mg/kg

FIG.3

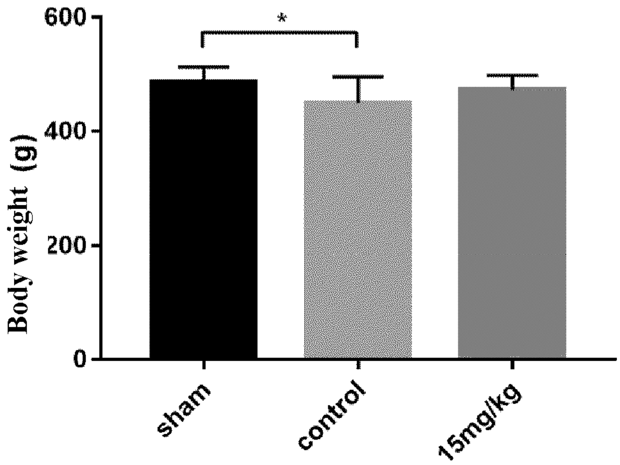
600
400
200
0
Body weight (g)
*
sham
control
15mg/kg

FIG.4

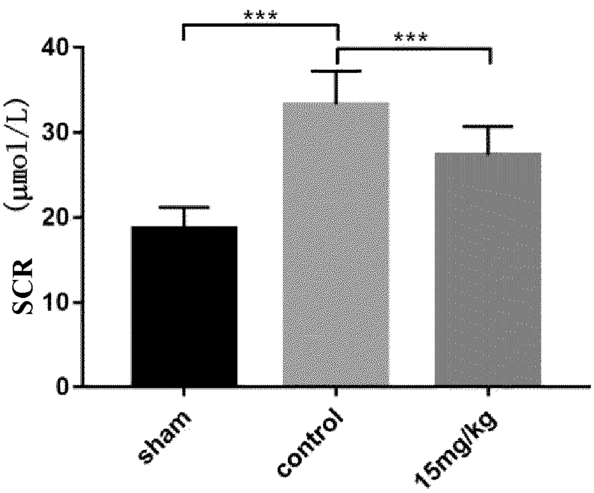
***
***
40
30
20
10
0
SCR (μmol/L)
sham
control
15mg/kg

FIG.5

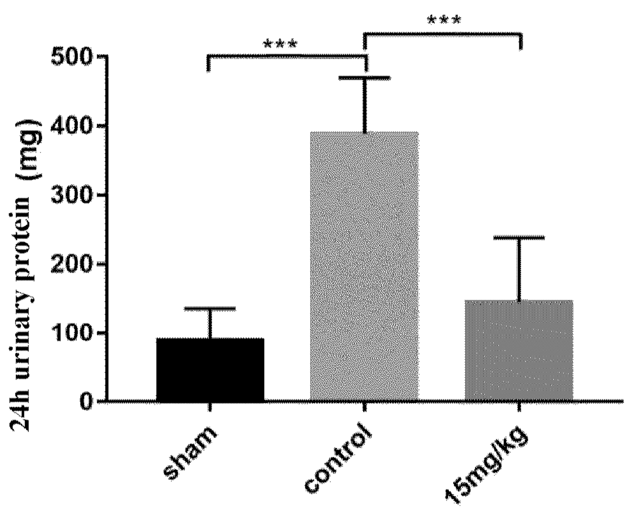
***
***
500
400
300
200
100
0
24h urinary protein (mg)
sham
control
15mg/kg

FIG.6

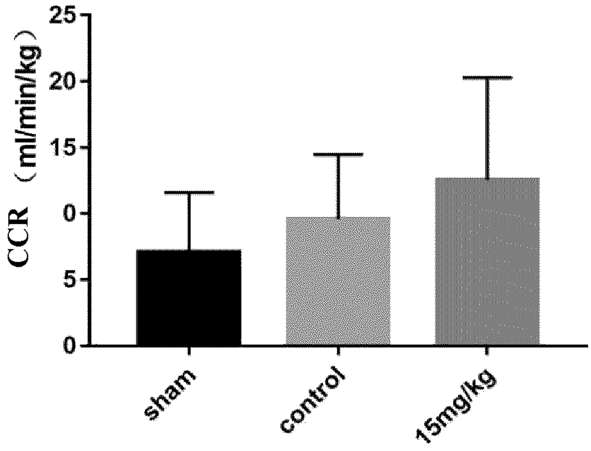


FIG.7

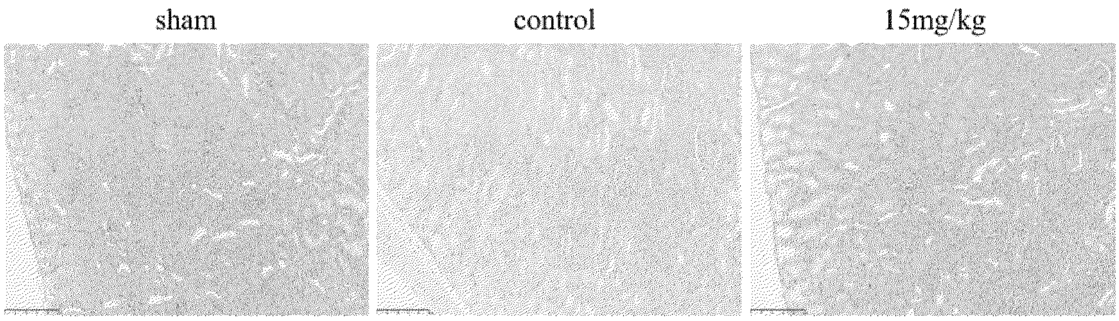


FIG.8

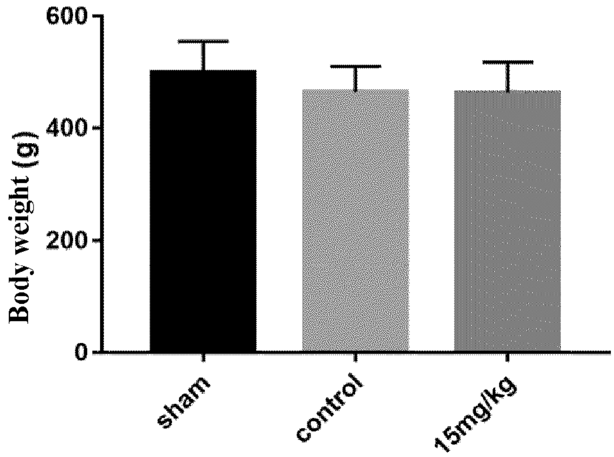


FIG.9

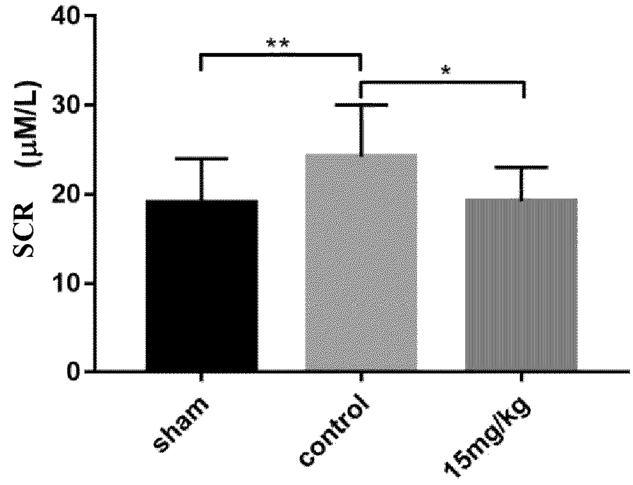
40
30
20
10
0
SCR (μM/L)
**
*
sham
control
15mg/kg

FIG.10

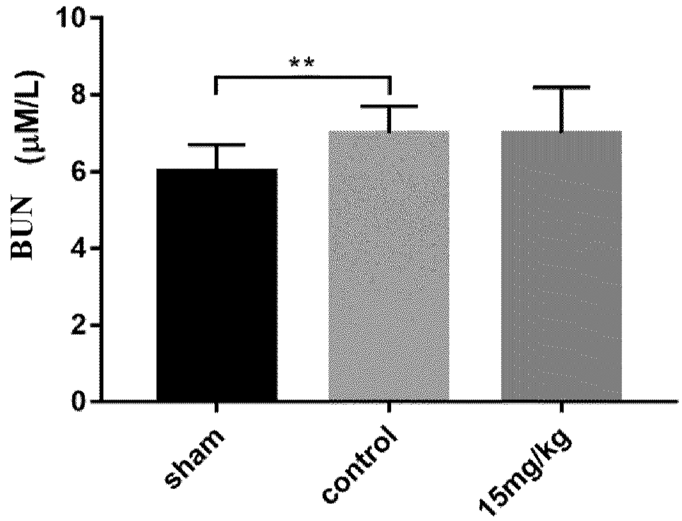
10
8
6
4
2
0
BUN (μM/L)
**
sham
control
15mg/kg

FIG.11

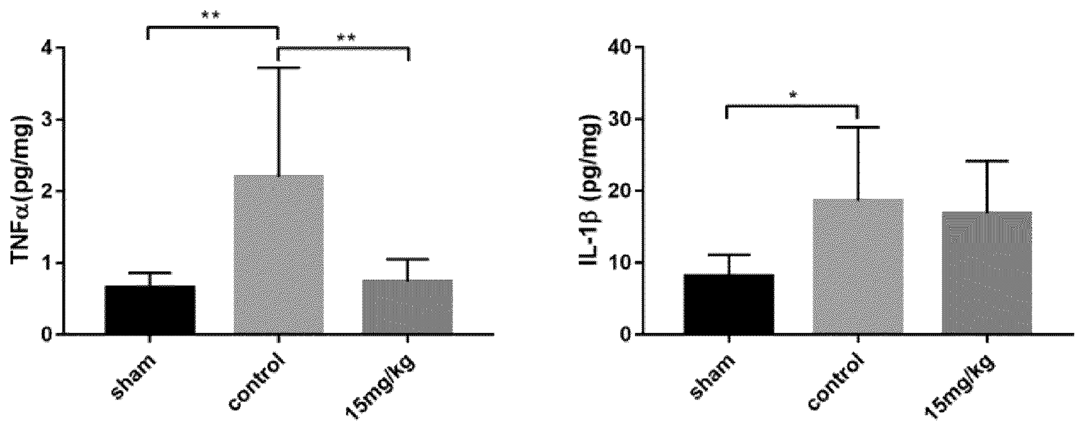


FIG.12

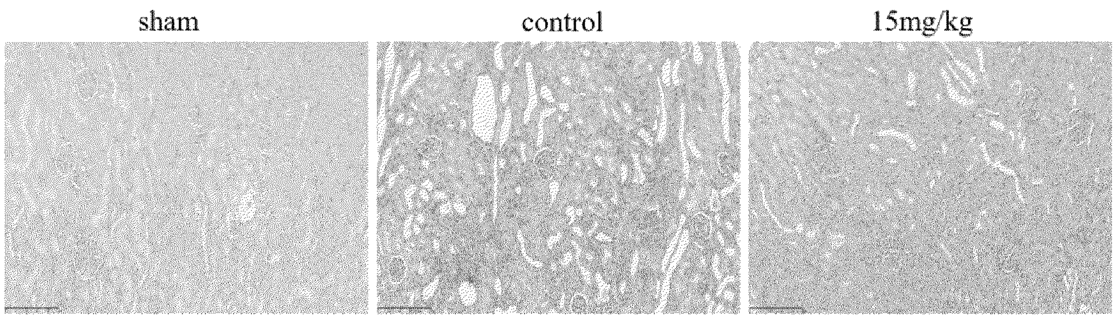


FIG.13

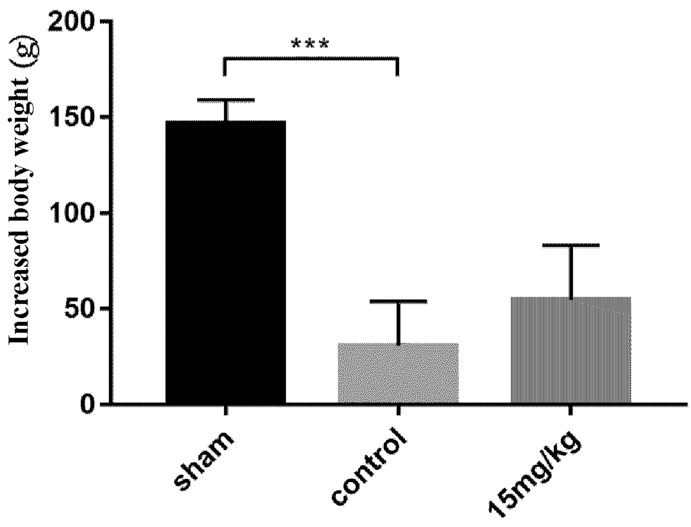


FIG.14

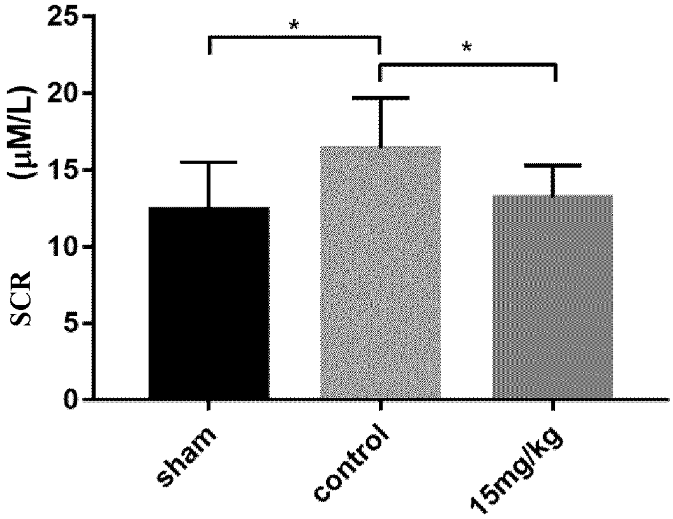
25
20
15
10
5
0
SCR (μM/L)
*
*
sham
control
15mg/kg

FIG.15

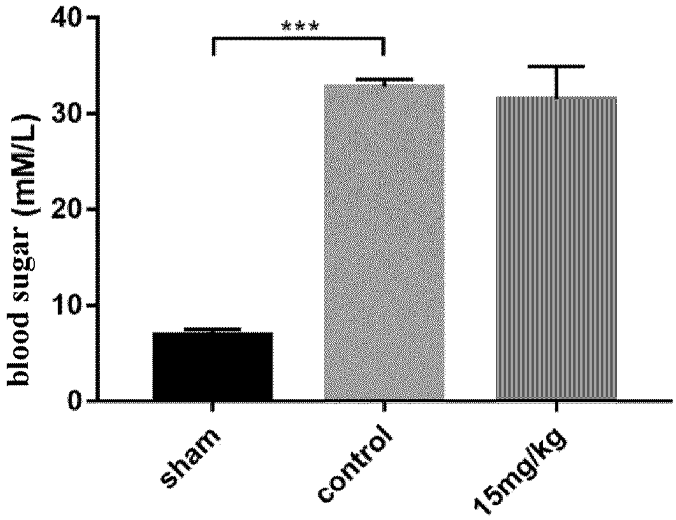
40
30
20
10
0
blood sugar (mM/L)
***
sham
control
15mg/kg

FIG.16

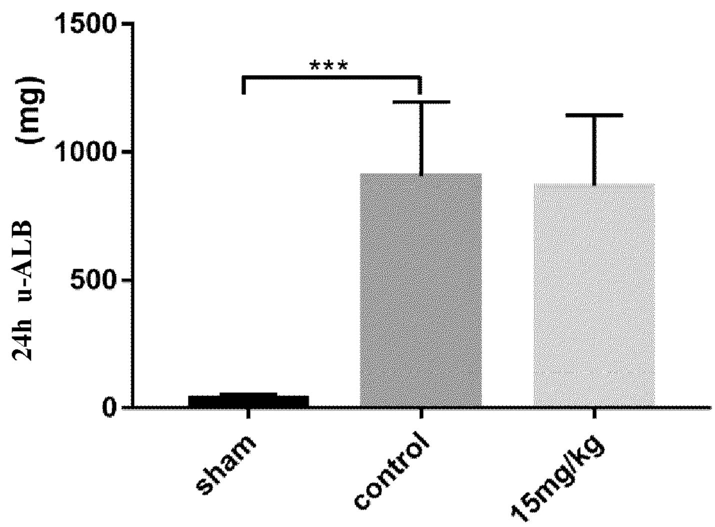


FIG.17

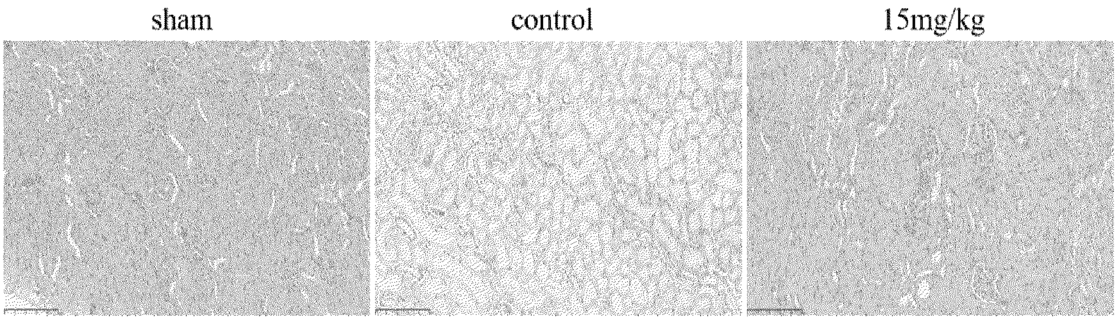


FIG.18

| Liver microsomal stability (half-life T1 / 2) | | |
|---|---|---|
| **Compound No.** | **mouse $t_{1/2}$ - min** | **human $t_{1/2}$ - min** |
| **UMI-77** | 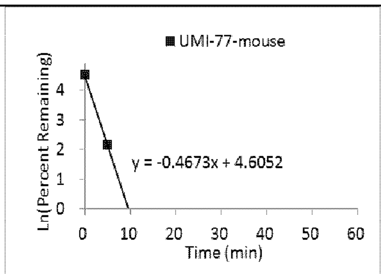  **1.5** | 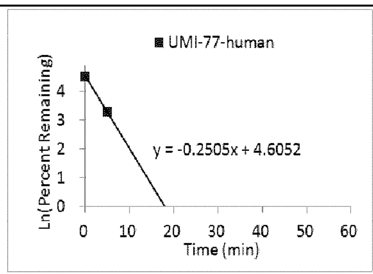  **2.8** |

| | | |
|---|---|---|
| I-2 | 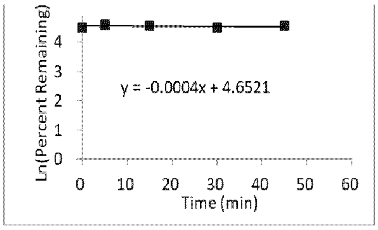<br>>120 | 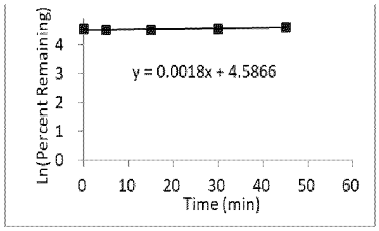<br>>120 |
| I-1 | 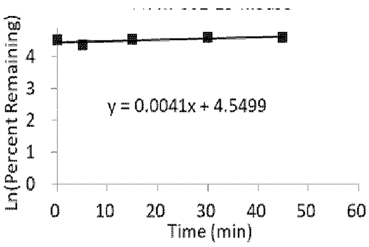<br>>120 | 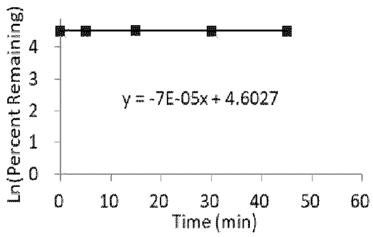<br>>120 |

FIG.19

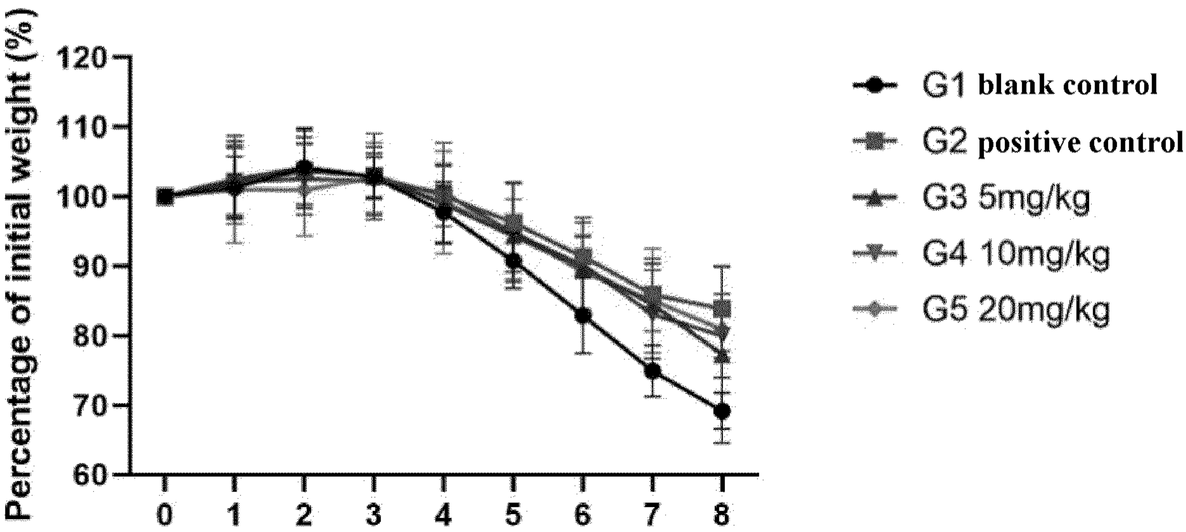


FIG.20

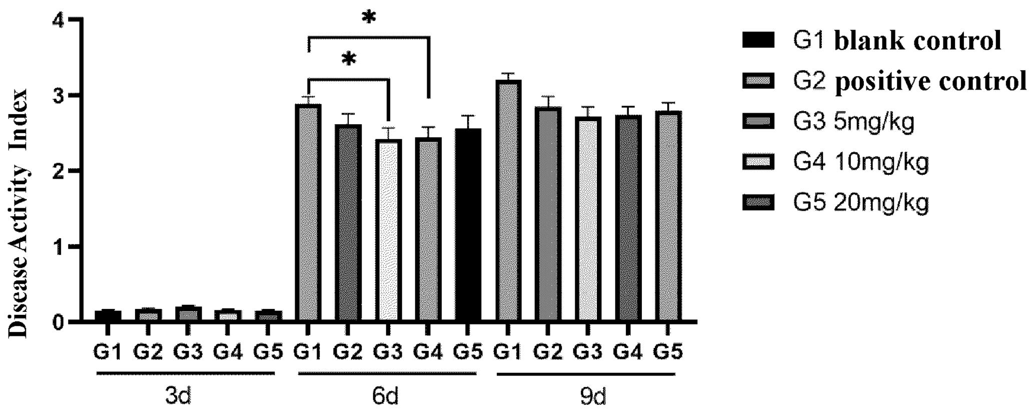


FIG.21

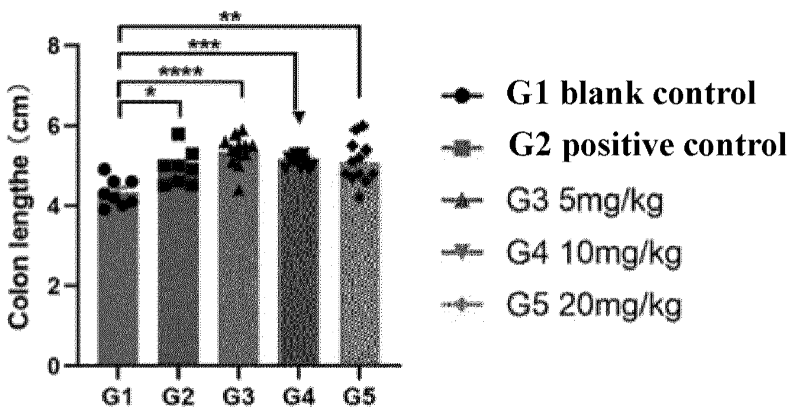


FIG.22

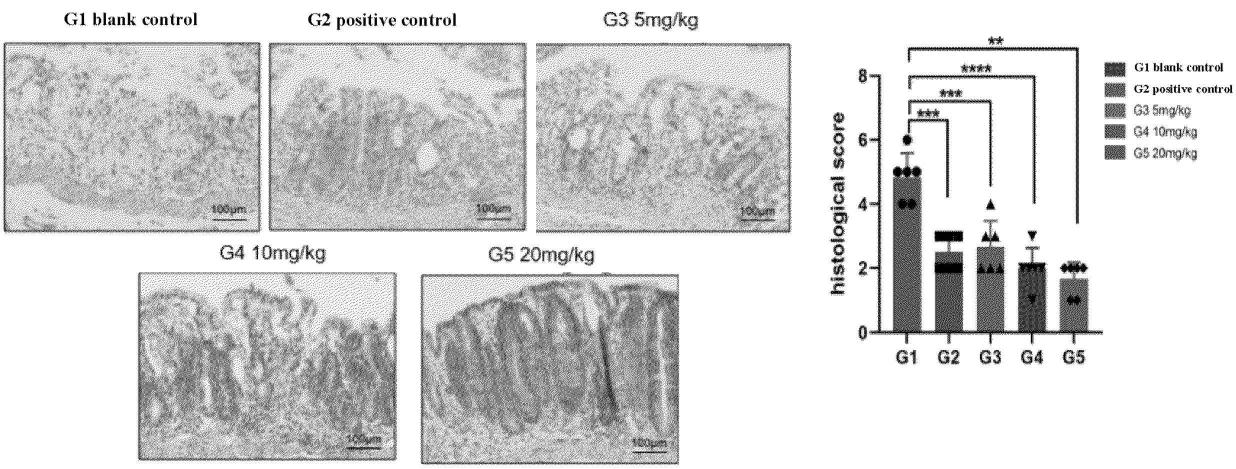


FIG.23

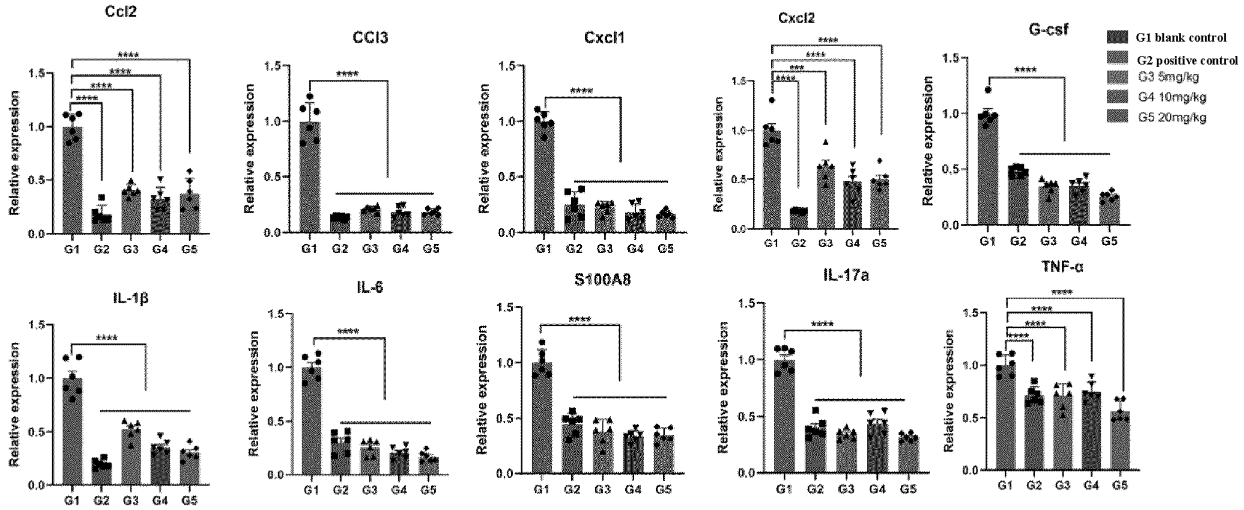
Ccl2
CCl3
Cxcl1
Cxcl2
G-csf
G1 blank control
G2 positive control
G3 5mg/kg
G4 10mg/kg
G5 20mg/kg
IL-1β
IL-6
S100A8
IL-17a
TNF-α
Relative expression
G1 G2 G3 G4 G5

FIG.24

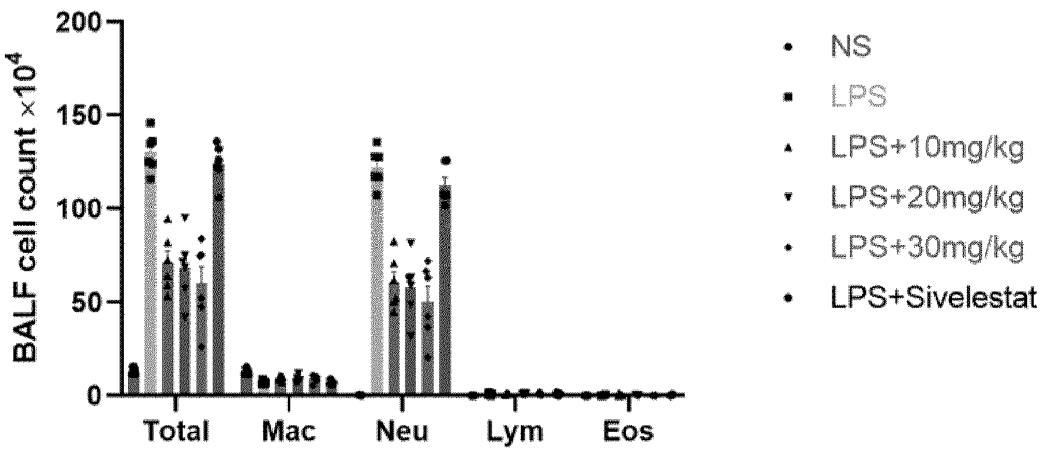
BALF cell count ×10^4
Total
Mac
Neu
Lym
Eos
NS
LPS
LPS+10mg/kg
LPS+20mg/kg
LPS+30mg/kg
LPS+Sivelestat

FIG.25

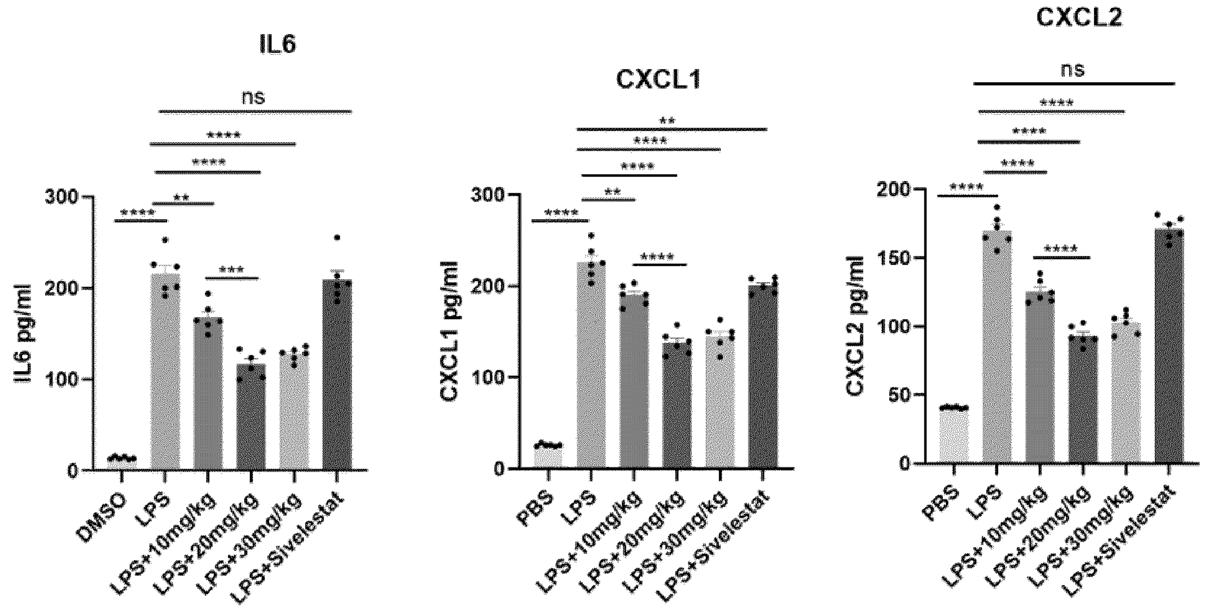
IL6
CXCL1
CXCL2
IL6 pg/ml
CXCL1 pg/ml
CXCL2 pg/ml
DMSO
PBS
LPS
LPS+10mg/kg
LPS+20mg/kg
LPS+30mg/kg
LPS+Sivelestat

FIG.26

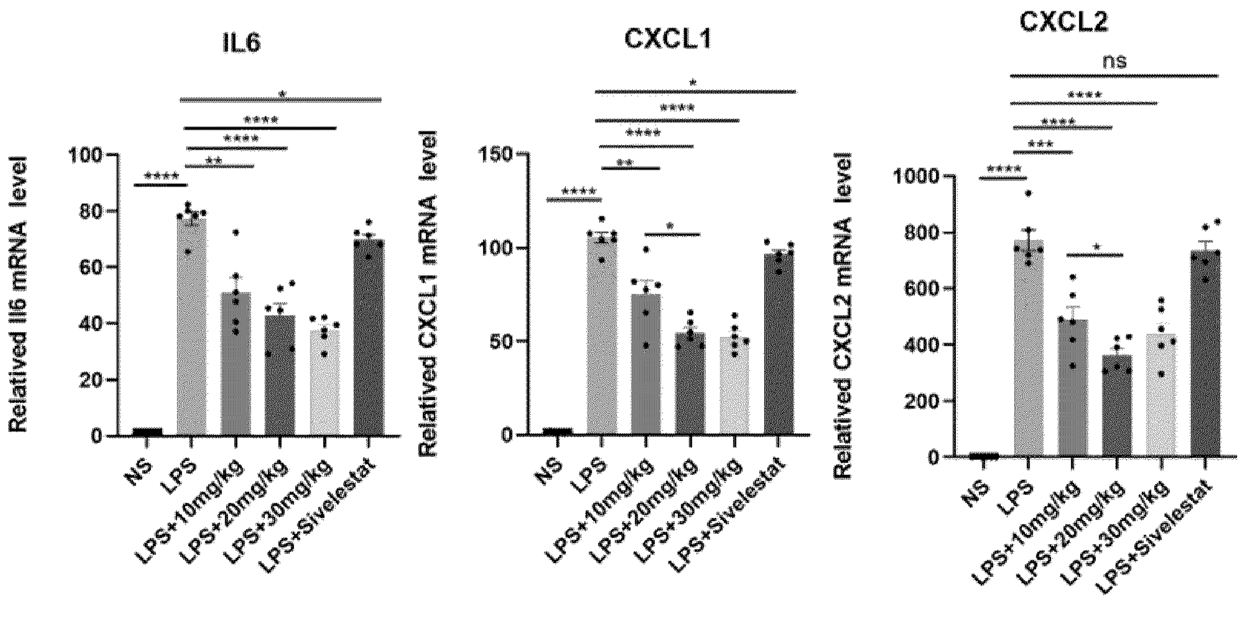
IL6
CXCL1
CXCL2
Relatived Il6 mRNA level
Relatived CXCL1 mRNA level
Relatived CXCL2 mRNA level
NS
LPS
LPS+10mg/kg
LPS+20mg/kg
LPS+30mg/kg
LPS+Sivelestat

FIG.27

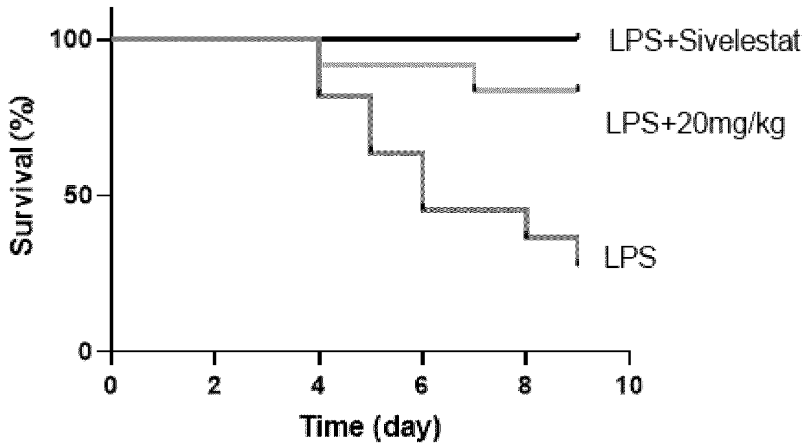


FIG.28

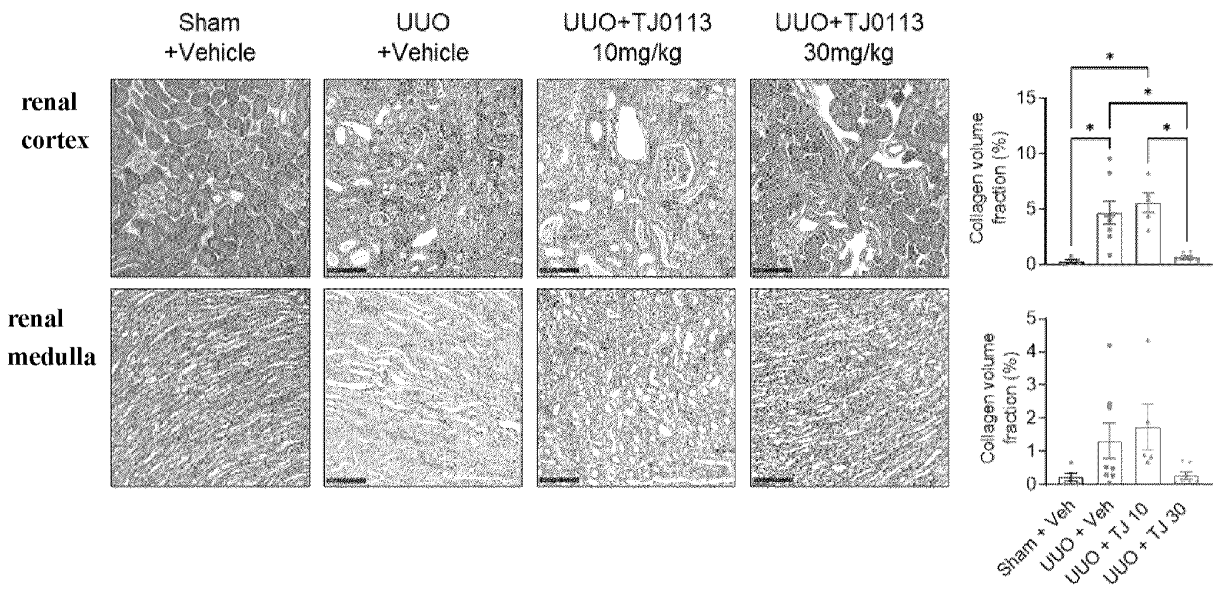


FIG.29

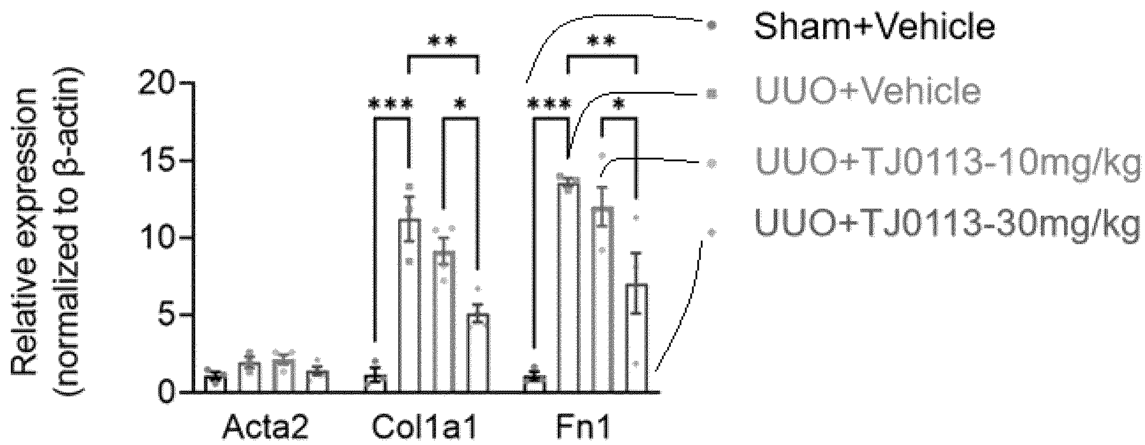


FIG.30

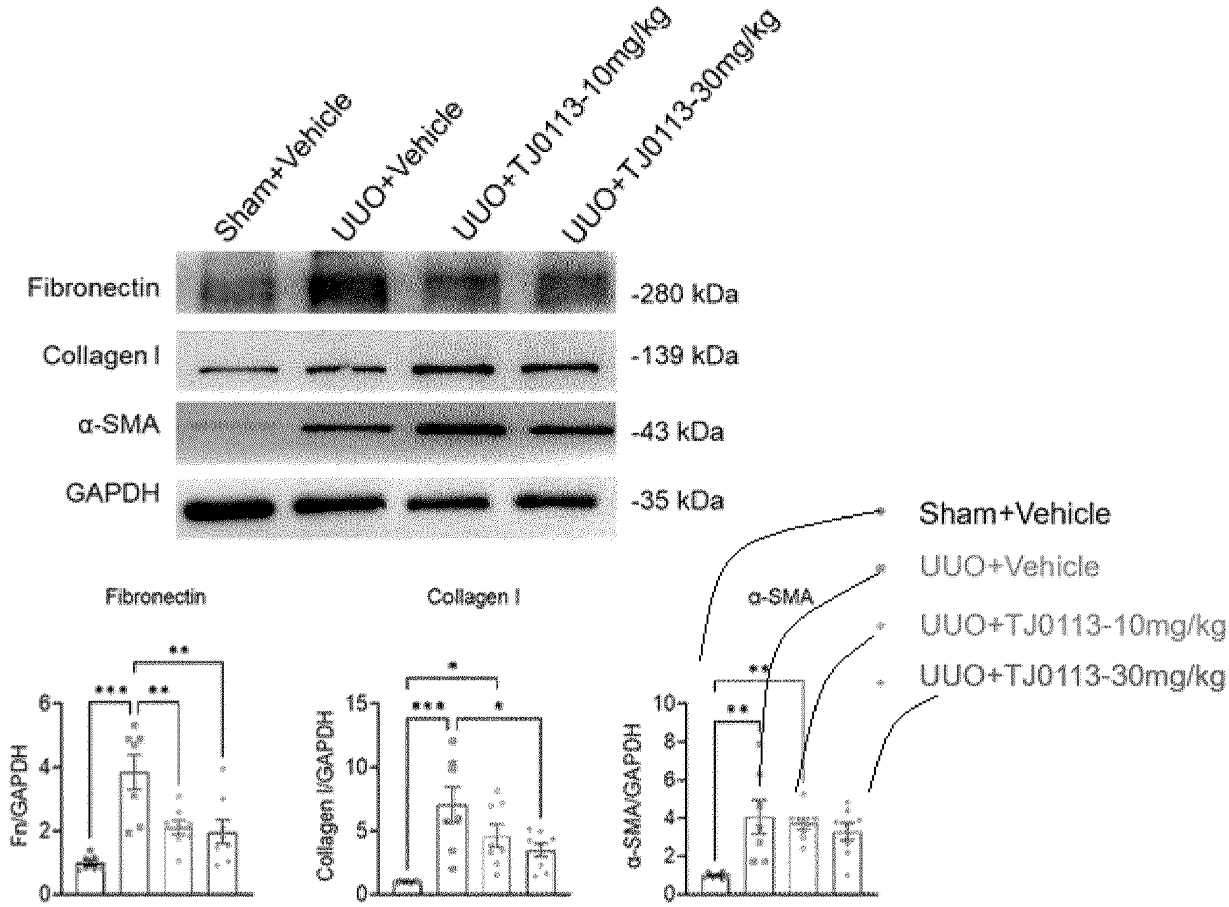
Sham+Vehicle
UUO+Vehicle
UUO+TJ0113-10mg/kg
UUO+TJ0113-30mg/kg
Fibronectin
Collagen I
α-SMA
GAPDH
-280 kDa
-139 kDa
-43 kDa
-35 kDa
Fn/GAPDH
Collagen I/GAPDH
α-SMA/GAPDH

FIG.31

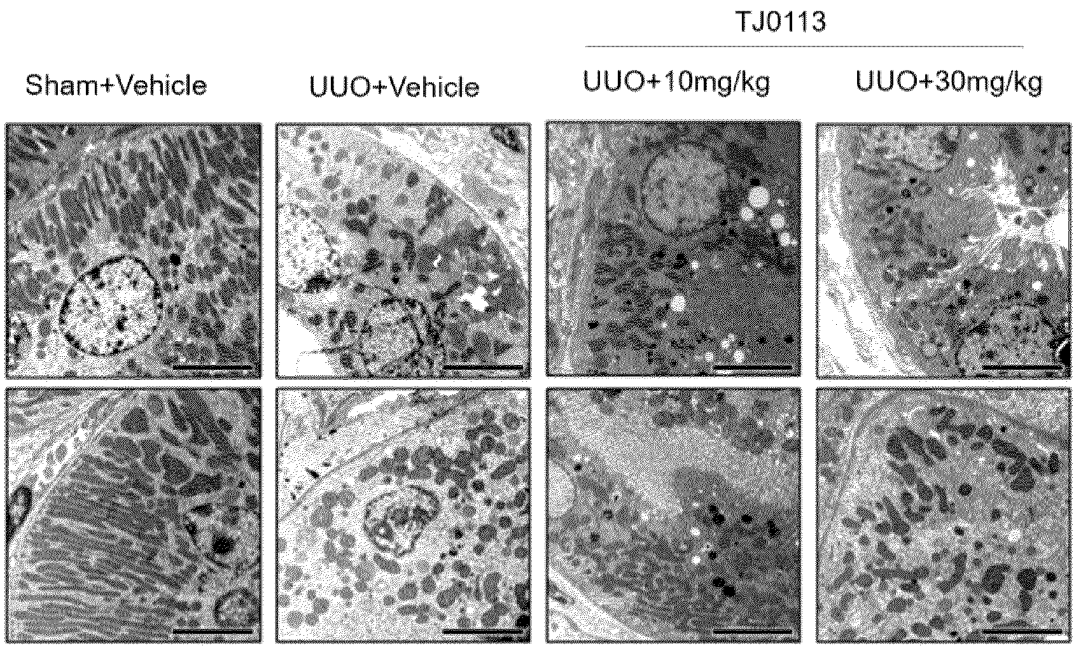
TJ0113
Sham+Vehicle
UUO+Vehicle
UUO+10mg/kg
UUO+30mg/kg

FIG.32

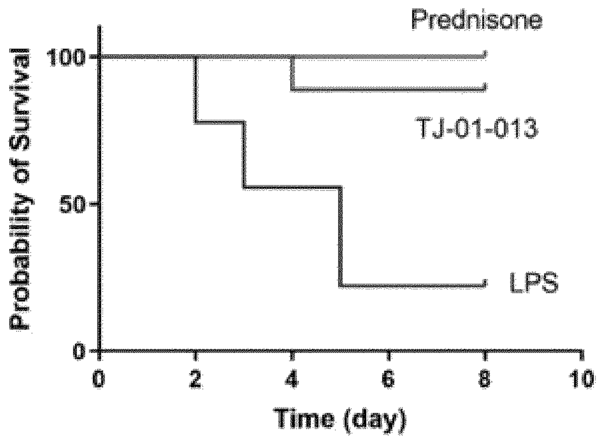


FIG.33

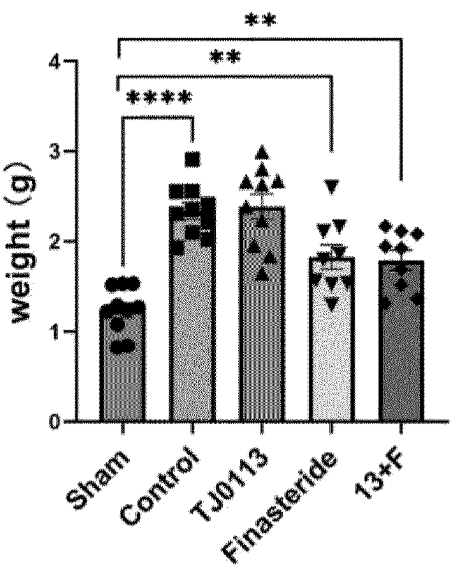


FIG.34

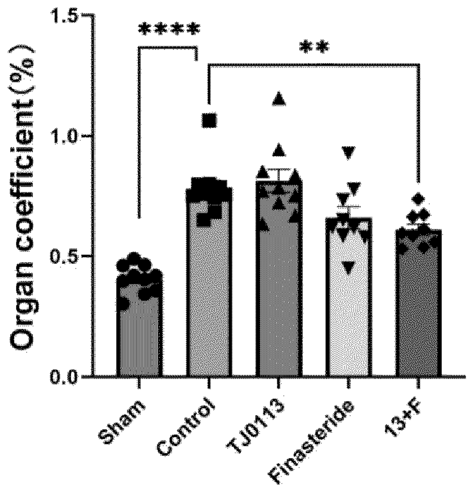


FIG.35

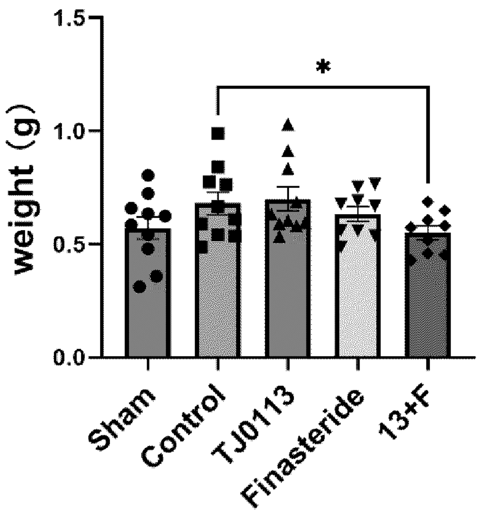


FIG.36

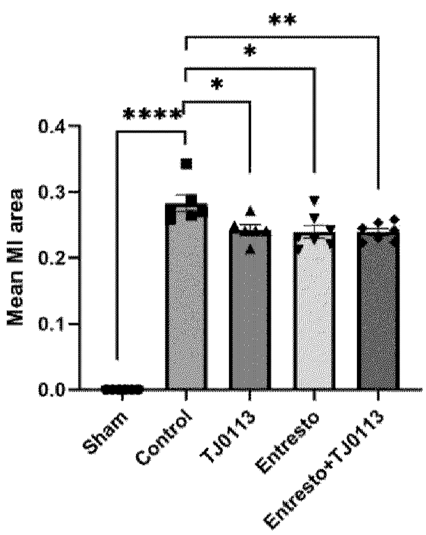


FIG.37

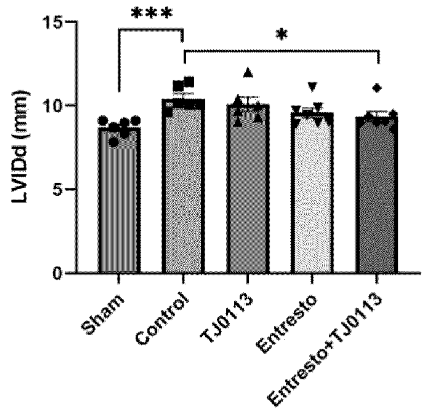


FIG.38

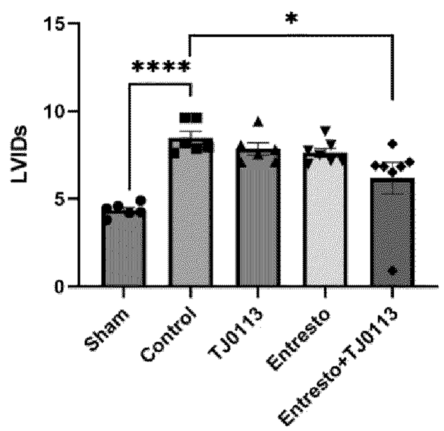


FIG.39

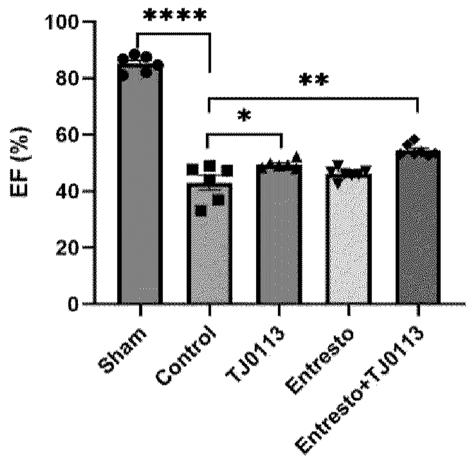


FIG.40

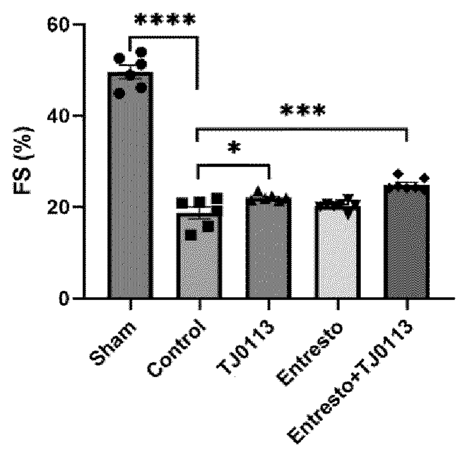


FIG.41

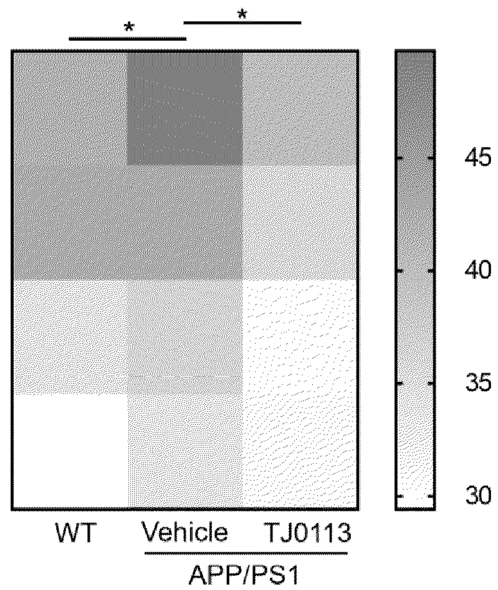


FIG.42

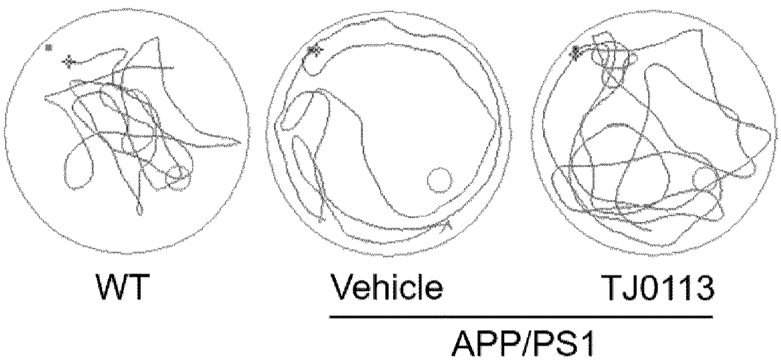


FIG.43

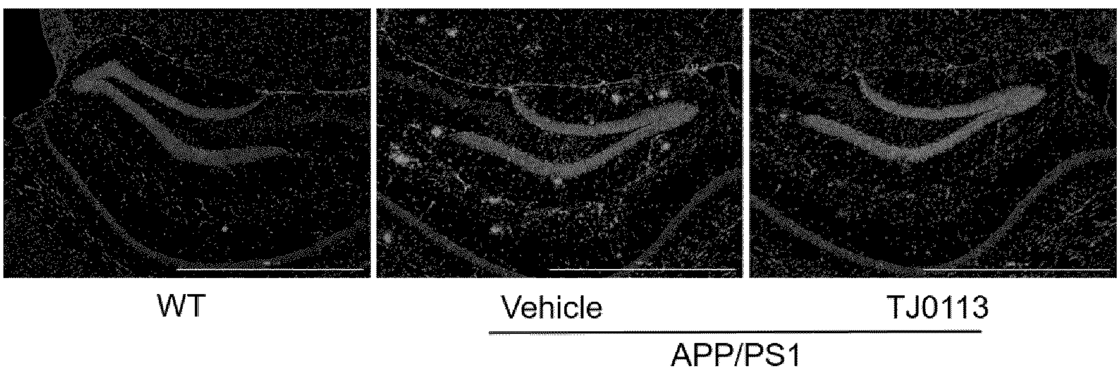


FIG.44

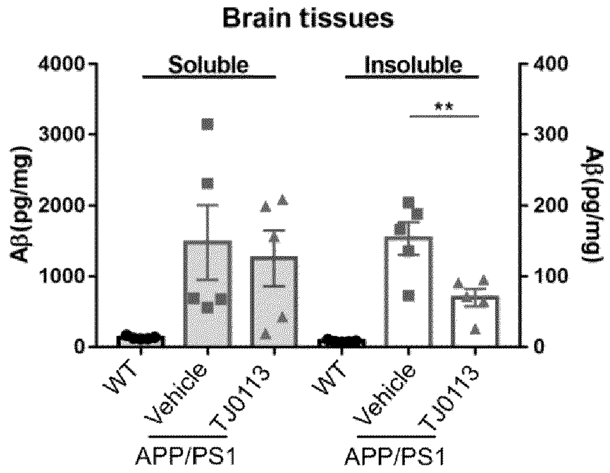


FIG.45

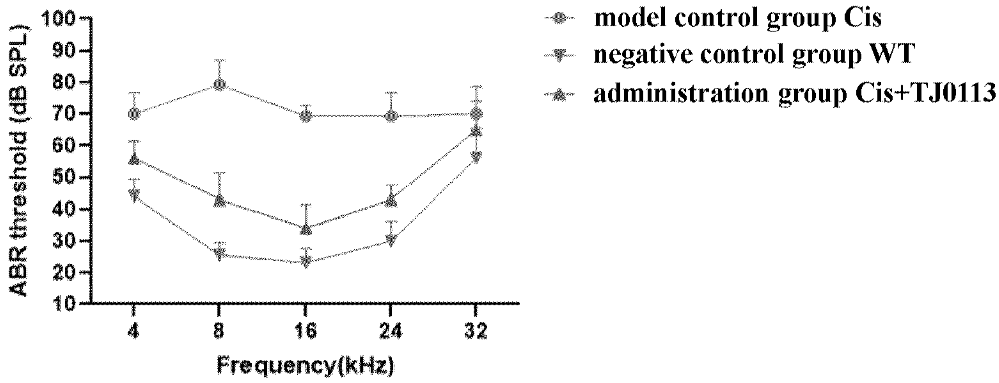


FIG.46

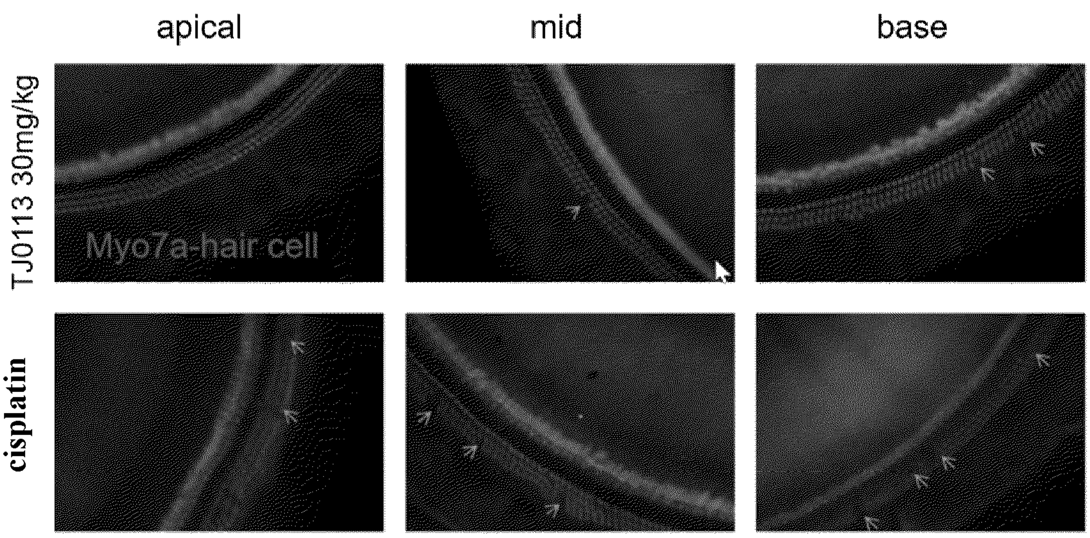


FIG.47

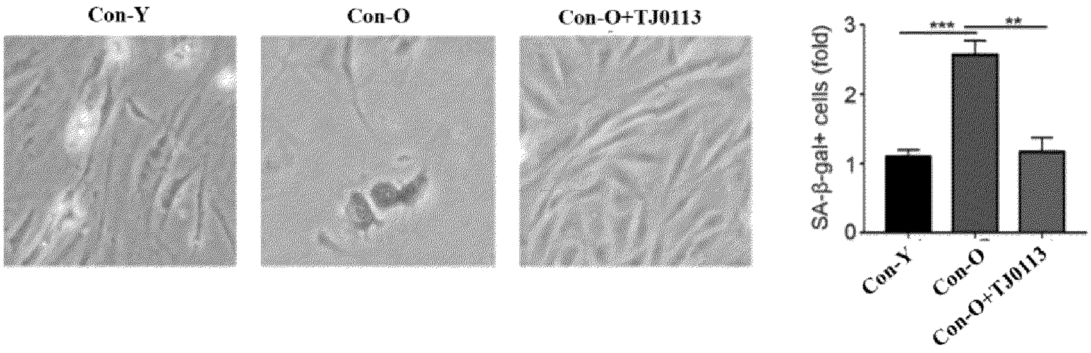


FIG.48

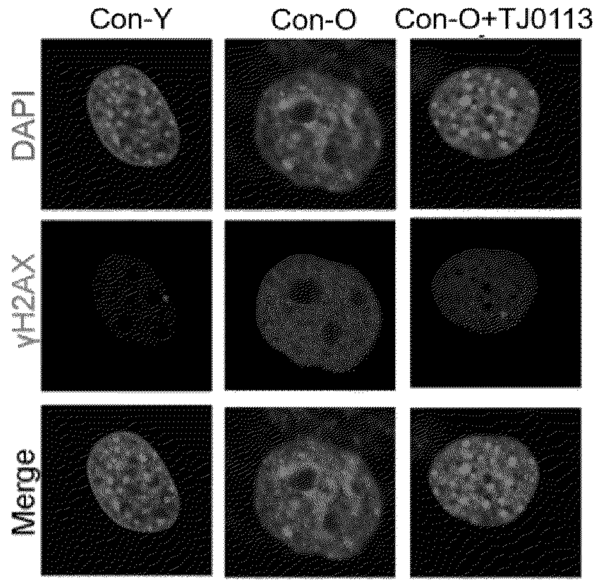


FIG.49

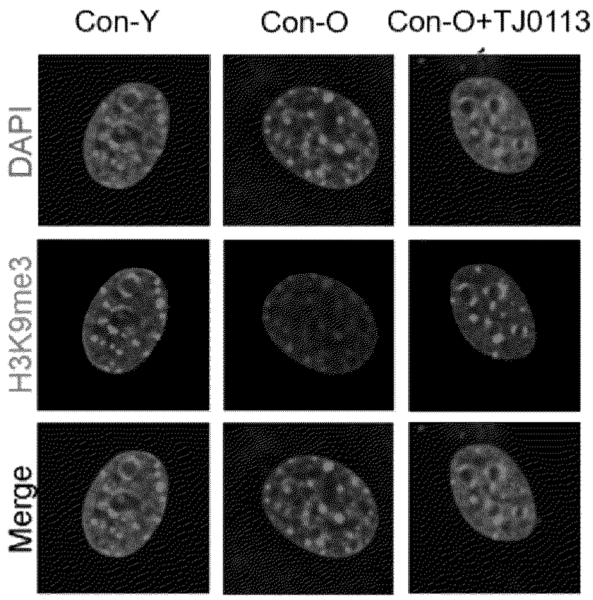


FIG.50

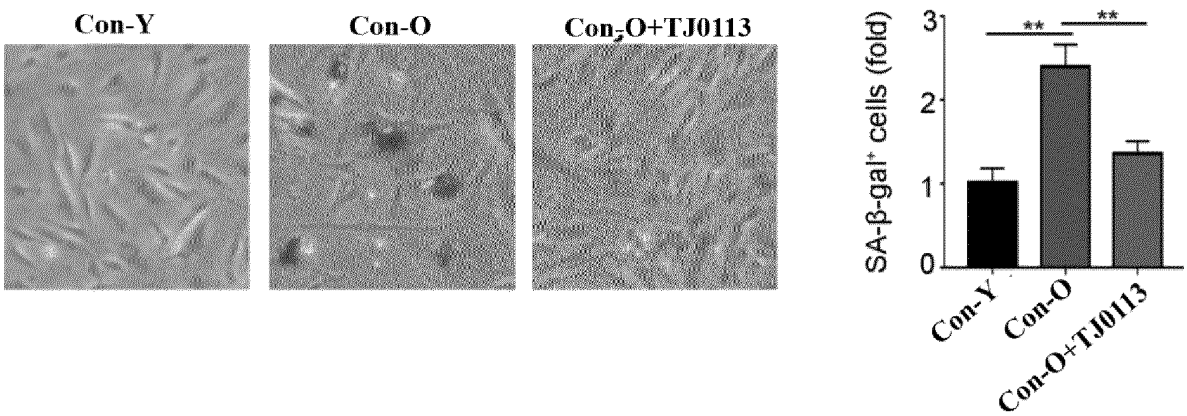


FIG.51

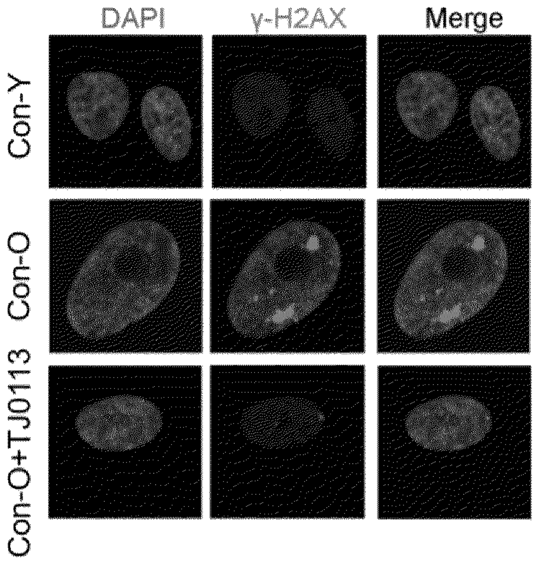


FIG.52

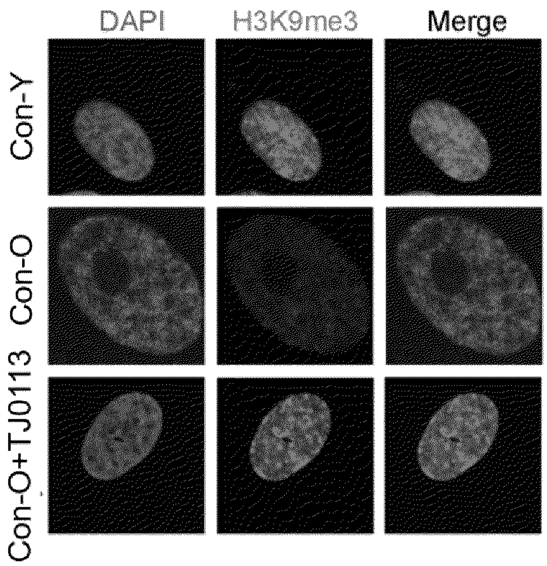


FIG.53

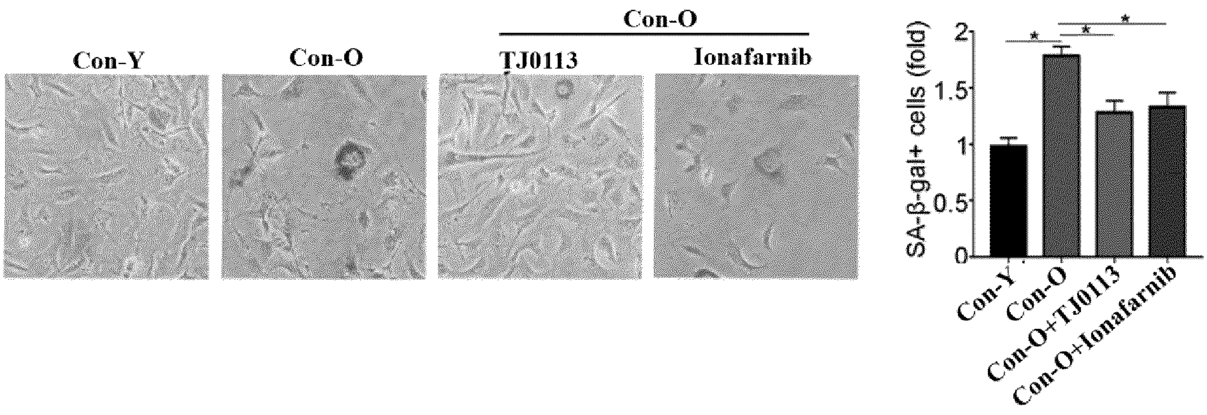


FIG.54

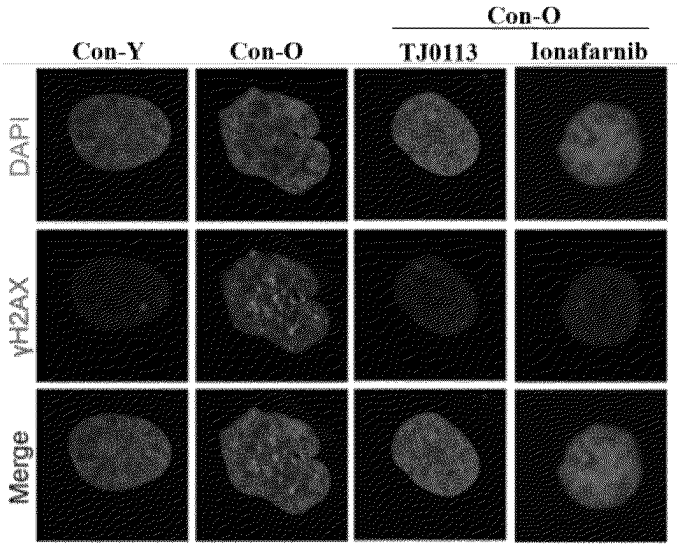


FIG.55

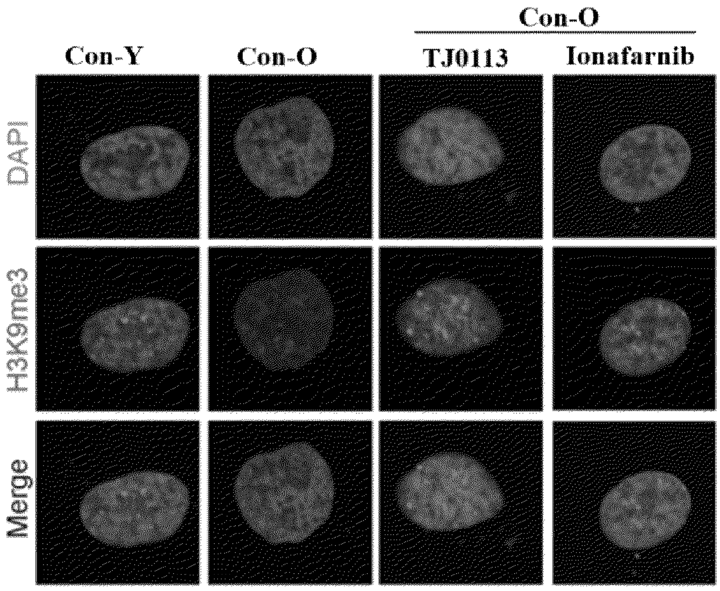


FIG.56

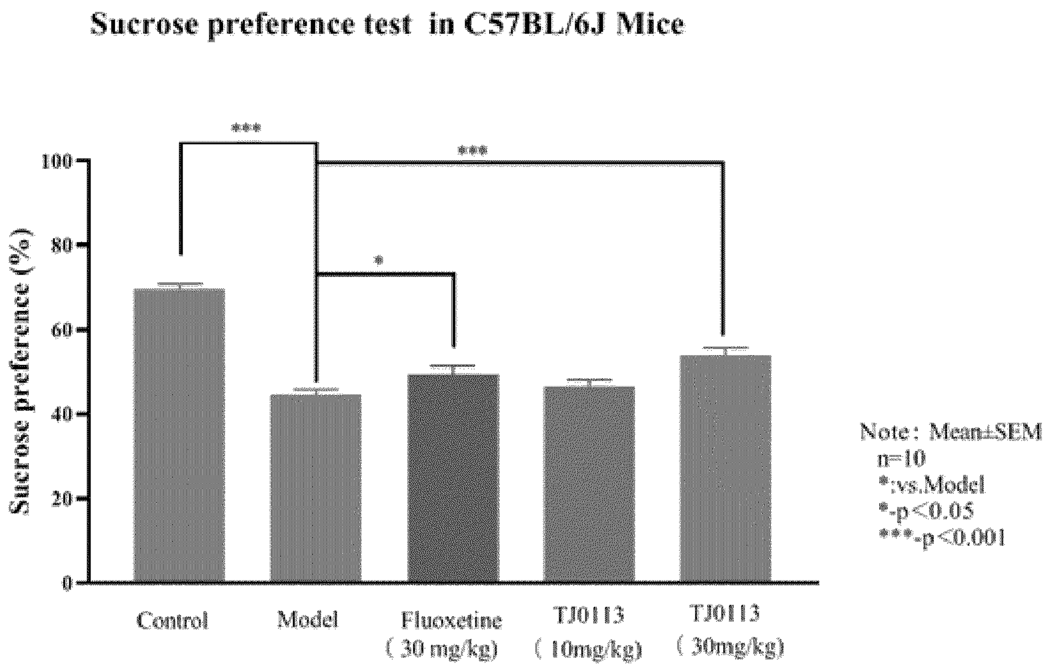
Sucrose preference test in C57BL/6J Mice
Sucrose preference (%)
100
80
60
40
20
0
***
***
*
Control
Model
Fluoxetine
( 30 mg/kg)
TJ0113
( 10mg/kg)
TJ0113
( 30mg/kg)
Note: Mean±SEM
n=10
*:vs.Model
*-p<0.05
***-p<0.001

FIG.57

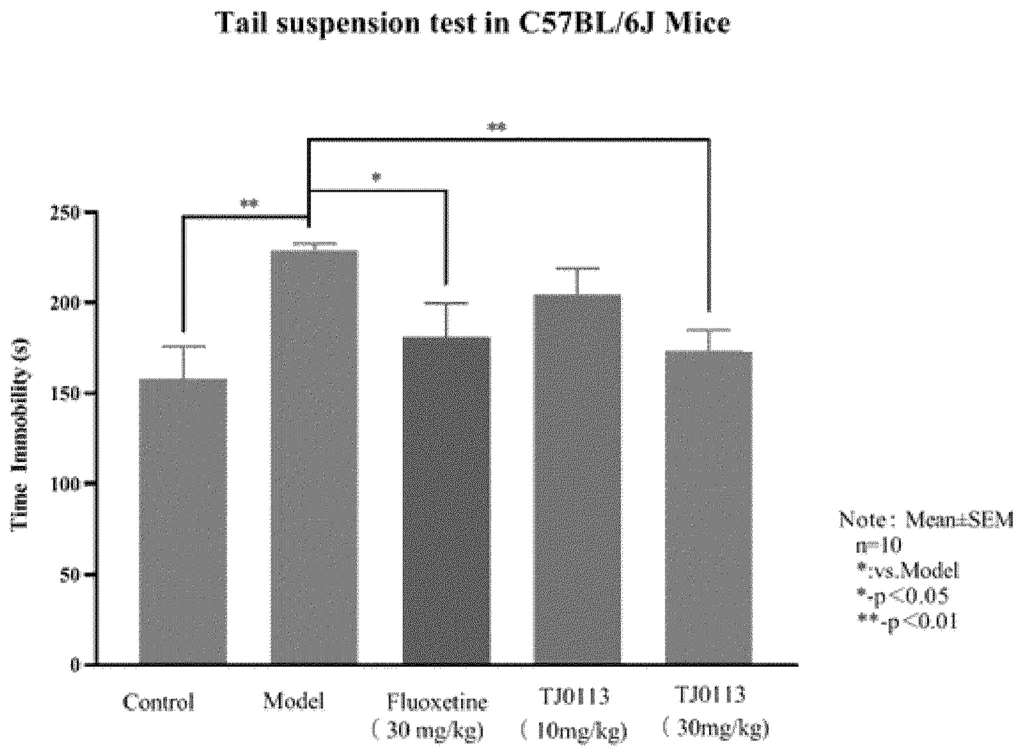
Tail suspension test in C57BL/6J Mice
Time Immobility (s)
250
200
150
100
50
0
**
**
*
Control
Model
Fluoxetine
( 30 mg/kg)
TJ0113
( 10mg/kg)
TJ0113
( 30mg/kg)
Note: Mean±SEM
n=10
*:vs.Model
*-p<0.05
**-p<0.01

FIG.58

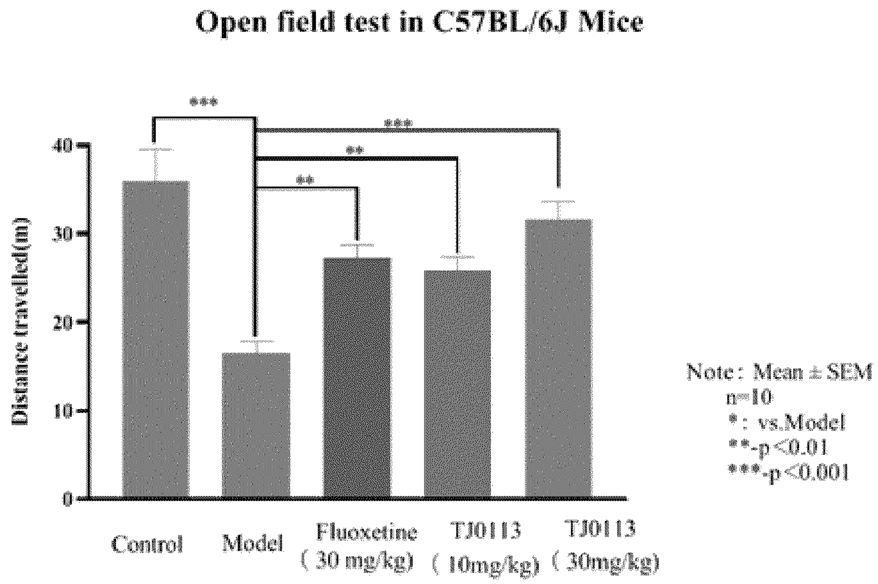
Open field test in C57BL/6J Mice
Distance travelled(m)
0
10
20
30
40
***
***
**
**
Control
Model
Fluoxetine (30 mg/kg)
TJ0113 (10mg/kg)
TJ0113 (30mg/kg)
Note: Mean ± SEM
n=10
*: vs.Model
**-p<0.01
***-p<0.001

FIG.59

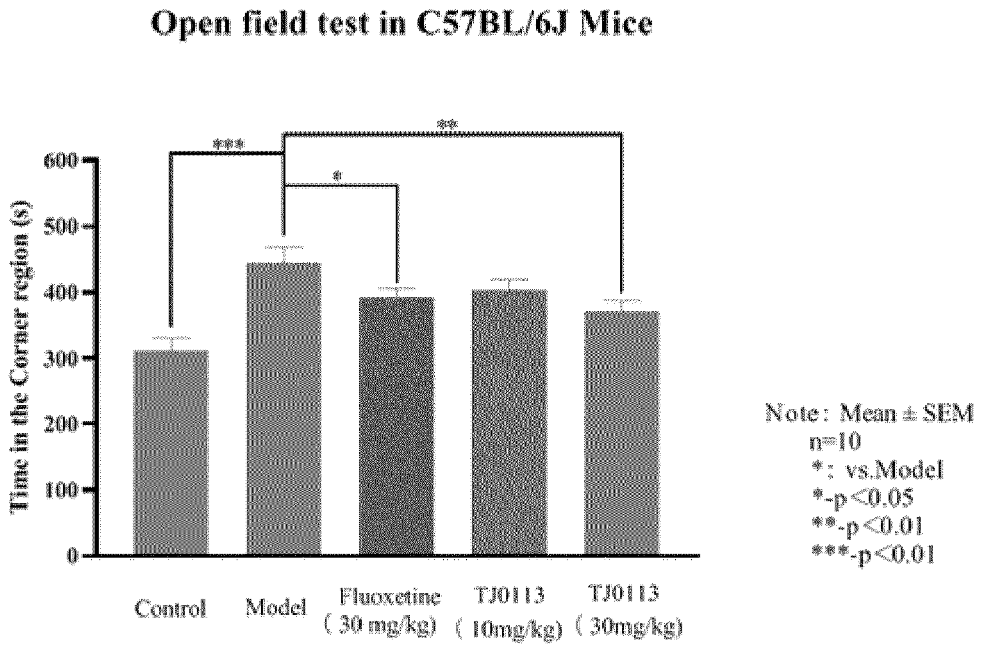
Open field test in C57BL/6J Mice
Time in the Corner region (s)
0
100
200
300
400
500
600
***
**
*
Control
Model
Fluoxetine (30 mg/kg)
TJ0113 (10mg/kg)
TJ0113 (30mg/kg)
Note: Mean ± SEM
n=10
*: vs.Model
*-p<0.05
**-p<0.01
***-p<0.01

FIG.60

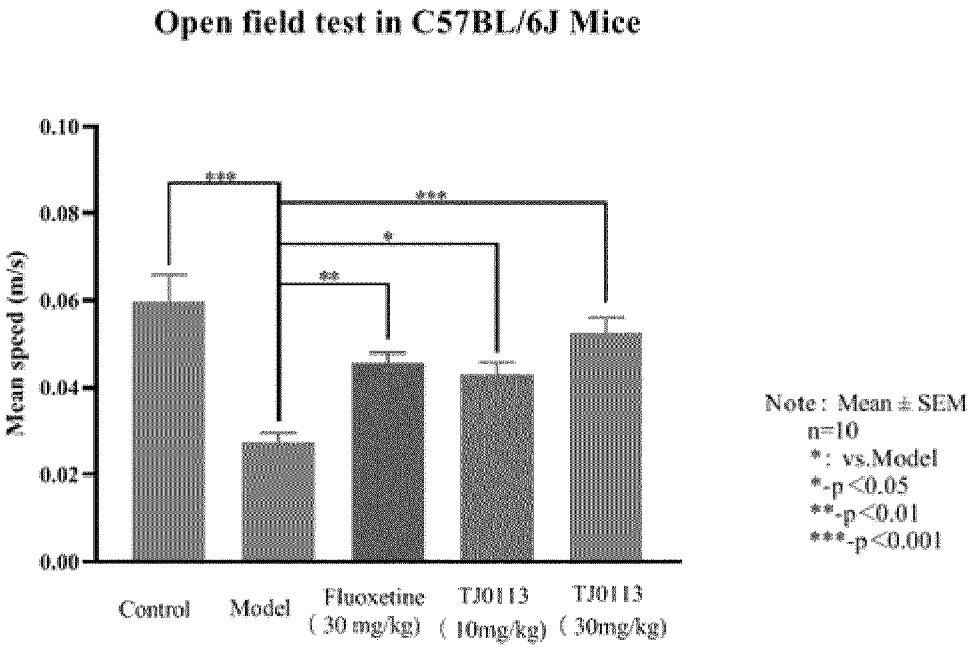
Open field test in C57BL/6J Mice
Mean speed (m/s)
0.10
0.08
0.06
0.04
0.02
0.00
***
***
*
**
Control
Model
Fluoxetine (30 mg/kg)
TJ0113 (10mg/kg)
TJ0113 (30mg/kg)
Note: Mean ± SEM
n=10
*: vs.Model
*-p<0.05
**-p<0.01
***-p<0.001

FIG.61

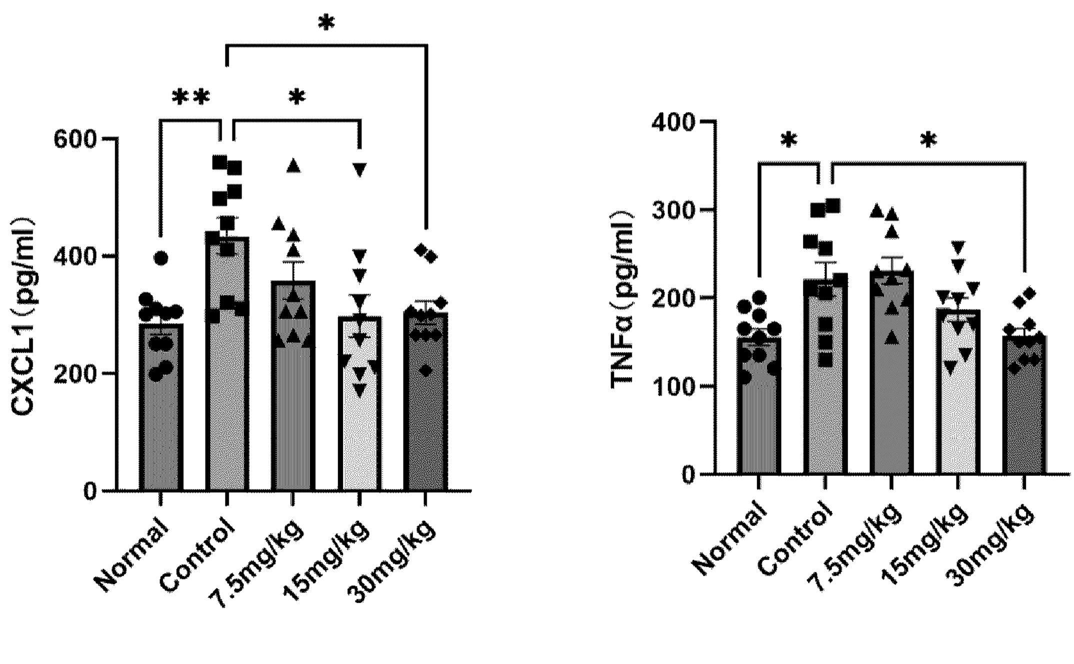
CXCL1 (pg/ml)
600
400
200
0
*
**
*
Normal
Control
7.5mg/kg
15mg/kg
30mg/kg
TNFα (pg/ml)
400
300
200
100
0
*
*
Normal
Control
7.5mg/kg
15mg/kg
30mg/kg

FIG.62

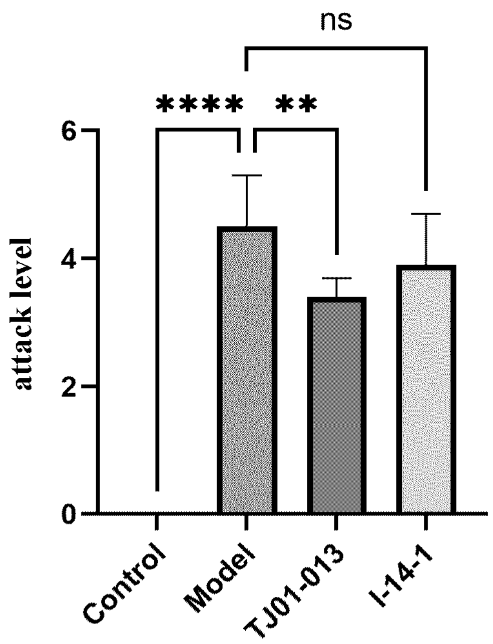
ns
****
**
6
4
2
0
attack level
Control
Model
TJ01-013
I-14-1

FIG.63

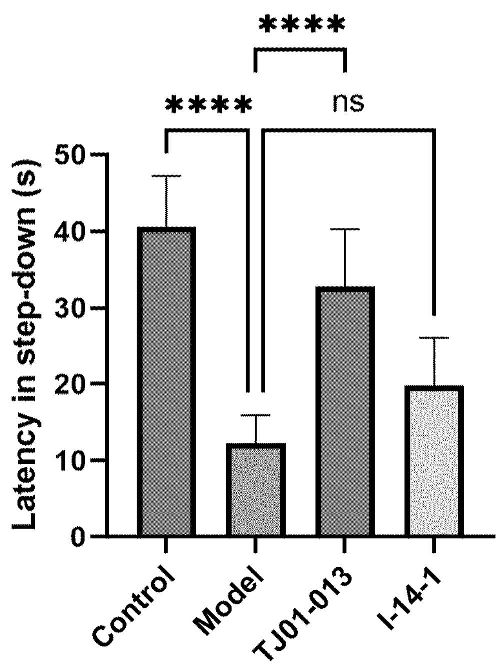
****
****
ns
50
40
30
20
10
0
Latency in step-down (s)
Control
Model
TJ01-013
I-14-1

FIG.64

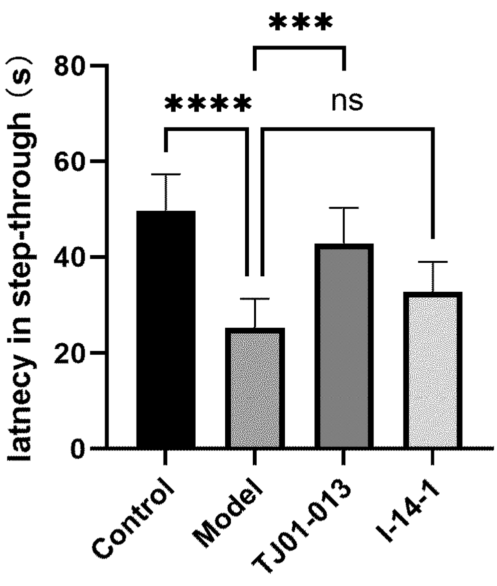

***
****
ns
80
60
40
20
0
latnecy in step-through（s）
Control
Model
TJ01-013
I-14-1


FIG.65

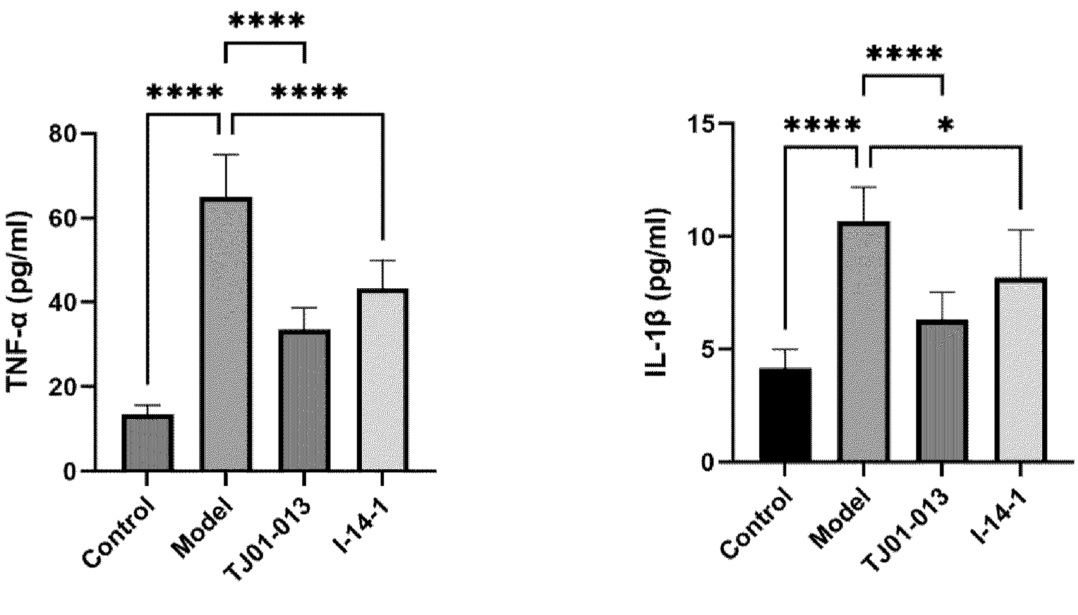

****
****
****
80
60
40
20
0
TNF-α (pg/ml)
Control
Model
TJ01-013
I-14-1
****
****
*
15
10
5
0
IL-1β (pg/ml)
Control
Model
TJ01-013
I-14-1


FIG.66

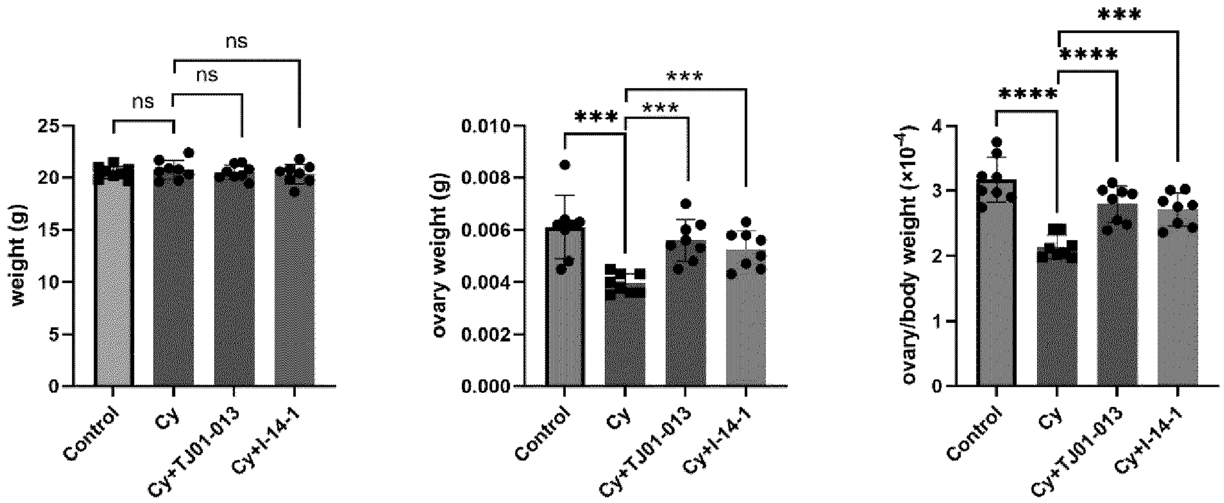
ns
ns
ns
25
20
15
10
5
0
weight (g)
Control
Cy
Cy+TJ01-013
Cy+I-14-1
***
***
***
0.010
0.008
0.006
0.004
0.002
0.000
ovary weight (g)
Control
Cy
Cy+TJ01-013
Cy+I-14-1
***
****
****
4
3
2
1
0
ovary/body weight (×10$^{-4}$)
Control
Cy
Cy+TJ01-013
Cy+I-14-1

FIG.67

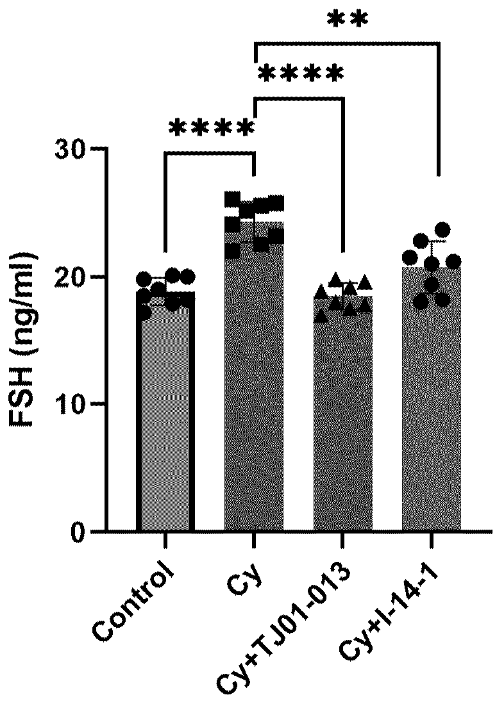
**
****
****
30
20
10
0
FSH (ng/ml)
Control
Cy
Cy+TJ01-013
Cy+I-14-1

FIG.68

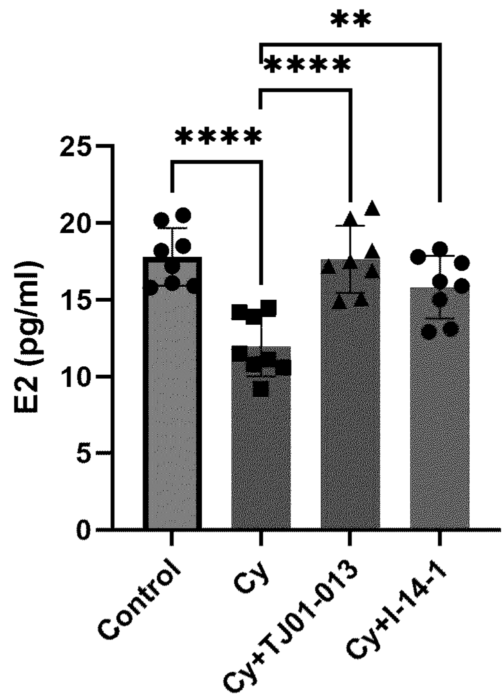
**
****
****
25
20
15
10
5
0
E2 (pg/ml)
Control
Cy
Cy+TJ01-013
Cy+I-14-1

FIG.69

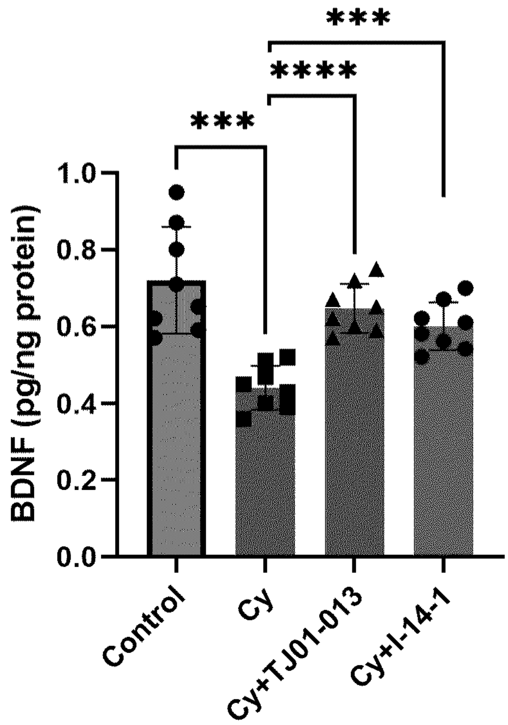
***
****
***
1.0
0.8
0.6
0.4
0.2
0.0
BDNF (pg/ng protein)
Control
Cy
Cy+TJ01-013
Cy+I-14-1

FIG.70

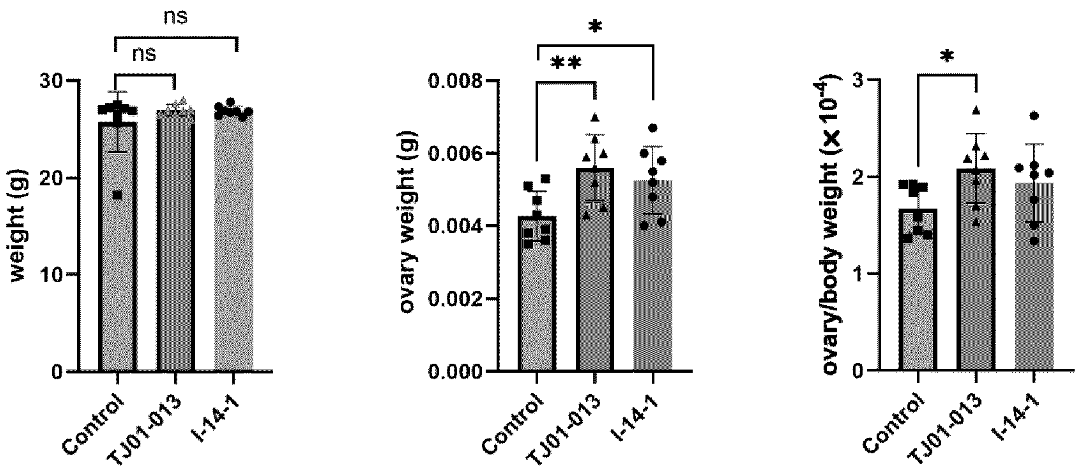
ns
ns
30
20
10
0
weight (g)
Control
TJ01-013
I-14-1
*
**
0.008
0.006
0.004
0.002
0.000
ovary weight (g)
Control
TJ01-013
I-14-1
*
3
2
1
0
ovary/body weight (×10⁻⁴)
Control
TJ01-013
I-14-1

FIG.71

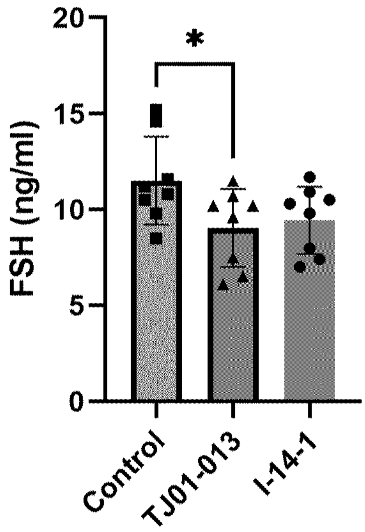
20
15
10
5
0
*
FSH (ng/ml)
Control
TJ01-013
I-14-1

FIG.72

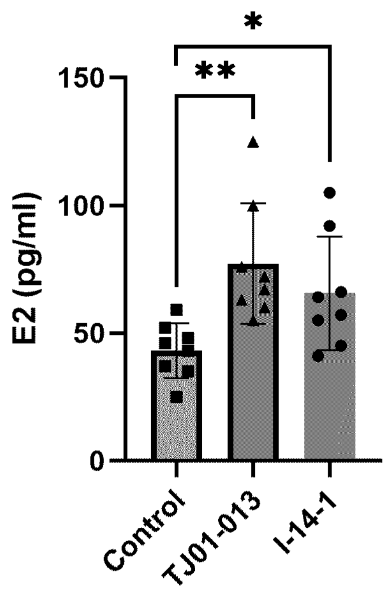
E2 (pg/ml)
150
100
50
0
**
*
Control
TJ01-013
I-14-1

FIG.73

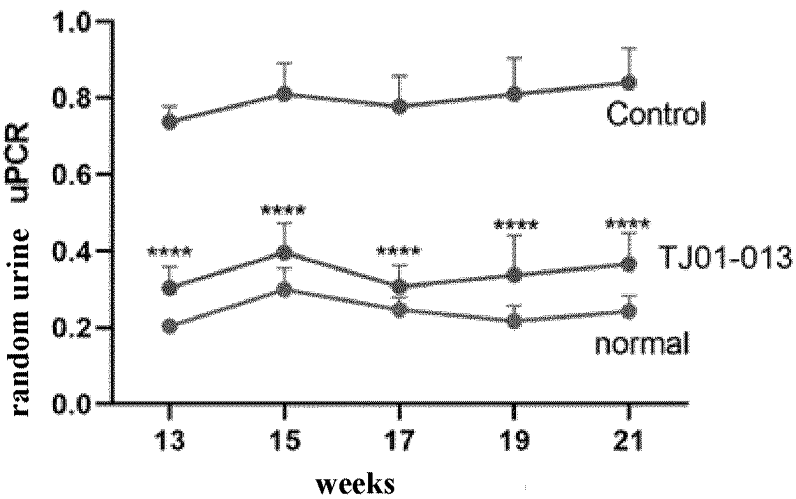
random urine uPCR
1.0
0.8
0.6
0.4
0.2
0.0
13
15
17
19
21
weeks
Control
TJ01-013
normal
****
****
****
****
****

FIG.74

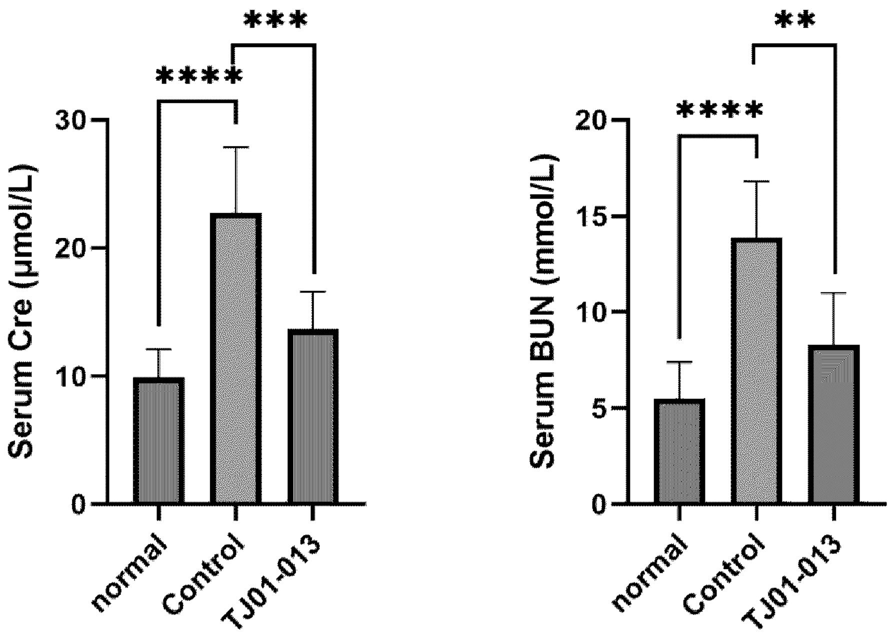
Serum Cre (μmol/L)
0
10
20
30
****
***
normal
Control
TJ01-013
Serum BUN (mmol/L)
0
5
10
15
20
****
**

FIG.75

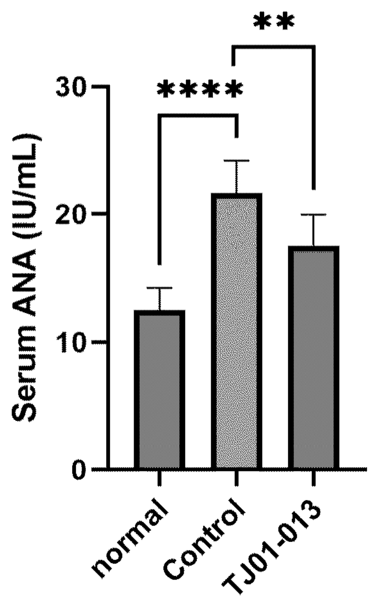
Serum ANA (IU/mL)
0
10
20
30
****
**
normal
Control
TJ01-013

FIG.76

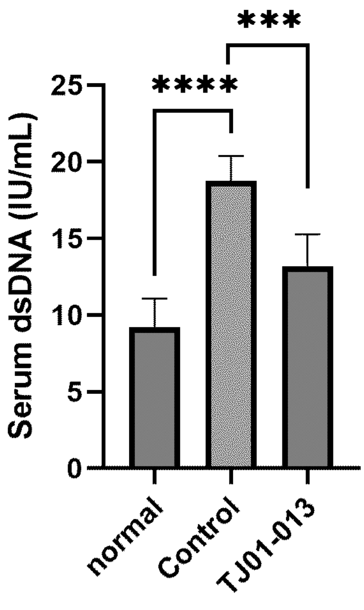
***
****
25
20
15
10
5
0
Serum dsDNA (IU/mL)
normal
Control
TJ01-013

FIG.77

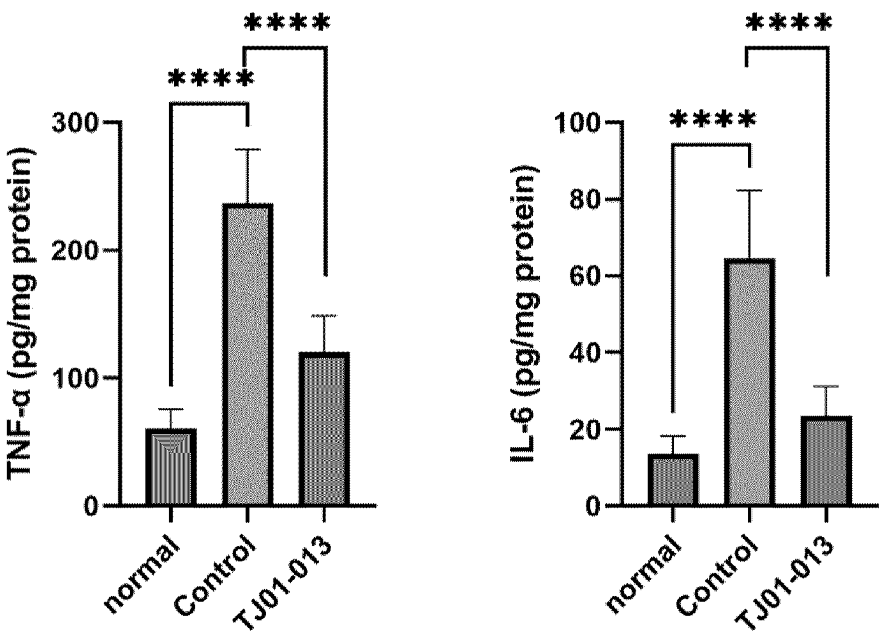
****
****
300
200
100
0
TNF-α (pg/mg protein)
normal
Control
TJ01-013
****
****
100
80
60
40
20
0
IL-6 (pg/mg protein)
normal
Control
TJ01-013

FIG.78

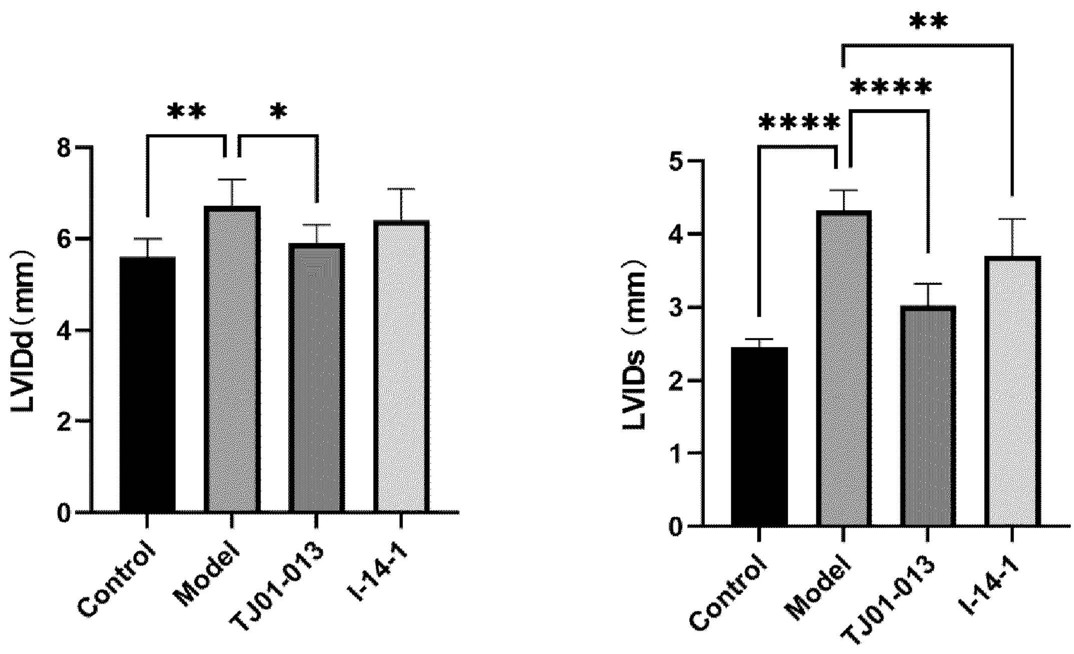
**
*
8
6
4
2
0
LVIDd（mm）
Control
Model
TJ01-013
I-14-1
**
****
****
5
4
3
2
1
0
LVIDs（mm）
Control
Model
TJ01-013
I-14-1

FIG.79

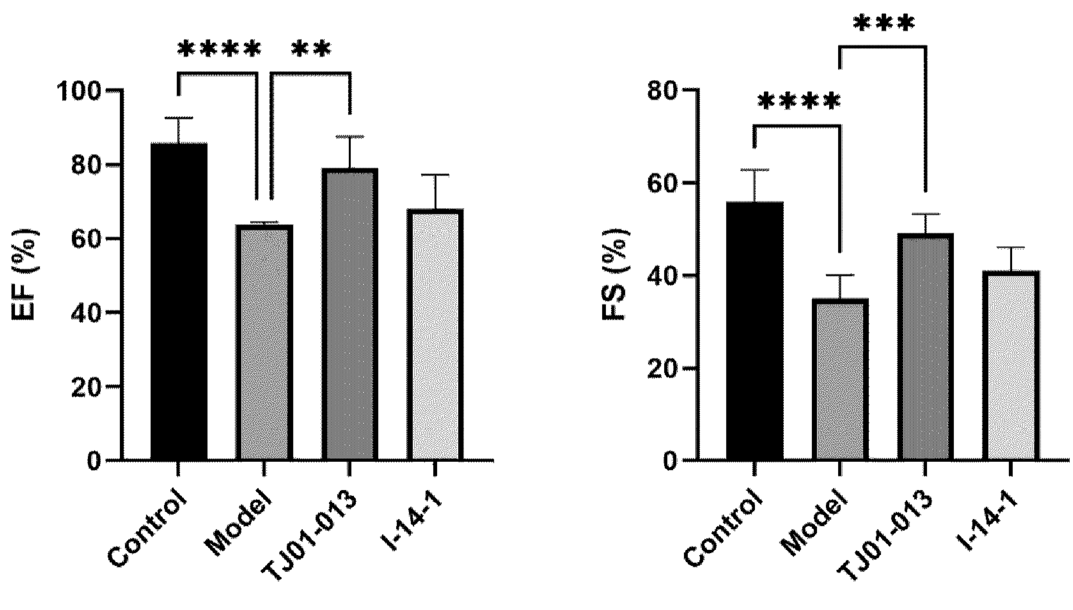
****
**
100
80
60
40
20
0
EF (%)
Control
Model
TJ01-013
I-14-1
***
****
80
60
40
20
0
FS (%)
Control
Model
TJ01-013
I-14-1

FIG.80

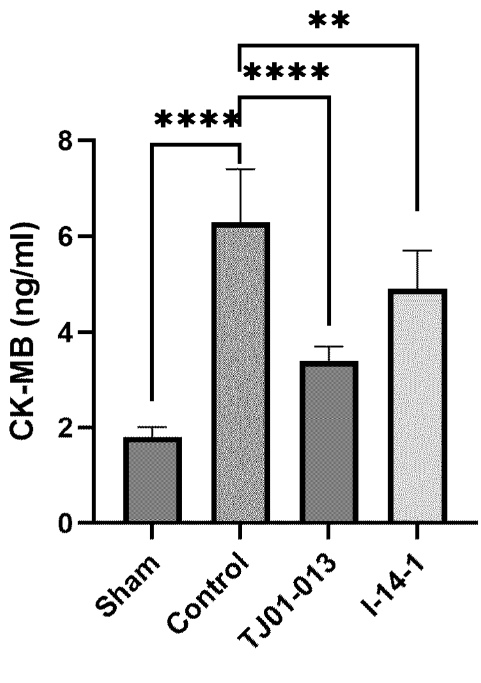
**
****
****
8
6
4
2
0
CK-MB (ng/ml)
Sham
Control
TJ01-013
I-14-1

FIG.81

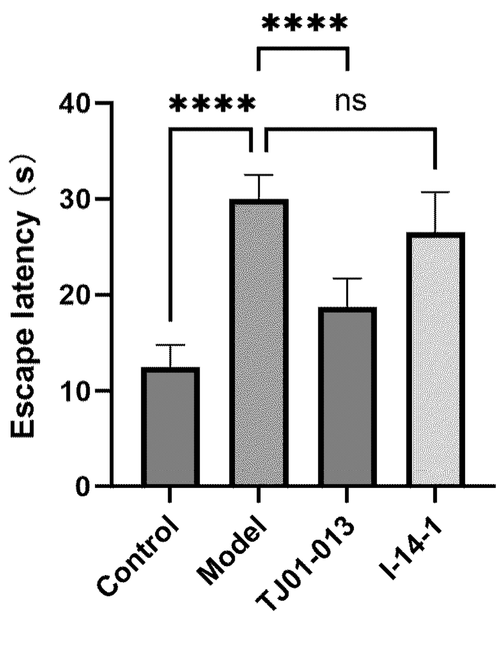
****
****
ns
40
30
20
10
0
Escape latency（s）
Control
Model
TJ01-013
I-14-1

FIG.82

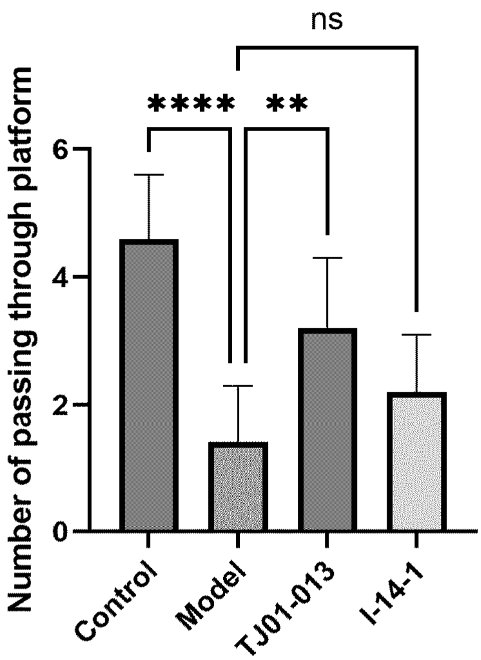
ns
****
**
Number of passing through platform
6
4
2
0
Control
Model
TJ01-013
I-14-1

FIG.83

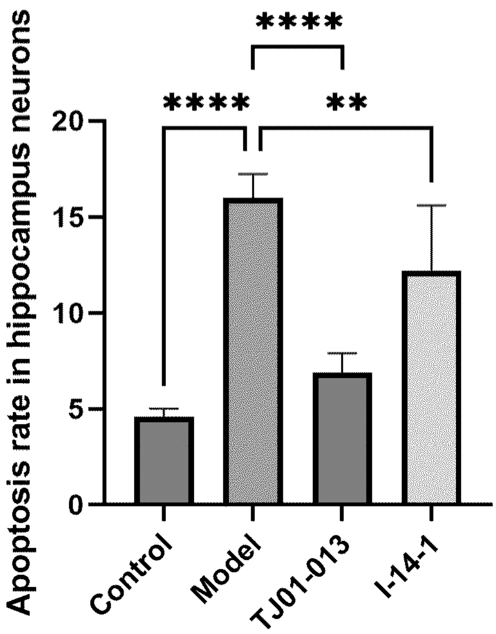
****
****
**
Apoptosis rate in hippocampus neurons
20
15
10
5
0
Control
Model
TJ01-013
I-14-1

FIG.84

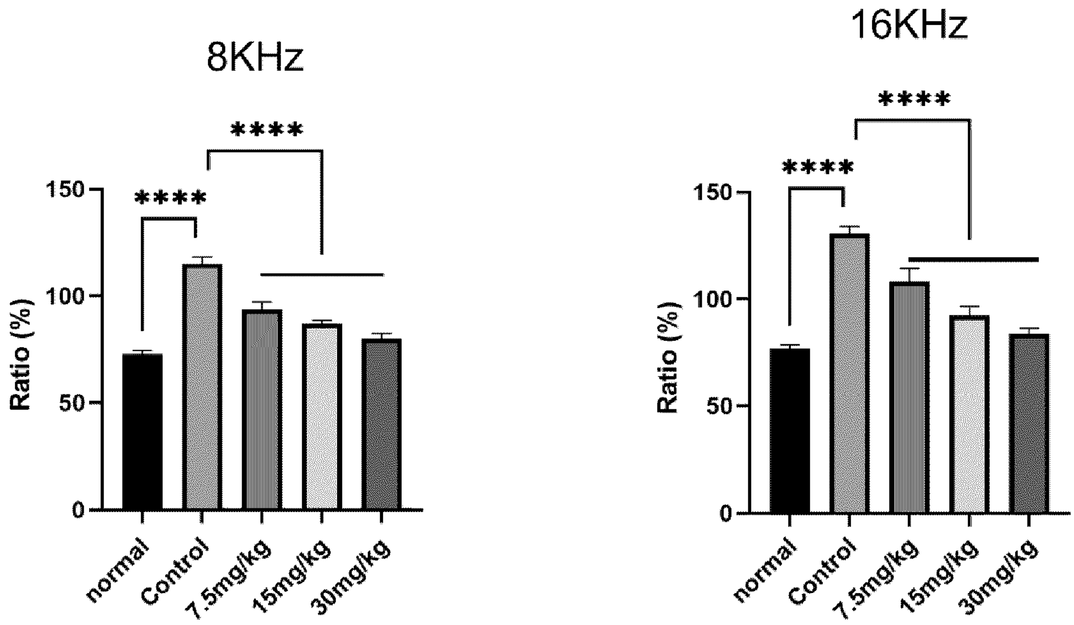
8KHz
****
****
150
100
50
0
Ratio (%)
normal
Control
7.5mg/kg
15mg/kg
30mg/kg
16KHz
****
****
150
100
50
0
Ratio (%)
normal
Control
7.5mg/kg
15mg/kg
30mg/kg

FIG.85

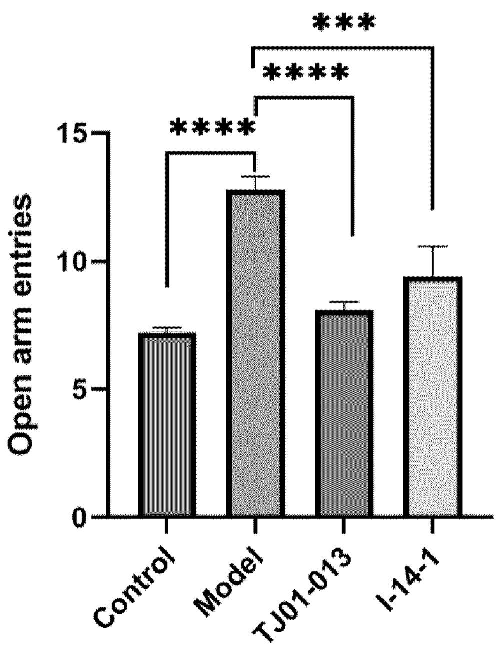
***
****
****
15
10
5
0
Open arm entries
Control
Model
TJ01-013
I-14-1

FIG.86

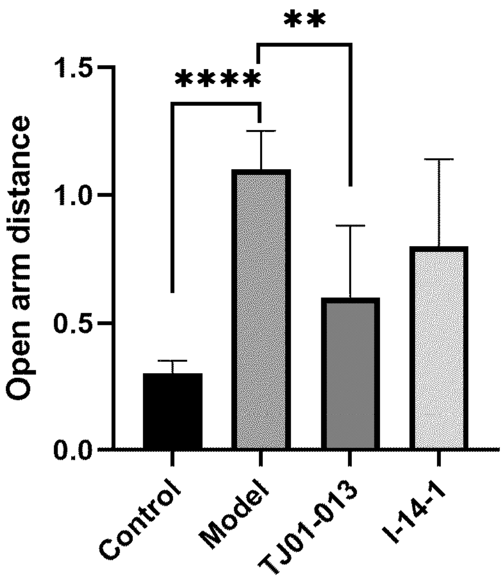
**
****
1.5
1.0
0.5
0.0
Open arm distance
Control
Model
TJ01-013
I-14-1

FIG.87

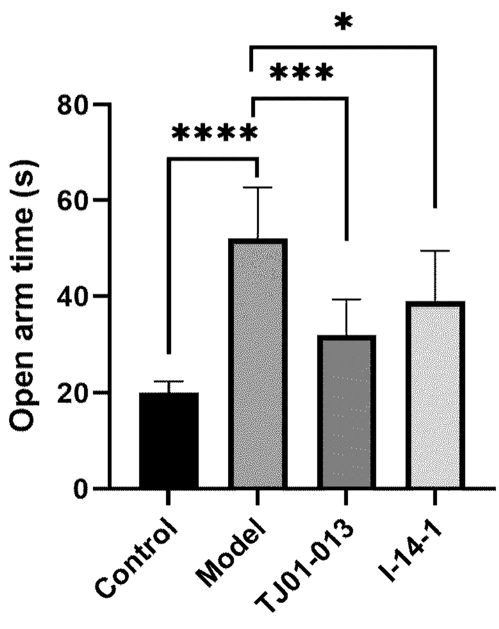
*
***
****
80
60
40
20
0
Open arm time (s)
Control
Model
TJ01-013
I-14-1

FIG.88

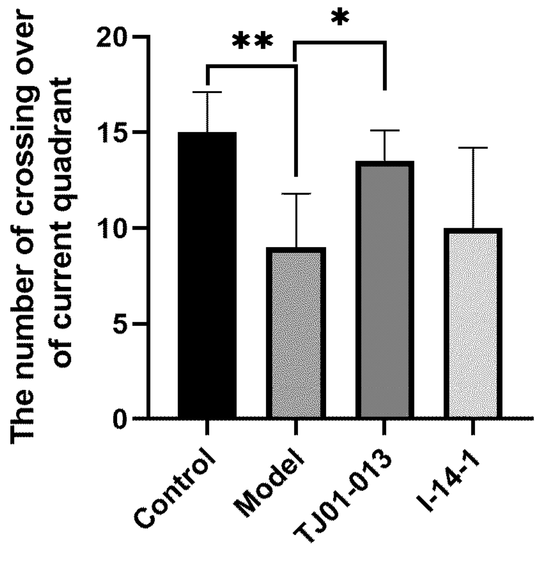
The number of crossing over of current quadrant
20
15
10
5
0
**
*
Control
Model
TJ01-013
I-14-1

FIG.89

## INTERNATIONAL SEARCH REPORT

International application No.
**PCT/CN2024/082357**

**A. CLASSIFICATION OF SUBJECT MATTER**

A61K31/18(2006.01)i; A61K31/4196(2006.01)i; A61P13/12(2006.01)i; A61Q1/00(2006.01)i

According to International Patent Classification (IPC) or to both national classification and IPC

**B. FIELDS SEARCHED**

Minimum documentation searched (classification system followed by classification symbols)

A61K, A61P, A61Q

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 杭州天玑济世生物科技有限公司, 南京天玑济世生物科技有限公司, 蛋白酶体, 线粒体, 肾脏损伤, 线粒体自噬, proteasome, mitochondrial, Mitophagy, mitochondrial autophagy, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2023045909 A1 (HANGZHOU PHECDAMED CO., LTD.) 30 March 2023 (2023-03-30) abstract, and claims 1-11 | 1-8, 11-14 |
| PX | WO 2023045910 A1 (HANGZHOU PHECDAMED CO., LTD.) 30 March 2023 (2023-03-30) abstract, and claims 1-10 | 1-8, 11-14 |
| Y | WO 2010005534 A2 (H. LEE MOFFITT CANCER CENTER AND RESEARCH INSTITUTE, INC. et al.) 14 January 2010 (2010-01-14) abstract, claims 1-21, and description, page 16, lines 5-13 | 1-8, 11-14 |
| Y | WO 9922729 A1 (CENTRE DE RECHERCHE DU CENTRE HOSPITALIER DE L'UNIVERSITE DE MONTREAL et al.) 14 May 1999 (1999-05-14) abstract, and claims 1-17 | 1-8, 11-14 |
| Y | WO 2010102286 A2 (H. LEE MOFFITT CANCER CENTER AND RESEARCH INSTITUTE, INC. et al.) 10 September 2010 (2010-09-10) abstract, and claims 1-29 | 1-8, 11-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 June 2024** | **21 June 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/082357**

**C. DOCUMENTS CONSIDERED TO BE RELEVANT**

| Category* | Citation of document, with indication, where appropriate, of the relevant passages | Relevant to claim No. |
|---|---|---|
| Y | GE, Y. et al. "Discovery and Synthesis of Hydronaphthoquinones as Novel Proteasome Inhibitors" *Journal of Medicinal Chemistry,* Vol. vol. 55, 05 January 2012 (2012-01-05), pp. 1978-1998 | 1-8, 11-14 |
| A | CN 111904958 A (ZHEJIANG UNIVERSITY) 10 November 2020 (2020-11-10) abstract, and claims 1-6 | 1-14 |

Form PCT/ISA/210 (second sheet) (July 2022)

## INTERNATIONAL SEARCH REPORT

International application No. **PCT/CN2024/082357**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-13**
because they relate to subject matter not required to be searched by this Authority, namely:

Claims 1-13 relate to the treatment of diseases, which belongs to subject matter for which no search is required by the International Searching Authority as defined in PCT Rule 39.1(iv). A search is performed on the basis that the designation of the subject matter thereof is a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The present application can be divided into the following groups of technical solutions:

Group 1: the use of a compound represented by general formula (I) or a pharmaceutically acceptable salt, stereoisomer, solvate or prodrug thereof, characterized by the use in: (i) preparation of a drug for preventing and/or treating diseases related to renal injury; and/or (ii) prevention and/or treatment of diseases related to renal injury.

Group 2: the use of a compound represented by general formula (I) or a pharmaceutically acceptable salt, stereoisomer, solvate or prodrug thereof, characterized by the use in: (iii) prevention and/or treatment of diseases related to mitochondrial dysfunction; and/or (iv) preparation of a drug for preventing and/or treating diseases related to mitochondrial dysfunction.

Group 3: the use of a compound represented by general formula (I) or a pharmaceutically acceptable salt, stereoisomer, solvate or prodrug thereof, characterized by the use in preparation of a cosmetic or a medical instrument.

The common technical feature among the above-mentioned three groups of technical solutions is: a compound represented by general formula (I) or a pharmaceutically acceptable salt, stereoisomer, solvate or prodrug thereof.

D1 (WO 2010005534 A2) discloses a proteasome inhibitor for selectively inducing apoptosis of cancer cells, and specifically discloses compounds such as compounds 9d, 9e, 9f, 10a, 10b and 10c (abstract, claims 1-21, and description, page 16, lines 5-13). These compounds fall within the scope of a compound represented by general formula (I) or a pharmaceutically acceptable salt, stereoisomer, solvate, or prodrug thereof.

It can be seen therefrom that the common technical feature among the technical solutions of groups 1-3 have been disclosed. Therefore, the above-mentioned three groups of technical solutions lack unity of invention and do not comply with PCT Rule 13.1.

**INTERNATIONAL SEARCH REPORT**

International application No.
**PCT/CN2024/082357**

**Box No. III Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2024/082357**

| Patent document cited in search report | Publication date (day/month/year) | Patent family member(s) | Publication date (day/month/year) |
|---|---|---|---|
| WO 2023045909 A1 | 30 March 2023 | IL 311496 A | 01 May 2024 |
| | | AU 2022350943 A1 | 21 March 2024 |
| | | CA 3230925 A1 | 03 March 2023 |
| WO 2023045910 A1 | 30 March 2023 | None | |
| WO 2010005534 A2 | 14 January 2010 | WO 2010005534 A3 | 08 April 2010 |
| | | US 2011201609 A1 | 18 August 2011 |
| | | US 8673910 B2 | 18 March 2014 |
| WO 9922729 A1 | 14 May 1999 | EP 0967976 A1 | 05 January 2000 |
| | | JP 2001508465 A | 26 June 2001 |
| | | AU 9731898 A | 24 May 1999 |
| | | AU 770798 B2 | 04 March 2004 |
| | | CA 2219867 A1 | 30 April 1999 |
| WO 2010102286 A2 | 10 September 2010 | WO 2010102286 A3 | 20 January 2011 |
| | | US 2012142917 A1 | 07 June 2012 |
| | | US 8466157 B2 | 18 June 2013 |
| CN 111904958 A | 10 November 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023102998399
- CN 2023115506763
- CN 202111108417